(19) Europäisches Patentamt
European Patent Office
Office européen des brevets

(11) EP 4 620 955 A1

(12) EUROPEAN PATENT APPLICATION
published in accordance with Art. 153(4) EPC

(43) Date of publication:
**24.09.2025 Bulletin 2025/39**

(21) Application number: **23890881.8**

(22) Date of filing: **17.11.2023**

(51) International Patent Classification (IPC):
***C07D 401/14*** (2006.01) ***C07D 413/14*** (2006.01)
***C07D 471/04*** (2006.01) ***C07D 498/04*** (2006.01)
***A61P 1/08*** (2006.01) ***A61K 31/4439*** (2006.01)
***A61P 3/04*** (2006.01) ***A61K 31/444*** (2006.01)
***A61K 31/437*** (2006.01) ***A61P 29/00*** (2006.01)

(52) Cooperative Patent Classification (CPC):
**A61K 31/437; A61K 31/4439; A61K 31/444; A61P 1/08; A61P 3/04; A61P 29/00; C07D 401/14; C07D 413/14; C07D 471/04; C07D 498/04**

(86) International application number:
**PCT/CN2023/132220**

(87) International publication number:
**WO 2024/104452 (23.05.2024 Gazette 2024/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2022 CN 202211461838**
**09.03.2023 CN 202310223815**
**12.04.2023 CN 202310388373**
**22.05.2023 CN 202310578810**
**20.07.2023 CN 202310895827**
**08.09.2023 CN 202311158862**

(71) Applicant: **Changchun Genescience Pharmaceutical Co., Ltd.**
**Changchun, Jilin 130012 (CN)**

(72) Inventors:
- **LU, Biao**
  **Changchun, Jilin 130012 (CN)**
- **YANG, Fanglong**
  **Changchun, Jilin 130012 (CN)**
- **ZHAO, Sheng**
  **Changchun, Jilin 130012 (CN)**
- **WANG, Siqin**
  **Changchun, Jilin 130012 (CN)**
- **JIN, Lei**
  **Changchun, Jilin 130012 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

Remarks:
The applicant has filed a text with which it is intended to bring the translation into conformity with the application as filed (Art. 14(2) EPC).

# (54) MC4R ANTAGONIST COMPOUND, PHARMACEUTICAL COMPOSITION AND PHARMACEUTICAL USE THEREOF

(57) A compound as shown in formula I, and a racemate, a stereoisomer, a tautomer, an isotopically labeled substance, a nitrogen oxide, a solvate, a polymorph, a metabolite, an ester, a prodrug, or a pharmaceutically acceptable salt thereof. The compound has a good MC4R antagonistic activity and good safety, improves transmembrane activity and drug bioavailability, and can be used as a drug for diagnosing, preventing, and/or treating MC4R-receptor-mediated diseases or conditions.



Processed by Luminess, 75001 PARIS (FR)

(Cont. next page)

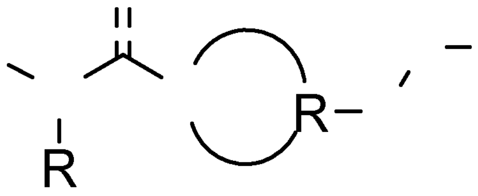

**Formula (I)**

## Description

### CROSS-REFERENCE TO RELATED APPLICATIONS

This application claims priorities to the following patent applications:

the earlier application No. 202211461838.1, filed with the China National Intellectual Property Administration on November 17, 2022 and entitled "MC4R ANTAGONIST COMPOUND, PHARMACEUTICAL COMPOSITION, AND PHARMACEUTICAL USE THEREOF";
the earlier application No. 202310223815.5, filed with the China National Intellectual Property Administration on March 9, 2023 and entitled "MC4R ANTAGONIST COMPOUND, PHARMACEUTICAL COMPOSITION, AND PHARMACEUTICAL USE THEREOF";
the earlier application No. 202310388373.X, filed with the China National Intellectual Property Administration on April 12, 2023 and entitled "MC4R ANTAGONIST COMPOUND, PHARMACEUTICAL COMPOSITION, AND PHARMACEUTICAL USE THEREOF";
the earlier application No. 202310578810.4, filed with the China National Intellectual Property Administration on May 22, 2023 and entitled "MC4R ANTAGONIST COMPOUND, PHARMACEUTICAL COMPOSITION, AND PHARMACEUTICAL USE THEREOF";
the earlier application No. 202310895827.2, filed with the China National Intellectual Property Administration on July 20, 2023 and entitled "MC4R ANTAGONIST COMPOUND, PHARMACEUTICAL COMPOSITION, AND PHARMACEUTICAL USE THEREOF"; and
the earlier application No. 202311158862.2, filed with the China National Intellectual Property Administration on September 8, 2023 and entitled "MC4R ANTAGONIST COMPOUND, PHARMACEUTICAL COMPOSITION, AND PHARMACEUTICAL USE THEREOF",
all of which are incorporated herein by reference in their entireties.

### TECHNICAL FIELD

The present invention relates to the field of pharmaceutical compounds, and specifically, to an MC4R antagonist compound, a pharmaceutical composition, and pharmaceutical use thereof.

### BACKGROUND

The melanocortin-4 receptor (MC4R) is a member of the melanocortin receptor family (MCRs) that is the class A subfamily of G-protein-coupled receptors (GPCRs) and that consists of five members (MC1R-MC5R), mediating various human physiological functions. MC4R, a seven-transmembrane GPCR expressed primarily in the hypothalamus, hippocampus, and thalamus, is a central regulator for body weight and energy homeostasis. MC1R, MC2R, MC3R, MC4R, and MC5R have been identified in mammals and are expressed in various tissues. MC1R is specifically expressed in melanocytes and melanoma. MC2R is an ACTH receptor expressed primarily in adrenal tissues. MC3R is expressed primarily in the brain and limbic system. MC4R is expressed widely in the brain and spinal cord. MC5R is expressed in the brain and in many peripheral tissues comprising skin, fat, skeletal muscle, and lymphoid tissue.
MC4R is an unusual GPCR due to the presence of both its endogenous agonist and its endogenous antagonist. Melanocortin ($\alpha$-MSH), derived from a hydrolysis product of proopiomelanocortin (POMC), acts as an endogenous ligand to activate the MC4R, suppressing appetite, thereby leading to weight loss. AgRP secreted by AgRP neurons can inhibit MC4R signaling, thereby promoting appetite and weight gain. MC4R loss-of-function mutations lead to obesity in mice: MC4R knockout mice gained weight at 5 weeks of age; at week 15, an average weight of homozygous female mice was twice that of wild-type mice from the same litter, and homozygous male mice were 50% heavier than the wild-type mice; and the weight of the MC4R knockout heterozygous mice was intermediate between the weight of the wild-type mice and the weight of the homozygous mice from the same litter. Therefore, MC4R knockout shows a gene dosage effect on weight regulation. Transgenic mice overexpressing AgRP exhibit obesity, increased food intake, and hyperinsulinemia. The MC4R loss-of-function mutations are associated with 6% to 8% of early severe obesity cases, and are the most common form causing monogenic obesity. Gain-of-function mutations are associated with low body mass index (BMI). In addition to maintaining food intake and energy homeostasis, MC4R also plays a role in other regions of the central nervous system, for example, is also of clinical significance in the regulation of pain perception, sexual function, anhedonia, blood pressure, and the like.
Based on important functions of the MC4R signaling pathway involved in the regulation of food intake, energy homeostasis, and growth, MC4R has become a target for the treatment of obesity. The MC4R agonist Setmelanotide has been FDA-approved for marketing for patients with POMC deficiency, leptin receptor deficiency, and other forms of severe

genetic obesity.

In recent years, several small-molecule MC4R antagonists have been reported in the literature and patent applications. These MC4R antagonists can be used for the treatment and/or prevention of MC4R-related diseases, comprising cachexia (cancer-associated cachexia, acquired immunodeficiency syndrome (AIDS)-associated cachexia, congestive heart failure (CHF)-associated cachexia, chronic kidney disease (CKD)-associated cachexia, and cachexia associated with the treatment of other chronic diseases); anorexia or anorexia nervosa (anorexia of aging, and anorexia associated with chemotherapy and/or radiotherapy); nausea and vomiting; weight loss (involuntary weight loss); failure to thrive; sarcopenia; muscular dystrophy; muscle weakness; fragility; osteoporosis; bone disorders (bone loss); pain (neuropathic pain); anxiety (post-traumatic stress disorder or PTSD); depression; hypertension; malnutrition in obesity (for example, sarcopenia caused by chronic obesity); sexual dysfunction; inflammatory disorders (inflammatory disorders associated with anorexia, cachexia, sarcopenia, or muscular dystrophy); and the like.

Therefore, there is still an unmet medical need for the development of a new small-molecule MC4R antagonist. An objective is to obtain a small-molecule MC4R antagonist that is more potent, more selective, less toxic, more physicochemically stable, more orally bioavailable, and more metabolically stable, for use in the treatment or prevention of MC4R-related diseases.

## SUMMARY

To resolve the problem in the related art, the present invention provides a compound represented by formula I, and a racemate, a stereoisomer, a tautomer, an isotopic label, a nitrogen oxide, a solvate, a polymorph, a metabolite, an ester, a prodrug, or a pharmaceutically acceptable salt thereof:

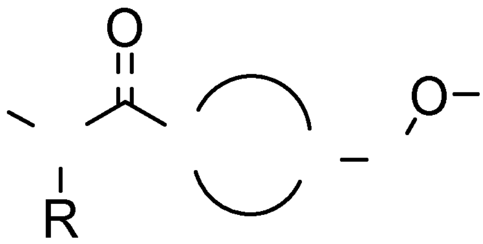

**formula I**

wherein X is selected from NR', O, or S; R' is selected from H, $C_{1-12}$ alkyl, or $C_{3-12}$ cycloalkyl;

Y is selected from CH or N;

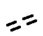 is present or absent;

R is selected from $C_{1-12}$ alkyl, deuterated $C_{1-12}$ alkyl, or halogenated $C_{1-12}$ alkyl;

A is selected from the following groups unsubstituted or optionally substituted with one, two, or more $R_a$: $C_{6-14}$ aryl, 5- to 14-membered heteroaryl, 3- to 14-membered heterocyclyl, and $C_{3-12}$ cycloalkyl; each $R_a$ is the same or different, and is independently selected from H, deuterium, halogen, CN, or the following groups unsubstituted or optionally substituted with one, two, or more $R_{a1}$: $C_{1-12}$ alkyl and $C_{1-12}$ alkoxy; each $R_{a1}$ is the same or different, and is independently selected from deuterium, halogen, CN, $C_{1-12}$ alkyl, or $C_{1-12}$ alkoxy;

E is selected from 3- to 14-membered N-containing heterocyclyl unsubstituted or optionally substituted with one, two, or more $R_e$; each $R_e$ is the same or different, and is independently selected from H, deuterium, halogen, CN, OH, oxo (=O), $C_{1-12}$ alkyl, or $C_{1-12}$ alkoxy; or two $R_e$ with an atom linked thereto form $C_{3-12}$ cycloalkyl or 3- to 14-membered heterocyclyl; N in E is linked to

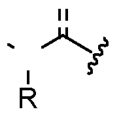

, and C in E is linked to X;

G is selected from the following groups unsubstituted or optionally substituted with one, two, or more $R_g$: $C_{6-14}$ aryl and 5- to 14-membered heteroaryl; each $R_g$ is the same or different, and is independently selected from H, deuterium, halogen, CN, or the following groups unsubstituted or optionally substituted with one, two, or more $R_{g1}$: amino, $C_{1-12}$ alkyl, $C_{1-12}$ alkoxy, $C_{3-12}$ cycloalkyl, $C_{6-14}$ aryl, and 5- to 14-membered heteroaryl; each $R_{g1}$ is the same or different, and is independently selected from deuterium, halogen, CN, $C_{1-12}$ alkyl, or $C_{1-12}$ alkoxy; and

M is absent or is selected from the following groups unsubstituted or optionally substituted with one, two, or more $R_m$: $C_{6-14}$ aryl, 5- to 14-membered heteroaryl, 3- to 14-membered heterocyclyl, and $C_{3-12}$ cycloalkyl; each $R_m$ is the same or different, and is independently selected from H, deuterium, halogen, CN, or the following groups unsubstituted or optionally substituted with one, two, or more $R_{m1}$: $C_{1-12}$ alkyl, $C_{1-12}$ alkoxy, and $C_{3-12}$ cycloalkyl; each $R_{m1}$ is the same or different, and is independently selected from deuterium, halogen, CN, OH, $C_{1-12}$ alkyl, or $C_{1-12}$ alkoxy.

In some embodiments, X is selected from NR', O, or S; R' is selected from H, $C_{1-6}$ alkyl, or $C_{3-8}$ cycloalkyl, such as methyl, ethyl, or cyclopropyl.

In some embodiments, R is selected from $C_{1-6}$ alkyl, deuterated $C_{1-6}$ alkyl, or halogenated $C_{1-6}$ alkyl, such as methyl.

In some embodiments, A is selected from the following groups unsubstituted or optionally substituted with one, two, or more $R_a$: $C_{6-10}$ aryl, 5- to 10-membered heteroaryl, 3- to 10-membered heterocyclyl, and $C_{3-8}$ cycloalkyl; each $R_a$ is the same or different, and is independently selected from H, deuterium, halogen, CN, or the following groups unsubstituted or optionally substituted with one, two, or more $R_{a1}$: $C_{1-6}$ alkyl and $C_{1-6}$ alkoxy; each $R_{a1}$ is the same or different, and is independently selected from deuterium, halogen, CN, $C_{1-6}$ alkyl, or $C_{1-6}$ alkoxy.

In some embodiments, A is selected from the following groups unsubstituted or optionally substituted with one, two, or more $R_a$:

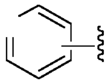, 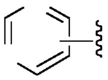, 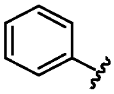, 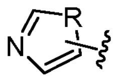, 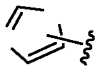,

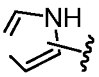, 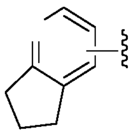, 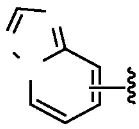, 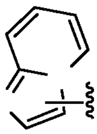, 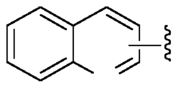, 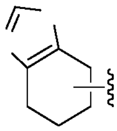, 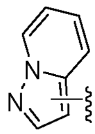,

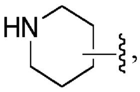, 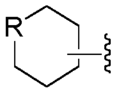, 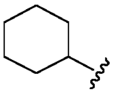, 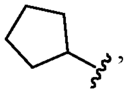, 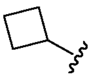, and 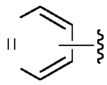;

each $R_a$ is the same or different, and is independently selected from H, F, Cl, Br, I, CN, $C_{1-6}$ alkyl, $C_{1-6}$ alkoxy, halogenated $C_{1-6}$ alkyl, or halogenated $C_{1-6}$ alkoxy.

In some embodiments, each $R_a$ is the same or different, and is independently selected from H, F, Cl, Br, CN, methyl, methoxy, trifluoromethyl, or difluoromethoxy.

In some embodiments, A is selected from

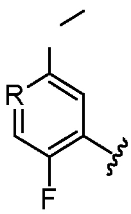, 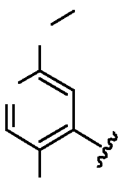, 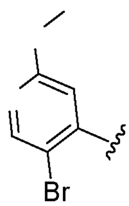,

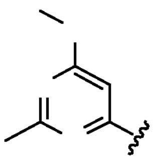, 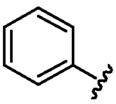, 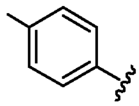, 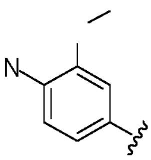, 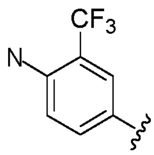, 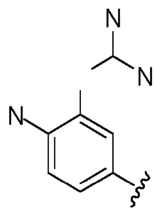,

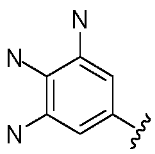, 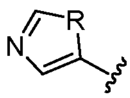, 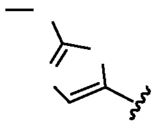, 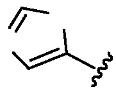, 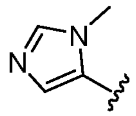, 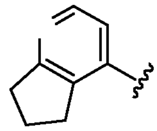, 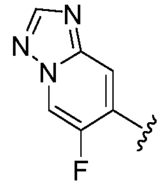,

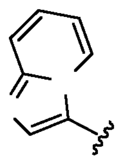, 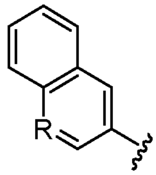, 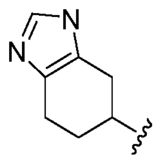, 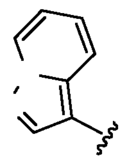, 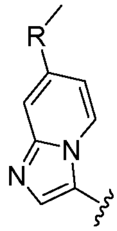, 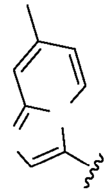, 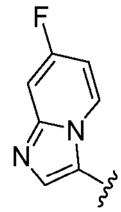,

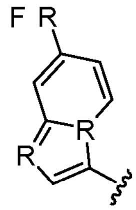, 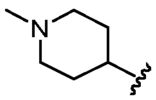, 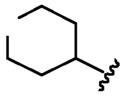, 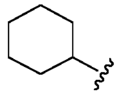, 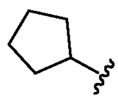, 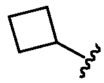, 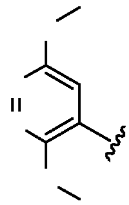, 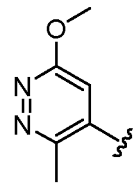,

or

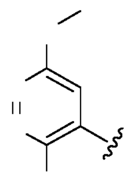.

In some embodiments, E is selected from 3- to 8-membered N-containing heterocyclyl unsubstituted or optionally substituted with one, two, or more $R_e$; each $R_e$ is the same or different, and is independently selected from H, deuterium, halogen, CN, OH, oxo (=O), $C_{1-6}$ alkyl, or $C_{1-6}$ alkoxy; or two $R_e$ with an atom linked thereto form $C_{3-8}$ cycloalkyl; N in E is linked to

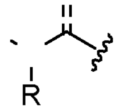,

and C in E is linked to X.

In a preferred embodiment, E is selected from the following groups unsubstituted or optionally substituted with one, two, or more $R_e$:

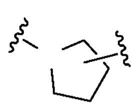, 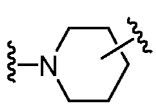, 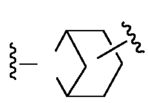,

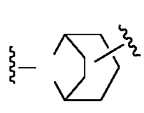, 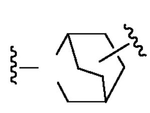, 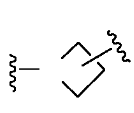, 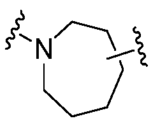, and 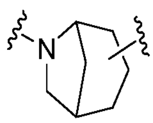;

each $R_e$ is the same or different, and is independently selected from H, F, Cl, Br, I, CN, OH, oxo (=O), or $C_{1-6}$ alkyl; or two $R_e$ with an atom linked thereto form $C_{3-8}$ cycloalkyl.

In a preferred embodiment, each $R_e$ is the same or different, and is independently selected from H, F, CN, OH, oxo

(=O), or methyl; or two $R_e$ with an atom linked thereto form cyclopropyl.

In a preferred embodiment, E is selected from

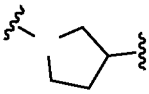, 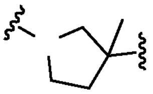, 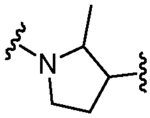,

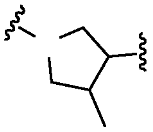, 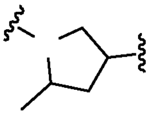, 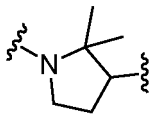, 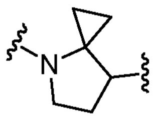, 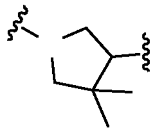, 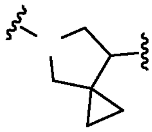,

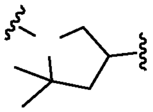, 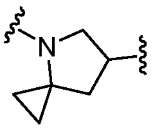, 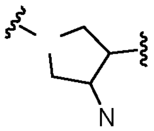, 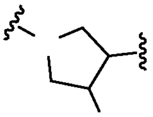, 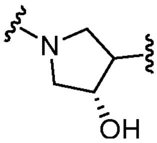, 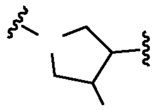,

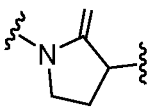, 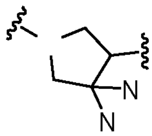, 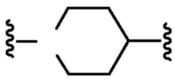, 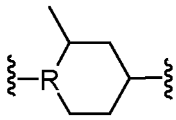, 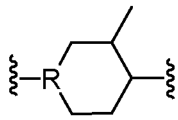, 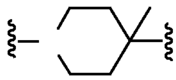,

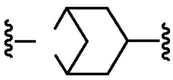, 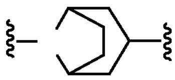, 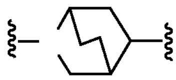, 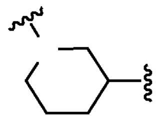, 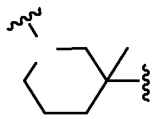, 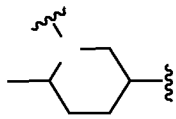,

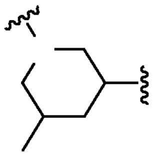, 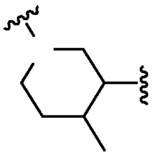, 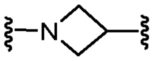, 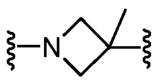, 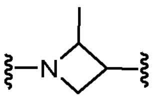, 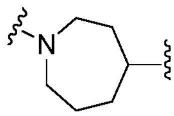,

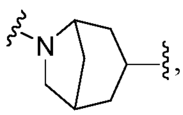, or 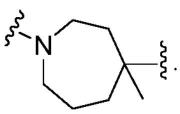.

In some embodiments, G is selected from the following groups unsubstituted or optionally substituted with one, two, or more $R_g$: $C_{6-10}$ aryl and 5- to 10-membered heteroaryl; each $R_g$ is the same or different, and is independently selected from H, deuterium, halogen, CN, or the following groups unsubstituted or optionally substituted with one, two, or more $R_{g1}$: amino, $C_{1-6}$ alkyl, $C_{1-6}$ alkoxy, $C_{3-8}$ cycloalkyl, $C_{6-10}$ aryl, and 5- to 10-membered heteroaryl; each $R_{g1}$ is the same or different, and is independently selected from deuterium, $C_{1-6}$ alkyl, or $C_{1-6}$ alkoxy.

In a preferred embodiment, G is selected from the following groups unsubstituted or optionally substituted with one, two, or more $R_g$:

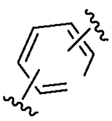, 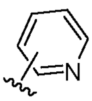, 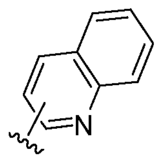, 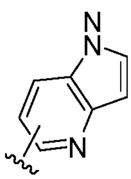,

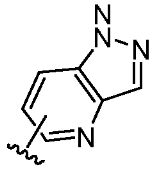, 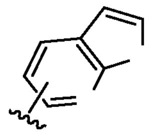, 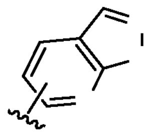, 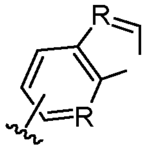, 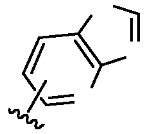, 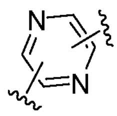,

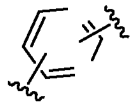, 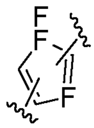, and 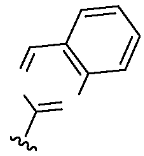

; each $R_g$ is the same or different, and is independently selected from H, halogen, CN, $C_{1-6}$ alkyl, $C_{1-6}$ alkoxy, $C_{3-8}$ cycloalkyl, amino, $C_{1-6}$ alkylamino, $(C_{1-6}$ alkyl$)_2$amino, $C_{6-10}$ aryl, or $C_{1-6}$ alkyl 5- to 8-membered heteroaryl.

In a preferred embodiment, each $R_g$ is the same or different, and is independently selected from H, F, methyl, ethyl, cyclopropyl, cyclobutyl, methoxy, methylamino ($CH_3NH$-), dimethylamino ($(CH_3)_2N$-), phenyl, or

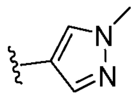.

In a preferred embodiment, G is selected from

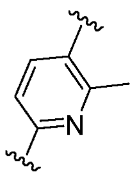, 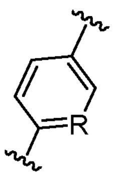, 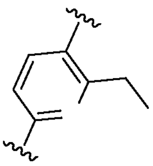,

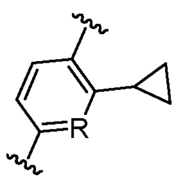, 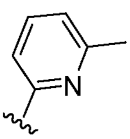, 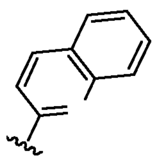, 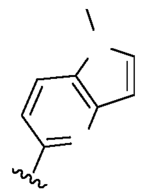, 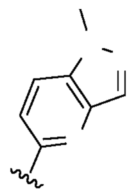, 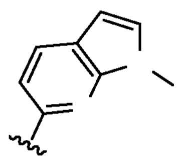,

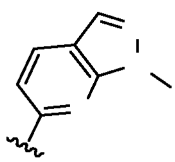, 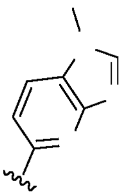, 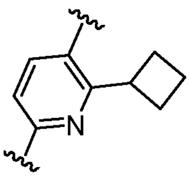, 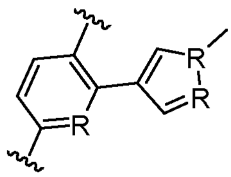, 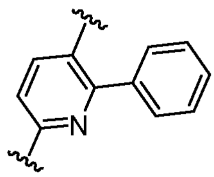,

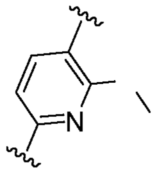, 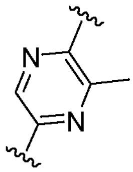, 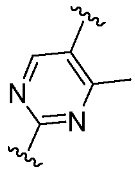, 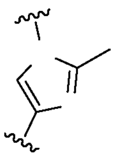, 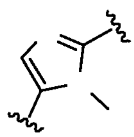, 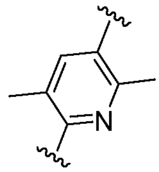, 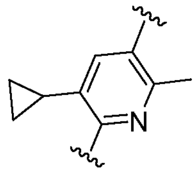,

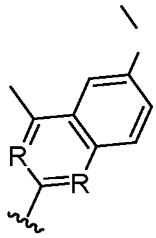 , 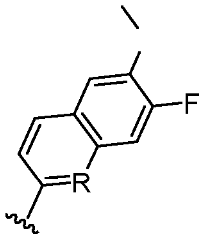 , 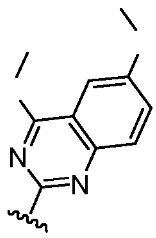 , 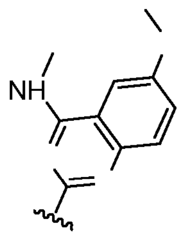 , 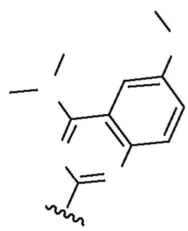 ,

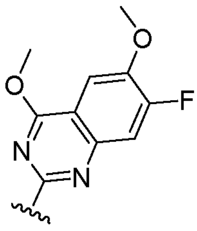 , 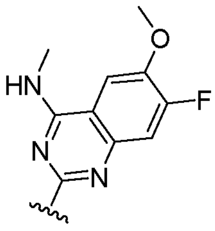 , 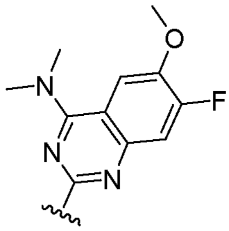 , or 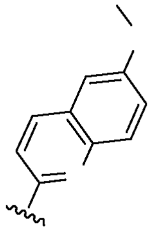 .

In some embodiments, M is absent or is selected from the following groups unsubstituted or optionally substituted with one, two, or more $R_m$: $C_{6-10}$ aryl, 5- to 10-membered heteroaryl, and 3- to 8-membered heterocyclyl; each $R_m$ is the same or different, and is independently selected from H, deuterium, halogen, CN, or the following groups unsubstituted or optionally substituted with one, two, or more $R_{m1}$: $C_{1-6}$ alkyl, $C_{1-6}$ alkoxy, and $C_{3-6}$ cycloalkyl; each $R_{m1}$ is the same or different, and is independently selected from deuterium, halogen, CN, OH, $C_{1-6}$ alkyl, or $C_{1-6}$ alkoxy.

In some embodiments, M is absent or is selected from the following groups unsubstituted or optionally substituted with one, two, or more $R_m$: pyridinyl, pyrimidinyl, phenyl, oxazolyl, pyrazolyl, triazolyl, 1,2,3-triazolyl, tetrazolyl, thiazolyl, tetrahydropyrrolyl, piperidinyl, morpholinyl, piperazinyl, naphthyl, quinolyl, azepanyl, imidazolyl,

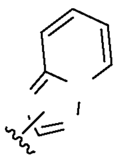 , 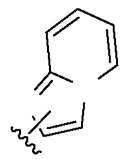 ,

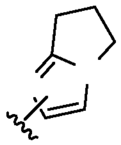 , and 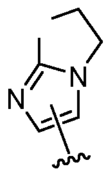

; each $R_m$ is the same or different, and is independently selected from H, deuterium, F, Cl, Br, I, CN, $C_{1-6}$ alkyl, $C_{1-6}$ alkoxy, $C_{3-6}$ cycloalkyl, halogenated $C_{1-6}$ alkyl, halogenated $C_{1-6}$ alkoxy, or hydroxyl-$C_{1-6}$ alkyl.

In a preferred embodiment, each $R_m$ is the same or different, and is independently selected from H, F, CN, methyl, methoxy, difluoromethyl, difluoromethoxy, trifluoromethyl, cyclopropyl, or

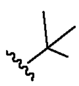 .

In a preferred embodiment, each $R_m$ is the same or different, and is independently selected from H, F, CN, methyl, methoxy, difluoromethyl, difluoromethoxy, trifluoromethyl, or cyclopropyl.

In a preferred embodiment, $R_m$ is selected from CN.

In preferred embodiments, M is absent or is selected from

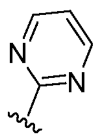 , 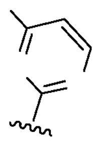 , 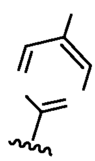 ,

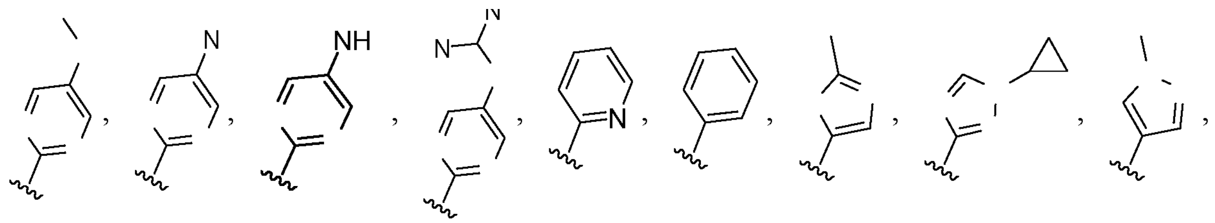

, , , , , , , , ,

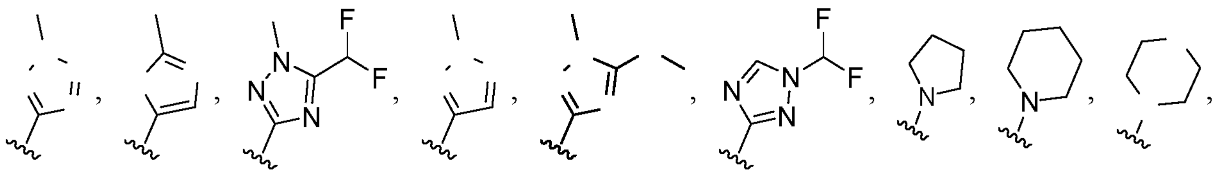

, , , , , , , , ,

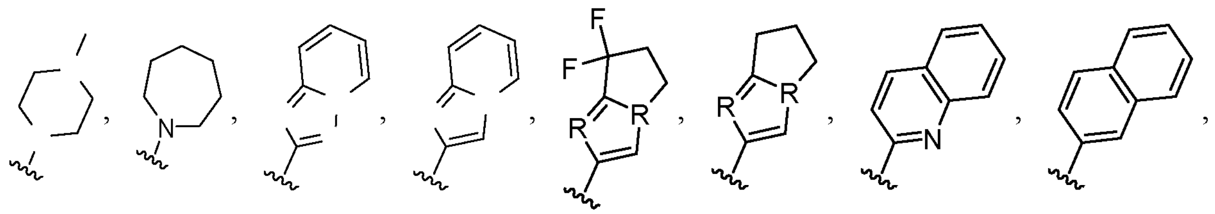

, , , , , , , ,

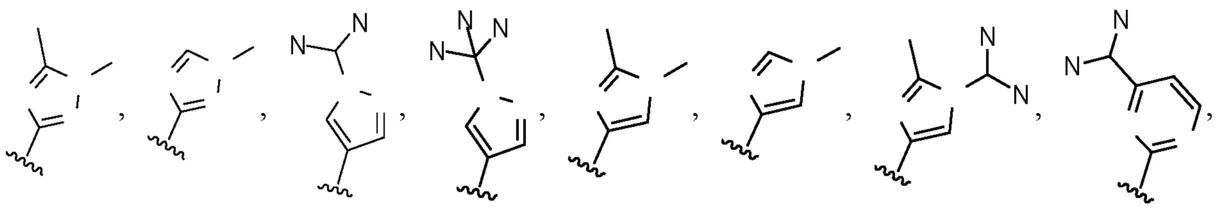

, , , , , , , ,

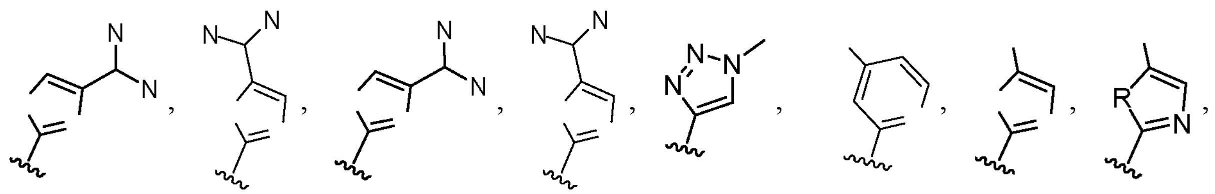

, , , , , , , ,

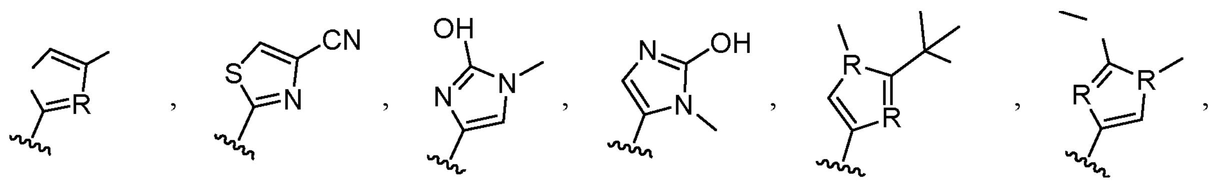

, , , , , ,

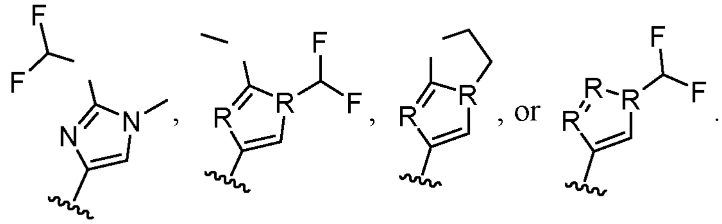

, , , or .

In preferred embodiments, M is selected from 5- and 6-membered heteroaryl substituted with one, two, or more $R_m$, wherein at least one of $R_m$ is CN, and the other of $R_m$ is present or absent, when the other of $R_m$ is present, the other of $R_m$ is the same or different, and is independently selected from halogen, CN, $C_{1-6}$ alkyl, $C_{1-6}$ alkoxy, $C_{3-6}$ cycloalkyl, halogenated $C_{1-6}$ alkyl, or halogenated $C_{1-6}$ alkoxy.

In a preferred embodiment, M is selected from 5- and 6-membered heteroaryl substituted with CN.

In some embodiments, the compound represented by formula I is selected from the following structures:

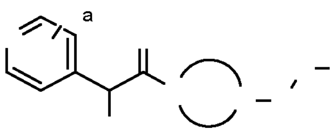

formula I-1

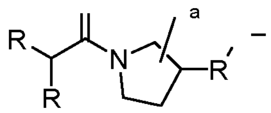

formula I-2

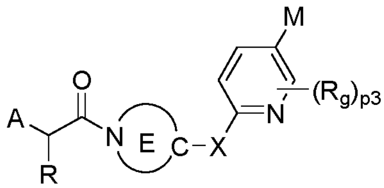

formula I-3

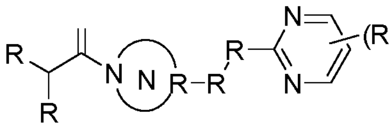

formula I-4

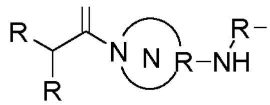

formula I-5

wherein X, R, A, E, G, M, $R_a$, $R_e$, $R_g$, and $R_m$ are as defined as described above; and p1, p2, p3, and p4 are each independently selected from 0, 1, 2, 3, 4, or 5.

In a preferred embodiment, the compound represented by formula I has a structure represented by the following formula II:

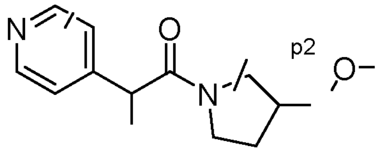

formula II

wherein R, M, G, $R_a$, $R_e$, p1, and p2 are as defined as described above.

In a preferred embodiment, the compound represented by formula I has a structure represented by the following formula II-1, II-2, or II-3:

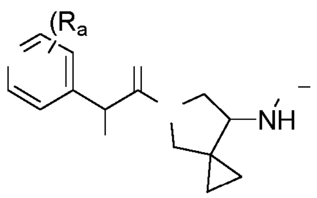

formula II-1

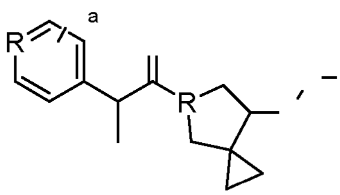

formula II-2

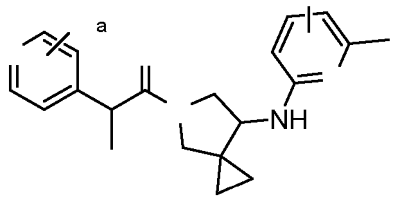

formula II-3

wherein R, M, G, $R_a$, and p1 are as defined as described above.

In a preferred embodiment, the compound represented by formula I has a structure represented by the following formula III:

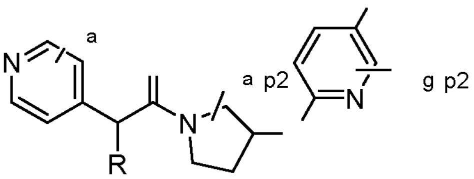

formula III

wherein M, R, $R_a$, $R_e$, $R_g$, p1, p2, and p3 are as defined as described above.

In a preferred embodiment, the compound represented by formula I is selected from the following structures:

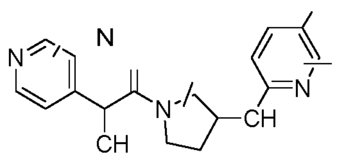
formula III-1

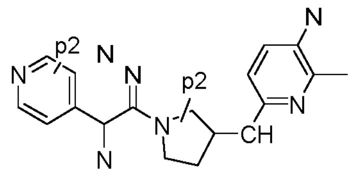
formula III-2

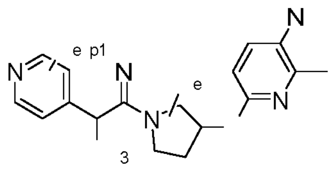
formula III-3

wherein M, R, $R_a$, $R_e$, $R_g$, p1, p2, and p3 are as defined as described above.

In a preferred embodiment, the compound represented by formula I has a structure represented by the following formula IV:

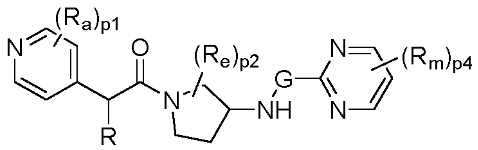
formula IV

wherein R, G, $R_a$, $R_e$, $R_m$, p1, p2, and p4 are as defined as described above.

In a preferred embodiment, the compound represented by formula I has a structure represented by the following formula V:

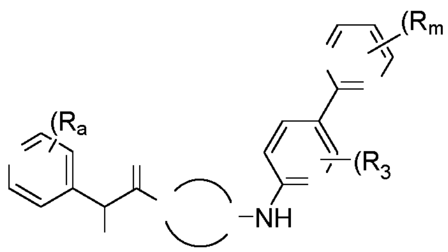
formula V

wherein R, E, $R_a$, $R_g$, $R_m$, p1, p3, and p4 are as defined as described above.

In a preferred embodiment, the compound represented by formula I has a structure represented by the following formula VI:

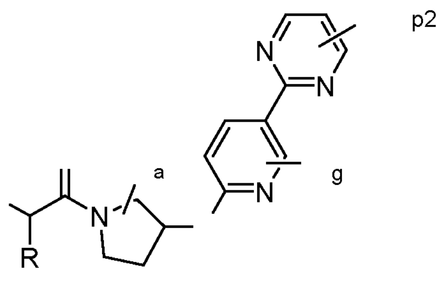
formula VI

wherein A, R, $R_e$, $R_g$, $R_m$, p2, p3, and p4 are as defined as described above.

In a preferred embodiment, the compound represented by formula I has a structure represented by the following formula VII:

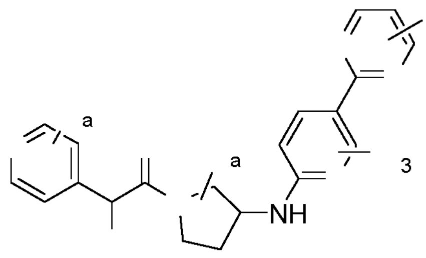

formula VII

wherein R, $R_a$, $R_e$, $R_g$, $R_m$, p1, p2, p3, and p4 are as defined as described above.

In a preferred embodiment, the compound represented by formula I has a structure represented by the following formula VIII or VIII-A:

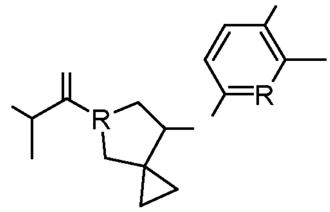

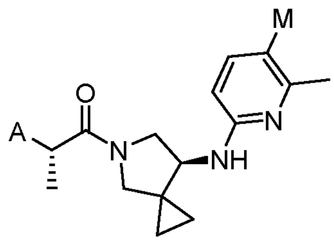

formula VIII formula VIII-A

wherein A and M are as defined as described above.

In a preferred embodiment, the compound represented by formula I has a structure represented by the following formula VIII-1 or VIII-2:

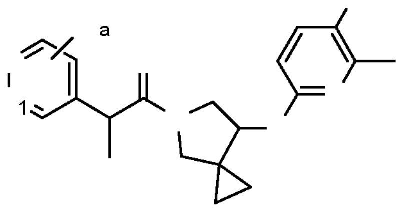

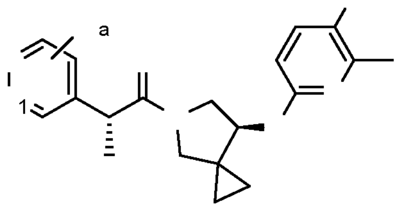

formula VIII-1 formula VIII-2

wherein $R_a$, p1, and M are as defined as described above; and $Y_1$ is selected from CH or N.

In a preferred embodiment, the compound represented by formula I has a structure represented by the following formula IX or IX-1:

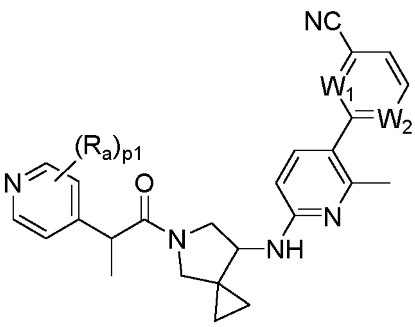

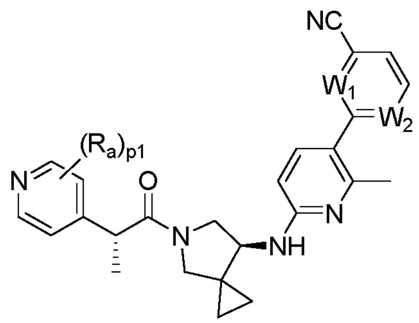

formula IX formula IX-1

wherein $R_a$ and p1 are as defined as described above; and $W_1$ and $W_2$ are the same or different, and are independently

selected from CH or N.

In a preferred embodiment, the compound represented by formula I is selected from the following structures:

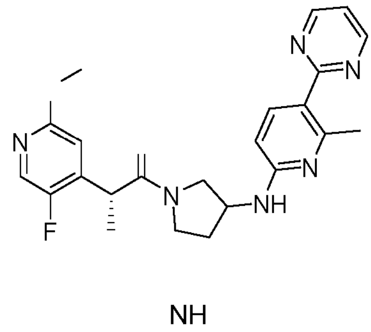

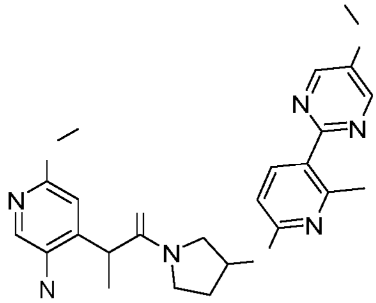

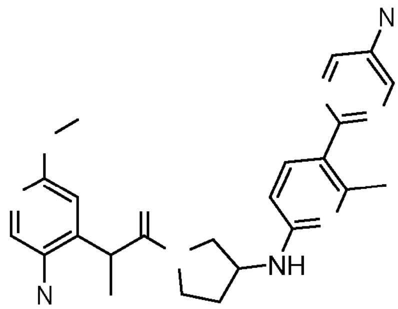

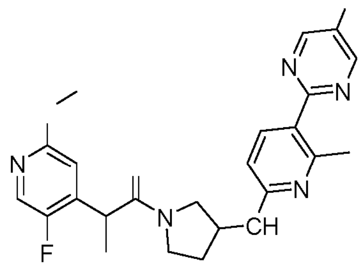

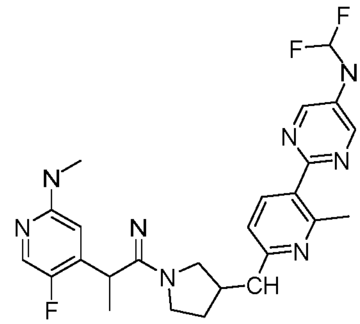

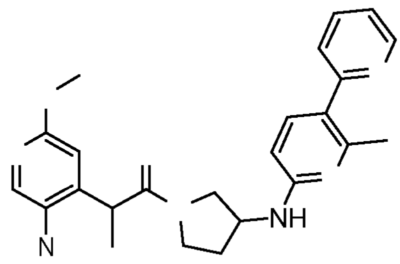

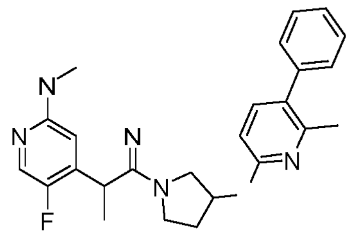

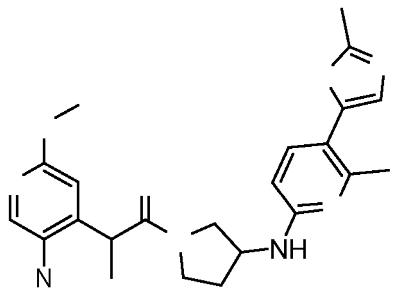

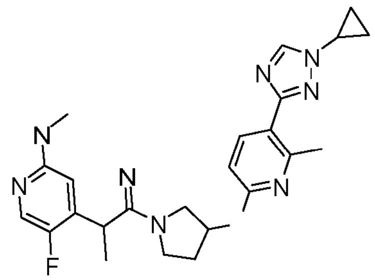

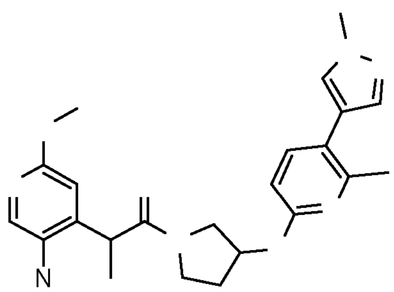

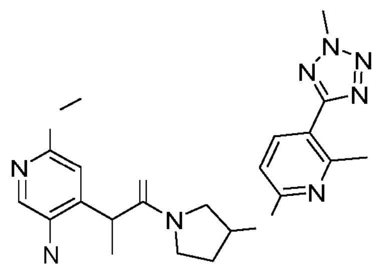

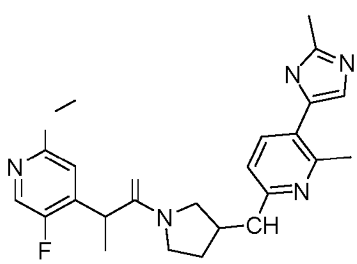

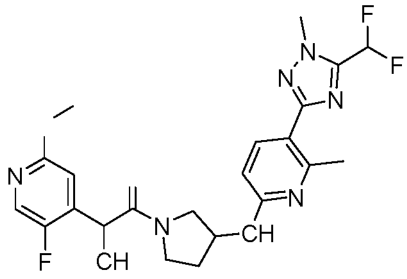
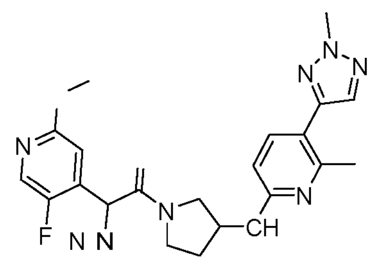
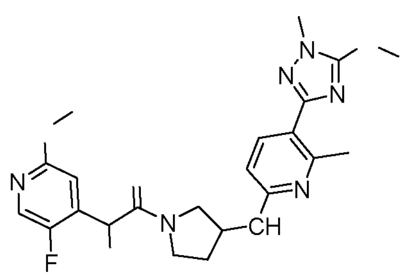

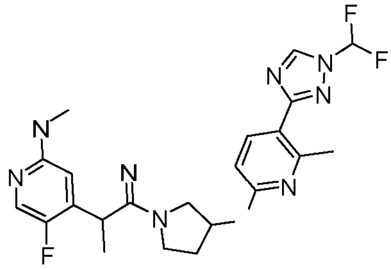
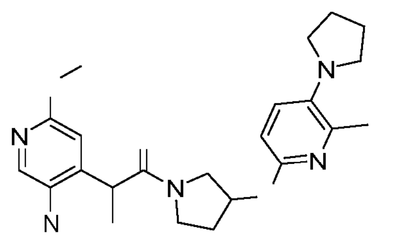
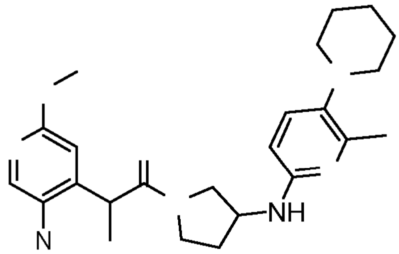

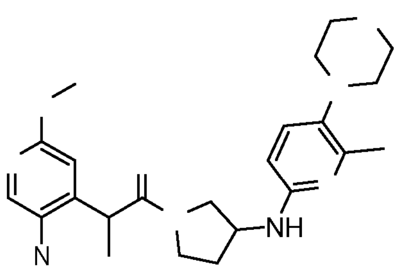
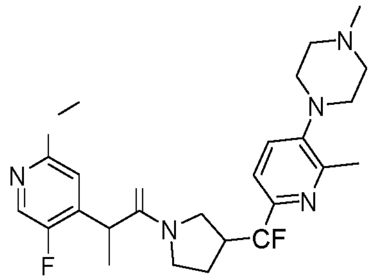
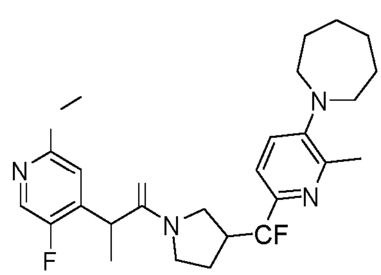

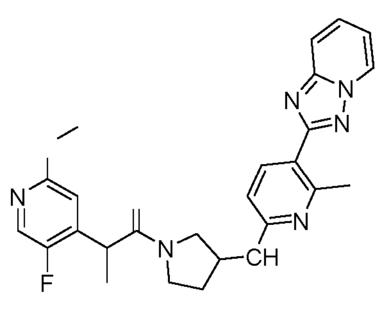
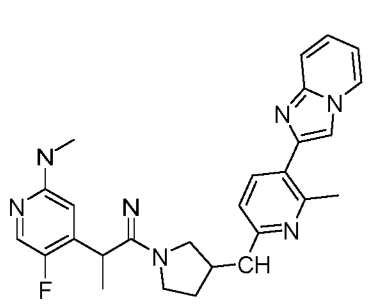
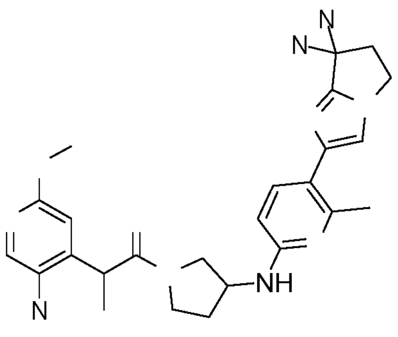

024

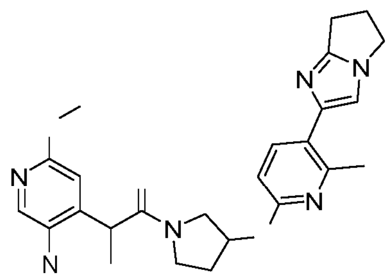
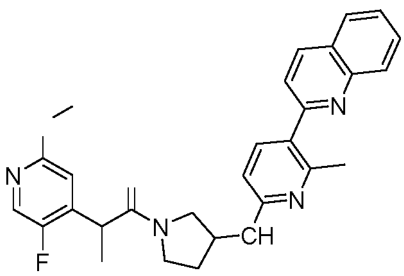
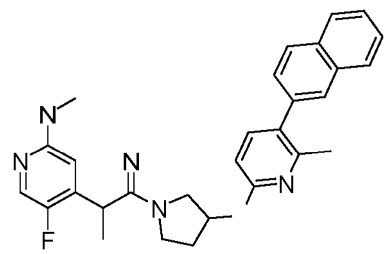

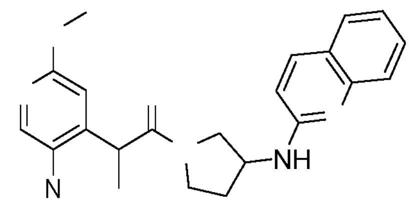

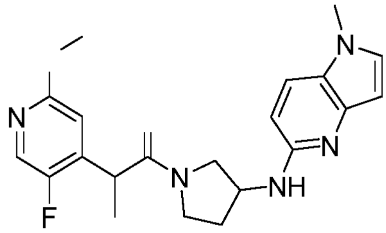

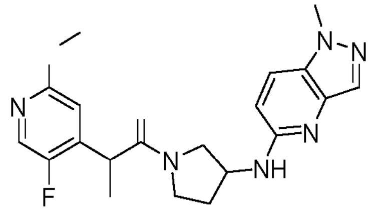

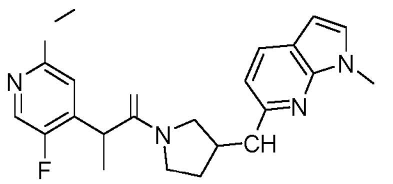

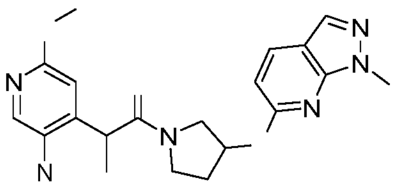

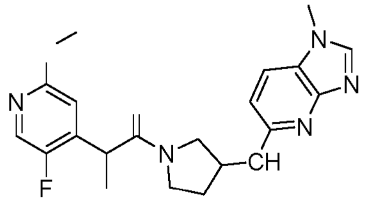

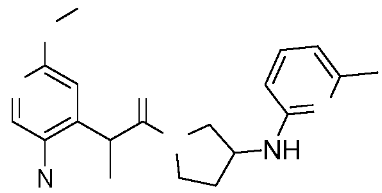

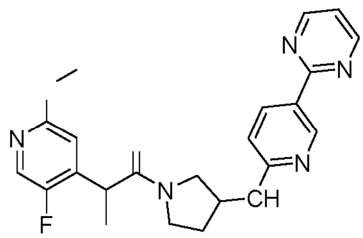

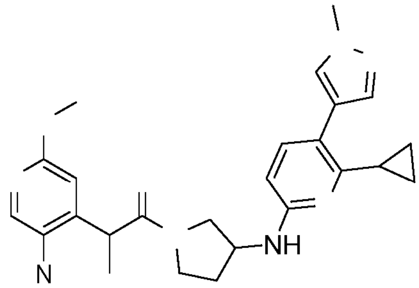

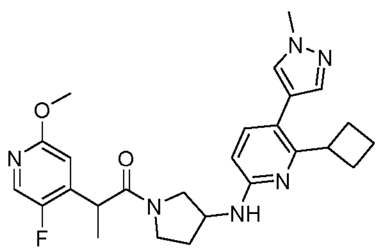

037

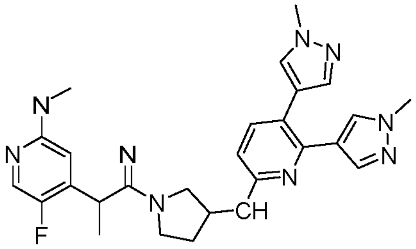

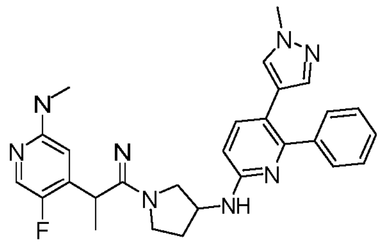

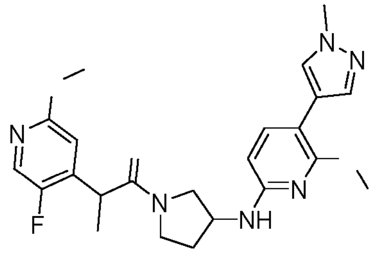

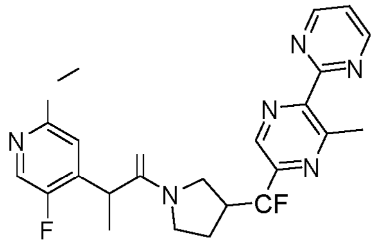

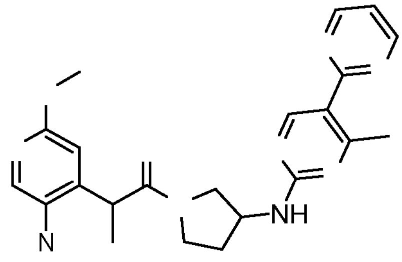

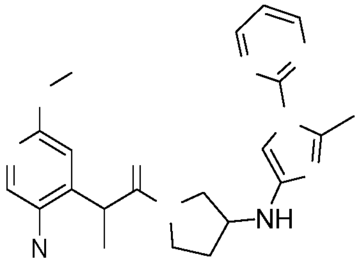

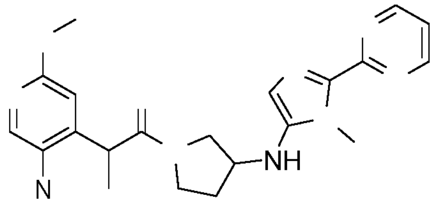

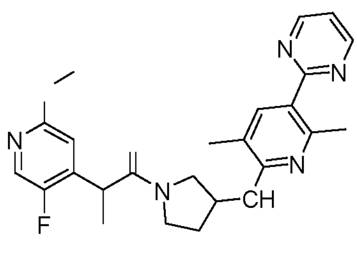

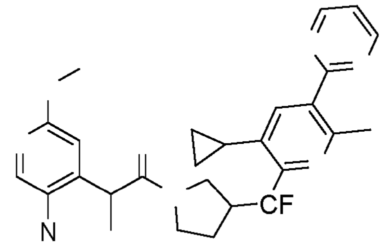

024

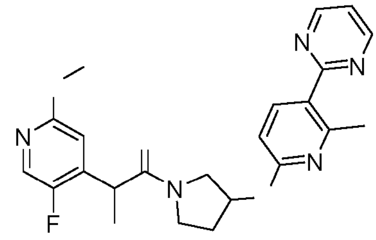

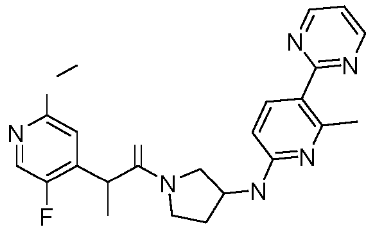

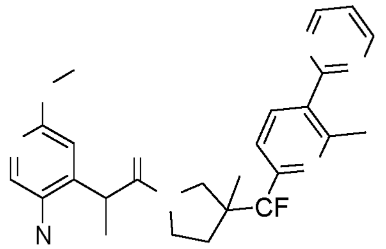

024

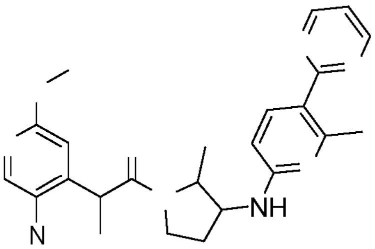

024

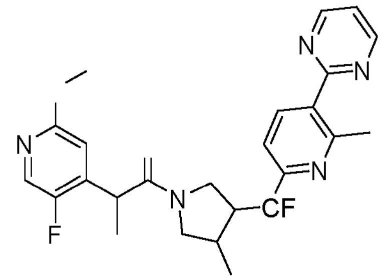

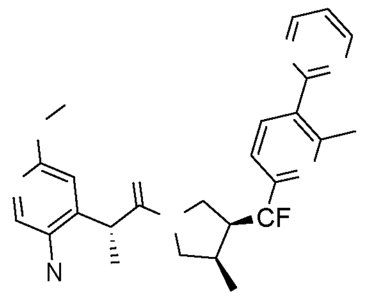

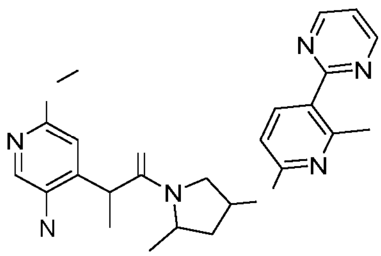

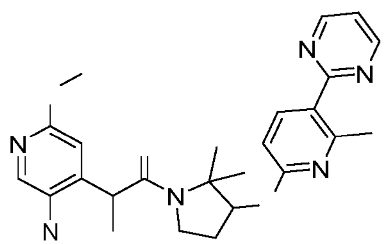

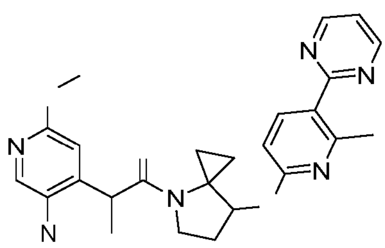

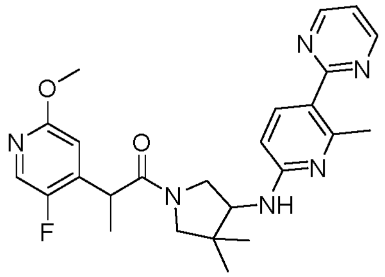

055

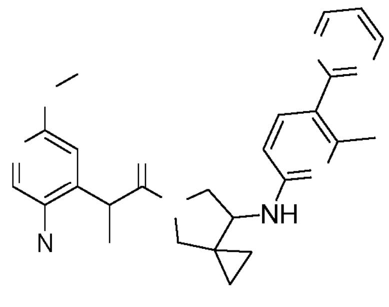

024

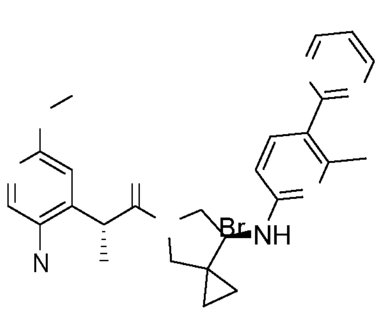

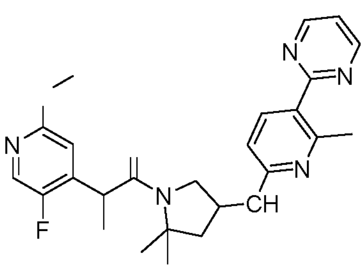

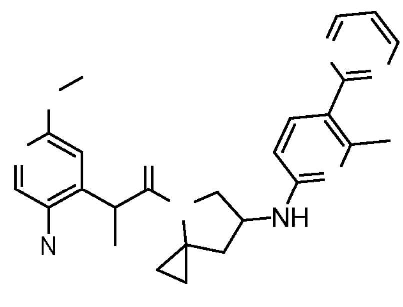

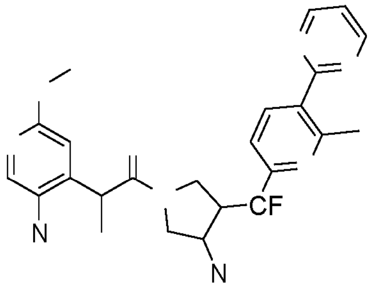 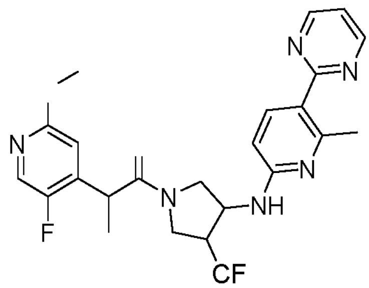 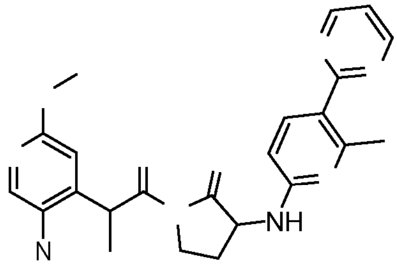

024

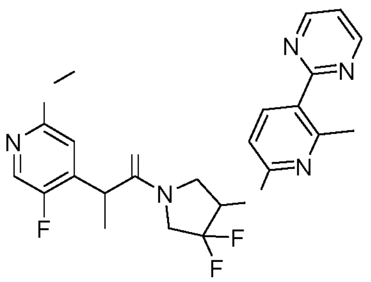 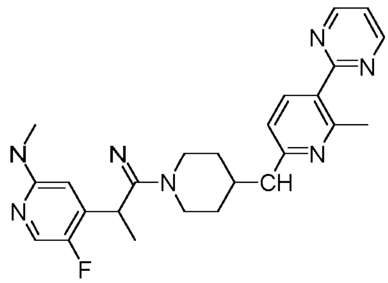 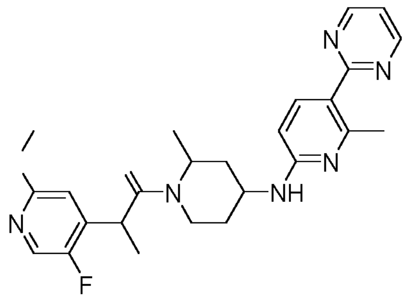

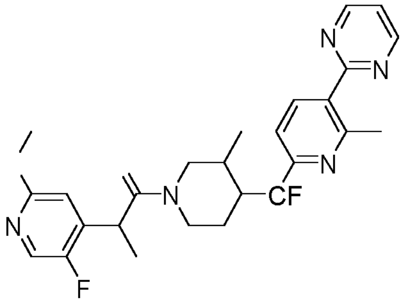 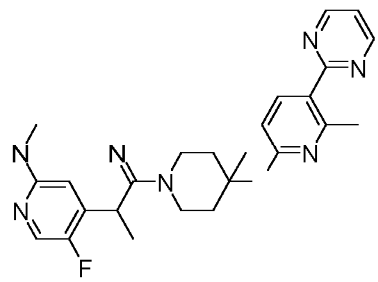 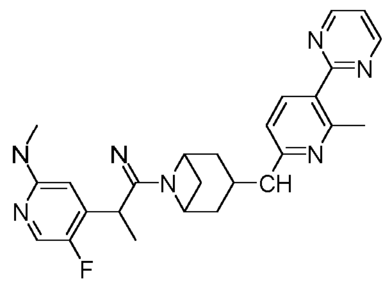

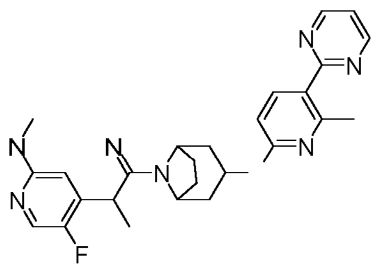 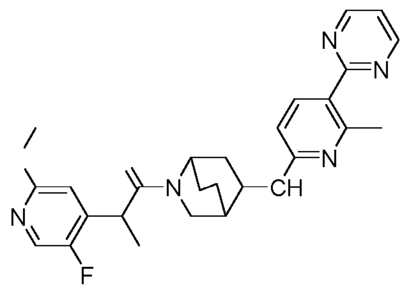 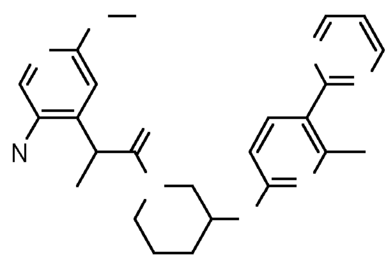

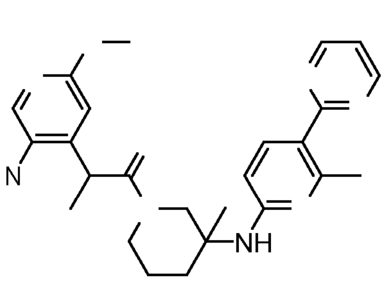 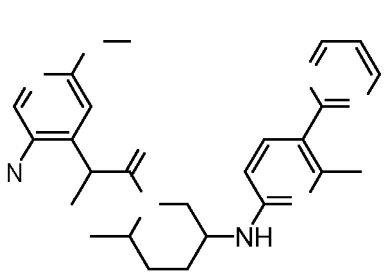 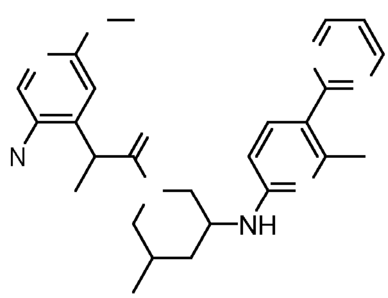

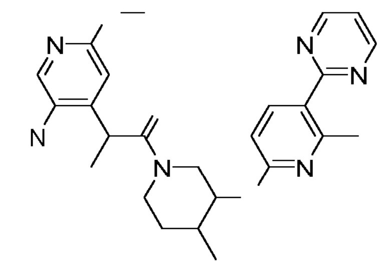
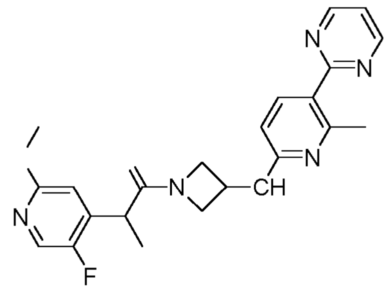
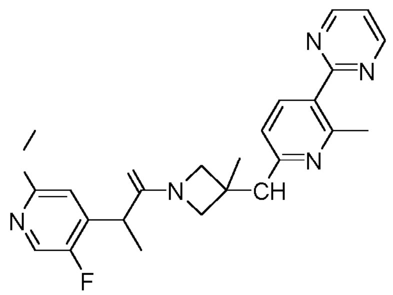

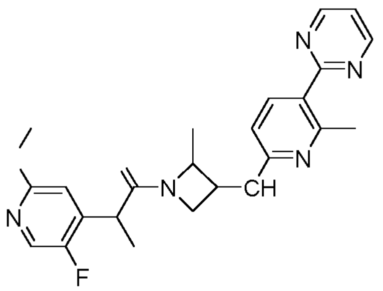
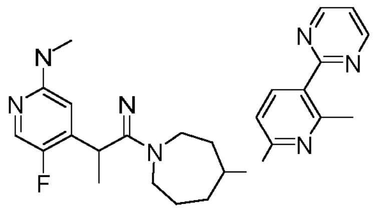
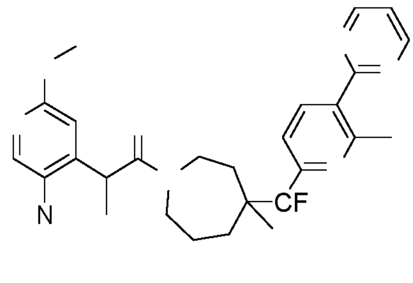

024

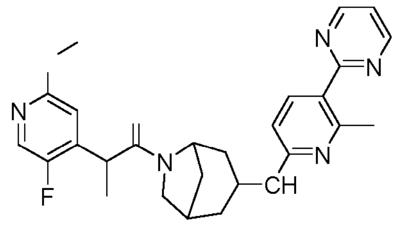
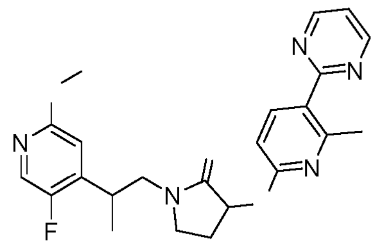
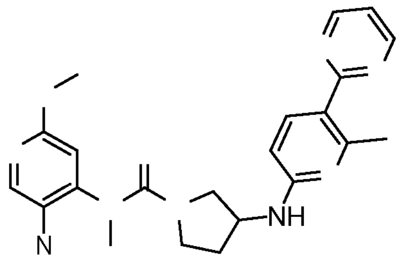

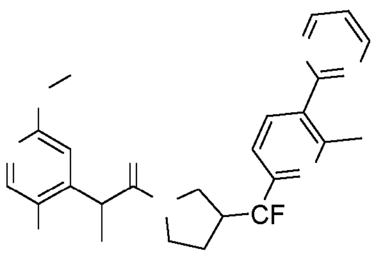

024

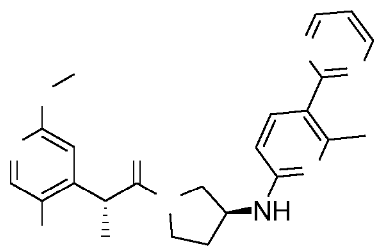
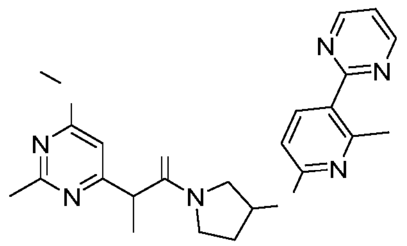

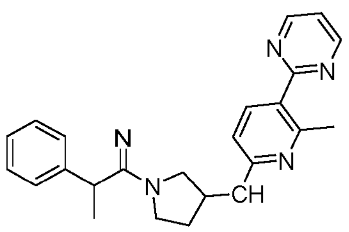
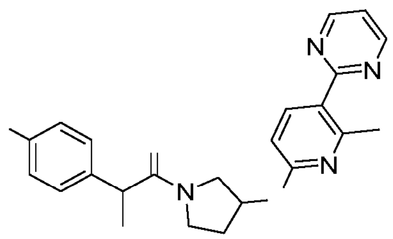
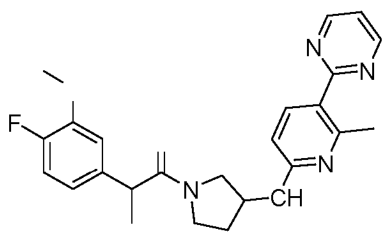

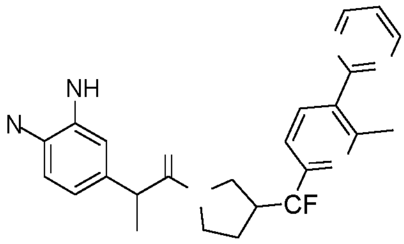
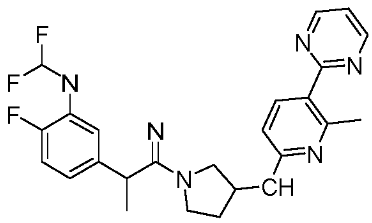
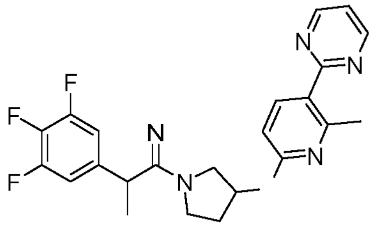

024

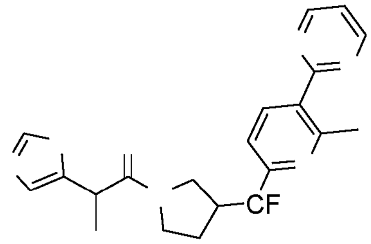
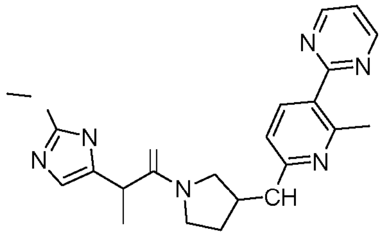
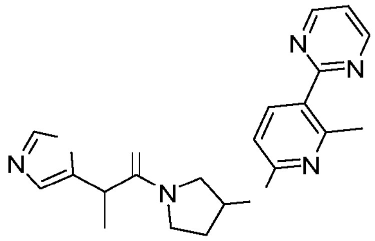

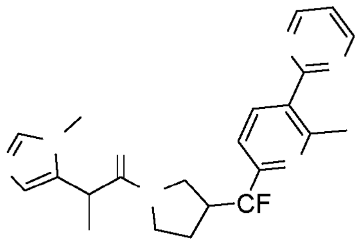
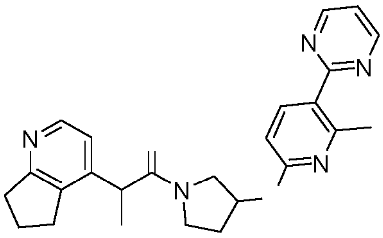
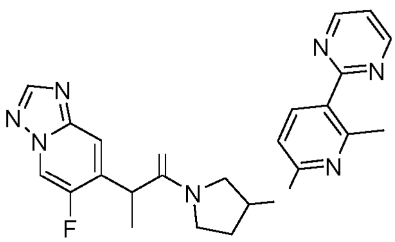

024

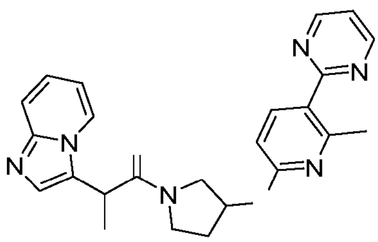
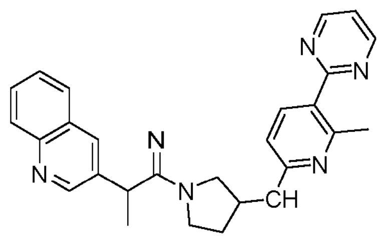
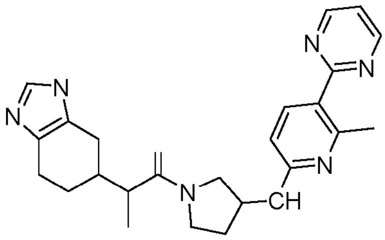

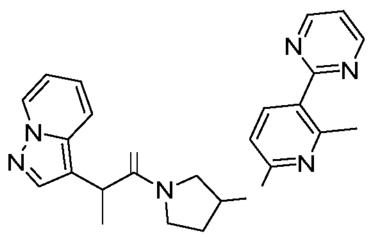
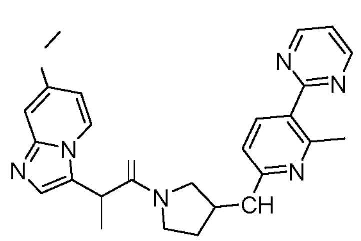
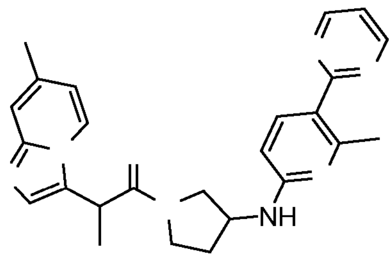

024

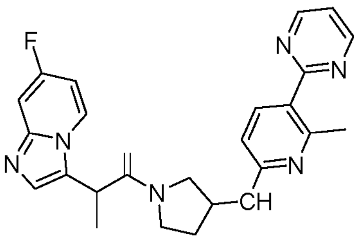

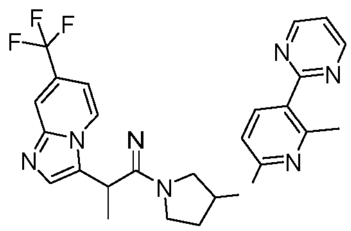

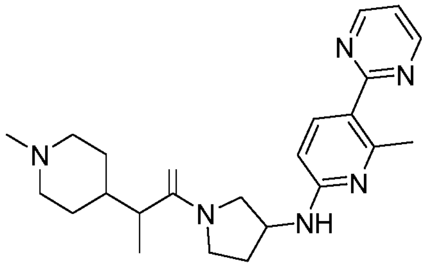

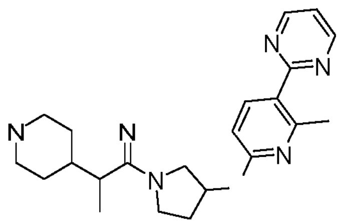

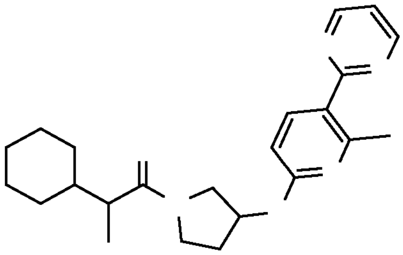

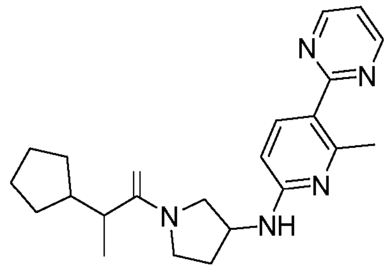

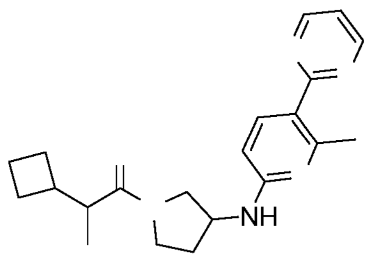

024

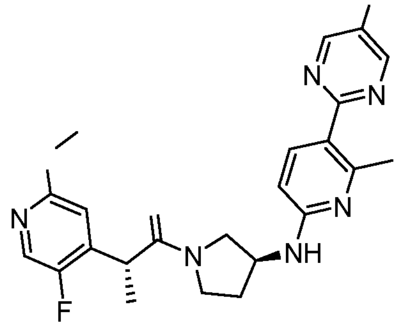

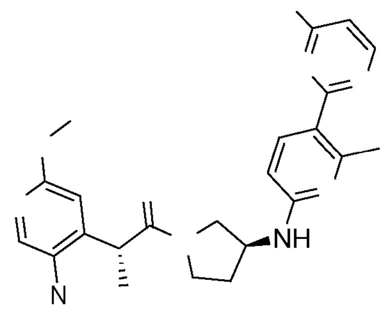

CF

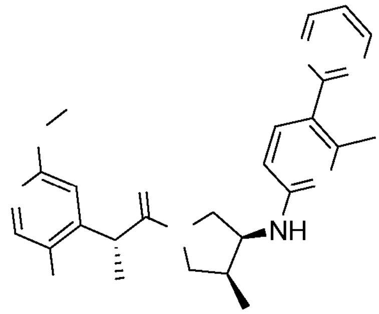

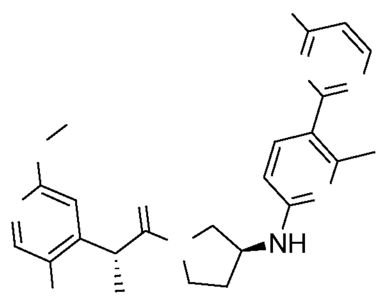

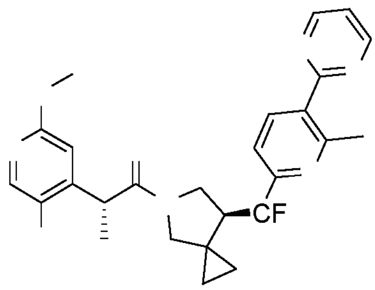

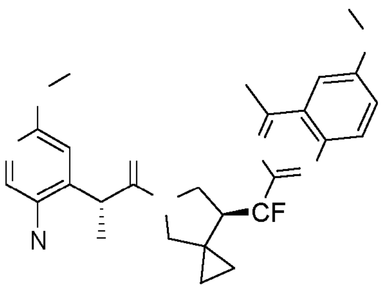

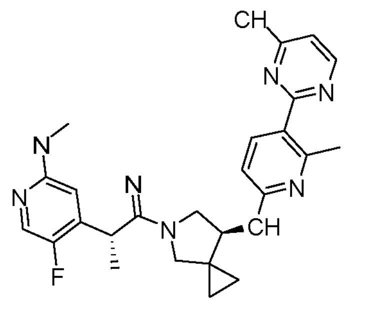

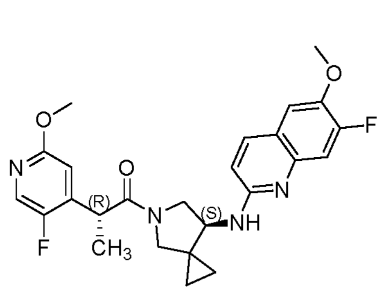

117

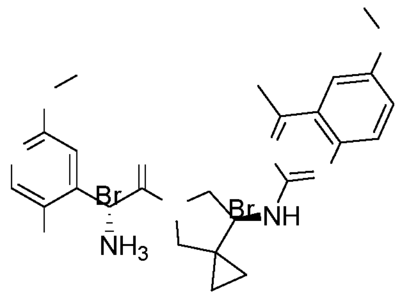

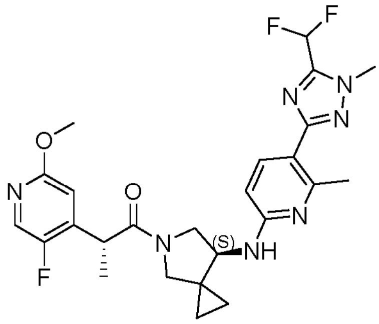

119

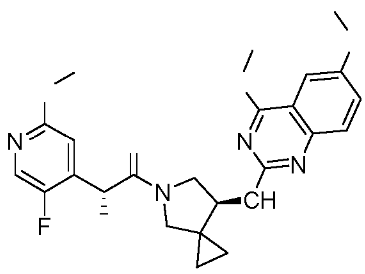

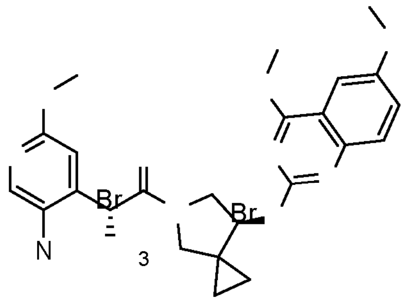

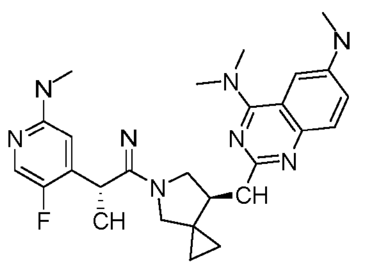

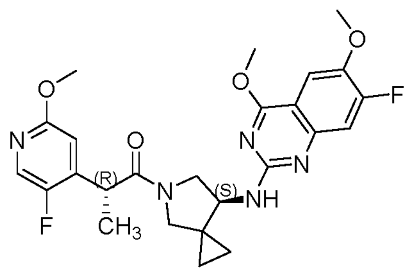

123

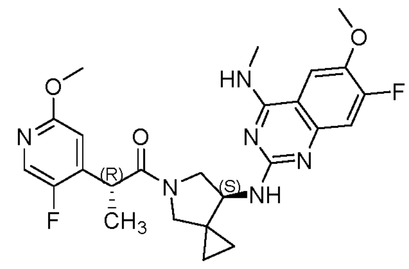

124

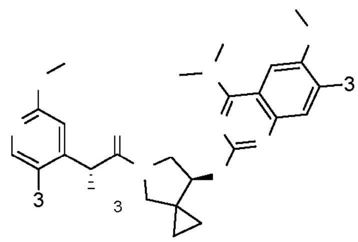

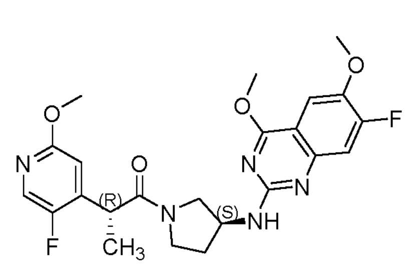

126

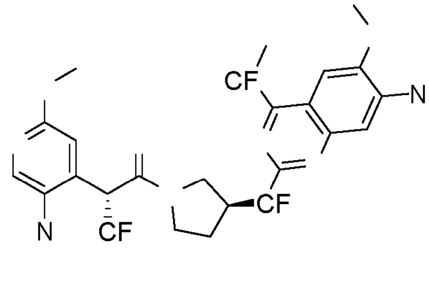

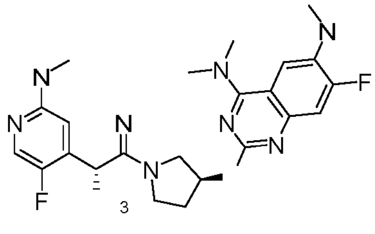

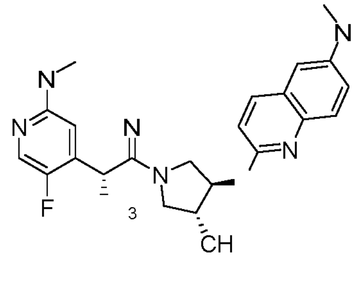

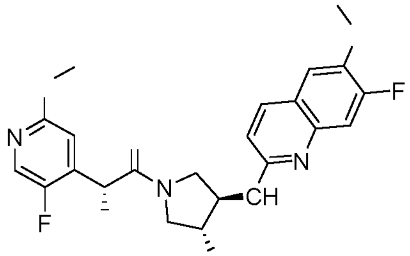

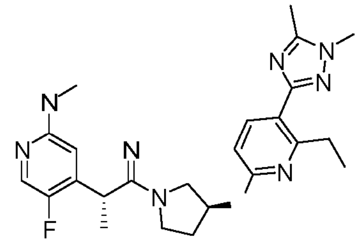

CH

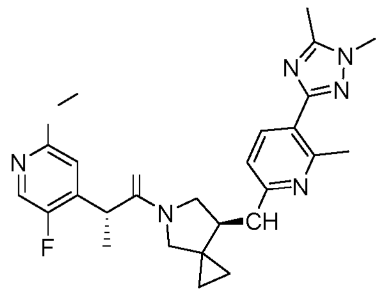

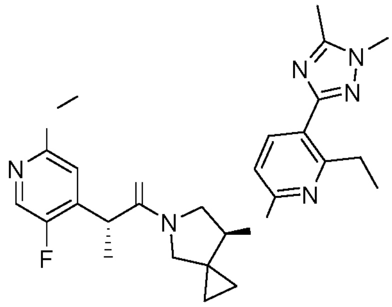

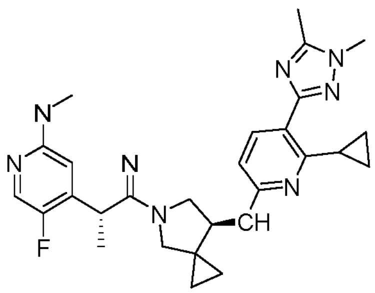

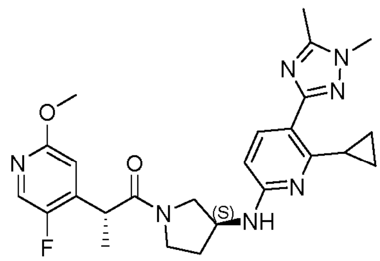

135

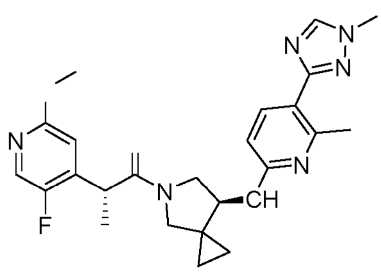

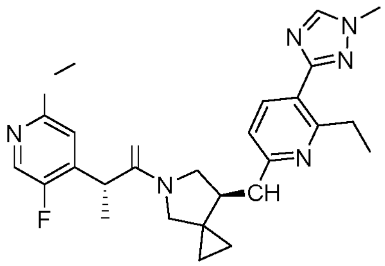

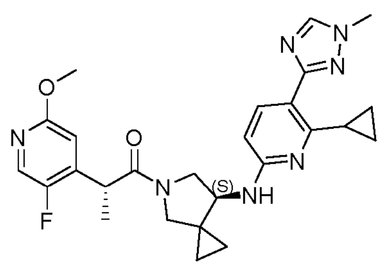

138

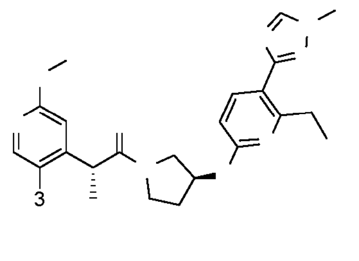

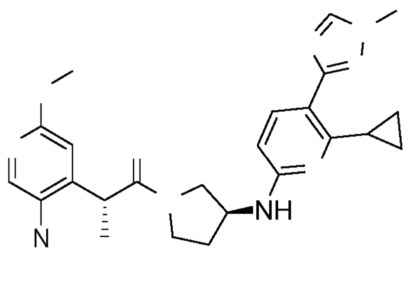

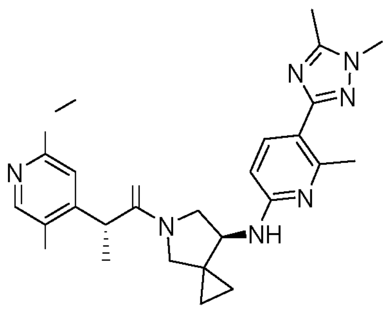

NH

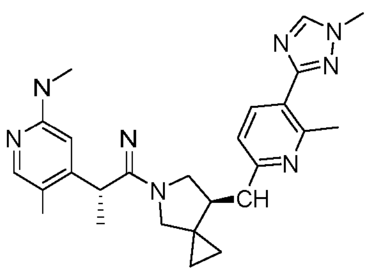

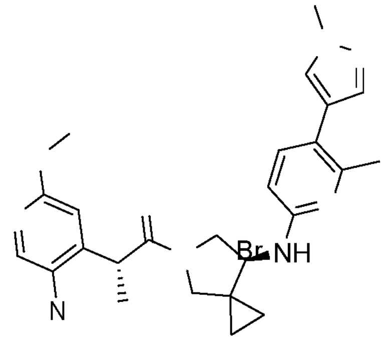

024

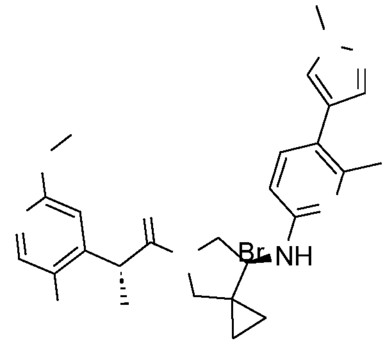

024

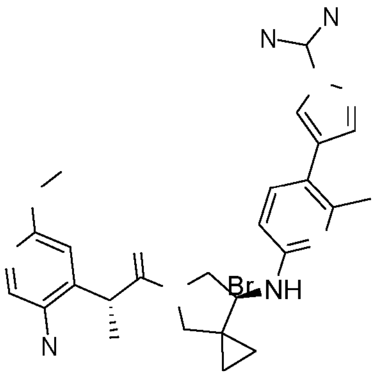

024

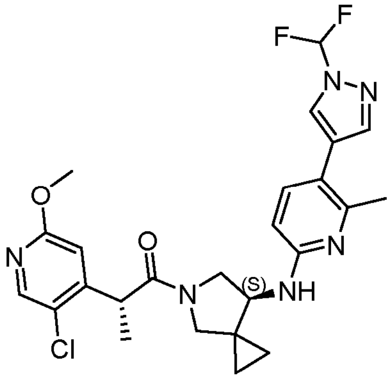

146

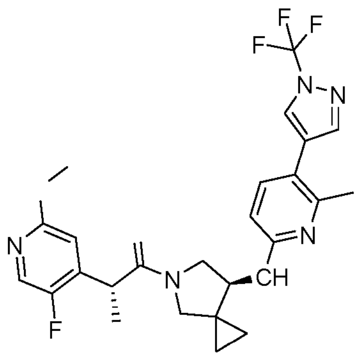

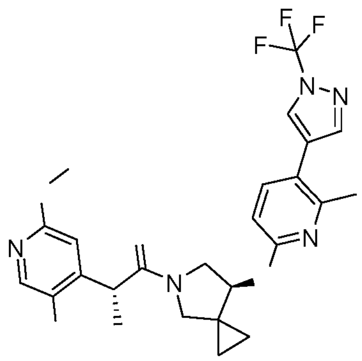

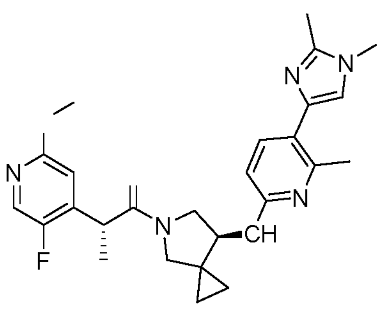

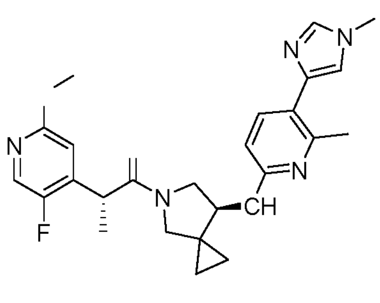

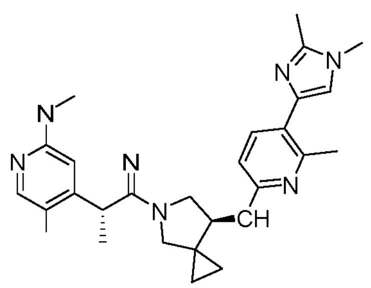

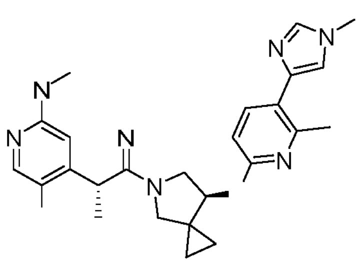

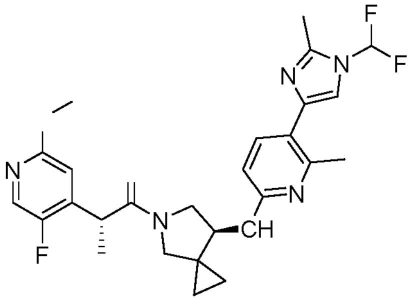

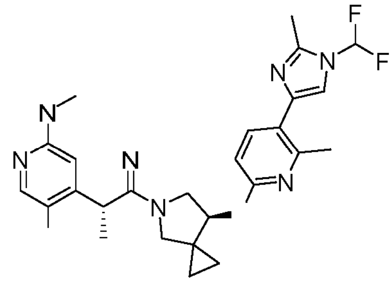

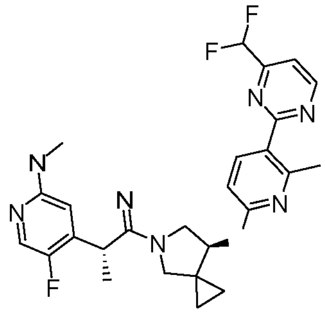

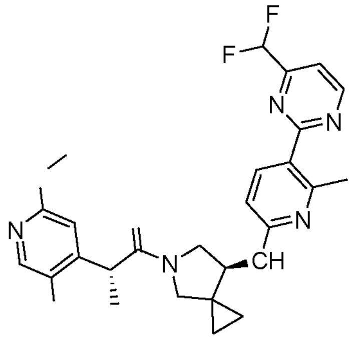

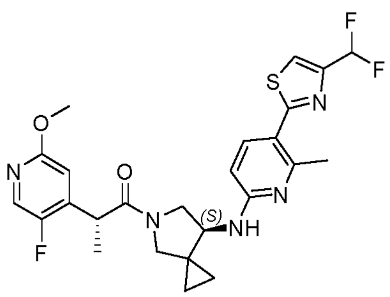

157

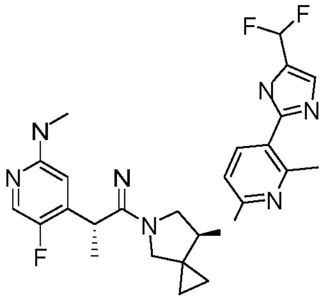

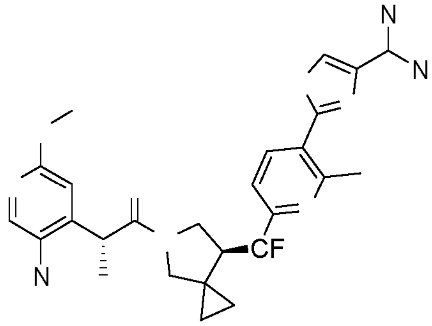

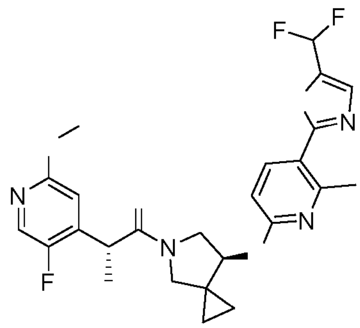

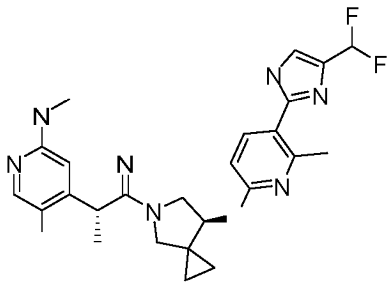

CH

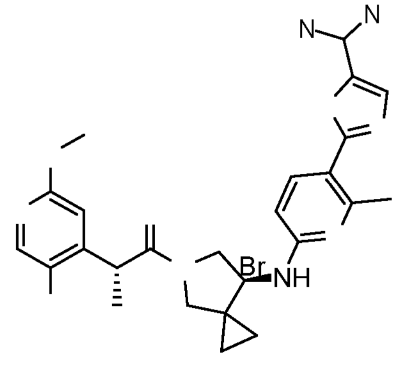

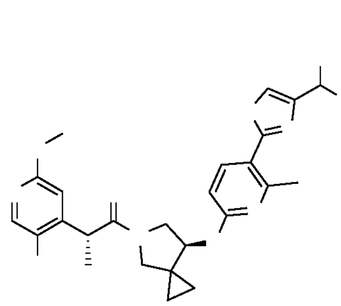

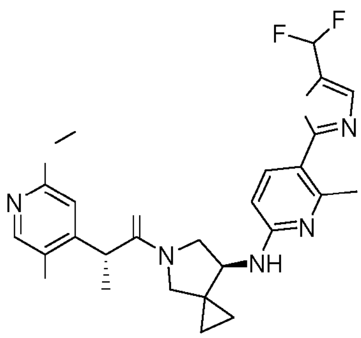

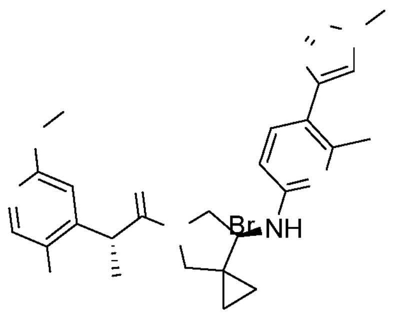

024

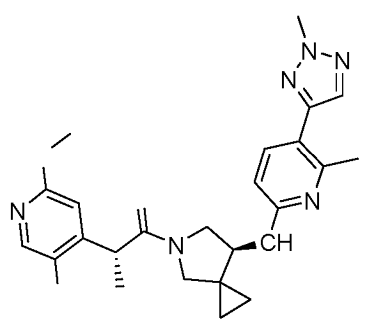

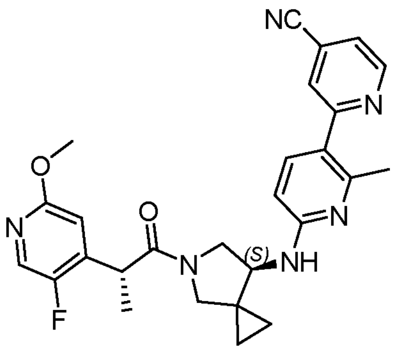

167

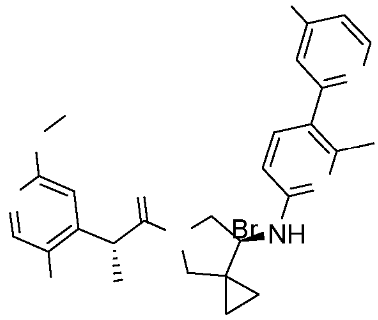

024

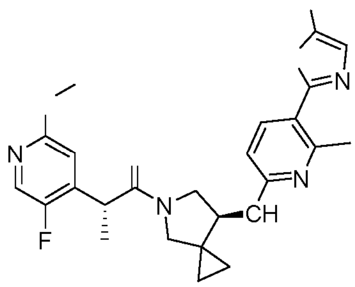

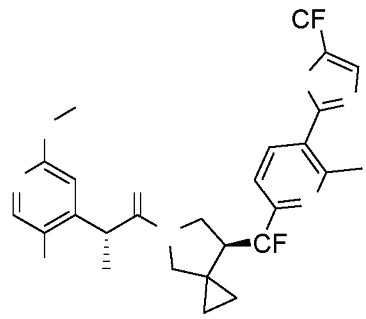

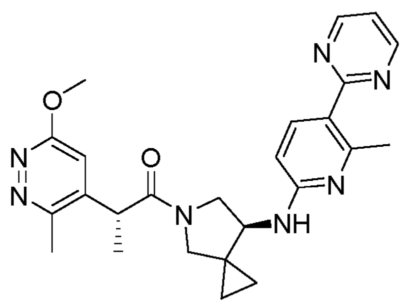

171

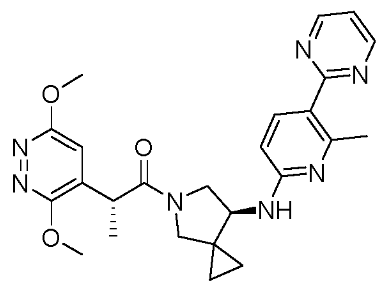

172

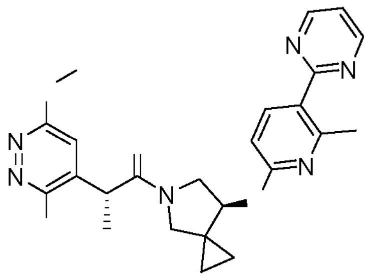

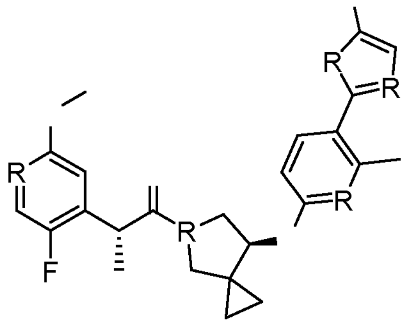

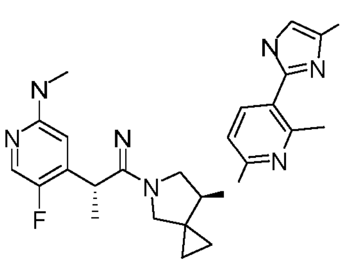

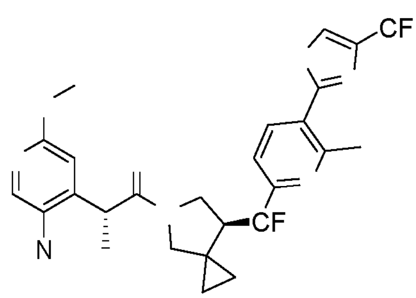

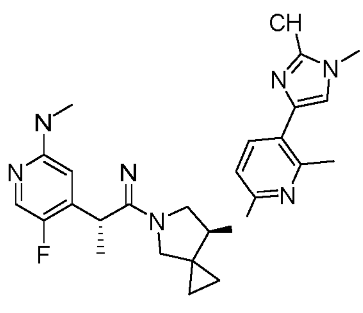

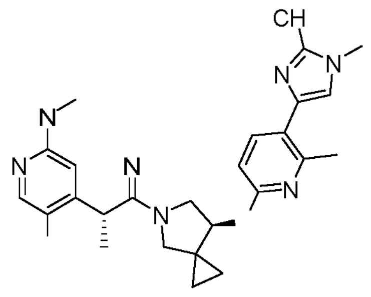

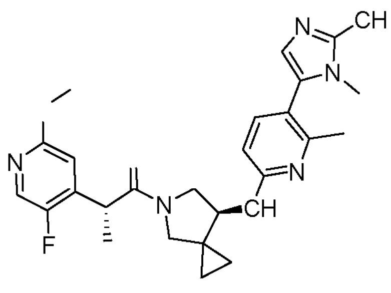

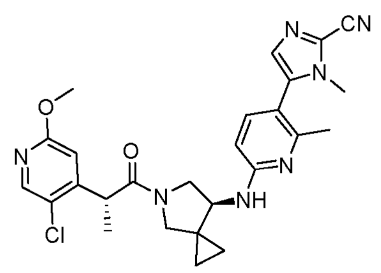

180

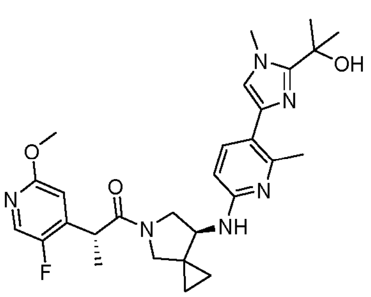

181

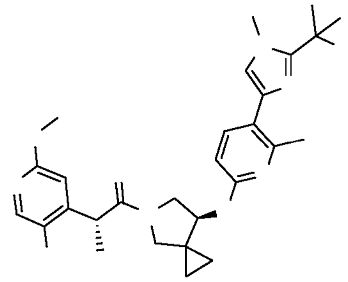

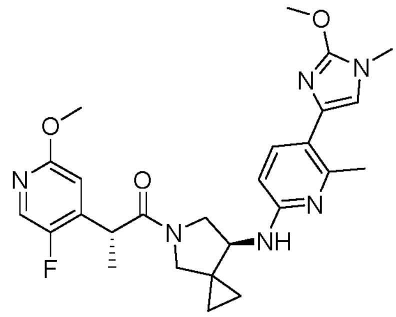

183

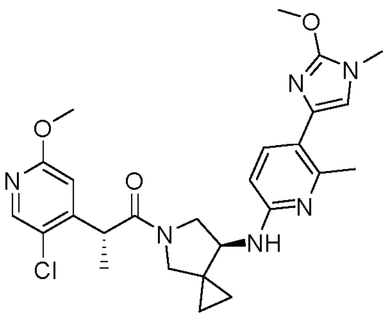

184

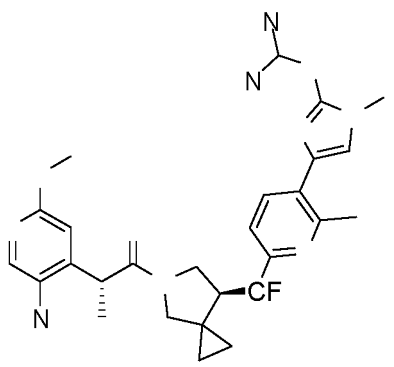

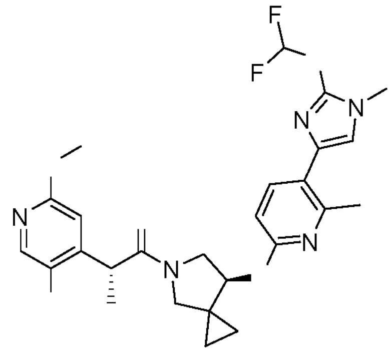

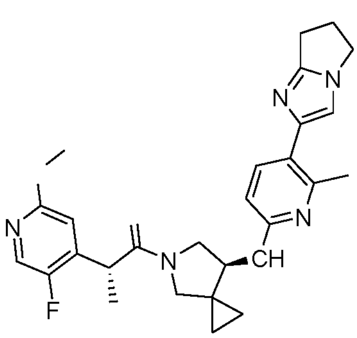

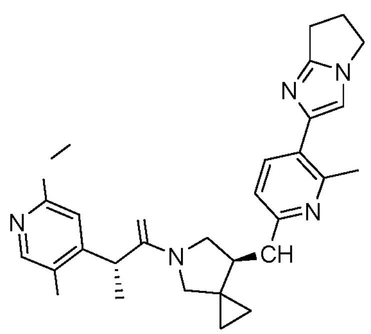

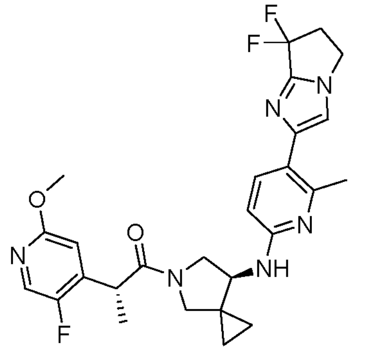

189

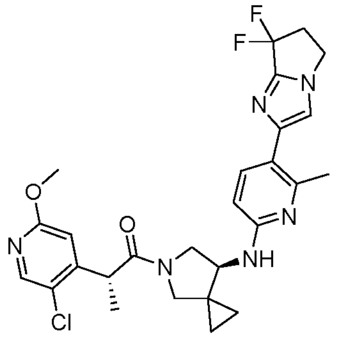

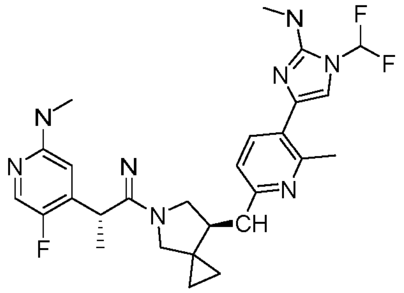

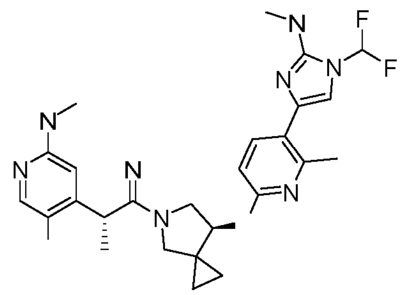

190

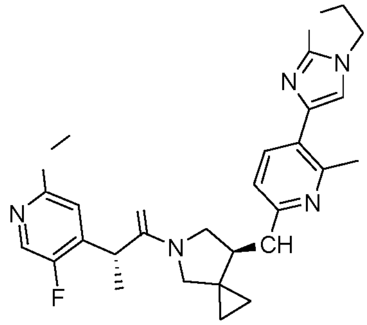

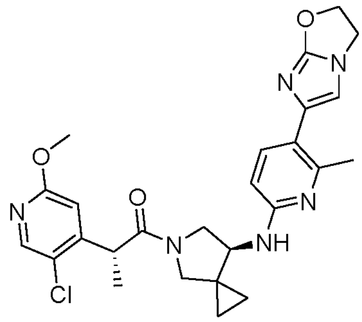

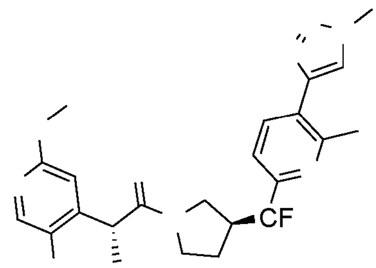

194

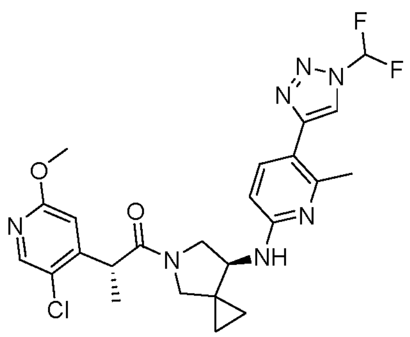

196

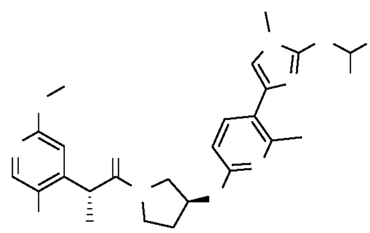

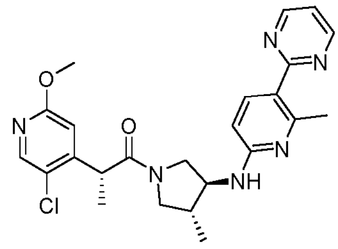

198

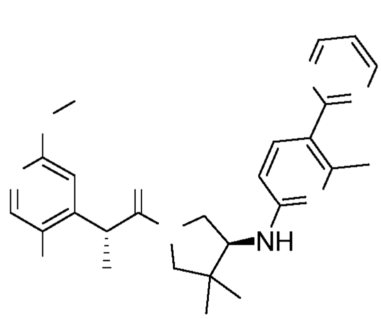

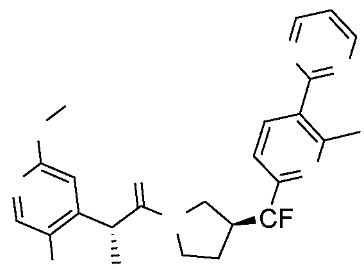

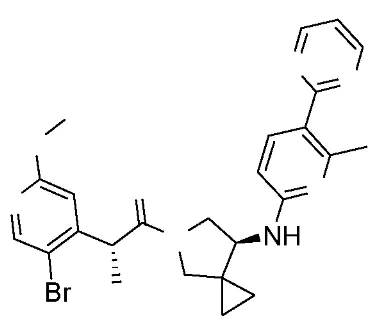

024

.

In some embodiments, the compound represented by formula I is selected from the following structures:

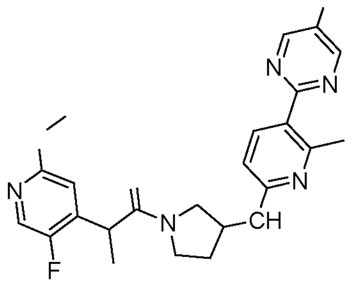

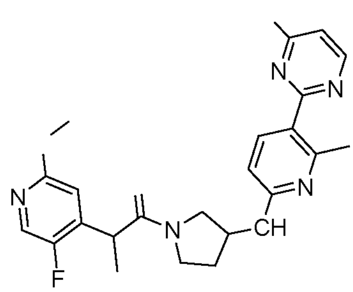

111-X

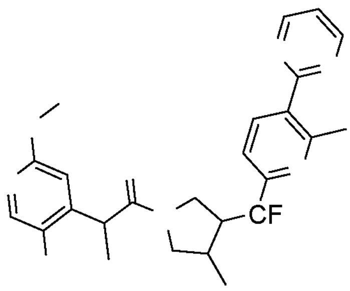

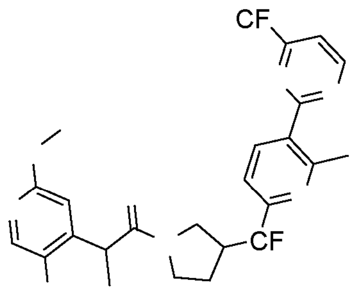

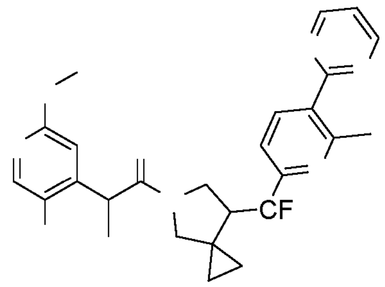

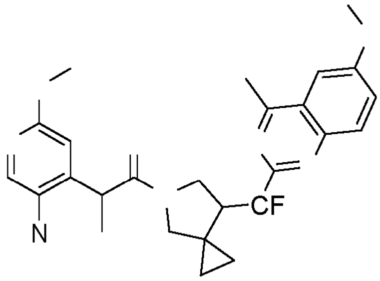

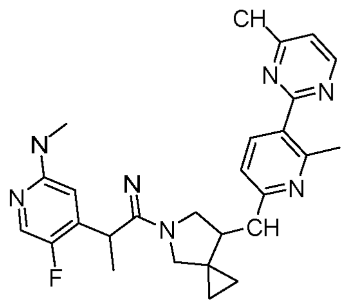

111-X

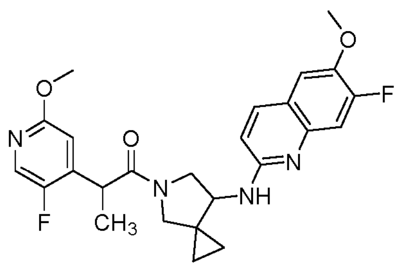

117-X

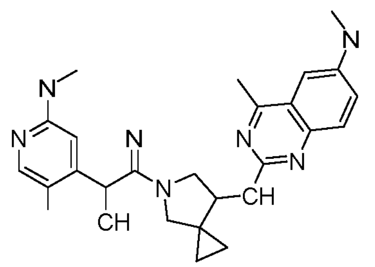

111-X

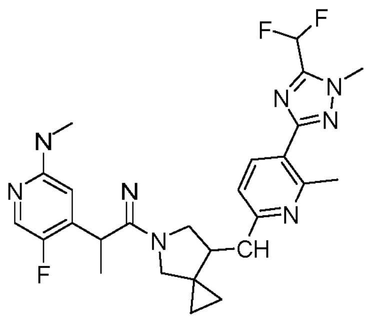

111-X

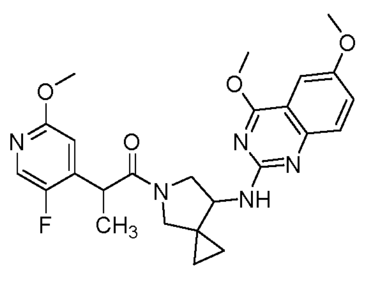

120-X

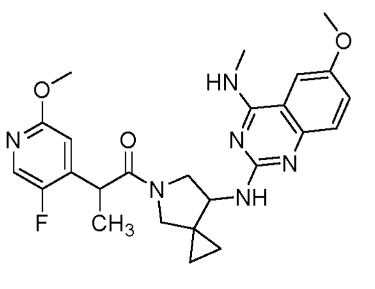

121-X

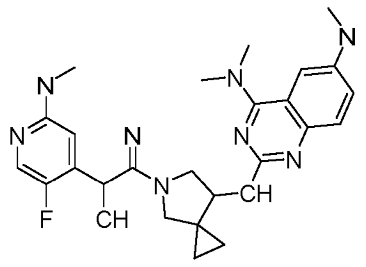

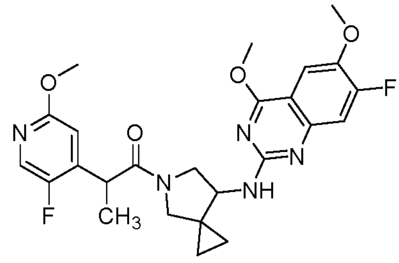

123-X

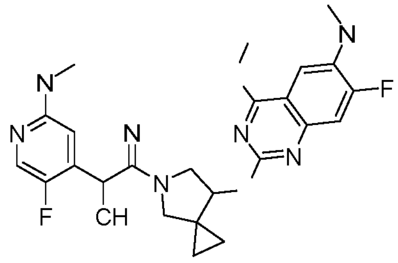

111-X

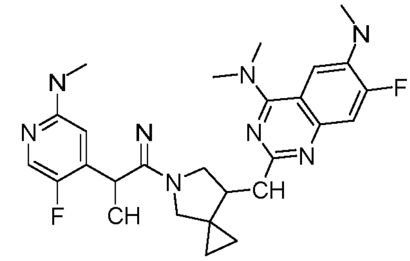

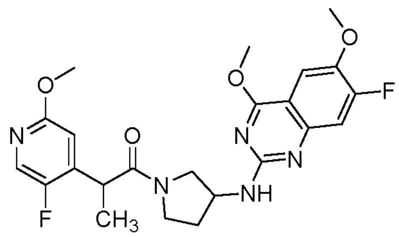

126-X

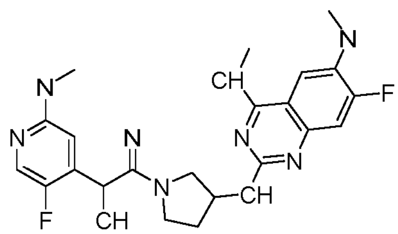

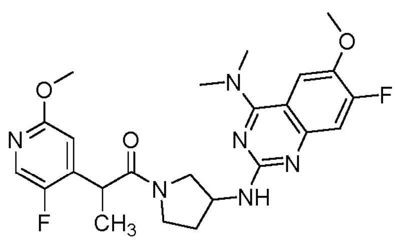

128-X

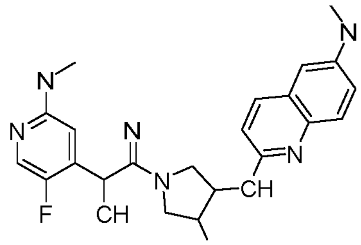

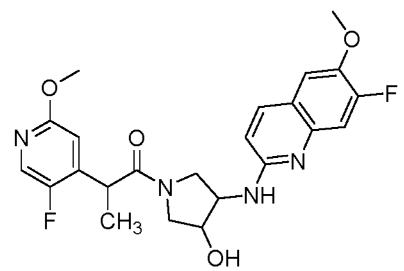

130-X

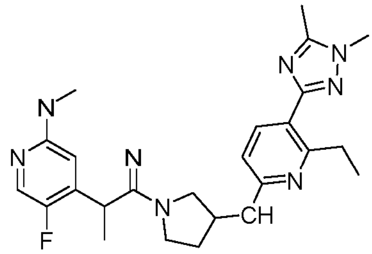

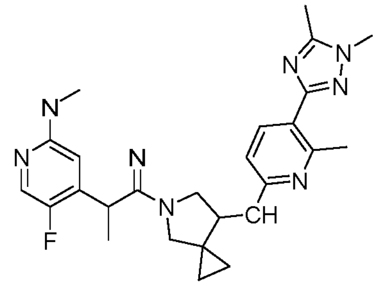

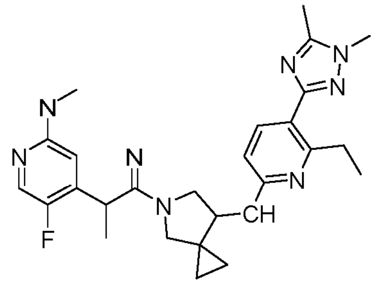

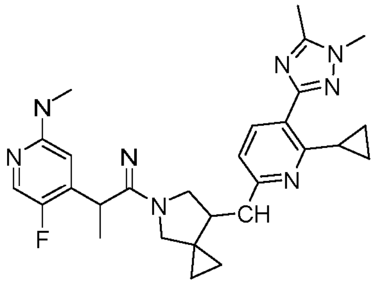

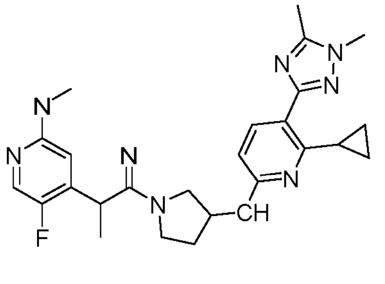

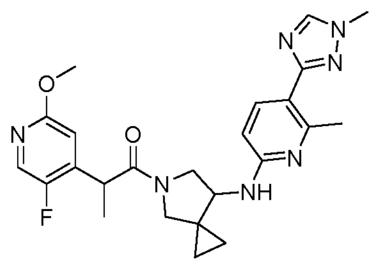

136-X

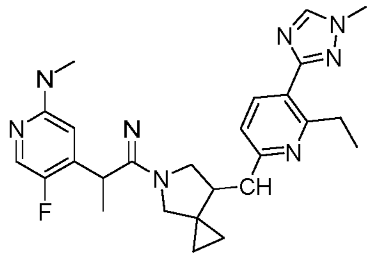

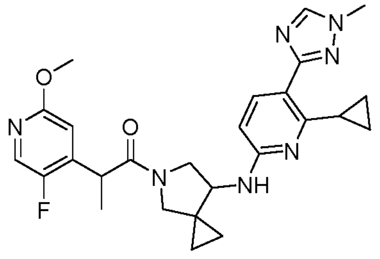

138-X

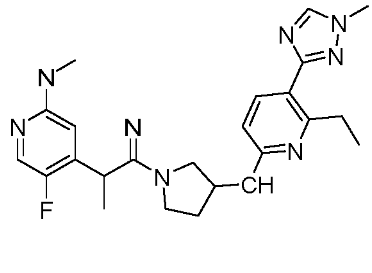

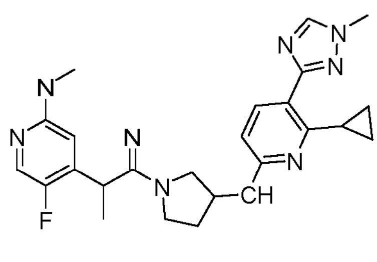

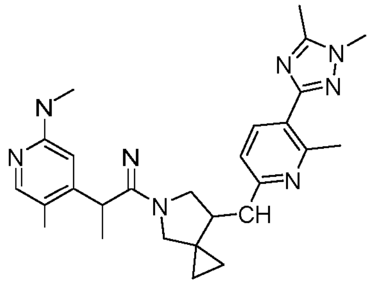

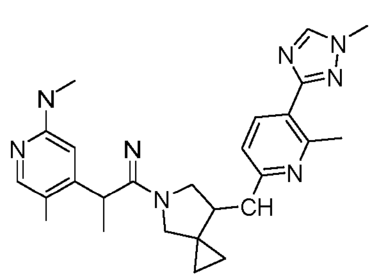

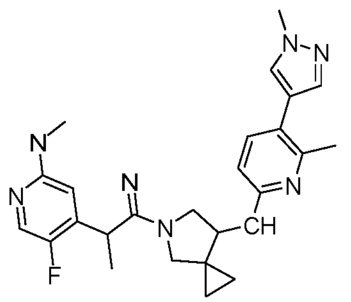

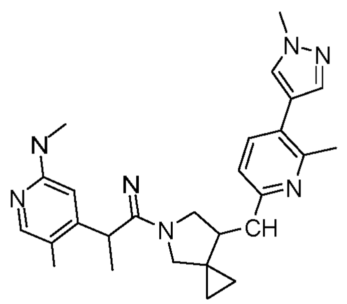

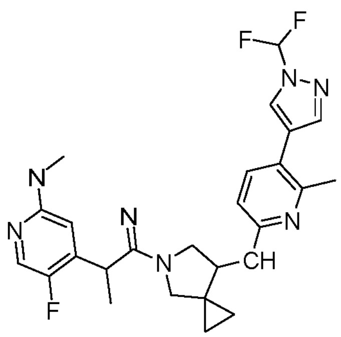

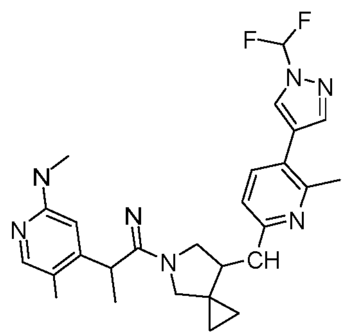

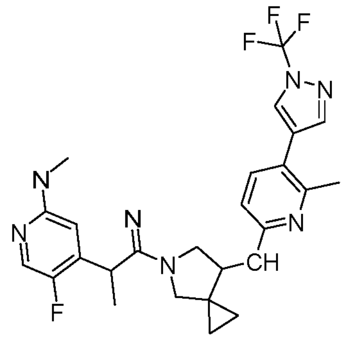

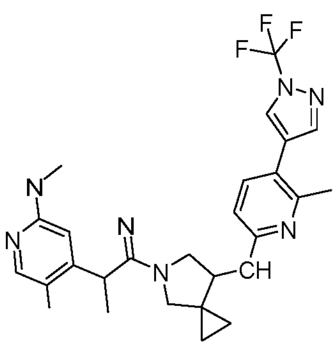

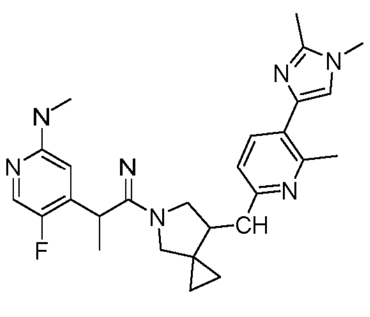

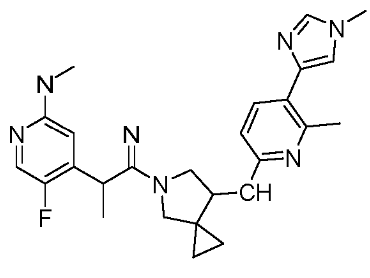

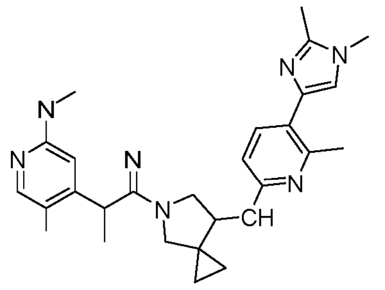

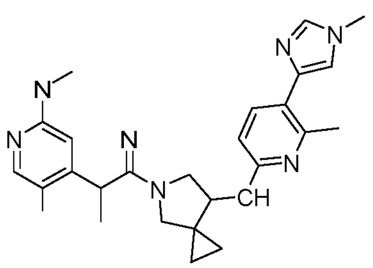

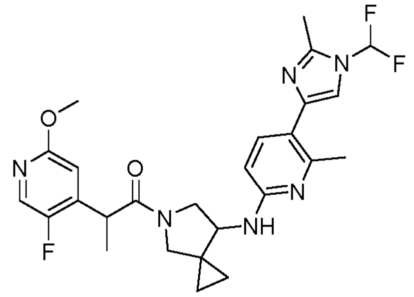

153-X

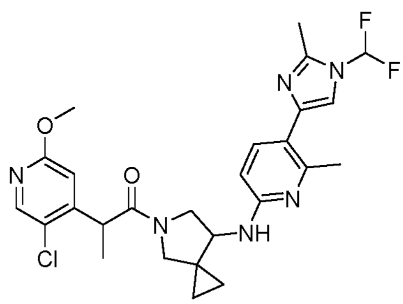

154-X

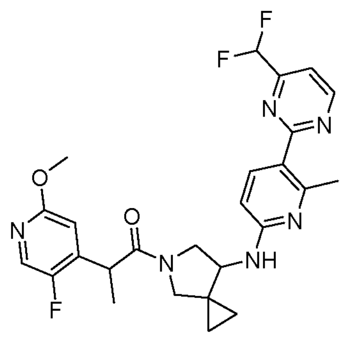

155-X

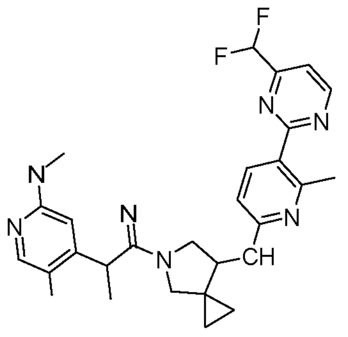

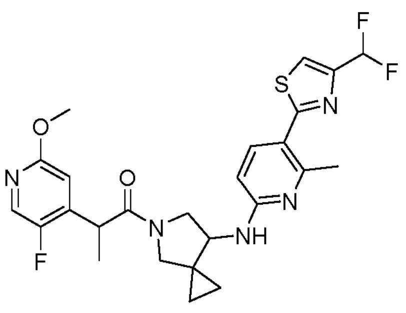

157-X

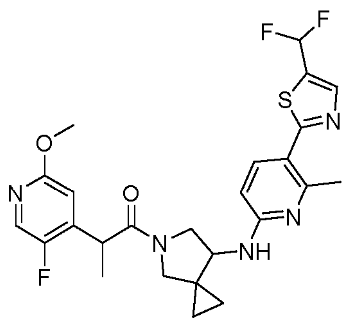

158-X

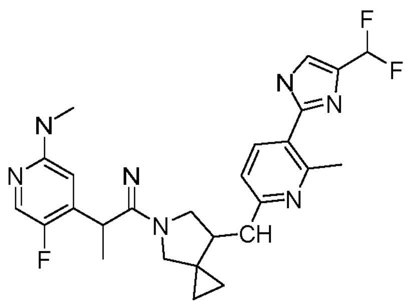

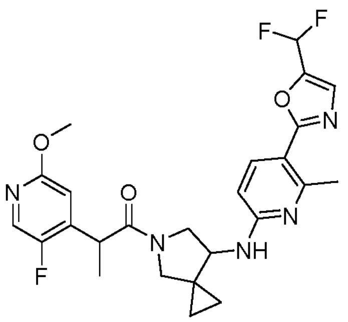

160-X

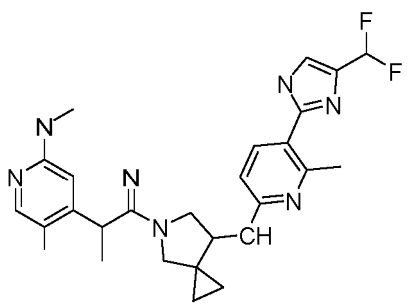

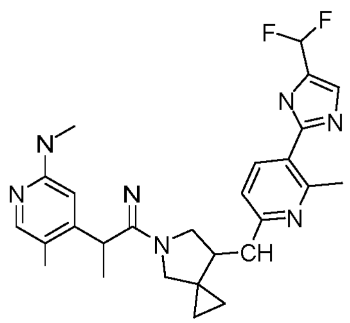

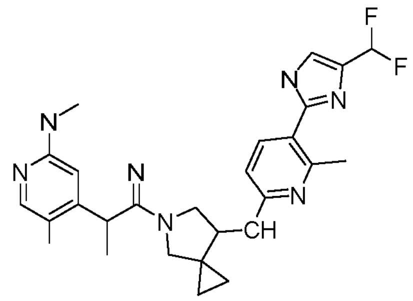

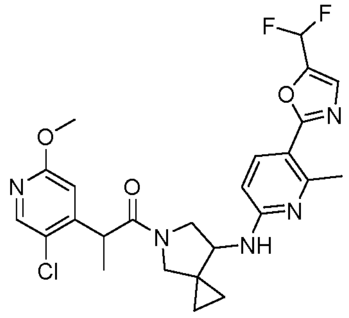

164-X

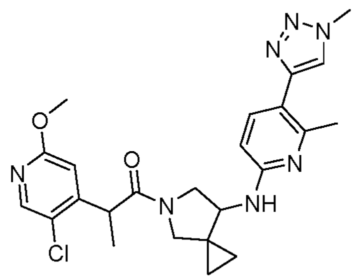

165-X

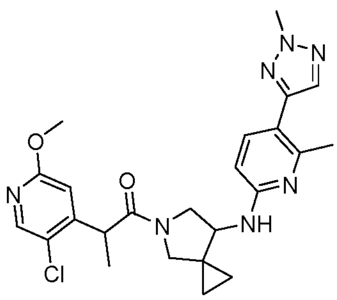

166-X

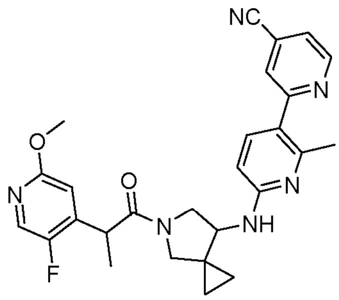

167-X

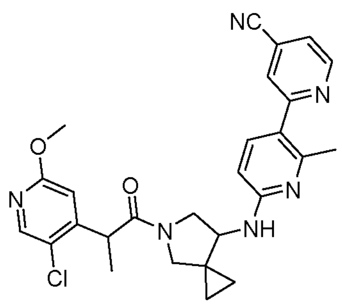

168-X

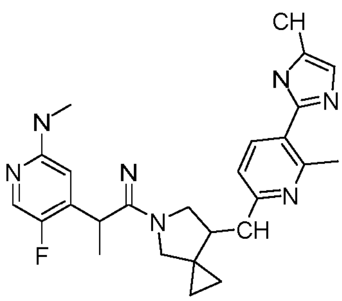

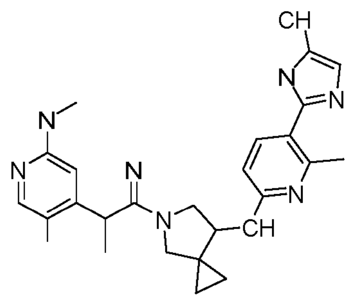

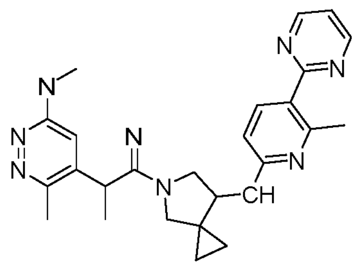

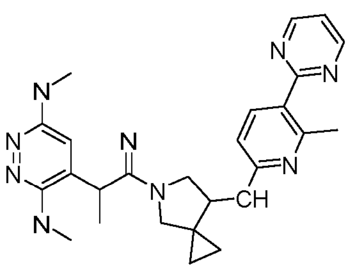

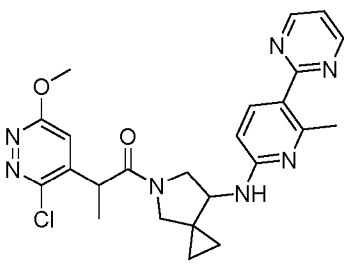

173-X

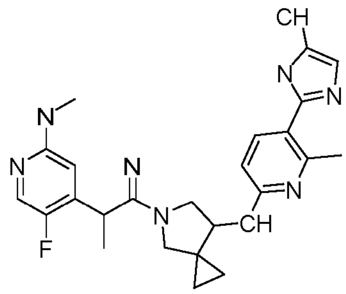

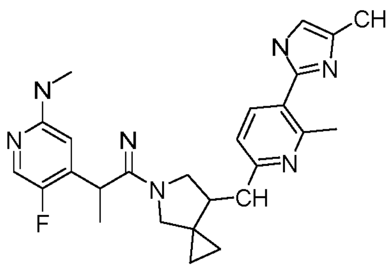

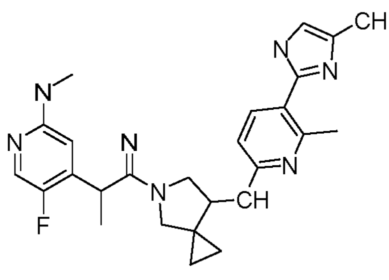

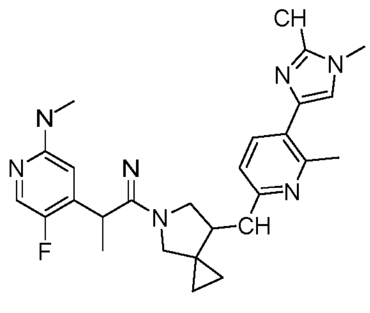

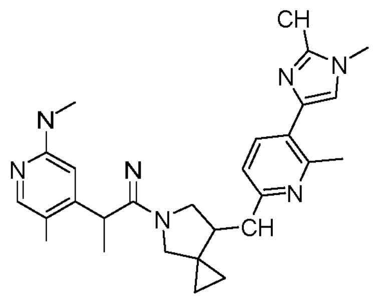

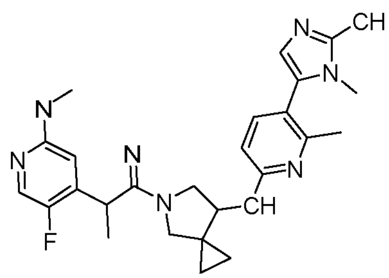

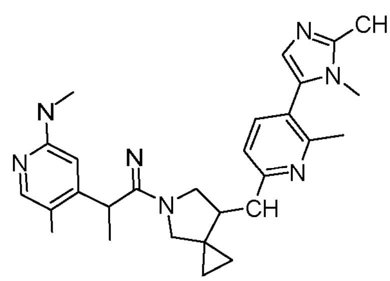

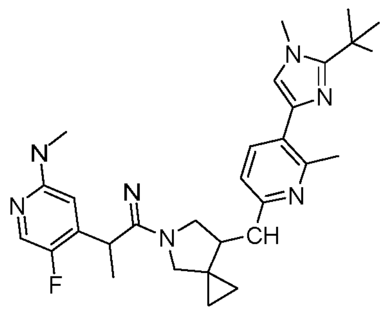

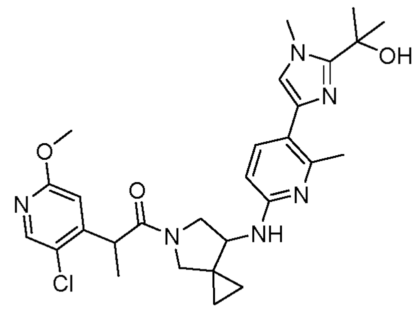

182-X

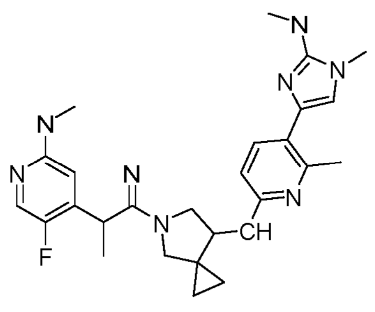

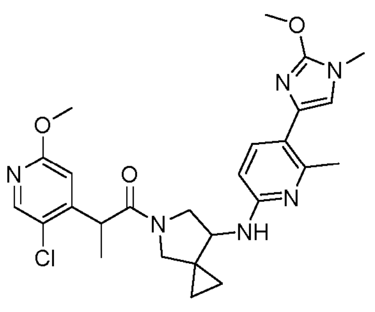

184-X

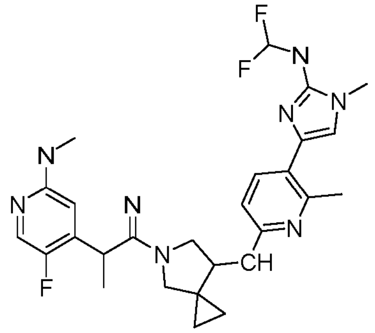

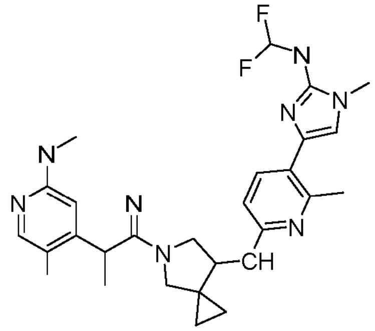
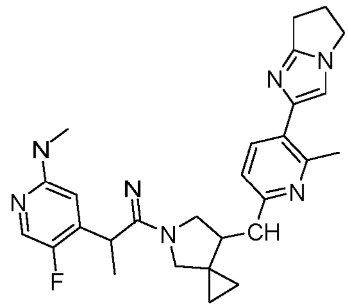
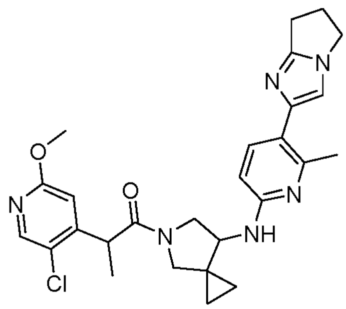

188-X

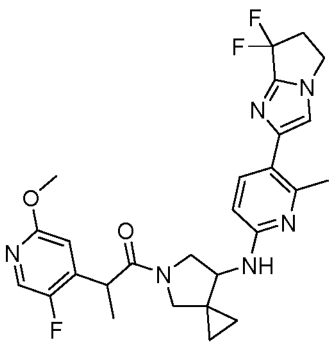

189-X

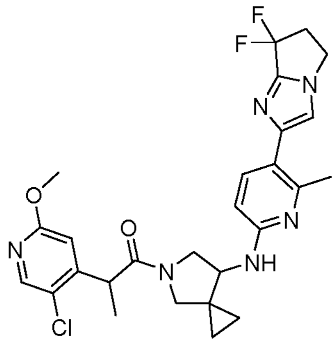

190-X

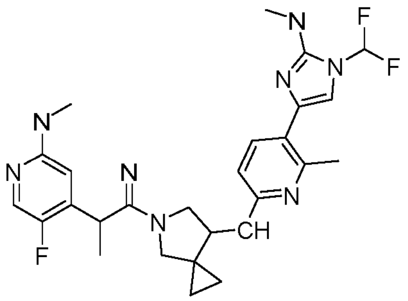

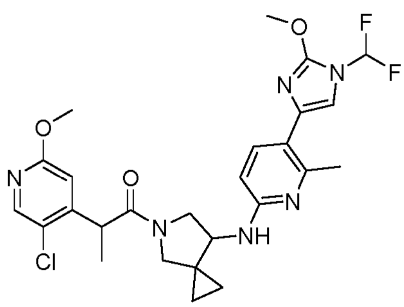

192-X

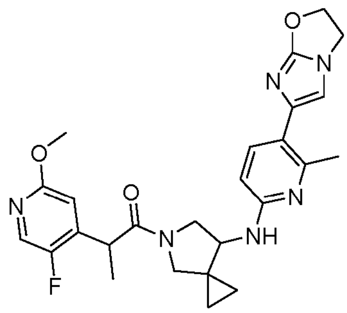

193-X

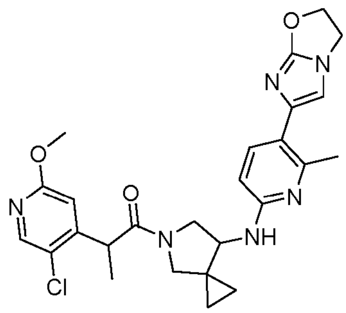

194-X

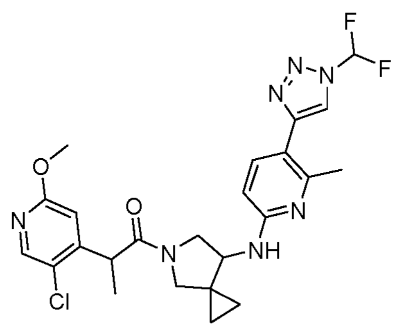

196-X

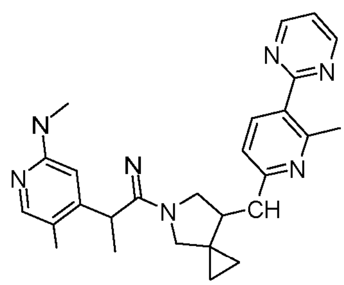

.

The present invention further provides a method for preparing the compound represented by formula I, comprising the following step:

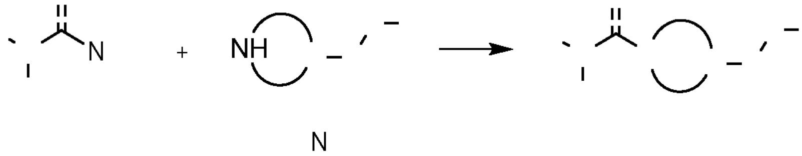

wherein X, Y, R, A, E, G, and M are as defined as described above; and L is selected from a leaving group, such as OH, OTf, F, Cl, Br, I, tributyltin ($SnBu_3$), 4,4,5,5-tetramethyl-1,3,2-dioxaborolan-2-yl, or methylsulfonyl.

In a preferred embodiment, the preparation method of the present invention exemplarily comprises the following step:

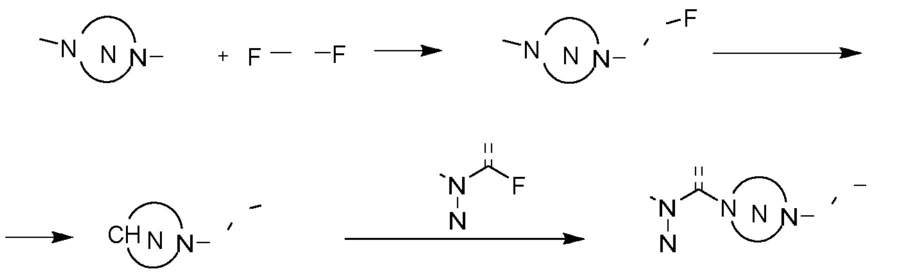

wherein X, Y, R, A, E, G, and M are as defined as described herein; L, $L_1$, $L_2$, and $L_3$ are each selected from a leaving group, such as OH, OTf, F, Cl, Br, I, tributyltin ($SnBu_3$), 4,4,5,5-tetramethyl-1,3,2-dioxaborolan-2-yl, or methylsulfonyl; and PG is selected from an amino-protecting group, such as benzyloxycarbonyl (Cbz), tert-butoxycarbonyl (Boc), fluorenylmethoxycarbonyl (Fmoc), benzyl (Bn), or p-methoxybenzyl (PMB).

In some embodiments, the preparation method of the present invention exemplarily comprises the following step:

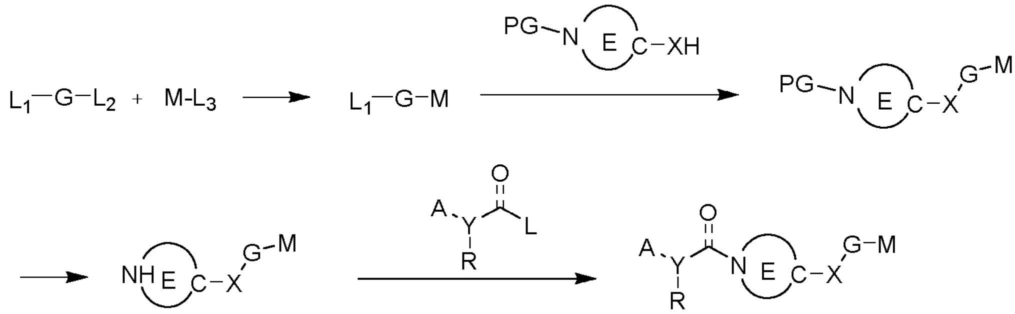

wherein X, Y, R, A, E, G, and M are as defined as described herein; L, $L_1$, $L_2$, and $L_3$ are each selected from a leaving group, such as OH, OTf, F, Cl, Br, I, tributyltin ($SnBu_3$), 4,4,5,5-tetramethyl-1,3,2-dioxaborolan-2-yl, or methylsulfonyl; and PG is selected from an amino-protecting group, such as benzyloxycarbonyl (Cbz), tert-butoxycarbonyl (Boc), fluorenylmethoxycarbonyl (Fmoc), benzyl (Bn), or p-methoxybenzyl (PMB).

In a preferred embodiment, the preparation method of the present invention exemplarily comprises the following step:

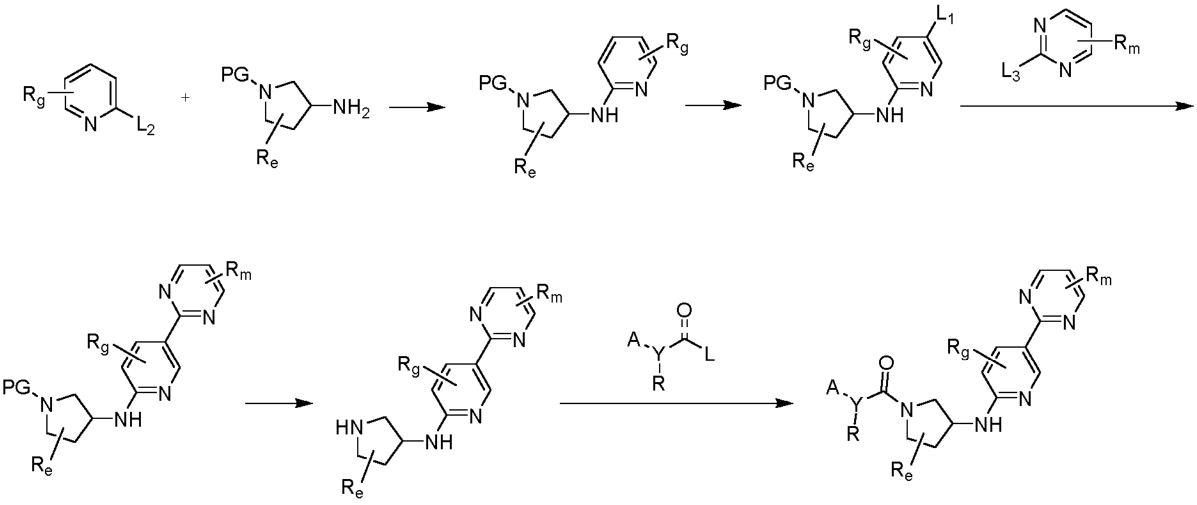

wherein Y, R, A, $R_e$, $R_g$, $R_m$, PG, L, $L_1$, $L_2$, and $L_3$ are as defined as described herein.

The present invention further provides a pharmaceutical composition, comprising the compound represented by formula I in the present invention, and a racemate, a stereoisomer, a tautomer, an isotopic label, a nitrogen oxide, a solvate, a polymorph, a metabolite, an ester, a prodrug, or a pharmaceutically acceptable salt thereof.

In some embodiments, the pharmaceutical composition of the present invention further comprises a therapeutically effective amount of the compound of formula I in the present invention and a racemate, a stereoisomer, a tautomer, an isotopic label, a nitrogen oxide, a solvate, a polymorph, a metabolite, an ester, a prodrug, or a pharmaceutically acceptable salt thereof, and a pharmaceutically acceptable carrier.

The carrier in the pharmaceutical composition is "acceptable", is compatible with (preferably, can stabilize) an active ingredient of the composition, and is not harmful to a to-be-treated subject. An active compound may be delivered using one or more drug excipients.

The present invention further provides use of the compound represented by formula I and a racemate, a stereoisomer, a tautomer, an isotopic label, a nitrogen oxide, a solvate, a polymorph, a metabolite, an ester, a prodrug, or a pharmaceutically acceptable salt thereof, or the pharmaceutical composition for the manufacture of a medicament.

In some embodiments, the medicament is for diagnosis, prevention, and/or treatment of an MC4R-mediated disease or condition.

In some embodiments, the medicament is an MC4R antagonist.

In some embodiments, the disease or condition is cachexia (cancer-associated cachexia, acquired immunodeficiency syndrome (AIDS)-associated cachexia, congestive heart failure (CHF)-associated cachexia, chronic kidney disease (CKD)-associated cachexia, and cachexia associated with the treatment of other chronic diseases); anorexia or anorexia nervosa (anorexia of aging, and anorexia associated with chemotherapy and/or radiotherapy); nausea and vomiting; weight loss (involuntary weight loss); failure to thrive; sarcopenia; muscular dystrophy; muscle weakness; fragility; osteoporosis; bone disorders (bone loss); pain (neuropathic pain); anxiety (post-traumatic stress disorder or PTSD); depression; hypertension; malnutrition in obesity (for example, sarcopenia caused by chronic obesity); sexual dysfunction; and inflammatory disorders (inflammatory disorders associated with anorexia, cachexia, sarcopenia, or muscular dystrophy).

The present invention further provides a method for diagnosing, preventing, and/or treating an MC4R-mediated disease or condition. The method comprises administering to a patient in need of such treatment a therapeutically effective amount of at least one compound of the present invention alone or optionally in combination with another compound of the present invention and/or at least one therapeutic agent of another type.

According to the present invention, the disease or condition is cachexia (cancer-associated cachexia, acquired immunodeficiency syndrome (AIDS)-associated cachexia, congestive heart failure (CHF)-associated cachexia, chronic kidney disease (CKD)-associated cachexia, and cachexia associated with the treatment of other chronic diseases); anorexia or anorexia nervosa (anorexia of aging, and anorexia associated with chemotherapy and/or radiotherapy); nausea and vomiting; weight loss (involuntary weight loss); failure to thrive; sarcopenia; muscular dystrophy; muscle weakness; fragility; osteoporosis; bone disorders (bone loss); pain (neuropathic pain); anxiety (post-traumatic stress disorder or PTSD); depression; hypertension; malnutrition in obesity (for example, sarcopenia caused by chronic obesity); sexual dysfunction; inflammatory disorders (inflammatory disorders associated with anorexia, cachexia, sarcopenia, or muscular dystrophy); and the like.

In some embodiments, as an MC4R antagonist, the compound is used for, comprising but not limited to: cachexia (cancer-associated cachexia, acquired immunodeficiency syndrome (AIDS)-associated cachexia, congestive heart

failure (CHF)-associated cachexia, chronic kidney disease (CKD)-associated cachexia, and cachexia associated with the treatment of other chronic diseases); and anorexia or anorexia nervosa (anorexia of aging, and anorexia associated with chemotherapy and/or radiotherapy).

The compound of the present invention may be used in combination with another therapeutic agent.

## Beneficial Effects

The compound provided in the present invention has good MC4R antagonistic activity. The compound of the present invention not only has good biological activity and a favorable safety profile, but also improves transmembrane activity and drug bioavailability.

In the present invention, through structure optimization, in vivo pharmacokinetic properties of the compound are significantly improved, and the inhibition of CYP 3A4M by the compound is reduced, lowering the risk of drug-drug interactions, and improving the druggability of the compound.

## Term Definition

Unless otherwise indicated, the definitions of groups and terms recorded in the specification and claims of this application comprise their definitions as examples, exemplary definitions, preferred definitions, definitions recorded in tables, definitions of specific compounds in the embodiments, and the like, which may be arbitrarily combined with each other. The group definitions and compound structures obtained through such combinations should be understood to be within the scope of the specification and/or claims of this application.

In this application, the term "optional" (or "optionally") in definitions of general formulas means a case of substitution with zero, one, or more substituents. For example, "optionally substituted with one, two, or more R" means "may not be substituted with R (no substitution) or may be selectively substituted with one, two, or more R".

"More" means three or more.

Unless otherwise indicated, a numerical range recorded in this specification and in the claims is equivalent to at least every specific integer value therein. For example, the numerical range "1-12" is equivalent to every integer value in the numerical range "1-12", that is, 1, 2, 3, 4, 5, 6, 7, 8, 9, 10, 11, and 12.

It should be understood that the term "$C_{1-12}$ alkyl" indicates straight- and branched-chain alkyl groups with 1-12 carbon atoms, "$C_{1-8}$ alkyl" indicates straight- and branched-chain alkyl groups with 1, 2, 3, 4, 5, 6, 7, or 8 carbon atoms, and "$C_{1-6}$ alkyl" indicates straight- and branched-chain alkyl groups with 1, 2, 3, 4, 5, or 6 carbon atoms. The alkyl group is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or an isomer thereof.

The term "$C_{3-12}$ cycloalkyl" should be understood as indicating saturated monovalent monocyclic and bicyclic (such as fused ring, bridged ring, or spiro ring) hydrocarbons or tricyclic alkanes, with 3-12 carbon atoms, preferably "$C_{3-10}$ cycloalkyl", more preferably "$C_{3-8}$ cycloalkyl". The term "$C_{3-12}$ cycloalkyl" should be understood as indicating saturated monovalent monocyclic and bicyclic (such as bridged ring or spiro ring) hydrocarbons or tricyclic alkanes, with 3, 4, 5, 6, 7, 8, 9, 10, 11, or 12 carbon atoms. The $C_{3-12}$ cycloalkyl may be a monocyclic hydrocarbyl group, such as cyclopropyl, cyclobutyl, cyclopentyl, cyclohexyl, cycloheptyl, cyclooctyl, cyclononyl, or cyclodecyl, or may be a bicyclic hydrocarbyl group, such as bornyl, indolyl, hexahydroindolyl, tetrahydronaphthyl, decahydronaphthyl, bicyclo[2.1.1]hexyl, bicyclo [2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2] octyl, 2,7-diazaspiro[3,5]nonyl, or 2,6-diazaspiro[3,4]octyl, or may be a tricyclic hydrocarbyl group, such as adamantyl.

The term "$C_{6-14}$ aryl" should be understood as preferably indicating monovalent aromatic or partially aromatic monocyclic, bicyclic (such as fused ring, bridged ring, or spiro ring), or tricyclic hydrocarbons, with 6-14 carbon atoms, which may be monoaromatic rings or fused polyaromatic rings, preferably "$C_{6-10}$ aryl". The term "$C_{6-14}$ aryl" should be understood as preferably indicating monovalent aromatic or partially aromatic monocyclic, bicyclic, or tricyclic hydrocarbons ("$C_{6-14}$ aryl") with 6, 7, 8, 9, 10, 11, 12, 13, or 14 carbon atoms, especially a ring ("$C_6$ aryl") with 6 carbon atoms, such as phenyl or biphenyl, or a ring ("$C_9$ aryl") with 9 carbon atoms, such as indanyl or indenyl, or a ring ("$C_{10}$ aryl") with 10 carbon atoms, such as tetrahydronaphthyl, dihydronaphthyl, or naphthyl, or a ring ("$C_{13}$ aryl") with 13 carbon atoms, such as fluorenyl, or a ring ("$C_{14}$ aryl") with 14 carbon atoms, such as anthryl. The $C_{6-20}$ aryl group may be mono- or poly-substituted. In addition, there is no limitation on a substitution site, which may be, for example, an ortho-position, a para-position, or a meta-position.

The term "5- to 14-membered heteroaryl" should be understood as comprising such monovalent monocyclic, bicyclic (such as fused ring, bridged ring, or spiro ring), or tricyclic aromatic ring systems, with 5-14 ring atoms and 1-5 heteroatoms independently selected from N, O, and S, for example, "5- to 10-membered heteroaryl". The term "5- to 14-membered heteroaryl" should be understood as comprising such monovalent monocyclic, bicyclic, or tricyclic aromatic

ring systems, with 5, 6, 7, 8, 9, 10, 11, 12, 13, or 14 ring atoms, especially 5, 6, 9, or 10 carbon atoms, and 1-5, preferably 1-3, heteroatoms independently selected from N, O, and S, and may be benzo-fused in each case. The "heteroaryl" also refers to a group in which a heteroaromatic ring is fused with one or more aromatic, alicyclic, or heterocyclic rings, wherein a root or site for linking is on the heteroaromatic ring. Non-limiting examples comprise 1-, 2-, 3-, 5-, 6-, 7-, or 8-indolizinyl, 1-, 3-, 4-, 5-, 6-, or 7-isoindolyl, 2-, 3-, 4-, 5-, 6-, or 7-indolyl, 2-, 3-, 4-, 5-, 6-, or 7-indazolyl, 2-, 4-, 5-, 6-, 7-, or 8-purinyl, 1-, 2-, 3-, 4-, 6-, 7-, 8-, or 9-quinolizinyl, 2-, 3-, 4-, 5-, 6-, 7-, or 8-quinolinyl, 1-, 3-, 4-, 5-, 6-, 7-, or 8-isoquinolinyl, 1-, 4-, 5-, 6-, 7-, or 8-phthalazinyl, 2-, 3-, 4-, 5-, or 6-naphthyridinyl, 2-, 3-, 5-, 6-, 7-, or 8-quinazolinyl, 3-, 4-, 5-, 6-, 7-, or 8-cinnolinyl, 2-, 4-, 6-, or 7-pteridinyl, 1-, 2-, 3-, 4-, 5-, 6-, 7-, or 8-4aH-carbazolyl, 1-, 2-, 3-, 4-, 5-, 6-, 7-, or 8-carbazolyl, 1-, 3-, 4-, 5-, 6-, 7-, 8-, or 9-carbolinyl, 1-, 2-, 3-, 4-, 6-, 7-, 8-, 9-, or 10-phenanthridinyl, 1-, 2-, 3-, 4-, 5-, 6-, 7-, 8-, or 9-acridinyl, 1-, 2-, 4-, 5-, 6-, 7-, 8-, or 9-dine, 2-, 3-, 4-, 5-, 6-, 8-, 9-, or 10-phenanthrolinyl, 1-, 2-, 3-, 4-, 6-, 7-, 8-, or 9-phenazinyl, 1-, 2-, 3-, 4-, 6-, 7-, 8-, 9-, or 10-phenothiazinyl, 1-, 2-, 3-, 4-, 6-, 7-, 8-, 9-, or 10-phenazinyl, 2-, 3-, 4-, 5-, 6-, or 1-, 3-, 4-, 5-, 6-, 7-, 8-, 9-, or 10-benzoisoquinolinyl, 2-, 3-, or 4-thieno[2,3-b]furanyl, 2-, 3-, 5-, 6-, 7-, 8-, 9-, 10-, or 11-7H-pyrazino[2,3-c]carbazolyl, 2-, 3-, 5-, 6-, or 7-2H-furo[3,2-b]-pyranyl, 2-, 3-, 4-, 5-, 7-, or 8-5H-pyrido[2,3-d]-o-azine, 1-, 3-, or 5-1H-pyrazolo[4,3-d]-azole, 2-, 4-, or 5-4H-imidazo[4,5-d]thiazolyl, 3-, 5-, or 8-pyrazino[2,3-d]pyridazinyl, 2-, 3-, 5-, or 6-imidazo[2,1-b]thiazolyl, 1-, 3-, 6-, 7-, 8-, or 9-furo[3,4-c]cinnolinyl, 1-, 2-, 3-, 4-, 5-, 6-, 8-, 9-, 10-, or 11-4H-pyrido[2,3-c]carbazolyl, 2-, 3-, 6-, or 7-imidazo[1,2-b][1,2,4]triazinyl, 7-benzo[b]thienyl, 2-, 4-, 5-, 6-, or 7-benzazolyl, 2-, 4-, 5-, 6-, or 7-benzimidazolyl, 2-, 3-, 4-, 5-, 6-, or 7-benzothiazolyl, 1-, 2-, 4-, 5-, 6-, 7-, 8-, or 9-benzoxapinyl, 2-, 4-, 5-, 6-, 7-, or 8-benzazinyl, and 1-, 2-, 3-, 5-, 6-, 7-, 8-, 9-, 10-, or 11-4H-pyrrolo[1,2-b][2]benzazapinyl. Exemplary fused heteroaryl comprises, but is not limited to, 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, 2-, 4-, 5-, 6-, or 7-benzazolyl, 2-, 4-, 5-, 6-, or 7-benzimidazolyl, and 2-, 4-, 5-, 6-, or 7-benzothiazolyl. When the 5- to 14-membered heteroaryl is linked to another group to form the compound of the present invention, the another group may be linked to a carbon atom on the 5- to 14-membered heteroaryl ring, or the another group may be linked to a heteroatom on the 5- to 14-membered heteroaryl ring. The 5- to 14-membered heteroaryl group may be mono- or poly-substituted. In addition, there is no limitation on a substitution site, for example, which may be hydrogen linked to a carbon atom on the heteroaryl ring, or hydrogen linked to a heteroatom on the heteroaryl ring.

Unless otherwise defined, the term "3- to 14-membered heterocyclyl" refers to a saturated or unsaturated non-aromatic ring or ring system, for example, a 4-, 5-, 6-, or 7-membered monocyclic, 7-, 8-, 9-, 10-, 11-, or 12-membered bicyclic (such as fused ring, bridged ring, or spiro ring), or 10-, 11-, 12-, 13-, or 14-membered tricyclic ring system, containing at least one, for example, 1, 2, 3, 4, or 5, heteroatom selected from O, S, and N, wherein N and S may be optionally oxidized to various oxidation states to form a nitrogen oxide, -S(O)-, or $-S(O)_2-$. For example, the "3- to 14-membered heterocyclyl" may be 3- to 14-membered N-containing heterocyclyl (containing at least one N). Preferably, the heterocyclyl may be selected from "3-to 10-membered heterocyclyl". The term "3- to 10-membered heterocyclyl" refers to a saturated or unsaturated non-aromatic ring or ring system, containing at least one heteroatom selected from O, S, and N. The heterocyclyl may be linked to the rest of a molecule by any one of carbon atoms or a nitrogen atom (if present). The heterocyclyl may comprise a fused or bridged ring and a spiro ring. Particularly, the heterocyclyl may comprise, but is not limited to: a 4-membered ring such as azetidinyl or oxetanyl; a 5-membered ring such as tetrahydrofuranyl, dioxol, pyrrolidinyl, imidazolidinyl, pyrazolidinyl, or pyrrolinyl; a 6-membered ring such as tetrahydropyranyl, piperidinyl, morpholinyl, dithianyl, thiomorpholinyl, piperazinyl, or trithianyl; or a 7-membered ring such as diazepanyl. Optionally, the heterocyclyl may be benzo-fused. The heterocyclyl may be bicyclic, for example, but not limited to, 5- and 5-membered rings, such as a hexahydrocyclopenta[c]pyrrole-2(1H)-yl ring, or 5- and 6-membered rings, such as a hexahydropyrrolo [1,2-a]pyrazine-2(1H)-yl ring. The heterocyclyl may be partially unsaturated, that is, may comprise one or more double bonds, such as, but not limited to, dihydrofuranyl, dihydropyranyl, 2,5-dihydro-1H-pyrrolyl, 4H-[1,3,4]thiadiazinyl, 1,2,3,5-tetrahydrooxazolyl, or 4H-[1,4]thiazinyl, or may be benzo-fused, such as, but not limited to, dihydroisoquinolinyl. When the 3- to 14-membered heterocyclyl is linked to another group to form the compound of the present invention, the another group may be linked to a carbon atom on the 3- to 14-membered heterocyclyl, or the another group may be linked to a heterocyclic atom on the 3- to 14-membered heterocyclyl ring. For example, when the 3- to 14-membered heterocyclyl is selected from piperazinyl, the another group may be linked to a nitrogen atom on the piperazinyl. Alternatively, when the 3- to 14-membered heterocyclyl is selected from piperidinyl, the another group may be linked to a nitrogen atom on the piperidinyl ring and a carbon atom on its para-position.

The term "spiro ring" refers to a ring system in which two rings share one ring-forming atom.

The term "fused ring" refers to a ring system in which two rings share two ring-forming atoms.

The term "bridged ring" refers to a ring system in which two rings share three or more ring-forming atoms.

The term "halogen" indicates fluorine, chlorine, bromine, and iodine.

"Halogenated" refers to substitution with one or more halogens.

The wavy line (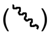) intersecting a chemical bond indicates a position of a group linked to another atom in a molecular structure.

A person skilled in the art may understand that the compound represented by formula I may exist in the form of various pharmaceutically acceptable salts. If these compounds have basic sites, the compounds can form acid addition

salts. If these compounds have acidic sites, the compounds can form base addition salts. If these compounds have both acidic sites (for example, carboxyl) and basic sites (for example, amino), the compounds can further form inner salts.

The compound of the present invention may exist in the form of a solvate (for example, a hydrate). The compound of the present invention comprises a polar solvent as a structural element of the lattice of the compound, for example, especially water, methanol, or ethanol. The amount of the polar solvent, especially water, may be in a stoichiometric or non-stoichiometric ratio.

The compound of the present invention may be chiral depending on its molecular structure, and thus may have various enantiomeric forms. In this case, these compounds may exist in racemic forms or in optically active forms. The compound of the present invention covers isomers whose chiral carbon is in the R or S configuration, mixtures thereof, and racemates. The compound of the present invention or an intermediate thereof may be separated by a chemical or physical method known to a person skilled in the art to form an enantiomeric compound, or may be used in this form for synthesis. In the case of racemic amines, a diastereoisomer is obtained from a mixture through reaction with an optically active resolving agent. Examples of suitable resolving agents are optically active acids, such as tartaric acids in the R and S forms, diacetyltartaric acid, dibenzoyltartaric acid, mandelic acid, malic acid, lactic acid, suitable N-protected amino acids (such as N-benzoylproline or N-benzenesulfonylproline), or various optically active camphorsulfonic acids. Enantioresolution by chromatography may also be carried out advantageously with the help of an optically active resolving agent (such as dinitrobenzoylphenylglycine, cellulose triacetate, another carbohydrate derivative, or a chirally derivatized methacrylate polymer that is immobilized on silica gel). A suitable eluent for this purpose is a water-containing or alcohol-containing solvent mixture, for example, hexane/isopropanol/acetonitrile.

A corresponding stable isomer may be separated by a known method such as extraction, filtration, or column chromatography.

The term "patient" refers to any animal comprising mammals, preferably mice, rats, other rodents, rabbits, dogs, cats, pigs, cows, sheep, horses, or primates, most preferably humans.

The term "therapeutically effective amount" refers to an amount of an active compound or drug that is being sought by researchers, veterinarians, physicians, or other clinicians in tissues, systems, animals, individuals, or humans, to elicit a biological or medical response, comprising one or more of the following: (1) disease prevention: for example, the prevention of diseases, disorders, or conditions in individuals who are susceptible to the diseases, disorders, or conditions but who have not experienced or developed pathologies or symptoms of the diseases; (2) disease suppression: for example, the suppression of diseases, disorders, or conditions in individuals who are experiencing or developing pathologies or symptoms of the diseases, disorders, or conditions (that is, the prevention of further progression of the pathologies and/or symptoms); and (3) disease remission: for example, the remission of diseases, disorders, or conditions in individuals who are experiencing or developing pathologies or symptoms of the diseases, disorders, or conditions (that is, the reversal of the pathologies and/or symptoms).

## DETAILED DESCRIPTION

The following further describes in detail the technical solutions of the present invention using specific examples. It should be understood that the following examples are merely for exemplarily illustrating and explaining the present invention, but should not be construed as a limitation on the protection scope of the present invention. Any technique implemented based on the foregoing content of the present invention falls within the protection scope of the present invention.

The structure of the compound of the present invention is determined by nuclear magnetic resonance (NMR) and/or liquid chromatography-mass spectrometry (LC-MS). An NMR chemical shift ($\delta$) is given in parts per million (ppm). Determination by NMR is carried out by using a Bruker AVANCE-400 NMR spectrometer. Solvents for determination are deuterated dimethyl sulfoxide (DMSO-d6), deuterated methanol ($CD_3OD$), and deuterated chloroform ($CDCl_3$). An internal standard is tetramethylsilane (TMS).

Determination by LC-MS is carried out by using an Agilent 1200 Infinity Series mass spectrometer. Determination by HPLC is carried out by using an Agilent 1200DAD high-pressure liquid chromatograph (Sunfire C18 150 $\times$ 4.6 mm chromatographic column) and a Waters 2695-2996 high-pressure liquid chromatograph (Gimini C18 150 $\times$ 4.6 mm chromatographic column).

A silica gel plate for thin-layer chromatography (TLC) is HSGF254 from Huanghai, Yantai and GF254 from Qingdao, with a size for TLC of 0.15 mm to 0.20 mm and a size for separation and purification of products by TLC of 0.4 mm to 0.5 mm. For column chromatography, 200-to 300-mesh silica gel from Huanghai, Yantai is usually used as a carrier.

Unless otherwise indicated, raw materials and reagents used in the following examples are commercially available or may be prepared by known methods. In the absence of special instructions, all reactions in the present invention are carried out with continuous magnetic stirring under dry nitrogen or argon atmosphere, solvents are dried, and reaction temperature is in degrees Celsius.

**Example 1**

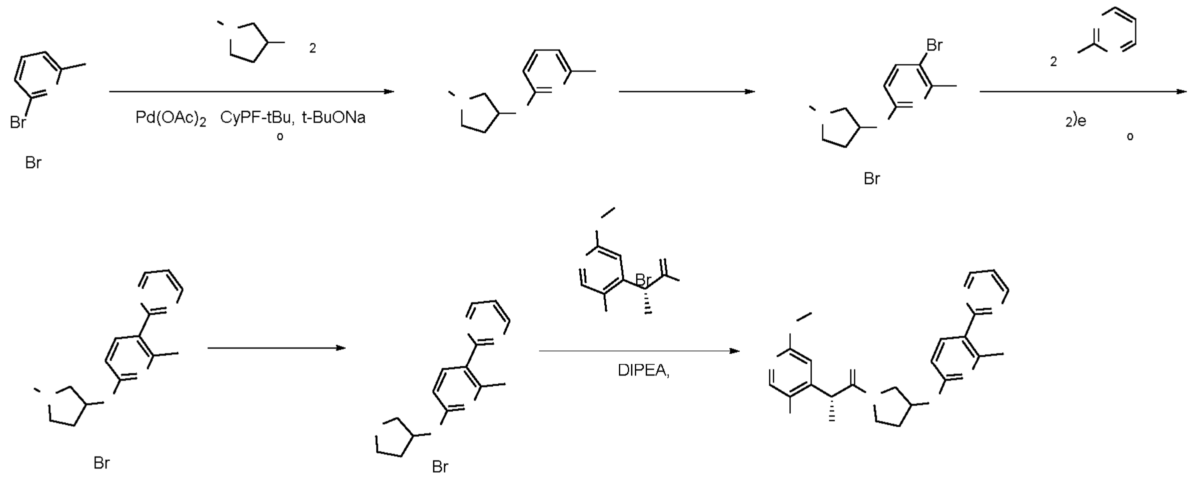


Step 1: 3-((6-methylpyridine-2-yl)amino)pyrrolidine-1-tert-butyl formate 1b

To a 40 mL sample vial were added 2-bromo-6-methylpyridine (1.71 g), 3-aminopyrrolidine-1-tert-butyl formate (2.23 g), palladium acetate (0.2 g), (R)-(-)-1-[(S)-2-(dicyclohexylphosphine)ferrocenyl]ethyl-di-tert-butylphosphine (54.8 mg), and sodium tert-butoxide (1.33 g) at room temperature, and to the above reaction mixture was added ethylene glycol dimethyl ether (15 mL). The mixture was stirred overnight at 90°C under nitrogen atmosphere. The progress of the reaction was monitored by LC-MS. After completion of the reaction, the solvent was removed under reduced pressure, and the resulting residue was purified by silica gel column chromatography with the eluent of ethyl acetate/petroleum ether (0-50%) to give the compound 1b (2.32 g).
LC-MS (ESI): *m/z* = 278.05 $[M+H]^+$

Step 2: 3-((5-bromo-6-methylpyridine-2-yl)amino)pyrrolidine-1-tert-butyl formate 1c

To a solution of compound 1b (1.4 g) in tetrahydrofuran/dichloromethane (1:1, 10 mL) was added dropwise a solution of N-bromosuccinimide (0.90 g) in acetonitrile (5 mL) at 0°C. After the addition, the reaction mixture was transferred to react at room temperature for 2 h. The progress of the reaction was monitored by LC-MS. After completion of the reaction, the solvent was removed under reduced pressure, and the resulting residue was purified by silica gel column chromatography with the eluent of ethyl acetate/petroleum ether (0-50%) to give the compound 1c (1.41 g).
LC-MS (ESI): *m/z* = 355.95 $[M+H]^+$

Step 3: 3-((6-methyl-5-(pyrimidine-2-yl)pyridine-2-yl)amino)pyrrolidine-1-tert-butyl formate 1d

To a 40 mL sample vial were added the compound 1c (700 mg), 2-(tributyltin)pyrimidine (1087.95 mg), tetrakis(triphenylphosphine)palladium (113.53 mg), cuprous iodide (18.71 mg), and anhydrous lithium chloride (83.29 mg) at room temperature. The vial was purged with nitrogen for three times, and then anhydrous toluene (15 mL) was added. The reaction mixture was stirred overnight at 120°C. The progress of the reaction was monitored by LC-MS. After completion of the reaction, the solvent was removed under reduced pressure, and the resulting residue was purified by silica gel column chromatography with the eluent of ethyl acetate/petroleum ether (20%-80%), to give the compound 1d (500 mg).
LC-MS (ESI): *m/z* = 355.90 $[M+H]^+$

Step 4: 6-methyl-5-(pyrimidine-2-yl)-N-(pyrrolidine-3-yl)pyridine-2-amine 1e

To a solution of compound 1d (135 mg) in dichloromethane (1 mL) was added trifluoroacetic acid (0.2 mL) at room temperature, the mixture was stirred at room temperature for 2 h. The progress of the reaction was monitored by LC-MS. After completion of the reaction, the resulting residue was concentrated under reduced pressure, to give a trifluoroacetate salt (97 mg) of the compound 1e.

LC-MS (ESI): $m/z$ = 256.05 $[M+H]^+$

Step 5: (2R)-2-(5-fluoro-2-methoxypyridine-4-yl)-1-(3-((6-methyl-5-(pyrimidine-2-yl)pyridine-2-yl)amino)pyrrolidine-1-yl)propyl-1-one 001

To a solution of the trifluoroacetate salt (97 mg) of compound 1e in N,N-dimethylformamide (1 mL) were added (R)-2-(5-fluoro-2-methoxypyridine-4-yl)propanoic acid (37.61 mg, prepared with reference to "WO2021250541A1"), N,N-diisopropylethylamine (245.51 mg), and 2-(7-azabenzotriazol-1-yl)-N,N,N',N'-tetramethyluronium hexafluorophosphate (216.68 mg) under nitrogen protection at room temperature, the mixture was stirred for 2 h to give a product, the progress of the reaction was monitored by LC-MS.. The crude product was purified by preparative high-performance liquid chromatography (chromatographic column configurations: Kinetex EVO C18 Column, 30 mm × 150 mm, 5 μm; mobile phase A: water (10 mmol/L ammonium bicarbonate), mobile phase B: acetonitrile; flow rate: 60 mL/min; elution gradient: 10% B to 45% B) to give the compound 001.

LC-MS (ESI): $m/z$ = 436.85 $[M+H]^+$
$^1$H NMR (400 MHz, DMSO-$d_6$) δ 8.85-8.80 (m, 2H), 8.14-7.93 (m, 2H), 7.33-7.28 (m, 1H), 7.17-6.92 (m, 1H), 6.74-6.61 (m, 1H), 6.48-6.32 (m, 1H), 4.49-4.31 (m, 1H), 4.20-4.02 (m, 1H), 3.91-3.73 (m, 4H), 3.70-3.53 (m, 1H), 3.45-3.36 (m, 1H), 3.28-3.14 (m, 1H), 2.65-2.58 (m, 3H), 2.28-2.04 (m, 1H), 1.99-1.73 (m, 1H), 1.37-1.28 (m, 3H).

**Example 2:** (R)-2-(5-fluoro-2-methoxypyridine-4-yl)-1-((3 S,4S)-3-methyl-4-(6-methyl-5-(pyrimidine-2-yl)pyridine-2-yl-amino)pyrrolidine-1-yl)propane-1-one (051-1)

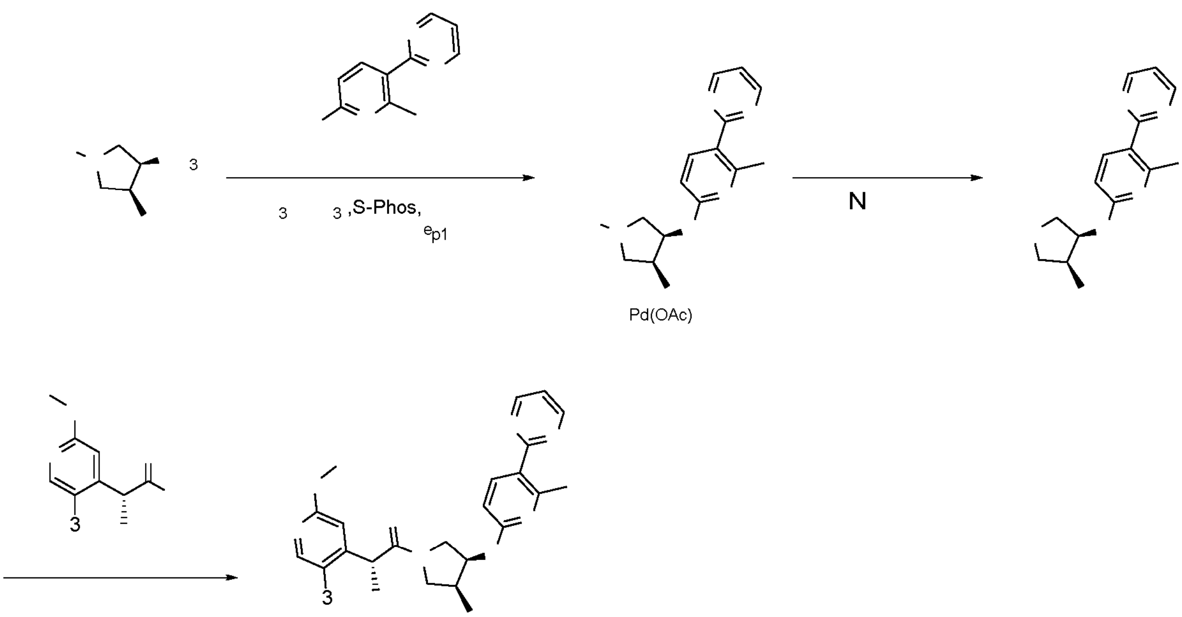


Step 1: (3S,4S)-3-methyl-4-(6-methyl-5-(pyrimidine-2-yl)pyridine-2-yl-amino)pyrrolidine-1-tert-butyl formate (051-1b)

To a solution of 051-1a (549 mg) and 083-1c (470 mg) in toluene (5 mL) were added tris(dibenzylideneacetone) dipalladium (209 mg), sodium tert-butoxide (439 mg), and 2-dicyclohexylphosphino-2',6'-dimethoxybiphenyl (187 mg) under nitrogen protection at room temperature. The resulting mixture was stirred at 80°C for 2 h. After the mixture was cooled down to room temperature and diluted with water (50 mL), the reaction mixture was subjected to extraction with ethyl acetate (3 × 50 mL). Then the organic phases were combined, backwashed with saturated saline (1 × 20 mL), and dried over anhydrous sodium sulfate. The resulting mixture was filtered, and then the filtrate was concentrated under reduced pressure. The resulting residue was purified by silica gel column chromatography (petroleum ether/ethyl acetate = 1:1) to give the compound 051-1b (500 mg).
LC-MS: (ESI, m/z) = 369.95 $[M+H]^+$

Step 2: 6-methyl-N-((3S,4S)-4-methylpyrrolidine-3-yl)-5-(pyrimidine-2-yl)pyridine-2-amine (051-1c)

051-1b (120 mg) was dissolved in a solution of hydrogen chloride in 1,4-dioxane (1 mL) and stirred for 30 min at room temperature. The reaction mixture was concentrated directly to give a hydrochloride salt (100 mg, crude product) of the compound 051-1c. The crude product was not further purified and was directly subjected to next step.
LC-MS: (ESI, m/z) = 270.10 $[M+H]^+$

Step 3: (R)-2-(5-fluoro-2-methoxypyridine-4-yl)-1-((3 S,4S)-3-methyl-4-(6-methyl-5-(pyrimidine-2-yl)pyridine-2-yl-amino)pyrrolidine-1-yl)propane-1-one (051-1)

To a solution of hydrochloride salt (35 mg) of 051-1c and 1f (26 mg) in N,N-dimethylacetamide (1 mL) was added N,N-diisopropylethylamine (336 mg) under nitrogen protection at room temperature. After the mixture was stirred for 5 min, 2-(7-azabenzotriazol)-N,N,N',N'-tetramethyluronium hexafluorophosphate (74.11 mg) was added in batches to the mixture at room temperature. The resulting mixture was stirred at room temperature for 30 min. The crude product was purified by high-performance liquid chromatography (chromatographic column configurations: YMC Triart C18 ExRs, 30 mm × 150 mm, 5 μm; mobile phase A: water (10 mmol/L aqueous ammonium bicarbonate solution), mobile phase B: acetonitrile; flow rate: 60 mL/min; gradient: 25% B to 60% B) to give the compound 051-1 (9.17 mg).

LC-MS: (ESI, m/z) = 450.95 $[M+H]^+$
$^1$H NMR (400 MHz, DMSO-d6) δ 8.84-8.80 (m, 2H), 8.12-8.09 (m, 1H), 8.05-8.00 (m, 1H), 7.32-7.27 (m, 1H), 7.02-6.89 (m, 1H), 6.75-6.69 (m, 1H), 6.55-6.46 (m, 1H), 4.63-4.47 (m, 1H), 4.19-4.03 (m, 1H), 3.82 (s, 3H), 3.69-3.47 (m, 2H), 3.32-3.09 (m, 2H), 2.61 (d, 3H), 2.59-2.38 (m, 1H), 1.38-1.28 (m, 3H), 0.97-0.79 (m, 3H).

**Example** 3: (R)-2-(5-chloro-2-methoxypyridine-4-yl)-1-((S)-3-((6-methyl-5-(pyrimidine-2-yl)pyridine-2-yl)amino)pyrrolidine-1-yl)propane-1-one (083-1)

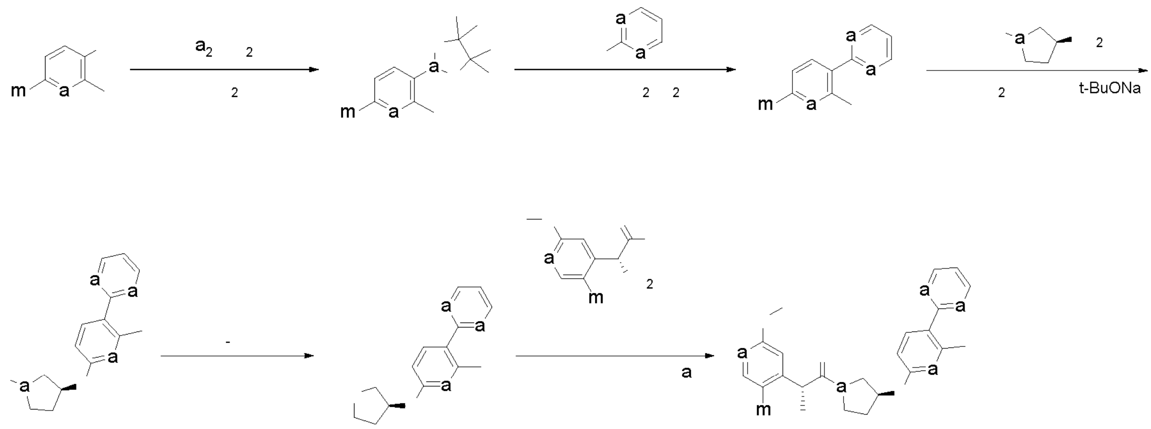


Step 1: 6-chloro-2-methyl-3-(4,4,5,5-tetramethyl-1,3,2-dioxaborolan-2-yl)pyridine (083-1b)

To a solution of 083-1a (50 g) and bis(pinacolato)diboron (122.99 g) in 1,4-dioxane were added [1,1-bis(diphenylphosphino)ferrocene]dichloropalladium(II) (3.54 g) and potassium acetate (52.29 g) at room temperature. The mixture was purged with nitrogen and stirred at 100°C for 2 h. The reaction solution was concentrated under reduced pressure, and then dissolved in N, N-dimethylformamide (30 mL). The resulting residue was purified by reversed-phase column chromatography under the following conditions: chromatographic column configurations (C18, 330 g), mobile phases: water and acetonitrile, 0% to 100%, and a UV254 nano-detector. The resulting residue was concentrated under reduced pressure to give the compound 083-1b (28 g).
LC-MS: (ESI, m/z) = 254.25 $[M+H]^+$

Step 2: 2-(6-chloro-2-methylpyridine-3-yl)pyrimidine (083-1c)

To a solution of compound 083-1b (23 g) and 2-bromopyrimidine (17.31 g) in 1,4-dioxane (400 mL) were added 1,1'-bis(diphenylphosphino)ferrocene-dichloropalladium(II) (6.64 g) and an aqueous sodium carbonate solution (68 mL, 4

M). The mixture was purged with nitrogen, and then reacted at 100°C for 2 h. After cooling down to room temperature, the mixture was diluted with water (200 mL) and subjected to extraction with ethyl acetate (300 mL × 3). The resulting organic phase was washed once with saturated saline, dried over anhydrous sodium sulfate, filtered, and concentrated under reduced pressure. The resulting residue was purified by silica gel column chromatography with petroleum ether/ethyl acetate (4:1) to give the compound 083-1c (18 g).
LC-MS: (ESI, m/z) = 206.05 $[M+H]^+$

Step 3: (S)-3-((6-methyl-5-(pyrimidine-2-yl)pyridine-2-yl)amino)pyrrolidine-1-tert-butyl formate (083-1d)

To a solution of 083-1c (10 g) and (S)-3-aminopyrrolidine-1-tert-butyl formate (13.59 g) in toluene (150 mL) were added palladium acetate (1.09 g), 2-dicyclohexylphosphino-2',6'-diisopropoxybiphenyl (2.27 g), and sodium tert-butoxide (9.35 g) at room temperature under nitrogen protection. The mixture was purged with nitrogen, and then reacted at 90°C for 3 h to give the desired product, which was found in the liquid phase. The resulting residue was concentrated under reduced pressure. The resulting residue was purified by silica gel column chromatography with petroleum ether/ethyl acetate (2:1) to give the compound 083-1d (12 g).
LC-MS: (ESI, m/z) = 356.15 $[M+H]^+$

Step 4: (S)-6-methyl-5-(pyrimidine-2-yl)-N-(pyrrolidine-3-yl)pyridine-2-amine (083-1e)

To 083-1d (1.0 g) was added a solution of hydrogen chloride in 1,4-dioxane (4 M, 10 mL) at room temperature. After the addition, the mixture was stirred at room temperature for 30 min to give the desired product, which was found in the liquid phase. The reaction mixture was directly concentrated under reduced pressure to give the hydrochloride salt (850 mg) of the compound 083-1e. The crude product was not further purified and was directly subjected to next step.
LC-MS: (ESI, m/z): 255.95 $[M+H]^+$

Step 5: (R)-2-(5-chloro-2-methoxypyridine-4-yl)-1-((S)-3-((6-methyl-5-(pyrimidine-2-yl)pyridine-2-yl)amino)pyrrolidine-1-yl)propane-1-one (083-1)

To a solution of the hydrochloride salt (850 mg) of the compound 083-1e and 083-1f (700 mg, synthesized with reference to the method in the patent WO2021250541A1) in N,N-dimethylacetamide (10 mL) was added N,N-diisopropylethylamine (2.10 g) at room temperature, the mixture was stirred at room temperature for 5 min. Then, 2-(7-aza-1H-benzotriazole-1-yl)-1,1,3,3-tetramethyluronium hexafluorophosphate (1.85 g) was added in batches to the mixture at room temperature. After the addition, the mixture was stirred at room temperature for 2 h to give the desired product, which was found in the liquid phase. The crude product was purified by high-performance liquid chromatography (chromatographic column: Ultimate @ XB-C18; mobile phase A: water (10 mmol/L ammonium bicarbonate), mobile phase B: acetonitrile; gradient: 25% B to 55% B) to give the compound 083-1.

LC-MS (ESI): m/z = 452.95 $[M+H]^+$
$^1$H NMR (400 MHz, DMSO-d6) δ 8.84-8.81 (m, 2H), 8.23 (d, 1H), 8.05-8.00 (m, 1H), 7.33-7.27 (m, 1H), 7.18-7.00 (m, 1H), 6.72 (d, 1H), 6.46-6.38 (m, 1H), 4.41-4.28 (m, 1H), 4.20-4.05 (m, 1H), 3.84 (d, 3H), 3.74-3.50 (m, 2H), 3.43-3.24 (m, 2H), 2.61 (d, 3H), 2.28-2.04 (m, 1H), 1.95-1.81 (m, 1H), 1.34-1.27 (m, 3H).

Example 4: 2-(6-(((S)-1-((R)-2-(5-fluoro-2-methoxypyridine-4-yl)propionyl)pyrrolidine-3-yl)amino)-2-methylpyridine-3-yl)pyrimidine-5-nitrile (110)

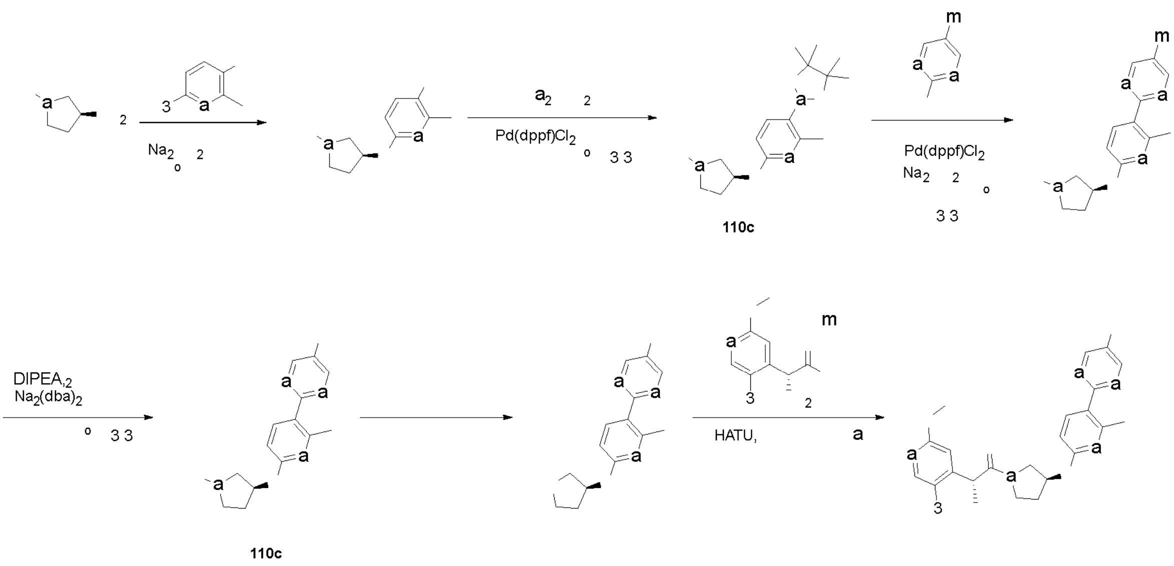

110c

110c

Step 1:(S)-3-((5-bromo-6-methylpyridine-2-yl)amino)pyrrolidine-1-tert-butyl formate (110b)

To a solution of 110a (10 g) and cesium carbonate (34.99 g) in N,N-dimethylacetamide (54 mL) was added 3-bromo-6-fluoro-2-methylpyridine (9.18 g) at room temperature. After the addition, the mixture was purged with nitrogen, and then stirred overnight at 120°C to give a desired product, which was found in the liquid phase, then the reaction mixture was diluted with water (300 mL). After the reaction mixture was subjected to extraction with ethyl acetate (3 × 200 mL), the organic phases were combined, backwashed with saturated saline (2 × 100 mL), and dried over sodium sulfate. The resulting mixture was filtered, and then the filtrate was concentrated under reduced pressure. The resulting residue was purified by silica gel column chromatography with petroleum ether/ethyl acetate (0%-100%), to give the compound 110b (4.7 g).
LC-MS (ESI): m/z = 355.70 $[M+H]^+$

Step 2: (S)-3-((6-methyl-5-(4,4,5,5-tetramethyl-1,3,2-dioxaborolan-2-yl)pyridine-2-yl)amino)pyrrolidine-1-tert-butyl formate (110c)

To a solution of 110b (970 mg) and bis(pinacolato)diboron (1.38 g) in 1,4-dioxane (10 mL) were added [1,1'-bis(diphenylphosphino)ferrocene]dichloropalladium(II) (39.84 mg) and potassium acetate (587.86 mg) at room temperature. After the addition, the mixture was purged with nitrogen, then heated to 100°C and stirred for 2 h, to give the desired product, which was found in the liquid phase. The reaction mixture was cooled down to room temperature. The resulting residue was purified by reversed-phase column chromatography (C18 chromatographic column configurations: 330 g; mobile phases A: water (10 mmol/L ammonium bicarbonate) and B: acetonitrile; gradient: B: 0% to 100%) to give the compound 110c (850 mg).
LC-MS (ESI): m/z = 403.75 $[M+H]^+$

Step 3: (S)-3-((5-(5-chloropyrimidine-2-yl)-6-methylpyridine-2-yl)amino)pyrrolidine-1-tert-butyl formate (110d)

To a solution of 110c (200 mg) and 2-bromo-5-chloropyrimidine (287.75 mg) in 1,4-dioxane (4 mL) were added [1,1'-bis(diphenylphosphino)ferrocene]dichloropalladium(II) (72.57 mg) and an aqueous sodium carbonate solution (0.38 mL, 4 M) at room temperature. After the addition, the mixture was purged with nitrogen, and stirred at 100°C for 2 h to give the desired product, which was found in the liquid phase. The reaction mixture was diluted with water (10 mL), and then subjected to extraction with ethyl acetate (3 × 20 mL). The organic phases were combined, backwashed with saturated saline (2 × 10 mL), and dried over sodium sulfate. The resulting mixture was filtered, and then the filtrate was concentrated under reduced pressure. The resulting residue was purified by silica gel column chromatography with petroleum ether/ethyl acetate = 7:1 to give the compound 110d (150 mg).
LC-MS (ESI): m/z = 390.05 $[M+H]^+$

Step 4: (S)-3-((5-(5-cyanopyrimidine-2-yl)-6-methylpyridine-2-yl)amino)pyrrolidine-1-tert-butyl formate (110e)

To a solution of 110d (90 mg) and zinc cyanide (54.21 mg) in N,N-dimethylacetamide (2 mL) were added

tris(dibenzylideneacetone)dipalladium (21.14 mg) and 2-(dicyclohexylphosphino)-2',4',6'-triisopropyl-1,1'-biphenyl (22.01 mg) at room temperature. After the addition, the mixture was purged with nitrogen, and stirred at 120°C for 2 h to give the desired product, which was found in the liquid phase. The reaction mixture was diluted with water (10 mL), and then subjected to extraction with ethyl acetate (3 × 20 mL). The organic phases were combined, backwashed with saturated saline (2 × 10 mL), and dried over sodium sulfate. The resulting mixture was filtered, and then the filtrate was concentrated under reduced pressure. The resulting residue was purified by silica gel column chromatography with petroleum ether:ethyl acetate = 10:1 to give the compound 110e (80 mg).
LC-MS (ESI): m/z = 381.05 $[M+H]^+$

Step 5: (6-(((S)-1-((R)-2-(5-fluoro-2-methoxypyridine-4-yl)propionyl)pyrrolidine-3-yl)amino)-2-methylpyridine-3-yl)pyrimidine-5-nitrile (110f)

To a solution of 110e (50 mg) in dichloromethane (3 mL) was added dropwise trifluoroacetic acid (1 mL) at room temperature. After the addition, the mixture was stirred at room temperature for 30 min to give the desired product, which was found in the liquid phase. The reaction solution was directly concentrated under reduced pressure to give the trifluoroacetate salt (35 mg, crude product) of compound 110f. The resulting mixture was not further purified and was directly subjected to next step.
LC-MS (ESI): m/z = 281.05 $[M+H]^+$

Step 6: 2-(6-(((S)-1-((R)-2-(5-fluoro-2-methoxypyridine-4-yl)propionyl)pyrrolidine-3-yl)amino)-2-methylpyridine-3-yl) pyrimidine-5-nitrile (110)

To a solution of the trifluoroacetate salt (35 mg) of 110f and 1f (25 mg) in N,N-dimethylacetamide (2 mL) was added N,N-diisopropylethylamine (165 mg) at room temperature, after the mixture was stirred at room temperature for 5 min, 2-(7-aza-1H-benzotriazole-1-yl)-1,1,3,3-tetramethyluronium hexafluorophosphate (71.59 mg) was added. After the addition, the mixture was stirred at room temperature for 2 h, to give a desired product, which was found in the liquid phase. The reaction solution was directly purified by high-performance liquid chromatography (chromatographic column: Kinetex EVO C18, 30 mm × 150 mm, 5 μm; mobile phase A: water (10 mmol/L ammonium bicarbonate), mobile phase B: acetonitrile; flow rate: 60 mL/min; gradient: 25% B to 55% B) to give the compound 110 (24.24 mg).

LC-MS (ESI): m/z = 461.95 $[M+H]^+$
$^1$H NMR (400 MHz, DMSO-d6) δ 9.24 (d, 2H), 8.23-8.17 (m, 1H), 8.13-8.09 (m, 1H), 7.52-7.35 (m, 1H), 6.73-6.67 (m, 1H), 6.51-6.43 (m, 1H), 4.47-4.36 (m, 1H), 4.19-4.02 (m, 1H), 3.82 (d, 3H), 3.77-3.68 (m, 1H), 3.65-3.49 (m, 1H), 3.46-3.21 (m, 2H), 2.67 (d, 3H), 2.30-2.04 (m, 1H), 1.96-1.81 (m, 1H), 1.37-1.28 (m, 3H).

**Example 5:** 2-(6-(((S)-1-((R)-2-(5-fluoro-2-methoxypyridine-4-yl)propionyl)pyrrolidine-3-yl)amino)-2-methylpyridine-3-yl)pyrimidine-4-nitrile (111)

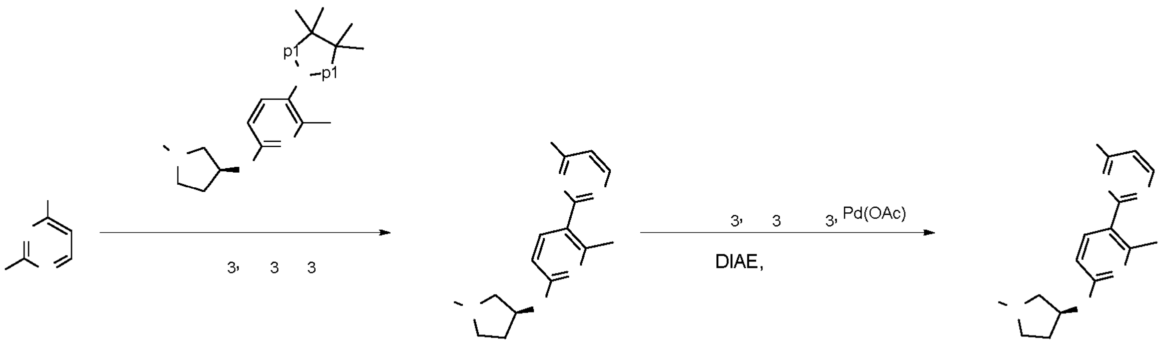


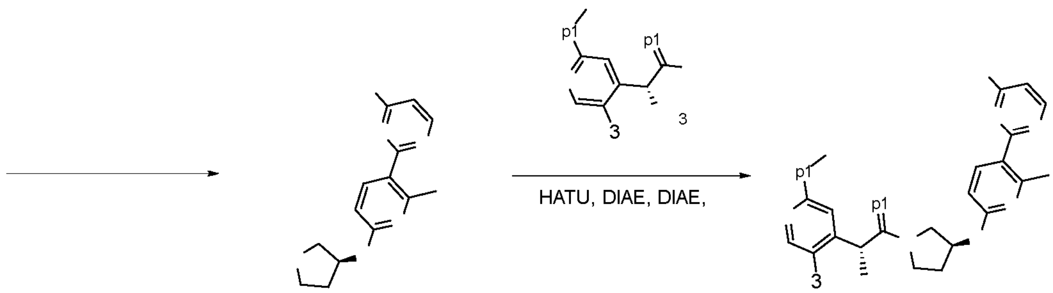


Step 1: (3S)-3-((5-(4-chloropyrimidine-2-yl)-6-methylpyridine-2-yl)amino)pyrrolidine-1-tert-butyl formate (111b)

To a solution of 111a (220 mg) and 110c (366.99 mg) in 1,4-dioxane (5 mL) were added an aqueous sodium carbonate solution (1.7 mL, 2 mol/L) and [1,1'-bis(diphenylphosphino)ferrocene]dichloropalladium(II) (83 mg) at room temperature. The mixture was purged with nitrogen for three times, and stirred at 100°C for 2 h, to give a product, the progress of the reaction was monitored by LC-MS. After the mixture was cooled down to room temperature and diluted with water (20 mL), the reaction mixture was subjected to extraction with ethyl acetate (3 × 20 mL). Then the organic phases were combined, backwashed with saturated saline (1 × 20 mL), and dried over anhydrous sodium sulfate. The resulting mixture was filtered, and then the filtrate was concentrated under reduced pressure. The resulting residue was purified by silica gel column chromatography (petroleum ether/ethyl acetate = 1:1) to give the compound 111b (160 mg).
LC-MS: (ESI, m/z) = 390.10 $[M+H]^+$

Step 2: (3S)-3-((5-(4-cyanopyrimidine-2-yl)-6-methylpyridine-2-yl)amino)pyrrolidine-1-tert-butyl formate (111c)

111b (160 mg) and zinc cyanide (96.37 mg) were dissolved in N,N-dimethylacetamide (2 mL) at room temperature, and tris(dibenzylideneacetone)dipalladium (37.58 mg) and 2-dicyclohexylphosphino-2,4,6-triisopropylbiphenyl (39.13 mg) were added. The mixture was purged with nitrogen for three times, stirred at 120°C for 2 h, cooled down to room temperature, and diluted with water (20 mL). The reaction mixture was subjected to extraction with ethyl acetate (3 × 20 mL). The organic phases were combined, backwashed with saturated saline (1 × 10 mL), and dried over anhydrous sodium sulfate. The resulting mixture was filtered, and then the filtrate was concentrated under reduced pressure. The resulting residue was purified by silica gel column chromatography (petroleum ether/ethyl acetate = 1:1) to give the compound 111c (80 mg).
LC-MS: (ESI, m/z) = 381.20 $[M+H]^+$

Step 3: (S)-2-(2-methyl-6-(pyrrolidine-3-yl-amino)pyridine-3-yl)pyrimidine-4-nitrile (111d)

To a reaction flask were added 111c (55 mg), dichloromethane (1 mL), and trifluoroacetic acid (0.5 mL) at room temperature, the mixture was stirred for 30 min to give a product. The progress of the reaction was monitored by LC-MS. Then the product was concentrated directly to give a trifluoroacetate salt (50 mg, crude product) of the compound 111d. The crude product was not further purified and was directly subjected to next step.
LC-MS: (ESI, m/z) = 281.05 $[M+H]^+$

Step 4: 2-(6-(((S)-1-((R)-2-(5-fluoro-2-methoxypyridine-4-yl)propionyl)pyrrolidine-3-yl)amino)-2-methylpyridine-3-yl) pyrimidine-4-nitrile (111)

The trifluoroacetate salt (50 mg, crude product) of 111d and N,N-dimethylacetamide (2 mL) were dissolved in N,N-diisopropylethylamine (454 mg) at room temperature, 1f (35 mg) was added, and stirred for 5 min. Then, 2-(7-azabenzotriazol)-N,N,N',N'-tetramethyluronium hexafluorophosphate (100 mg) was added at room temperature. The resulting residue was stirred for 30 min to give a product, which was found in the liquid phase. The crude product was purified by high-performance liquid chromatography (chromatographic column configurations: Kinetex 5 μm EVO C18, 30 mm × 150 mm, 5 μm; mobile phase A: water (10 mmol/L ammonium bicarbonate), mobile phase B: acetonitrile; flow rate: 60 mL/min; gradient: 25% B to 58% B) to give the compound 111 (9.17 mg).

LC-MS: (ESI, m/z) = 462.00 $[M+H]^+$

$^1$H NMR (400 MHz, DMSO-d6) δ 9.14-9.10 (m, 1H), 8.15-8.06 (m, 2H), 7.90-7.86 (m, 1H), 7.40-7.24 (m, 1H), 6.73-6.68 (m, 1H), 6.50-6.43 (m, 1H), 4.44-4.34 (m, 1H), 4.19-4.03 (m, 1H), 3.82 (d, 3H), 3.76-3.68 (m, 1H), 3.65-3.50 (m, 1H), 3.45-3.20 (m, 2H), 2.63 (d, 3H), 2.29-2.03 (m, 1H), 1.95-1.80 (m, 1H), 1.37-1.27 (3H).

**Example 6:** (R)-2-(5-chloro-2-methoxypyridine-4-yl)-1-((3 S,4S)-3-methyl-4-(6-methyl-5-(pyrimidine-2-yl)pyridine-2-yl-amino)pyrrolidine-1-yl)propane-1-one (112)

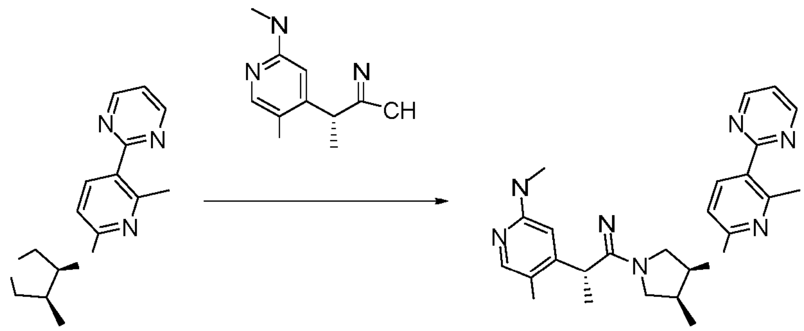

S-Phos,

To a solution of the hydrochloride salt (35 mg) of 051-1c and 083-1f (28 mg) in N,N-dimethylacetamide (1 mL) was added N,N-diisopropylethylamine (335 mg) under nitrogen protection at room temperature. After the mixture was stirred for 5 min, 2-(7-azabenzotriazol)-N,N,N',N'-tetramethyluronium hexafluorophosphate (74.11 mg) was added in batches to the mixture at room temperature. The resulting mixture was stirred at room temperature for 30 min. The crude product was purified by high-performance liquid chromatography (chromatographic column configurations: Kinetex EVO C18 Column, 30 mm × 150 mm, 5 μm; mobile phase A: water (10 mmol/L ammonium bicarbonate), mobile phase B: acetonitrile; flow rate: 60 mL/min; gradient: 30% B to 55% B) to give the compound 112 (16.19 mg).

LC-MS: (ESI, m/z) = 466.95 $[M+H]^+$

$^1$H NMR (400 MHz, DMSO-d6) δ 8.84-8.80 (m, 2H), 8.25-8.20 (m, 1H), 8.02 (d, 1H), 7.32-7.27 (m, 1H), 7.03-6.86 (m, 1H), 6.74 (d, 1H), 6.56-6.47 (m, 1H), 4.62-4.47 (m, 1H), 4.19-4.04 (m, 1H), 3.84 (s, 3H), 3.68-3.47 (m, 2H), 3.26-3.11 (m, 2H), 2.61 (d, 3H), 2.58-2.37 (m, 1H), 1.36-1.27 (m, 3H), 0.97-0.80 (m, 3H).

**Example 7:** 2-(6-(((S)-1-((R)-2-(5-chloro-2-methoxypyridine-4-yl)propionyl)pyrrolidine-3-yl)amino)-2-methylpyridine-3-yl)pyrimidine-4-nitrile (113)

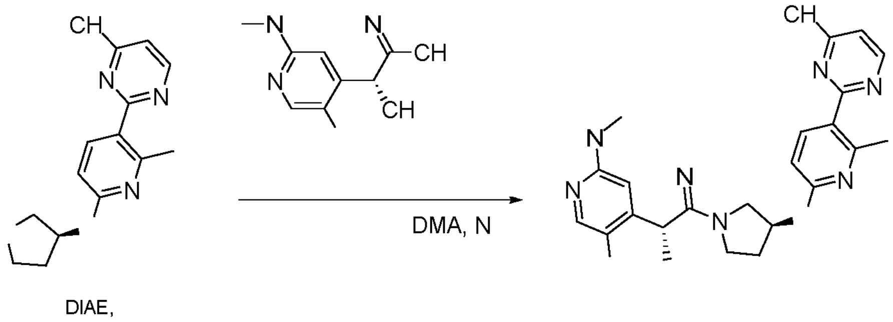


To a solution of the trifluoroacetate salt (35 mg) of 111d and 083-1f (27 mg) in N,N-dimethylacetamide (1 mL) was added N,N-diisopropylethylamine (322 mg) at room temperature. After the mixture was stirred for 5 min, 2-(7-azabenzotriazol)-N,N,N',N'-tetramethyluronium hexafluorophosphate (71 mg) was added to the mixture at room temperature. After 2 hours of reaction at room temperature, the completion of the reaction was detected in the liquid phase. The crude product was purified by high-performance liquid chromatography (chromatographic column configurations: Kinetex 5 μm EVO C18, 30 mm × 150 mm, 5 μm; mobile phase A: water (10 mmol/L aqueous ammonium bicarbonate solution), mobile phase B: acetonitrile; flow rate: 60 mL/min; gradient: 25% B to 58% B) to give the compound 113 (5 mg).

LC-MS: (ESI, m/z) = 477.95 $[M+H]^+$
$^1H$ NMR (400 MHz, DMSO-d6) δ 9.15-9.10 (m, 1H), 8.23 (d, 1H), 8.11-8.05 (m, 1H), 7.90-7.86 (m, 1H), 7.41-7.21 (m, 1H), 6.72 (d, 1H), 6.50-6.42 (m, 1H), 4.43-4.29 (m, 1H), 4.21-4.05 (m, 1H), 3.84 (d, 3H), 3.74-3.65 (m, 1H), 3.63-3.49 (m, 1H), 3.46-3.21 (m, 2H), 2.63 (d, 3H), 2.30-2.03 (m, 1H), 1.96-1.81 (m, 1H), 1.36-1.25 (m, 3H).

**Example 8:** (R)-2-(5-chloro-2-methoxypyridine-4-yl)-1-((S)-7-((6-methyl-5-(pyrimidine-2-yl)pyridine-2-yl)amino)-5-azaspiro[2.4]heptane-5-yl)propan-1-one (114)

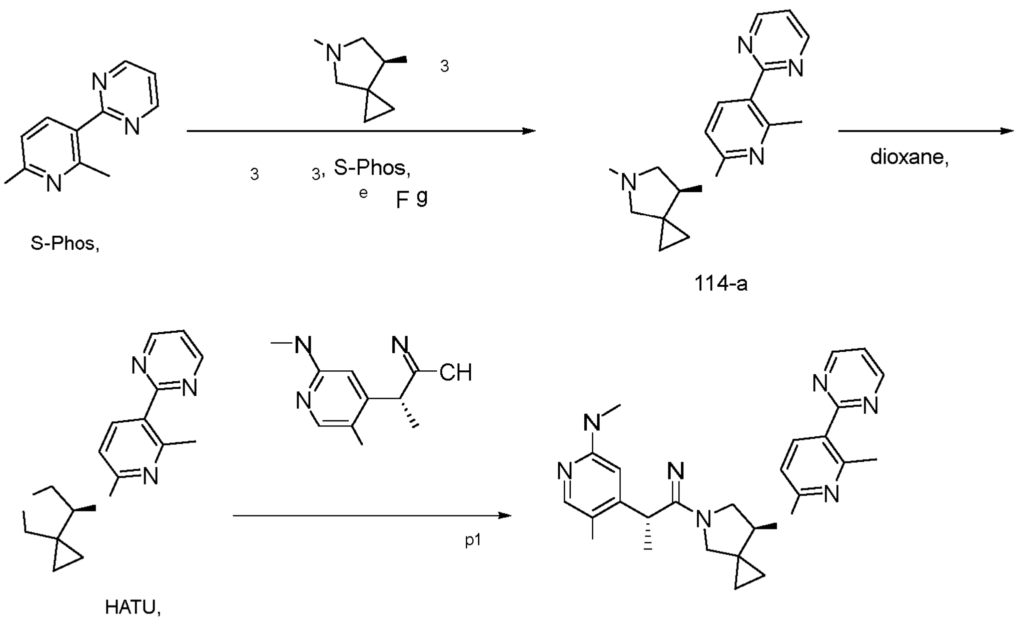

114-a

Step 1: (S)-7-((6-methyl-5-(pyrimidine-2-yl)pyridine-2-yl)amino)-5-azaspiro[2.4]heptane-5-tert-butyl formate (114- a)

To a 40 mL sample vial were added the compound 083-1c (500 mg), (S)-7-amino-5-azaspiro[2.4]heptane-5-tert-butyl formate (567.77 mg), tris(dibenzylideneacetone)dipalladium (222.64 mg), 2-dicyclohexylphosphino-2',6'-dimethoxy-1,1'-biphenyl (199.63 mg), and sodium tert-butoxide (467.32 mg). The vial was purged with nitrogen for three times, then toluene (5 mL) was added, and stirred at 100°C for 2 h, to give a desired product, which was found in the liquid phase. The mixture was cooled down to room temperature, diluted with water (20 mL), and subjected to extraction with ethyl acetate (3 × 20 mL). The organic phases were combined, and dried over sodium sulfate. The resulting mixture was

filtered, and then the filtrate was concentrated under reduced pressure. The resulting residue was purified by silica gel column chromatography with ethyl acetate/petroleum ether (0-50%) to give the compound 114-a (850 mg).
LC-MS: (ES, *m/z*) = 382.05 $[M+H]^+$

Step 2: (S)-N-(6-methyl-5-(pyrimidine-2-yl)pyridine-2-yl)-5-azaspiro[2.4]heptane-7-amine (114- b)

To a solution (4 M, 5 mL) of hydrogen chloride in 1,4-dioxane was added the compound 114-a (500 mg). The mixture was stirred at room temperature for 30 min, to give a product. The progress of the reaction was monitored by LC-MS. The reaction solution was concentrated under reduced pressure to give a hydrochloride salt (420 mg, crude product) of the compound 114-b. The crude product was not purified and was directly subjected to next step.
LC-MS: (ES, *m/z*) = 282.40 $[M+H]^+$

Step 3: (R)-2-(5-chloro-2-methoxypyridine-4-yl)-1-((S)-7-((6-methyl-5-(pyrimidine-2-yl)pyridine-2-yl)amino)-5-azaspiro[2.4]heptane-5-yl)propan-1-one (114)

The hydrochloride salt (130 mg) of the compound 114-b was dissolved in an N,N-dimethylacetamide (4 mL) solution, and the compound 083-1f (153.28 mg), N,N-diisopropylethylamine (275.62 mg), and 2-(7-azabenzotriazol-1-yl)-N,N,N',N'-tetramethyluronium hexafluorophosphate (405.42 mg) were added sequentially. The reaction solution was stirred at room temperature for 2 h. The progress of the reaction was monitored by LC-MS. After the completion of the reaction, the reaction solution was directly purified by high-performance liquid chromatography (chromatographic column: Kinetex 5 μm EVO C18, 30 mm × 150 mm; mobile phase A: water (10 mmol/L $NH_4HCO_3$), mobile phase B: acetonitrile; flow rate: 60 mL/min; gradient: 22% B to 57% B within 8 min) to give the compound 114 (195.78 mg).

LC-MS: (ES, *m/z*) = 478.95 $[M+H]^+$
$^1$H NMR (400 MHz, DMSO-$d_6$) δ 8.84-8.81 (m, 2H), 8.22 (d, 1H), 8.03-7.98 (m, 1H), 7.33-7.28 (m, 1H), 7.12-7.00 (m, 1H), 6.73 (d, 1H), 6.53-6.42 (m, 1H), 4.20-4.05 (m, 2H), 3.84 (d, 3H), 3.74-3.53 (m, 3H), 3.53-3.48 (m, 0.5H), 3.25 (d, 0.5H), 2.60 (d, 3H), 1.37-1.25 (m, 3H), 0.90-0.48 (m, 4H).

**Example 9:** (2R)-2-(5-fluoro-2-methoxypyridine-4-yl)-1-[(7S)-7-{[6-methyl-5-(pyrimidine-2-yl)pyridine-2-yl]amino}-5-azaspiro[2.4]heptane-5-yl]propan-1-one (056-1)

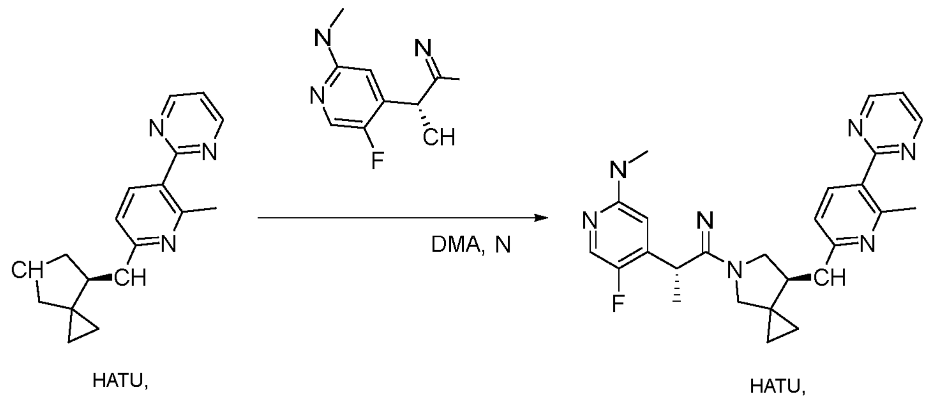


The hydrochloride salt (35 mg) of the compound 114-b and the compound 1f (25 mg) were dissolved in N,N-dimethylacetamide (3 mL) at room temperature, N,N-diisopropylethylamine (243.23 mg) was added, and then 2-(7-azabenzotriazol)-N,N,N',N'-tetramethyluronium hexafluorophosphate (71 mg) was added. After the addition, the mixture was stirred at room temperature for 2 h. The reaction solution was filtered using a filter head. The filter head was washed with N,N-dimethylacetamide (0.5 mL × 2). The filtrates were combined. The crude product was purified by high-performance liquid chromatography (chromatographic column configurations: Kinetex EVO C18 column, 30 mm × 150 mm, 5 μm; mobile phase A: water (10 mmol/L ammonium bicarbonate), mobile phase B: acetonitrile; flow rate: 60 mL/min; gradient: 20% B to 55% B within 8 min) to give the compound 056-1 (27.7 mg).

LC-MS (ESI, *m/z*): = 463.00 $[M+H]^+$
$^1$H NMR (400 MHz, DMSO-$d_6$) δ 8.84-8.80 (m, 2H), 8.13-8.09 (m, 1H), 8.03-7.98 (m, 1H), 7.32-7.27 (m, 1H), 7.10-6.97

(m, 1H), 6.73-6.68 (m, 1H), 6.52-6.42 (m, 1H), 4.26-4.04 (m, 2H), 3.82 (d, 3H), 3.79-3.69 (m, 2H), 3.57-3.46 (m, 1.5H), 3.24 (d, 0.5H), 2.60 (d, 3H), 1.37-1.27 (m, 3H), 0.89-0.46 (m, 4H).

**Example 10:** (R)-2-(5-fluoro-2-methoxypyridine-4-yl)-1-((S)-7-((6-methoxy-4-methylquinazoline-2-yl)amino)-5-azaspiro[2.4]heptane-5-yl)propan-1-one (115)

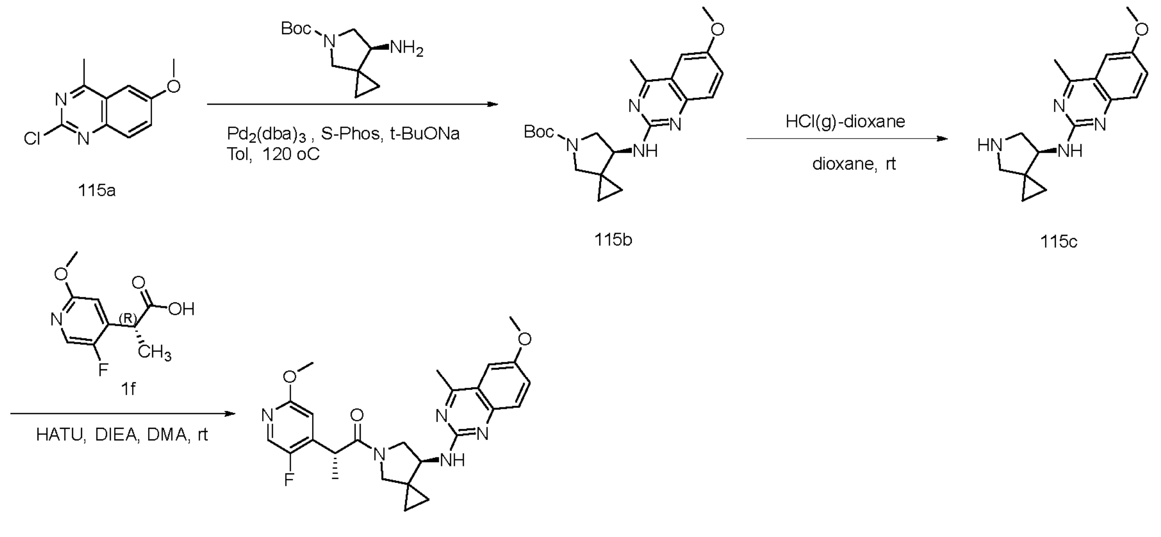

115a
115b
115c
1f
115

Step 1: (S)-7-((6-methoxy-4-methylquinazoline-2-yl)amino)-5-azaspiro[2.4]heptane-5-tert-butyl formate (115b)

To a reaction flask were added a compound 115a (100 mg, prepared with reference to "WO2012083165A1") and (S)-7-amino-5-azaspiro[2.4]heptane-5-tert-butyl formate (122 mg) under nitrogen protection, then tris(dibenzylideneacetone)dipalladium (43 mg), sodium tert-butoxide (92 mg), and 2-dicyclohexylphosphino-2',6'-dimethoxybiphenyl (39 mg) were added. The mixture was purged with nitrogen, and finally ultra-dry toluene (2 mL) was added to react at 120°C, to give a product. The progress of the reaction was monitored by LC-MS. The reaction mixture was diluted with water (50 mL), and subjected to extraction with ethyl acetate (3 × 50 mL). The organic phases were combined, backwashed with a saturated aqueous sodium chloride solution (1 × 50 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (50-100%), to give the compound 115b (100 mg). LC-MS: (ESI, *m/z*) = 385.10 $[M+H]^+$

Step 2: (S)-6-methoxy-4-methyl-N-(5-azaspiro[2.4]heptane-7-yl)quinazoline-2-amine (115c)

To a reaction flask were added the compound 115b (40 mg) and a solution (4 M, 1 mL) of hydrogen chloride in 1,4-dioxane at room temperature, to react at room temperature for 30 min, to give a product. The progress of the reaction was monitored by LC-MS. The reaction solution was concentrated under reduced pressure to give a hydrochloride salt of the compound 115c (35 mg, crude product). The crude product was not further purified and was directly subjected to next step. LC-MS: (ESI, *m/z*) = 285.00 $[M+H]^+$

Step 3: (R)-2-(5-fluoro-2-methoxypyridine-4-yl)-1-((S)-7-((6-methoxy-4-methylquinazoline-2-yl)amino)-5-azaspiro[2.4]heptane-5-yl)propan-1-one (115)

To a reaction flask were added the hydrochloride salt (35 mg) of the compound 115c and the compound 1f (25 mg) at room temperature. The mixture was dissolved in N,N-dimethylacetamide (1 mL), then N,N-diisopropylethylamine (94 mg) was added, and stirred for 5 min. Then, 2-(7-azabenzotriazol)-N,N,N',N'-tetramethyluronium hexafluorophosphate (70 mg) was added, and stirred at room temperature for 1 h, to give a product. The progress of the reaction was monitored by LC-MS. The crude product was purified by high-performance liquid chromatography (chromatographic column configurations: Kinetex EVO C18 column, 30 mm × 150 mm, 5 μm; mobile phase A: water (10 mmol/L ammonium bicarbonate), mobile phase B: acetonitrile; flow rate: 60 mL/min; gradient: 40% B to 64% B within 10 min), to give the

compound 115 (17.5 mg).

LC-MS: (ESI, *m/z*) = 466.00 [M+H]$^+$
$^1$H NMR (400 MHz, DMSO-$d_6$) δ 8.12-8.09 (m, 1H), 7.41-7.24 (m, 4H), 6.73-6.69 (m, 1H), 4.27-4.04 (m, 2H), 3.85 (d, 6H), 3.82 (d, 3H), 3.78-3.47 (m, 3.5H), 3.25-3.19 (m, 0.5H), 2.70 (d, 3H), 1.37-1.26 (m, 3H), 0.93-0.41 (m, 4H).

**Example 11:** 2-(6-(((S)-5-((R)-2-(5-fluoro-2-methoxypyridine-4-yl)propionyl)-5-azaspiro[2.4]heptane-7-yl)amino)-2-methylpyridine-3-yl)pyrimidine-4-nitrile (116)

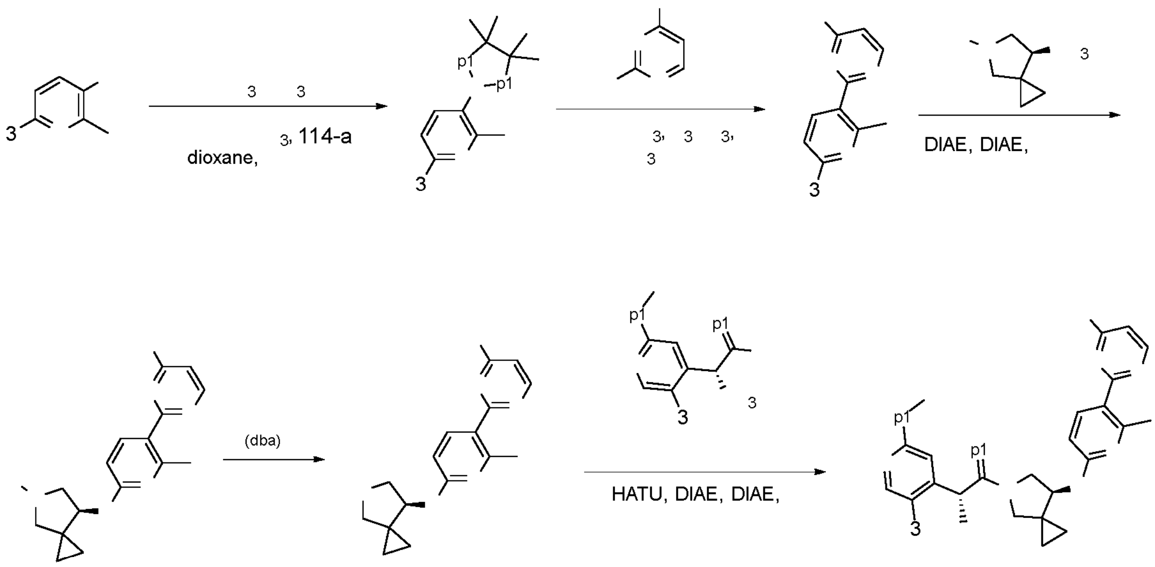


Step 1: 6-fluoro-2-methyl-3-(4,4,5,5-tetramethyl-1,3,2-dioxaborolan-2-yl)pyridine (116b)

A compound 116a (5 g) and bis(pinacolato)diboron (13.44 g) were dissolved in 1,4-dioxane (50 mL), then [1,1'-bis(diphenylphosphino)ferrocene]dichloropalladium(II) dichloromethane complex (432 mg) and potassium acetate (5.7 g) were added. The mixture was purged with nitrogen for three times, and heated to 100°C and stirred for 2 h. The reaction mixture was cooled down to room temperature and then filtered. The filter cake was washed with 1,4-dioxane (20 mL). The filtrate was concentrated under reduced pressure. The resulting residue was purified by reversed-phase column chromatography (C18 chromatographic column; mobile phases: acetonitrile and water; gradient: 0% to 75% within 25 min), to give the compound 116b (4.8 g).
LC-MS: (ESI, *m/z*) = 238.10 [M+H]$^+$

Step 2: 2-(6-fluoro-2-methylpyridine-3-yl)pyrimidine-4-nitrile (116c)

The compound 116b (500 mg) and 2-bromopyrimidine-4-nitrile (773 mg) were dissolved in a mixed solution of 1,4-dioxane (5 mL) and water (1.25 mL), then [1,1'-bis(diphenylphosphino)ferrocene]dichloropalladium(II) dichloromethane complex (86 mg) and potassium carbonate (874 mg) were added. The mixture was purged with nitrogen for three times, and heated to 100°C and stirred for 1 h. The reaction mixture was cooled down to room temperature and then diluted with water (50 mL). Then, the mixture was subjected to extraction with ethyl acetate (3 × 50 mL). The organic phases were combined, backwashed with a saturated sodium chloride solution (2 × 50 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-20%), to give the compound 116c (320 mg, 70.9%).
LC-MS: (ESI, *m/z*) = 215.00 [M+H]$^+$

Step 3: (S)-7-((5-(4-cyanopyrimidine-2-yl)-6-methylpyridine-2-yl)amino)-5-azaspiro[2.4]heptane-5-tert-butyl formate (116d)

The compound 116c (100 mg) and (S)-7-amino-5-azaspiro[2.4]heptane-5-tert-butyl formate (150 mg) were

dissolved in N,N-dimethylacetamide (2 mL), N,N-diisopropylethylamine (182 mg) was added at room temperature, and heated to 100°C and stirred overnight. The reaction mixture was cooled down to room temperature and then diluted with water (40 mL). Then, the mixture was subjected to extraction with ethyl acetate (3 × 50 mL). The organic phases were combined, backwashed with a saturated sodium chloride solution (2 × 50 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-37%), to give the compound 116d (120 mg).
LC-MS: (ESI, *m/z*) = 407.25 $[M+H]^+$

Step 4: (S)-2-(6-((5-azaspiro[2.4]hept-7-yl)amino)-2-methylpyridine-3-yl)pyrimidine-4-nitrile (116e)

The compound 116d (100 mg) was dissolved in dichloromethane (0.5 mL), and trifluoroacetic acid (1 mL) was added and stirred at room temperature for 1 h. The reaction mixture was concentrated under reduced pressure to give a trifluoroacetate salt (80 mg, crude product) of the compound 116e. The crude product was not further purified and was directly subjected to next step.
LC-MS: (ESI, *m/z*) = 307.10 $[M+H]^+$

Step 5: 2-(6-(((S)-5-((R)-2-(5-fluoro-2-methoxypyridine-4-yl)propionyl)-5-azaspiro[2.4]heptane-7-yl)amino)-2-methylpyridine-3-yl)pyrimidine-4-nitrile (116)

The trifluoroacetate salt (80 mg, crude product) of the compound 116e and the compound 1f (49 mg) were dissolved in N,N-dimethylacetamide (1 mL), and N,N-diisopropylethylamine (317 mg) was added to adjust the mixture to be basic. Then, 2-(7-azabenzotriazol)-N,N,N',N'-tetramethyluronium hexafluorophosphate (187 mg) was added, and stirred at room temperature for 1 h. The resulting mixture was filtered. The filter cake was washed with N,N-dimethylacetamide (2 × 1 mL). The filtrate was purified by high-performance liquid chromatography (chromatographic column configurations: Kinetex 5 μm EVO C18 column, 30 × 150 mm, 5 μm; mobile phase A: water (10 mmol/L ammonium bicarbonate), mobile phase B: acetonitrile; flow rate: 60 mL/min; gradient: 30% B to 65% B within 8 min), to give the compound 116 (26.8 mg).

LC-MS: (ESI, *m/z*) = 488.00 $[M+H]^+$
$^1H$ NMR (400 MHz, DMSO-$d_6$) δ 9.14-9.10 (m, 1H), 8.13-8.09 (m, 1H), 8.09-8.04 (m, 1H), 7.89-7.86 (m, 1H), 7.34-7.27 (m, 1H), 6.73-6.68 (m, 1H), 6.56-6.45 (m, 1H), 4.25-4.05 (m, 2H), 3.82 (d, 3H), 3.78-3.60 (m, 2H), 3.56-3.47 (m, 1.5H), 3.23 (d, 0.5H), 2.62 (d, 3H), 1.36-1.28 (m, 3H), 0.88-0.47 (m, 4H).

**Example 12:** (R)-2-(5-fluoro-2-methoxypyridine-4-yl)-1-((S)-7-((7-fluoro-6-methoxyquinoline-2-yl)amino)-5-azaspiro[2.4]heptane-5-yl)propan-1-one (117)

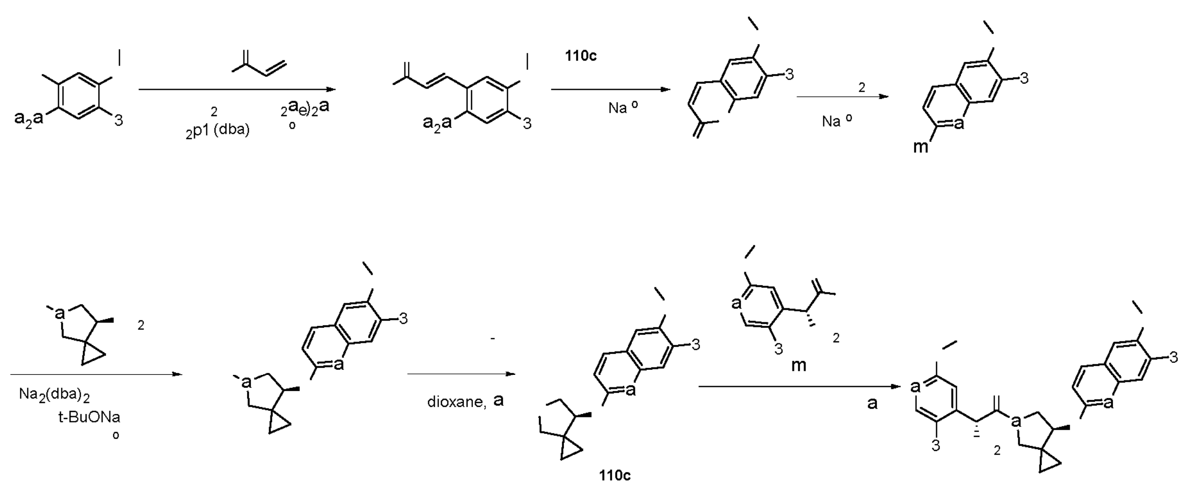

Step 1: (E)-3-(2-amino-4-fluoro-5-methoxyphenyl)ethyl acrylate (117b)

A compound 117a (2 g) and ethyl acrylate (1.1 g) were dissolved in N,N-dimethylformamide (20 mL), then palladium acetate (204 mg), tri(o-tolyl)phosphine (305 mg), and triethylamine (3.69 g) were added. The mixture was purged with nitrogen for three times, and heated to 105°C and stirred for 2 h. The mixture was cooled down to room temperature and then diluted with water (100 mL). Then, the mixture was subjected to extraction with ethyl acetate (3 × 200 mL). The organic phases were combined, backwashed with a saturated sodium chloride solution (2 × 200 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-25%), to give the compound 117b (1.4 g).
LC-MS: (ESI, *m/z*) = 239.95 $[M+H]^+$

Step 2: 7-fluoro-6-methoxyquinoline-2(1H)-one (117c)

The compound 117b (940 mg) was dissolved in anhydrous ethanol (10 mL), then a solution (2.4 g) of 21% sodium ethoxide in ethanol was added. The mixture was purged with nitrogen for three times, and heated to 90°C and stirred for 2 h. After the reaction mixture was concentrated under reduced pressure, a saturated ammonium chloride solution was added to adjust pH to neutral, and stirred for 10 min. After the resulting mixture was filtered, the filter cake was collected and concentrated under reduced pressure, to give the compound 117c (500 mg, crude product). The crude product was not further purified and was directly subjected to next step.
LC-MS: (ESI, *m/z*) = 194.15 $[M+H]^+$

Step 3: 2-chloro-7-fluoro-6-methoxyquinoline (117d)

The compound 117c (200 mg, crude product) was dissolved in phosphorus oxychloride (4 mL). The mixture was purged with nitrogen for three times, and heated to 80°C and stirred for 3 h. The reaction mixture was cooled down to room temperature and then added dropwise to ice water (40 mL) to be quenched. Then, the mixture was subjected to extraction with ethyl acetate (3 × 50 mL). The organic phases were combined, backwashed with a saturated sodium chloride solution (2 × 50 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-12%), to give the compound 117d (118 mg).
LC-MS: (ESI, *m/z*) = 212.10 $[M+H]^+$

Step 4: (S)-7-((7-fluoro-6-methoxyquinoline-2-yl)amino)-5-azaspiro[2.4]heptane-5-tert-butyl formate (117e)

The compound 117d (55 mg) and (S)-7-amino-5-azaspiro[2.4]heptane-5-tert-butyl formate (66 mg) were dissolved in toluene (2 mL), then tris(dibenzylideneacetone)dipalladium (24 mg), 2-dicyclohexylphosphino-2',6'-dimethoxy-biphenyl (21 mg), and sodium tert-butoxide (50 mg) were added. The mixture was purged with nitrogen for three times, and heated to 100°C and stirred for 2 h. The reaction mixture was cooled down to room temperature and then diluted with water (10 mL). Then, the mixture was subjected to extraction with ethyl acetate (3 × 20 mL). The organic phases were combined, backwashed with a saturated sodium chloride solution (2 × 20 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-13%), to give the compound 117e (60 mg).
LC-MS: (ESI, *m/z*) = 388.15 $[M+H]^+$

Step 5: (S)-7-fluoro-6-methoxy-N-(5-azaspiro[2.4]heptane-7-yl)quinoline-2-amine (117f)

To a reaction flask was added the compound 117e (48 mg), a solution (4 M, 2 mL) of hydrogen chloride in 1,4-dioxane was added at room temperature, and stirred for 1 h. The reaction mixture was concentrated under reduced pressure to give a hydrochloride salt (38 mg, crude product) of the compound 117f. The crude product was not further purified and was directly subjected to next step.
LC-MS: (ESI, *m/z*) = 288.05 $[M+H]^+$

Step 6: (R)-2-(5-fluoro-2-methoxypyridine-4-yl)-1-((S)-7-((7-fluoro-6-methoxyquinoline-2-yl)amino)-5-azaspiro[2.4]heptane-5-yl)propan-1-one (117)

The hydrochloride salt (38 mg, crude product) of the compound 117f and the compound 1f (25 mg) were dissolved

in N,N-dimethylacetamide (1 mL), and N,N-diisopropylethylamine (161 mg) was added at room temperature to adjust the mixture to be basic. Then, 2-(7-azabenzotriazol)-N,N,N',N'-tetramethyluronium hexafluorophosphate (95 mg) was added, and stirred at room temperature for 1 h. The resulting mixture was filtered. The filter cake was washed with N,N-dimethylacetamide (2 × 1 mL). The filtrate was purified by high-performance liquid chromatography (chromatographic column configurations: XBridge BEH Shield RP 18 column, 30 × 150 mm, 5 μm; mobile phase A: water (10 mmol/L ammonium bicarbonate), mobile phase B: acetonitrile; flow rate: 60 mL/min; gradient: 35% B to 75% B within 8 min), to give the compound 117 (17.3 mg).

LC-MS: (ESI, *m/z*) = 469.00 $[M+H]^+$
$^1$H NMR (400 MHz, DMSO-$d_6$) δ 8.13-8.08 (m, 1H), 7.85-7.78 (m, 1H), 7.34-7.28 (m, 1H), 7.24-7.17 (m, 1H), 7.15-7.00 (m, 1H), 6.83-6.66 (m, 2H), 4.26-4.05 (m, 2H), 3.87 (d, 3H), 3.82 (d, 3H), 3.77-3.61 (m, 2H), 3.56-3.50 (m, 1.5H), 3.24 (d, 0.5H), 1.37-1.25 (m, 3H), 0.89-0.48 (m, 4H).

**Example 13:** 2R-2-(5-fluoro-2-methoxypyridine-4-yl)-1-[(7S)-7-[(6-methoxy-4-methylquinazoline-2-yl)amino]-5-azaspiro[2.4]heptane-5-yl]propan-1-one (118)

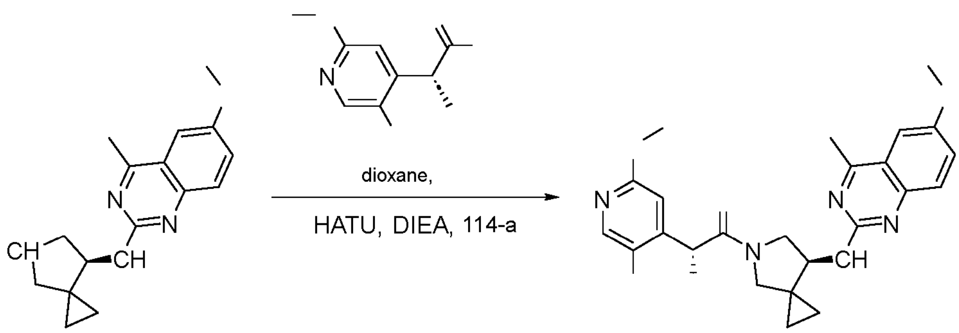


115c

To a solution of the hydrochloride salt (30 mg, crude product) of the compound 115c and the compound 083-1f (26.54 mg) in N,N-dimethylacetamide (2 mL) was added N,N-diisopropylethylamine (318.16 mg), the mixture was stirred for 5 min. Then, 2-(7-azabenzotriazol-1-yl)-N,N,N',N'-tetramethyluronium hexafluorophosphate (70.20 mg) was added and stirred at room temperature for 1 h. The crude product was purified by high-performance liquid chromatography (chromatographic column configurations: Kinetex EVO C18 chromatographic column, 30 mm × 150 mm, 5 μm; mobile phase A: water (10 mmol/L ammonium bicarbonate), mobile phase B: acetonitrile; flow rate: 60 mL/min; gradient: 38% B to 53% B), to give the compound 118 (7.36 mg).

LC-MS (ESI): *m/z* = 481.90 $[M+H]^+$
$^1$H NMR (400 MHz, DMSO-$d_6$) δ 8.21 (d, 1H), 7.42-7.22 (m, 4H), 6.73 (d, 1H), 4.25-4.06 (m, 2H), 3.89-3.80 (m, 6H), 3.78-3.49 (m, 3.5H), 3.23 (d, 0.5H), 2.70 (d, 3H), 1.35-1.24 (m, 3H), 0.93-0.41 (m, 4H).

**Example 14:** (R)-1-((S)-7-((5-(5-(difluoromethyl)-1-methyl-1H-1,2,4-triazole-3-yl)-6-methylpyridine-2-yl)amino)-5-azaspiro[2.4]heptane-5-yl)-2-(5-fluoro-2-methoxypyridine-4-yl)propan-1-one (119)

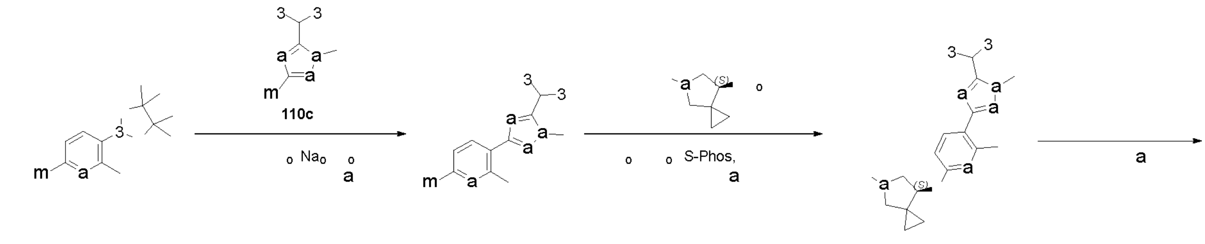


110c

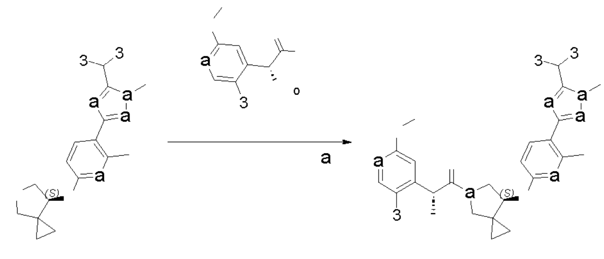

110c

Step 1: 6-chloro-3-(5-(difluoromethyl)-1-methyl-14-1,2,4-triazole-3-yl)-2-methylpyridine (119b)

The compound 083-1b (500 mg) was dissolved in 1,4-dioxane (5 mL), then [1,1'-bis(diphenylphosphino) ferrocene]dichloropalladium(II) dichloromethane complex (72.15 mg) and a 2 M sodium carbonate solution (627.07 mg) were added. The mixture was purged with nitrogen for three times. Then a compound 119a (501.71 mg) was added. The mixture was purged again with nitrogen for three times, and heated to 100°C and stirred for 2 h. The reaction mixture was cooled down to room temperature and then diluted with water (10 mL). Then, the mixture was subjected to extraction with ethyl acetate (3 × 20 mL). The organic phases were combined, backwashed with a saturated sodium chloride solution (2 × 20 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-20%), to give the compound 119b (400 mg).
LC-MS: (ESI, *m/z*) = 259.35 [M+H]$^+$

Step 2: (S)-7-((5-(5-(difluoromethyl)-1-methyl-1*H*-1,2,4-triazole-3-yl)-6-methylpyridine-2-yl)amino)-5-azaspiro[2.4]heptane-5-tert-butyl formate (119c)

To a reaction flask were added the compound 119b (200 mg), (7S)-7-amino-5-azaspiro[2.4]heptane-5-tert-butyl formate (180.56 mg), tris(dibenzylideneacetone)dipalladium (70.81 mg), 2-dicyclohexylphosphino-2',6'-dimethoxy-1,1'-biphenyl (63.49 mg), sodium tert-butoxide (148.62 mg), and toluene (2 mL). The mixture was purged with nitrogen for three times, and stirred at 110°C for 2 h. The reaction mixture was diluted with water (10 mL), and subjected to extraction with ethyl acetate (3 × 10 mL). The organic phases were combined, and dried over sodium sulfate. The resulting mixture was filtered, and then the filtrate was concentrated under reduced pressure. The resulting residue was purified by silica gel column chromatography with ethyl acetate/petroleum ether (0-20%), to give the compound 119c (400 mg).
LC-MS: (ESI, *m/z*) = 435.50 [M+H]$^+$

Step 3: (S)-N-(5-(5-(difluoromethyl)-1-methyl-1*H*-1,2,4-triazole-3-yl)-6-methylpyridine-2-yl)-5-azaspiro[2.4]heptane-7-amine (119d)

To a solution of hydrogen chloride in 1,4-dioxane (4 M, 2 mL) was added the compound 119c (60 mg), the mixture was stirred at room temperature for 30 min. The reaction solution was concentrated under reduced pressure to give a hydrochloride salt (45 mg, crude product) of the compound 119d. The crude product was not purified and was directly subjected to next step.
LC-MS: (ESI, *m/z*) = 335.50 [M+H]$^+$

Step 4: (R)-1-((S)-7-((5-(5-(difluoromethyl)-1-methyl-1*H*-1,2,4-triazole-3-yl)-6-methylpyridine-2-yl)amino)-5-azaspiro [2.4]heptane-5-yl)-2-(5-fluoro-2-methoxypyridine-4-yl)propan-1-one (119)

The hydrochloride salt (45 mg, crude product) of the compound 119d was dissolved in an N,N-dimethylacetamide (1 mL) solution, and the compound 1f (29.78 mg), N,N-diisopropylethylamine (57.98 mg), and 2-(7-azabenzotriazol-1-yl)-N,N,N',N'-tetramethyluronium hexafluorophosphate (85.29 mg) were added sequentially. The reaction solution was stirred at room temperature for 1 h. The reaction solution was directly purified by high-performance liquid chromatography (chromatographic column: XSelect CSH FluoroPhenyl 5 μm, 30 mm × 150 mm; mobile phase A: water (10 mmol/L ammonium bicarbonate), mobile phase B: acetonitrile; flow rate: 60 mL/min; gradient: 30% B to 55% B within 10 min), to give the compound 119 (40 mg).

LC-MS: (ESI, *m/z*) = 515.95 $[M+H]^+$
$^1H$ NMR (400 MHz, DMSO-$d_6$) δ 8.13-8.08 (m, 1H), 7.88-7.81 (m, 1H), 7.56-7.26 (m, 1H), 7.03-6.90 (m, 1H), 6.73-6.68 (m, 1H), 6.49-6.39 (m, 1H), 4.21-4.04 (m, 2H), 4.00 (s, 3H), 3.82 (d, 3H), 3.75-3.58 (m, 2H), 3.55-3.44 (m, 1.5 H), 3.26-3.20 (m, 0.5H), 2.57 (d, 3H), 1.35-1.28 (m, 3H), 0.88-0.45 (m, 4H).

Synthesis of the intermediate 119a

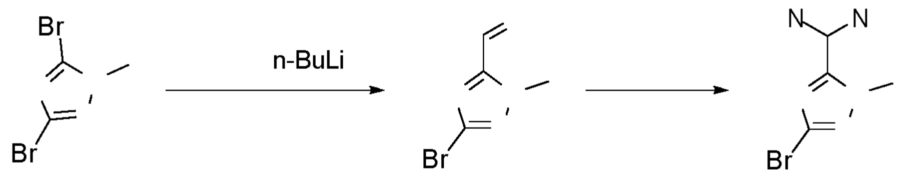


Step 1: 3-bromo-1-methyl-1H-1,2,4-triazole-5-carbaldehyde (119f)

To a 250 mL three-necked flask were added a compound 119e (14 g) and ultra-dry tetrahydrofuran (140 mL). The mixture was purged with nitrogen for three times. The mixture was cooled down to -45°C. N-butyllithium (4.47 g) was added dropwise and stirred at -45°C for 30 min, then cooled down to -75°C, and N,N-dimethylformamide (5.52 g) was added dropwise. After 15 min, the cooling bath was removed to naturally warm up the reaction mixture to room temperature, to react for 2 h. The mixture was poured into water (200 mL). The reaction mixture was subjected to extraction with ethyl acetate (4 × 150 mL). The organic phases were combined, and dried over sodium sulfate. The resulting mixture was filtered, and then the filtrate was concentrated under reduced pressure. The resulting residue was purified by silica gel column chromatography with ethyl acetate/petroleum ether (0-20%), to give the compound 119f (5.5 g).
LC-MS: (ESI, *m/z*) = 208.05 $[M+H_2O+H]^+$

Step 2: 3-bromo-5-difluoromethyl-1-methyl-1*H*-1,2,4-triazole (119a)

To a 100 mL three-necked flask were added the compound 119f (4 g) and diethylaminosulfur trifluoride (13.57 g) in an ice bath. Under nitrogen protection, at room temperature, the resulting residue was stirred overnight. The reaction mixture was quenched with a saturated aqueous ammonium bicarbonate solution (50 mL) at 0°C. The reaction mixture was subjected to extraction with dichloromethane (3 × 50 mL). The organic phases were combined, backwashed with saturated saline (50 mL), and dried over anhydrous sodium sulfate. The resulting mixture was filtered, and then the filtrate was concentrated under reduced pressure. purified by reversed-phase column chromatography (C18 chromatographic column; mobile phases: water (0.5% formic acid) and acetonitrile; gradient: 10% to 50% within 10 min), to give the intermediate 119a (1.6 g).
LC-MS: (ESI, *m/z*) = 212.30 $[M+H]^+$

**Example 15:** (2R)-1-[(7S)-7-{[5-(1,5-dimethyl-1*H*-1,2,4-triazole-3-yl)-6-methylpyridine-2-yl]amino}-5-azaspiro[2.4]heptane-5-yl]-2-(5-fluoro-2-methoxypyridine-4-yl)propan-1-one 132

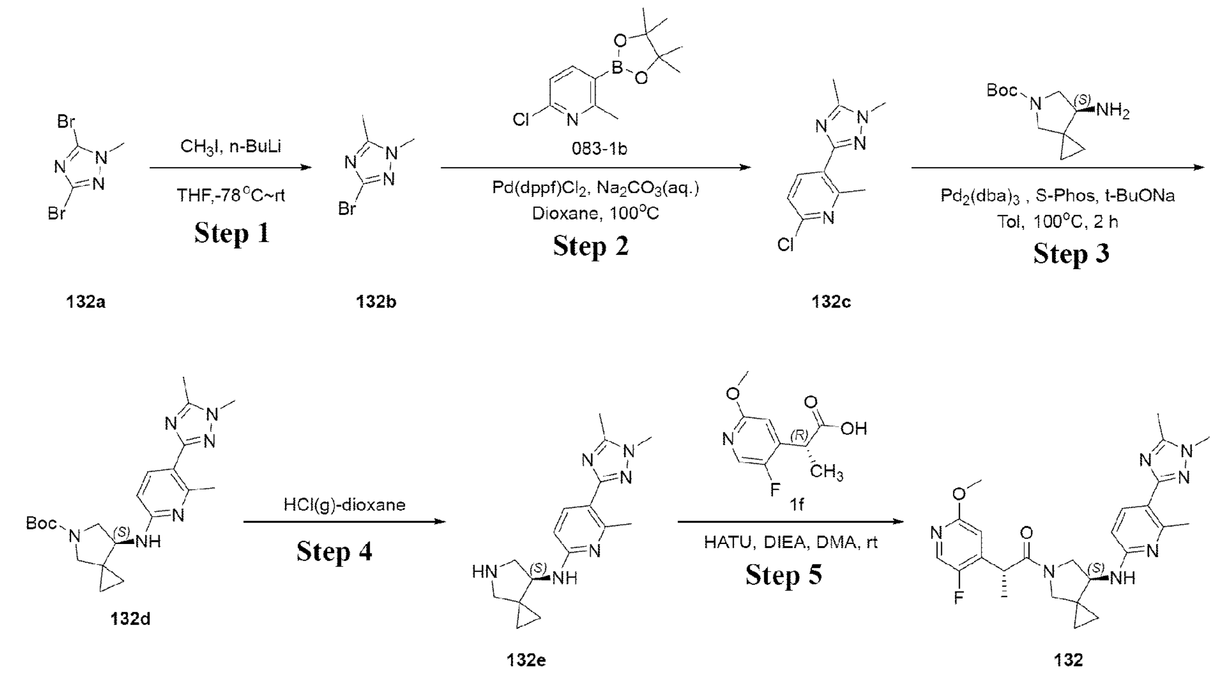


132a 132b 132c

132d 132e 132

Step 1: 3-bromo-1,5-dimethyl-1*H*-1,2,4-triazole 132b

To a solution of 132a (3 g) in tetrahydrofuran (30 mL) was added dropwise a solution (2.5 M, 6.00 mL) of n-butyllithium in n-hexane at -78°C, the mixture was stirred for 1 h. Then, iodomethane (2.30 g) was added dropwise at -40°C and stirred for 2 h, and then quenched with a saturated aqueous ammonium chloride solution (30 mL) at 0°C. The reaction mixture was subjected to extraction with ethyl acetate (50 mL × 3). The organic phases were combined, backwashed with saturated saline (50 mL), and dried over anhydrous sodium sulfate. After the resulting mixture was filtered, the filtrate was concentrated under reduced pressure, to give the compound 132b (2 g, crude product).
GC-MS: (EI, *m*/*z*) = 175.00 $[M]^+$

Step 2: 6-chloro-3-(1,5-dimethyl-1H-1,2,4-triazole-3-yl)-2-methylpyridine 132c

To a solution of 132b (2 g) and 083-1b (2.88 g) in 1,4-dioxane (30 mL) were added [1,1'-bis(diphenylphosphino) ferrocene]dichloropalladium(II) (831.41 mg) and a sodium carbonate solution (2 M, 10 mL), the mixture was stirred at 100°C under nitrogen protection for 2 h. The reaction mixture was subjected to extraction with ethyl acetate (30 mL × 3). The organic phases were combined, backwashed with saturated saline (20 mL), and dried over anhydrous sodium sulfate. The resulting mixture was filtered, and then the filtrate was concentrated under reduced pressure. The resulting residue was purified by silica gel column chromatography with petroleum ether/ethyl acetate (1:1), to give the compound 132c (300 mg).
LC-MS: (ESI, *m*/*z*) = 223.40 $[M+H]^+$

Step 3: (7S)-7-{[5-(1,5-dimethyl-1*H*-1,2,4-triazole-3-yl)-6-methylpyridine-2-yl]amino}-5-azaspiro[2.4]heptane-5-tert-butyl formate 132d

To a solution of the compound 132c (300 mg) and (S)-7-amino-5-azaspiro[2.4]heptane-5-tert-butyl formate (286 mg) in toluene (3 mL) were added tris(dibenzylideneacetone)dipalladium (61.68 mg), 2-dicyclohexylphosphino-2',6'-dimethoxybiphenyl (55.31 mg), and sodium tert-butoxide (258.95 mg). The mixture was purged with nitrogen, and stirred at 100°C for 2 h. Then, the mixture was cooled down to room temperature and diluted with water (10 mL). The mixture was subjected to extraction with ethyl acetate (20 mL × 3). The organic phases were combined, backwashed with saturated saline (20 mL), and dried over anhydrous sodium sulfate. The resulting mixture was filtered, and then the filtrate was concentrated under reduced pressure. The resulting residue was purified by silica gel column chromatography with dichloromethane/methanol (1:1), to give the compound 132d (330 mg).
LC-MS: (ESI, *m*/*z*) = 399.25 $[M+H]^+$

Step 4: (7S)-N-[5-(1,5-dimethyl-1,2,4-triazole-3-yl)-6-methylpyridine-2-yl]-5-azaspiro[2.4]hept-7-amine 132e

To a reaction flask were added 132d (330 mg) and a solution (4 M, 4 mL) of hydrogen chloride in 1,4-dioxane. The resulting residue was stirred for 1 h. The reaction solution was directly spin-dried to give a hydrochloride salt (270 mg, crude product) of the compound 132e. The crude product was not further purified and was directly subjected to next step. LC-MS: (ESI, *m/z)* = 299.10 $[M+H]^+$

Step 5: (2R)-1-[(7S)-7-{[5-(1,5-dimethyl-1*H*-1,2,4-triazole-3-yl)-6-methylpyridine-2-yl]amino}-5-azaspiro[2.4]heptane-5-yl]-2-(5-fluoro-2-methoxypyridine-4-yl)propan-1-one 132

To a solution of the hydrochloride salt (250 mg) of 132e and 1f (166.88 mg) in N,N-dimethylacetamide (5 mL) was added N,N-diisopropylethylamine (2165.71 mg), the mixture was stirred for 5 min. Then, 2-(7-azabenzotriazol-1-yl)-N,N,N',N'-tetramethyluronium hexafluorophosphate (477.85 mg) was added. The resulting residue was stirred for 1 h. The reaction solution was purified directly by high-performance liquid chromatography (chromatographic column configurations: XBridge BEH Shield RP18 column, 30 mm× 150 mm, 5 μm; mobile phase A: water (10 mmol/L ammonium bicarbonate), mobile phase B: acetonitrile; flow rate: 60 mL/min; gradient: 25% B to 50% B), to give the compound 132 (131.5 mg).

LC-MS (ESI): *m/z* = 480.20 $[M+H]^+$
$^1$H NMR (400 MHz, DMSO-$d_6$) δ 8.12-8.09 (m, 1H), 7.84-7.79 (m, 1H), 6.86-6.73 (m, 1H), 6.73-6.68 (m, 1H), 6.45-6.34 (m, 1H), 4.18-4.04 (m, 2H), 3.82 (d, 3H), 3.78 (d, 3H), 3.75-3.58 (m, 2H), 3.53 (s, 1H), 3.50-3.44 (m, 0.5H), 3.23 (d, 0.5H), 2.56 (d, 3H), 2.40 (d, 3H), 1.36-1.27 (m, 3H), 0.89-0.78 (m, 1H), 0.71-0.44 (m, 3H).

**Example 16:** (2R)-2-(5-fluoro-2-methoxypyridine-4-yl)-1-[(7S)-7-{[6-methyl-5-(1-methyl-1*H*-1,2,4-triazole-3-yl)pyridine-2-yl]amino}-5-azaspiro[2.4]heptane-5-yl]propan-1-one 136

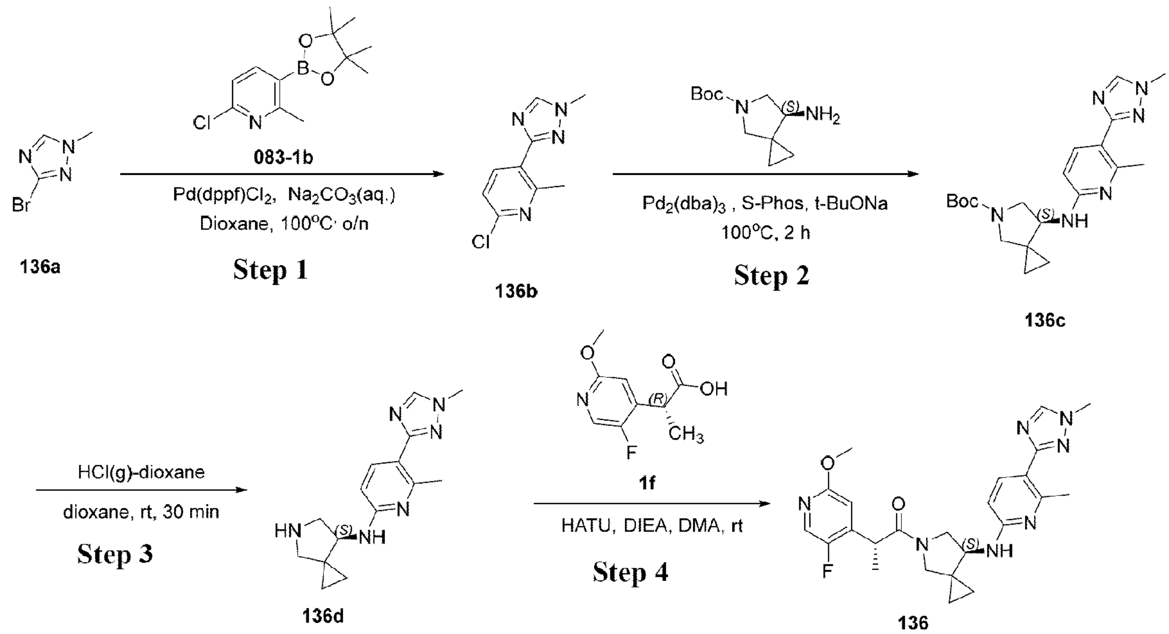


Step 1: 6-chloro-2-methyl-3-(1-methyl-1*H*-1,2,4-triazole-3-yl)pyridine 136b

To a reaction flask were added 136a (383 mg) and 083-1b (200 mg) under nitrogen protection, then 1,1'-bis(diphenylphosphino)ferrocene dichloropalladium(II) dichloromethane complex (64 mg) and an aqueous sodium carbonate solution (2 M, 1.2 mL) were added. The mixture was purged with nitrogen, and finally 1,4-dioxane (4 mL) was added, to react at 100°C overnight. The resulting mixture was diluted with water (50 mL), and subjected to extraction with ethyl acetate (3 × 50 mL). The organic phases were combined, backwashed with a saturated aqueous sodium chloride solution (1 × 50 mL), and dried over anhydrous sodium sulfate. The resulting mixture was filtered, and then the

filtrate was concentrated under reduced pressure. The resulting residue was purified by silica gel column chromatography with the eluent of ethyl acetate/petroleum ether (0-50%), to give the compound 136b (150 mg).
LC-MS: (ESI, *m/z*) = 208.95 $[M+H]^+$

Step 2: (S)-7-((6-methyl-5-(1-methyl-1*H*-1,2,4-triazole-3-yl)pyridine-2-yl)amino)-5-azaspiro[2.4]heptane-5-tert-butyl formate 136c

To a reaction flask were added 136b (130 mg) and (S)-7-amino-5-azaspiro[2.4]heptane-5-tert-butyl formate (132 mg) under nitrogen protection, then tris(dibenzylideneacetone)dipalladium (57 mg), sodium tert-butoxide (119 mg), and 2-dicyclohexylphosphino-2',6'-dimethoxybiphenyl (51 mg) were added. The mixture was purged with nitrogen, and finally ultra-dry toluene (4 mL) was added to react at 100°C for 2 h. The reaction mixture was diluted with water (50 mL), and subjected to extraction with ethyl acetate (3 × 50 mL). The organic phases were combined, backwashed with a saturated aqueous sodium chloride solution (1 × 50 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-50%), to give the compound 136c (120 mg, 50.05%).
LC-MS: (ESI, *m/z*) = 385.15 $[M+H]^+$

Step 3: (S)-N-(6-methyl-5-(1-methyl-1*H*-1,2,4-triazole-3-yl)pyridine-2-yl)-5-azaspiro[2.4]hept-7-amine 136d

To a reaction flask were added 136c (40 mg) and a solution (4 M, 1 mL) of hydrogen chloride in 1,4-dioxane at room temperature to react at room temperature for 30 min. The reaction solution was concentrated under reduced pressure, to give a hydrochloride salt (35 mg, crude product) of the compound 136d. The crude product was not further purified and was directly subjected to next step.
LC-MS: (ESI, *m/z*) = 285.05 $[M+H]^+$

Step 4: (2R)-2-(5-fluoro-2-methoxypyridine-4-yl)-1-[(7S)-7-{[6-methyl-5-(1-methyl-1*H*-1,2,4-triazole-3-yl)pyridine-2-yl]amino}-5-azaspiro[2.4]heptane-5-yl]propan-1-one 136

The hydrochloride salt (30 mg) of 136d and 1f (22 mg) were added to a reaction flask and dissolved in N,N-dimethylacetamide (2 mL) at room temperature, then N,N-diisopropylethylamine (40 mg) was added, and stirred for a few minutes. Finally, 2-(7-azabenzotriazol)-N,N,N',N'-tetramethyluronium hexafluorophosphate (60 mg) was added. The resulting mixture was stirred at room temperature for 2 h. The mixture was purified directly by high-performance liquid chromatography (chromatographic column configurations: XBridge BEH C18 column, 30 mm × 150 mm, 5 μm; mobile phase A: water (10 mmol/L ammonium bicarbonate), mobile phase B: acetonitrile; flow rate: 60 mL/min; gradient: 25% B to 45% B within 10 min), to give the compound 136 (26.9 mg).

LC-MS: (ESI, *m/z*) = 465.95 $[M+H]^+$
$^1$H NMR (400 MHz, DMSO-$d_6$) δ 8.43 (d, 1H), 8.13-8.08 (m, 1H), 7.87-7.82 (m, 1H), 6.90-6.76 (m, 1H), 6.73-6.68 (m, 1H), 6.47-6.37 (m, 1H), 4.19-4.04 (m, 2H), 3.88 (d, 3H), 3.82 (d, 3H), 3.76-3.58 (m, 2H), 3.53 (s, 1H), 3.50-3.43 (m, 0.5H), 3.23 (d, 0.5H), 2.57 (d, 3H), 1.37-1.26 (m, 3H), 0.89-0.78 (m, 1H), 0.72-0.43 (m, 3H).

**Example 17:** (2R)-2-(5-chloro-2-methoxypyridine-4-yl)-1-[(7S)-7-{[5-(1,5-dimethyl-1*H*-1,2,4-triazole-3-yl)-6-methylpyridine-2-yl]amino}-5-azaspiro[2.4]heptane-5-yl]propan-1-one 141

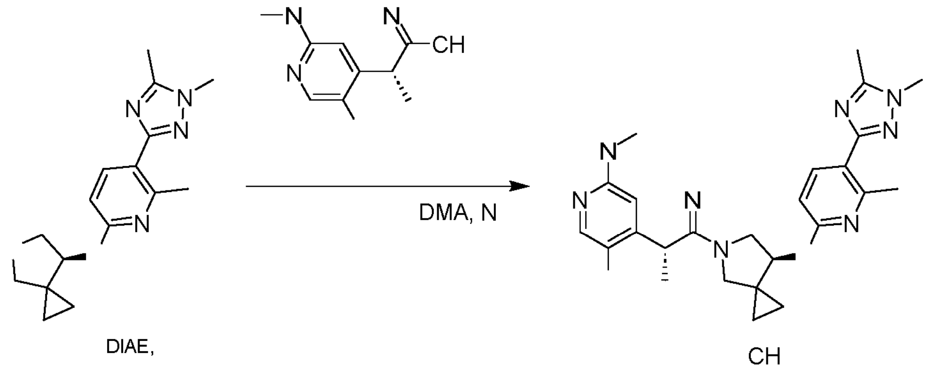


To a solution of the hydrochloride salt (50 mg) of 132e and 083-1f (36.13 mg) in N,N-dimethylacetamide (2 mL) was added N,N-diisopropylethylamine (433.14 mg), the mixture was stirred for 5 min. Then, 2-(7-azabenzotriazol-1-yl)-N,N,N',N'-tetramethyluronium hexafluorophosphate (95.57 mg) was added. The resulting residue was stirred for 1 h. The reaction solution was purified directly by high-performance liquid chromatography (chromatographic column configurations: XBridge BEH C18 OBD preparative column, 30 mm× 150 mm, 5 μm; mobile phase A: water (10 mmol/L ammonium bicarbonate), mobile phase B: acetonitrile; flow rate: 60 mL/min; gradient: 25% B to 51% B), to give the compound 141 (27.11 mg).

LC-MS (ESI): *m/z* = 496.10 $[M+H]^+$
$^1$H NMR (400 MHz, DMSO-$d_6$) δ 8.21 (d, 1H), 7.84-7.79 (m, 1H), 6.86-6.74 (m, 1H), 6.72 (d, 1H), 6.45-6.35 (m, 1H), 4.16-4.00 (m, 2H), 3.84 (d, 3H), 3.78 (d, 3H), 3.72-3.51 (m, 3H), 3.49 (d, 0.5H), 3.24 (d, 0.5H), 2.56 (d, 3H), 2.40 (d, 3H), 1.36-1.23 (m, 3H), 0.89-0.77 (m, 1H), 0.73-0.45 (m, 3H).

**Example 18:** (2R)-2-(5-chloro-2-methoxypyridine-4-yl)-1-[(7S)-7-{[6-methyl-5-(1-methyl-1*H*-1,2,4-triazole-3-yl)pyridine-2-yl]amino}-5-azaspiro[2.4]heptane-5-yl]propan-1-one 142

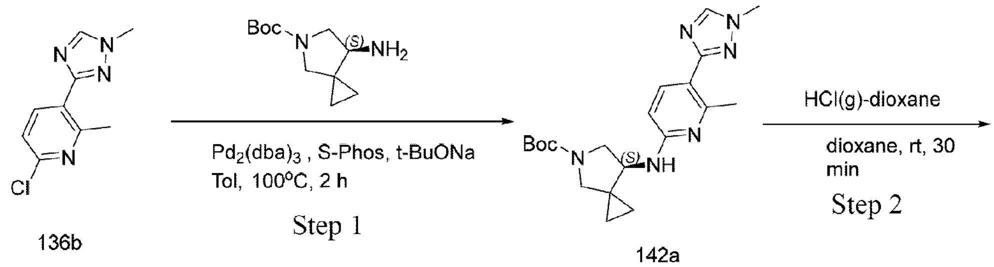

136b
142a

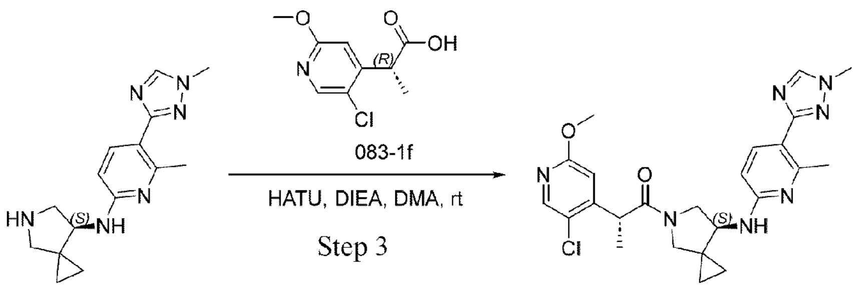

142b
142

Step 1: (S)-7-((6-methyl-5-(1-methyl-1*H*-1,2,4-triazole-3-yl)pyridine-2-yl)amino)-5-azaspiro[2.4]heptane-5-tert-butyl formate 142a

To a reaction flask were added 136b (250 mg) and (S)-7-amino-5-azaspiro[2.4]heptane-5-tert-butyl formate (254

mg) under nitrogen protection, then tris(dibenzylideneacetone)dipalladium (109 mg), sodium tert-butoxide (230 mg), and 2-dicyclohexylphosphino-2',6'-dimethoxybiphenyl (98 mg) were added, and ultra-dry toluene (4 mL) was added. The mixture was purged with nitrogen, to react at 100°C for 2 h. The reaction mixture was cooled down to room temperature, diluted with water (30 mL), and subjected to extraction with ethyl acetate (3 × 30 mL). The organic phases were combined, backwashed with a saturated aqueous sodium chloride solution (1 × 30 mL), and dried over anhydrous sodium sulfate. The resulting mixture was filtered, and then the filtrate was concentrated under reduced pressure. The resulting residue was purified by silica gel column chromatography with the eluent of methanol/dichloromethane (0-50%), to give the compound 142a (200 mg).
LC-MS: (ESI, *m*/*z*) = 385.10 $[M+H]^+$

Step 2: (S)-N-(6-methyl-5-(1-methyl-1*H*-1,2,4-triazole-3-yl)pyridine-2-yl)-5-azaspiro[2.4]hept-7-amine 142b

To a reaction flask were added 142a (40 mg) and a solution (4 M, 1 mL) of hydrogen chloride in 1,4-dioxane at room temperature, to react at room temperature for 30 min. The reaction solution was concentrated under reduced pressure to give a hydrochloride salt (35 mg, crude product) of the compound 142b. The crude product was not further purified and was directly subjected to next step.
LC-MS: (ESI, *m*/*z*) = 285.10 $[M+H]^+$

Step 3: (2R)-2-(5-chloro-2-methoxypyridine-4-yl)-1-[(7S)-7-{[6-methyl-5-(1-methyl-1*H*-1,2,4-triazole-3-yl)pyridine-2-yl] amino}-5-azaspiro[2.4]heptane-5-yl]propan-1-one 142

The hydrochloride salt (35 mg) of 142b and 083-1f (26 mg) were added to a reaction flask and dissolved in N,N-dimethylacetamide (2 mL) at room temperature, then N,N-diisopropylethylamine (94 mg) was added, and stirred for 5 min. Finally, 2-(7-azabenzotriazol)-N,N,N',N'-tetramethyluronium hexafluorophosphate (70 mg) was added. The mixture was stirred for 2 h. The crude product was purified by high-performance liquid chromatography (chromatographic column configurations: XBridge BEH C18 column, 30 mm × 150 mm, 5 μm; mobile phase A: water (10 mmol/L ammonium bicarbonate), mobile phase B: acetonitrile; flow rate: 60 mL/min; gradient: 16% B to 51% B within 10 min), to give the compound 142 (17.09 mg).

LC-MS: (ESI, *m*/*z*) = 481.90 $[M+H]^+$
$^1$H NMR (400 MHz, DMSO-$d_6$) δ 8.44 (d, 1H), 8.22 (d, 1H), 7.88-7.82 (m, 1H), 6.91-6.78 (m, 1H), 6.72 (d, 1H), 6.47-6.37 (m, 1H), 4.16-4.01 (m, 2H), 3.88 (s, 3H), 3.84 (d, 3H), 3.72-3.51 (m, 3H), 3.49 (d, 0.5H), 3.24 (d, 0.5H), 2.57 (d, 3H), 1.35-1.25 (m, 3H), 0.89-0.78 (m, 1H), 0.74-0.45 (m, 3H).

**Example 19:** (2R)-2-(5-fluoro-2-methoxypyridine-4-yl)-1-((7S)-7-((6-methyl-5-(1-methyl-1H-pyrazol-4-yl)pyridine-2-yl) amino)-5-azaspiro[2.4]heptane-5-yl)propan-1-one 143

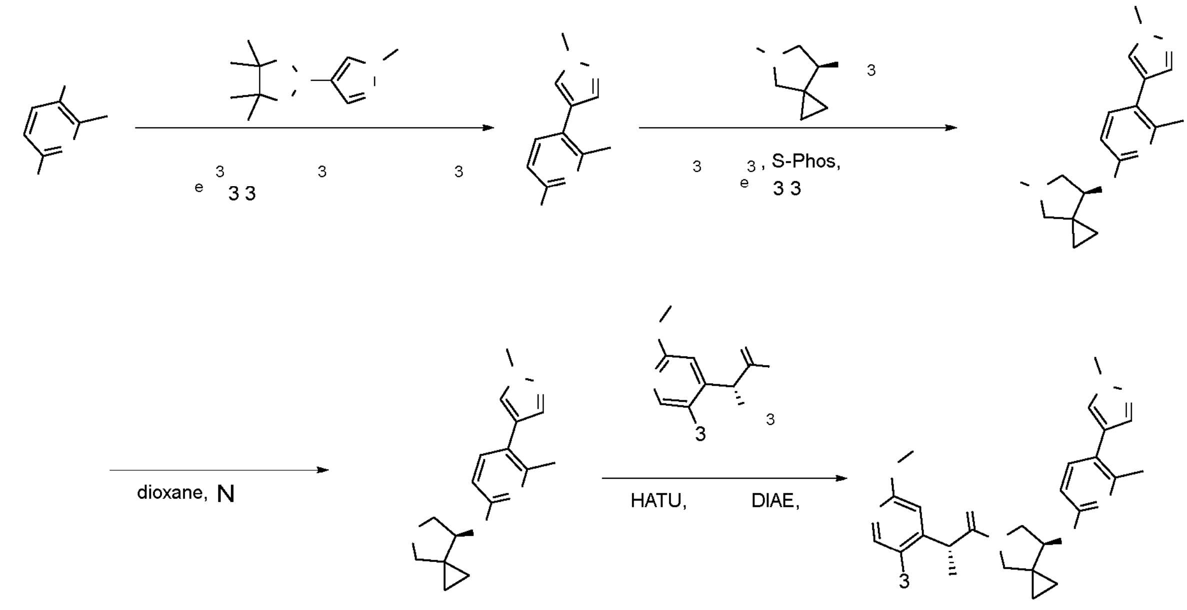


Step 1: 6-chloro-2-methyl-3-(1-methyl-1*H*-pyrazol-4-yl)pyridine 143a

To a solution of the compound 083-1a (1 g) and 1-methyl-4-(4,4,5,5-tetramethyl-1,3,2-dioxaboran-2-yl)-1H-pyrazole (1.01 g) in 1,4-dioxane (20 mL) and water (5 mL) were added cesium carbonate (4.73 g) and (2-dicyclohexylphosphino-2',6'-dimethoxy-1,1'-biphenyl)(2'-amino-1,1'-biphenyl-2-yl)palladium(II) methanesulfonate (188.95 mg). Under nitrogen protection, at 100°C, the resulting residue was stirred for 2 h. The reaction mixture was diluted with water (30 mL), and subjected to extraction with ethyl acetate (50 mL × 3). The organic phases were combined, backwashed with saturated saline (30 mL × 1), and dried over sodium sulfate. The resulting mixture was filtered, and then the filtrate was concentrated under reduced pressure. The resulting residue was purified by silica gel column chromatography with petroleum ether/ethyl acetate (1:1), to give the compound 143a (480 mg).
LC-MS: (ESI, *m/z*) = 208.10 $[M+H]^+$

Step 2: (S)-7-((6-methyl-5-(1-methyl-1*H*-pyrazol-4-yl)pyridine-2-yl)amino)-5-azaspiro[2.4]heptane-5-tert-butyl formate 143b

To a solution of 143a (250 mg) and (7S)-7-amino-5-azaspiro[2.4]heptane-5-tert-butyl formate (255.58 mg) in toluene (3 mL) were added tris(dibenzylideneacetone)dipalladium (55.12 mg), 2-dicyclohexylphosphino-2',6'-dimethoxybiphenyl (49.42 mg), and sodium tert-butoxide (231.40 mg). Under nitrogen protection, at 100°C, the mixture was stirred for 2 h. Then, the reaction mixture was cooled down to room temperature. The reaction mixture was diluted with water (20 mL), and subjected to extraction with ethyl acetate (20 mL × 3). The organic phases were combined, backwashed with saturated saline (20 mL × 1), and dried over sodium sulfate. The resulting mixture was filtered, and then the filtrate was concentrated under reduced pressure. The resulting residue was purified by silica gel column chromatography with petroleum ether/ethyl acetate (1:1), to give the compound 143b (320 mg).
LC-MS: (ESI, *m/z*) = 384.10 $[M+H]^+$

Step 3: (S)-N-(6-methyl-5-(1-methyl-1*H*-pyrazol-4-yl)pyridine-2-yl)-5-azaspiro[2.4]heptane-7-amine 143c

To a reaction flask were added 143b (70 mg) and a solution (4 M, 2 mL) of hydrogen chloride in 1,4-dioxane, the mixture was stirred at room temperature for 1 h, to give a hydrochloride salt (50 mg) of the compound 143c. The crude product was not further purified and was directly subjected to next step.
LC-MS: (ESI, *m/z*) = 284.45 $[M+H]^+$

Step 4: (2R)-2-(5-fluoro-2-methoxypyridine-4-yl)-1-((7S)-7-((6-methyl-5-(1-methyl-1*H*-pyrazol-4-yl)pyridine-2-yl)amino)-5-azaspiro[2.4]heptane-5-yl)propan-1-one 143

To a solution of the hydrochloride salt (25 mg) of 143c and the compound 1f (17.57 mg) in N,N-dimethylacetamide (1 mL) was added N,N-diisopropylethylamine (228.05 mg), the mixture was stirred for 5 min. Then, 2-(7-azabenzotriazol-1-yl)-N,N,N',N'-tetramethyluronium hexafluorophosphate (50.32 mg) was added. The resulting mixture was stirred for 1 h. The reaction solution was purified by high-performance liquid chromatography (chromatographic column configurations: YMC Triart C18 ExRs preparative column, 30 mm× 150 mm, 5 μm; mobile phase A: water (10 mmol/L ammonium bicarbonate), mobile phase B: acetonitrile; flow rate: 60 mL/min; gradient: 35% B to 57% B), to give the compound 143 (10.96 mg).

LC-MS (ESI): $m/z$ = 465.00 $[M+H]^+$
$^1$H NMR (400 MHz, DMSO-$d_6$) δ 8.13-8.09 (m, 1H), 7.79-7.75 (m, 1H), 7.54-7.50 (m, 1H), 7.36-7.30 (m, 1H), 6.72-6.68 (m, 1H), 6.61-6.47 (m, 1H), 6.43-6.32 (m, 1H), 4.15-4.01 (m, 2H), 3.85 (d, 3H), 3.82 (d, 3H), 3.75-3.48 (m, 3H), 3.48-3.41 (m, 0.5 H), 3.24 (d, 0.5H), 2.33 (d, 3H), 1.35-1.27 (m, 3H), 0.87-0.76 (m, 1H), 0.70-0.42 (m, 3H).

**Example 20:** (2R)-2-(5-chloro-2-methoxypyridine-4-yl)-1-((7S)-7-[6-methyl-5-(1-methyl-1*H*-pyrazol-4-yl)pyridine-2-yl) amino)-5-azaspiro[2.4]heptane-5-yl]propan-1-one 144

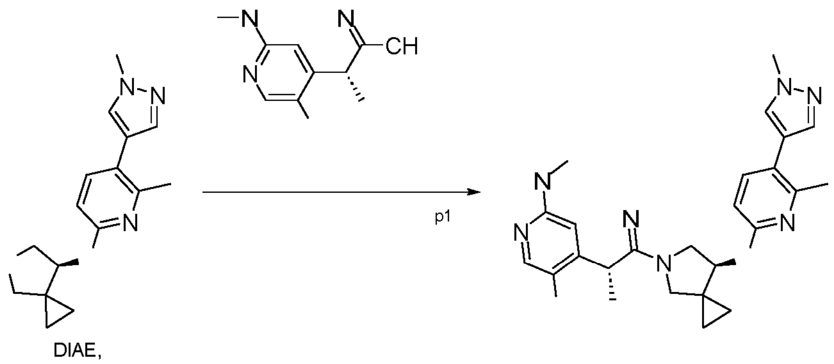


To a solution of the hydrochloride salt (25 mg) of the compound 143c and the compound 083-1f (19.02 mg) in N,N-dimethylacetamide (1 mL) was added N,N-diisopropylethylamine (228.05 mg), the mixture was stirred for 5 min. Then, 2-(7-azabenzotriazol-1-yl)-N,N,N',N'-tetramethyluronium hexafluorophosphate (50.32 mg) was added. The resulting residue was stirred for 1 h. The reaction solution was purified by high-performance liquid chromatography (chromatographic column configurations: YMC Triart C18 ExRs preparative column, 30 mm× 150 mm, 5 μm; mobile phase A: water (10 mmol/L ammonium bicarbonate), mobile phase B: acetonitrile; flow rate: 60 mL/min; gradient: 40% B to 60% B), to give the compound 144 (12.19 mg).

LC-MS (ESI): $m/z$ = 480.90 $[M+H]^+$
$^1$H NMR (400 MHz, DMSO-$d_6$) δ 8.21 (d, 1H), 7.79-7.76 (m, 1H), 7.53-7.51 (m, 1H), 7.36-7.30 (m, 1H), 6.72 (d, 1H), 6.60-6.48 (m, 1H), 6.43-6.33 (m, 1H), 4.13-3.98 (m, 2H), 3.88-3.81 (m, 6H), 3.70-3.47 (m, 3.5 H), 3.25 (d, 0.5H), 2.33 (d, 3H), 1.35-1.25 (m, 3H), 0.88-0.76 (m, 1H), 0.72-0.44 (m, 3H).

**Example 21:** (R)-1-((S)-7-((5-(1,2-dimethyl-1*H*-imidazol-4-yl)-6-methylpyridine-2-yl)amino)-5-azaspiro[2.4]hept-5-yl)-2-(5-fluoro-2-methoxypyridine-4-yl)propan-1-one 149

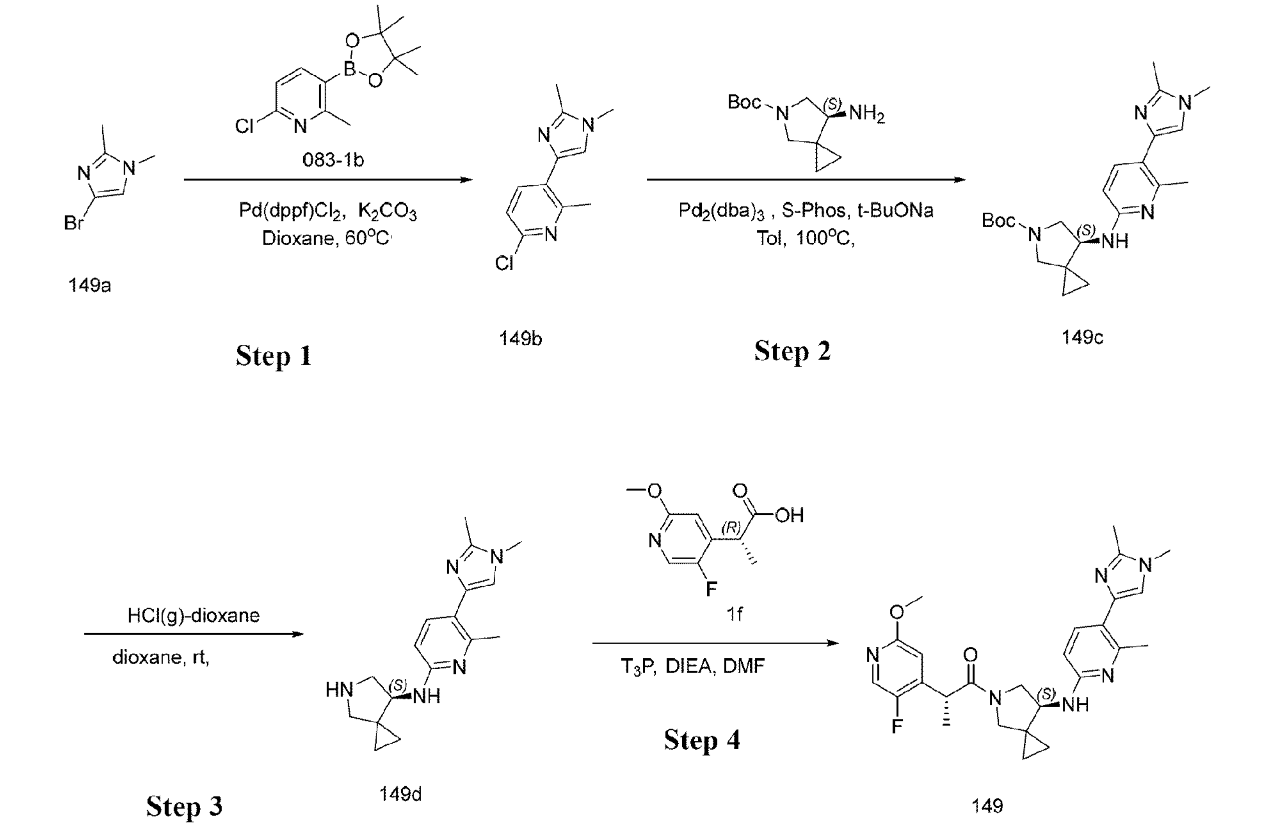


Step 1: 6-chloro-3-(1,2-dimethyl-1*H*-imidazol-4-yl)-2-methylpyridine 149b

A compound 149a (200 mg) was dissolved in a solution of 1,4-dioxane (2 mL) and water (0.2 mL), and 083-1b (144.85 mg), [1,1'-bis(diphenylphosphino)ferrocene]dichloropalladium(II) dichloromethane complex (41.81 mg), and potassium carbonate (157.92 mg) were added sequentially. The mixture was purged with nitrogen, to react at 60°C overnight. Then, the mixture was cooled down to room temperature, diluted with water (10 mL), and then subjected to extraction with ethyl acetate (3 × 10 mL). The organic phases were combined, and dried over anhydrous sodium sulfate. The resulting mixture was filtered, and then the filtrate was concentrated under reduced pressure. The resulting residue was purified by silica gel column chromatography with ethyl acetate/petroleum ether (0-100%), to give the compound 149b (130 mg).
LC-MS: (ES, m/z) = 221.90 $[M+H]^+$

Step 2: (S)-7-((5-(1,2-dimethyl-1*H*-imidazol-4-yl)-6-methylpyridine-2-yl)amino)-5-azaspiro[2.4]heptane-5-tert-butyl carboxylate 149c

To a sample vial were added sequentially the compound 149b (100 mg), (S)-7-amino-5-azaspiro[2.4]heptane-5-tert-butyl carboxylate (95.76 mg), tris(dibenzylideneacetone)dipalladium (41.31 mg), 2-dicyclohexylphosphino-2',6'-dimethoxy-1,1'-biphenyl (37.04 mg), and sodium tert-butoxide (86.70 mg), and then toluene (1 mL) was added. The mixture was purged with nitrogen, to react at 100°C for 2 h. Then, the mixture was cooled down to room temperature, diluted with water (5 mL), and then subjected to extraction with ethyl acetate (3 × 5 mL). The organic phases were combined, and dried over anhydrous sodium sulfate. The resulting mixture was filtered, and then the filtrate was concentrated under reduced pressure. The resulting residue was purified by reversed-phase column chromatography under the following conditions (C18 chromatographic column; mobile phases: water and methanol; gradient: 0% to 100% within 20 min), to give the compound 149c (30 mg).
LC-MS: (ES, m/z) = 398.25 $[M+H]^+$

Step 3: (S)-N-(5-(1,2-dimethyl-1*H*-imidazol-4-yl)-6-methylpyridine-2-yl)-5-azaspiro[2.4]hept-7-amine 149d

To a solution of hydrogen chloride in 1,4-dioxane (4 M, 1 mL) was added the compound 149c (30 mg), the mixture was stirred at room temperature for 30 min. Then, the reaction solution was concentrated under reduced pressure to give a

hydrochloride salt (20 mg) of the compound 149d. The crude product was not purified and was directly subjected to next step.
LC-MS: (ES, m/z) = 298.45 [M+H]$^+$

Step 4: (R)-1-((S)-7-((5-(1,2-dimethyl-1*H*-imidazol-4-yl)-6-methylpyridine-2-yl)amino)-5-azaspiro[2.4]hept-5-yl)-2-(5-fluoro-2-methoxypyridine-4-yl)propan-1-one 149

To a solution of the hydrochloride salt (20 mg) of the compound 149d and the compound 1f (20 mg) in N,N-dimethylformamide (1 mL) was added dropwise N,N-diisopropylethylamine (64.89 mg) at room temperature, the mixture was stirred for 5 min, and then propylphosphonic anhydride (79.87 mg) was added. The reaction mixture was stirred at room temperature for 1 h. The crude product was purified by high-performance liquid chromatography (YMC Triart C18 ExRs 5 μm, 30 mm × 150 mm; mobile phase A: water (10 mmol/L $NH_4HCO_3$), mobile phase B: ACN; flow rate: 60 mL/min; gradient: 30% B to 52% B within 10 min), to give the compound 149 (1 mg).

LC-MS: (ES, m/z) = 479.25 [M+H]$^+$
$^1$H NMR (400 MHz, DMSO-$d_6$) δ 8.12-8.08 (m, 1H), 7.72-7.66 (m, 1H), 7.09 (s, 1H), 6.72-6.67 (m, 1H), 6.56-6.43 (m, 1H), 6.42-6.31 (m, 1H), 4.16-4.02 (m, 2H), 3.82 (d, 3H), 3.74-3.67 (m, 0.5H), 3.65-3.41 (m, 6H), 3.24 (d, 0.5H), 2.39 (d, 3H), 2.30 (s, 3H), 1.36-1.26 (m, 3H), 0.88-0.75 (m, 1H), 0.69-0.42 (m, 3H).

**Example 22:** (*R*)-2-(5-fluoro-2-methoxypyridine-4-yl)-1-((*S*)-7-((6-methyl-5-(1-methyl-1*H*-imidazol-4-yl)pyridine-2-yl)amino)-5-azaspiro[2.4]hept-5-yl)propan-1-one 150

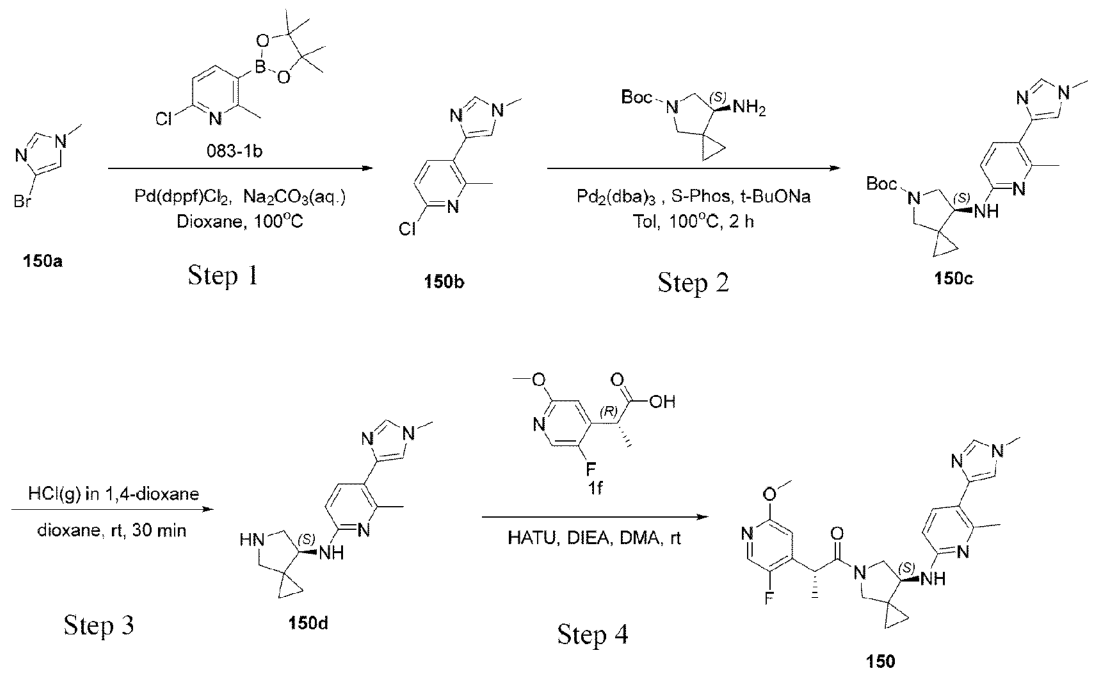

150a 150b 150c 150d 150

Step 1: 6-chloro-2-methyl-3-(1-methyl-1*H*-imidazol-4-yl)pyridine 150b

To a solution of a compound 150a (200 mg) and the compound 083-1b (314.94 mg) in 1,4-dioxane (5 mL) were added 1,1'-bis(diphenylphosphino)ferrocene dichloropalladium(II) dichloromethane complex (90.89 mg) and an aqueous sodium carbonate solution (2 M, 1 mL), the mixture was stirred at 100°C under nitrogen protection for 2 h. The reaction mixture was diluted with water (20 mL), and then subjected to extraction with ethyl acetate (20 mL × 3). The organic phases were combined, backwashed with saturated saline (20 mL), and dried over anhydrous sodium sulfate. The resulting mixture was filtered, and then concentrated under reduced pressure. The resulting residue was purified by silica gel column chromatography with petroleum ether/ethyl acetate (1:1), to give the compound 150b (45 mg).
LC-MS: (ESI, *m/z*) = 208.05 [M+H]$^+$

Step 2: (*S*)-7-((6-methyl-5-(1-methyl-1*H*-imidazol-4-yl)pyridine-2-yl)amino)-5-azaspiro[2.4]heptane-5-tert-butyl carboxylate 150c

To a solution of 150b (45 mg) and (S)-7-amino-5-azaspiro[2.4]heptane-5-tert-butyl carboxylate (46.0 mg) in toluene (2 mL) were added tris(dibenzylideneacetone)dipalladium (19.84 mg), 2-dicyclohexylphosphino-2',6'-dimethoxybiphenyl (17.79 mg), and sodium tert-butoxide (41.65 mg), the mixture was stirred at 100°C under nitrogen protection for 2 h. Then, the reaction mixture was cooled down to room temperature. The reaction mixture was diluted with water (5 mL), and then subjected to extraction with ethyl acetate (5 mL × 3). The organic phases were combined, backwashed with saturated saline (5 mL), and dried over anhydrous sodium sulfate. The resulting mixture was filtered, then concentrated under reduced pressure, and then purified by silica gel column chromatography with petroleum ether/ethyl acetate (1:1), to give the compound 150c (70 mg).
LC-MS: (ESI, *m*/*z*) = 383.95 $[M+H]^+$

Step 3: (*S*)-*N*-(6-methyl-5-(1-methyl-1*H*-imidazol-4-yl)pyridine-2-yl)-5-azaspiro[2.4]hept-7-amine 150d

To a reaction flask were added the compound 150c (70 mg) and a solution (2 mL, 4 M) of hydrogen chloride in 1,4-dioxane. The resulting mixture was stirred for 1 h. The resulting residue was concentrated under reduced pressure, to give a hydrochloride salt of the compound 150d (70 mg). The crude product was not further purified and was directly subjected to next step.
LC-MS: (ESI, *m*/*z*) = 284.15 $[M+H]^+$

Step 4: (*R*)-2-(5-fluoro-2-methoxypyridine-4-yl)-1-((*S*)-7-((6-methyl-5-(1-methyl-1*H*-imidazol-4-yl)pyridine-2-yl)amino)-5-azaspiro[2.4]hept-5-yl)propan-1-one 150

To a solution of the hydrochloride salt of 150d (50 mg) in N,N-dimethylacetamide (2 mL) were added the compound 1f (31.14 mg) and N,N-diisopropylethylamine (101.02 mg), the mixture was stirred for 5 min. Then, 2-(7-azabenzotriazol)-N,N,N',N'-tetramethyluronium hexafluorophosphate (89.16 mg) was added. The resulting residue was stirred for 1 h. The reaction solution was purified directly by high-performance liquid chromatography (chromatographic column configurations: XBridge BEH C18 OBD Prep Column, 30 mm× 150 mm, 5 μm; mobile phase A: water (10 mmol/L ammonium bicarbonate), mobile phase B: acetonitrile; flow rate: 60 mL/min; gradient: 23% B to 47% B), to give the compound 150 (10.3 mg).

LC-MS (ES+): *m*/*z* = 465.20 $[M+H]^+$
$^1$H NMR (400 MHz, DMSO-$d_6$, *ppm*) δ 8.12-8.08 (m, 1H), 7.74-7.68 (m, 1H), 7.60-7.57 (m, 1H), 7.18-7.15 (m, 1H), 6.72-6.68 (m, 1H), 6.57-6.43 (m, 1H), 6.43-6.33 (m, 1H), 4.17-4.03 (m, 2H), 3.82 (d, 3H), 3.75-3.57 (m, 5H), 3.56-3.42 (m, 1.5H), 3.24 (d, 0.5H), 2.41 (d, 3H), 1.35-1.28 (m, 3H), 0.89-0.77 (m, 1H), 0.70-0.42 (m, 3H).

**Example 23:** (R)-2-(5-chloro-2-methoxypyridine-4-yl)-1-((S)-7-((5-(4-(difluoromethyl)pyrimidine-2-yl)-6-methylpyridine-2-yl)amino)-5-azaspiro[2.4]hept-5-yl)propan-1-one 156

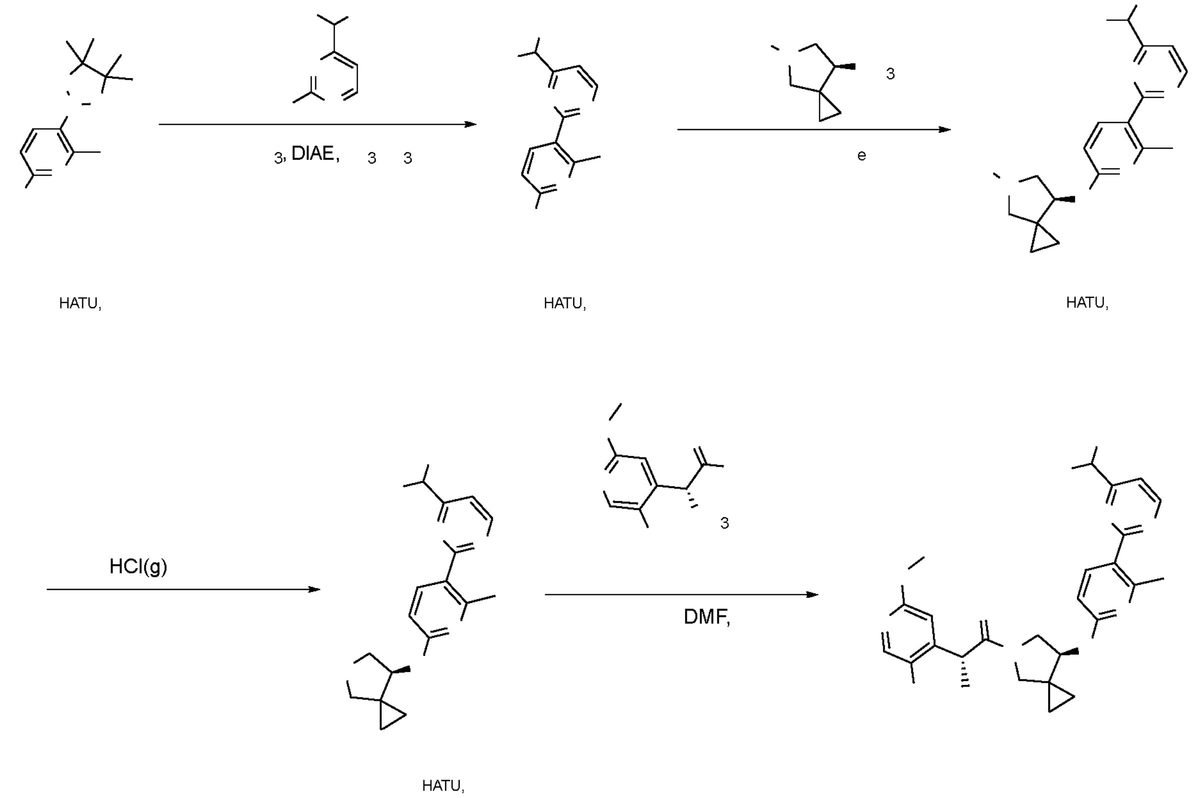


Step 1: 4-(difluoromethyl)-2-(6-fluoro-2-methylpyridine-3-yl)pyrimidine 156b

A compound 156a (1.08 g) and 2-chloro-4-(difluoromethyl)pyrimidine (500 mg) were dissolved in a mixed solution of 1,4-dioxane (10 mL) and water (3 mL), then [1,1'-bis(diphenylphosphino)ferrocene]dichloropalladium(II) dichloromethane complex (249 mg), cuprous chloride (60 mg), and sodium carbonate (970 mg) were added. The mixture was purged with nitrogen for three times, and heated to 100°C and stirred for 2 h. Then, the reaction mixture was cooled down to room temperature and diluted with water (50 mL). Then, the mixture was subjected to extraction with ethyl acetate (3 × 50 mL). The organic phases were combined, backwashed with a saturated sodium chloride solution (2 × 50 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-21%), to give the compound 156b (550 mg).
LC-MS: (ESI, *m*/*z*) = 240.05 [M+H]$^+$

Step 2: (S)-7-((5-(4-(difluoromethyl)pyrimidine-2-yl)-6-methylpyridine-2-yl)amino)-5-azaspiro[2.4]heptane-5-tert-butyl carboxylate 156c

To a reaction flask were added 156b (202 mg) and (S)-7-amino-5-azaspiro[2.4]heptane-5-tert-butyl carboxylate (150 mg) under nitrogen protection, then N,N-diisopropylethylamine (913 mg) was added. The mixture was purged with nitrogen, and finally methylpyrrolidone (3 mL) was added to react at 140°C overnight. Then, the reaction mixture was diluted with water (30 mL), and subjected to extraction with ethyl acetate (3 × 30 mL). The organic phases were combined, backwashed with a saturated aqueous sodium chloride solution (1 × 30 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-50%), to give the compound 156c (60 mg).
LC-MS: (ESI, *m*/*z*) = 432.45 [M+H]$^+$

Step 3: (S)-*N*-(5-(4-(difluoromethyl)pyrimidine-2-yl)-6-methylpyridine-2-yl)-5-azaspiro[2.4]hept-7-amine 156d

To a reaction flask were added the compound 156c (60 mg) and a solution (4 M, 1 mL) of hydrogen chloride in 1,4-dioxane at room temperature, to react at room temperature for 30 min. The reaction solution was concentrated under reduced pressure to give a hydrochloride salt (45 mg, crude product) of the compound 156d. The crude product was not

further purified and was directly subjected to next step.
LC-MS: (ESI, *m/z*) = 332.10 [M+H]+

Step 4: (R)-2-(5-chloro-2-methoxypyridine-4-yl)-1-((S)-7-((5-(4-(difluoromethyl)pyrimidine-2-yl)-6-methylpyridine-2-yl)amino)-5-azaspiro[2.4]hept-5-yl)propan-1-one 156

The compound 156d (40 mg) and the compound 083-1f (26 mg) were added to a reaction flask and dissolved in N,N-dimethylformamide (2 mL) at room temperature, then N,N-diisopropylethylamine (156 mg) was added, and stirred for 5 min. Finally, propylphosphonic anhydride (192 mg) was added, and stirred at room temperature for 2 h. The crude product was purified by high-performance liquid chromatography (chromatographic column configurations: XBridge BEH C18 column, 30 × 150, 5 μm; mobile phase A: water (10 mmol/L ammonium bicarbonate), mobile phase B: acetonitrile; flow rate: 60 mL/min; gradient: 45% B to 70% B within 8 min), to give the compound 156 (27.9 mg).

LC-MS: (ESI, *m/z*) = 528.80 [M+H]+
$^1$H NMR (400 MHz, DMSO-d6) δ 9.06-9.02 (m, 1H), 8.22 (d, 1H), 8.10-8.04 (m, 1H), 7.56-7.52 (m, 1H), 7.24-6.83 (m, 2H), 6.73 (d, 1H), 6.56-6.45 (m, 1H), 4.22-4.05 (m, 2H), 3.84 (d, 3H), 3.75-3.47 (m, 3.5H), 3.25 (d, 0.5H), 2.63 (d, 3H), 1.36-1.26 (m, 3H), 0.90-0.78 (m, 1H), 0.75-0.48 (m, 3H).

**Example 24:** (R)-2-(5-fluoro-2-methoxypyridine-4-yl)-1-((S)-7-((5-(2-(2-hydroxypropan-2-yl)-1-methyl-1H-imidazol-4-yl)-6-methylpyridine-2-yl)amino)-5-azaspiro[2.4]hept-5-yl)propan-1-one 181

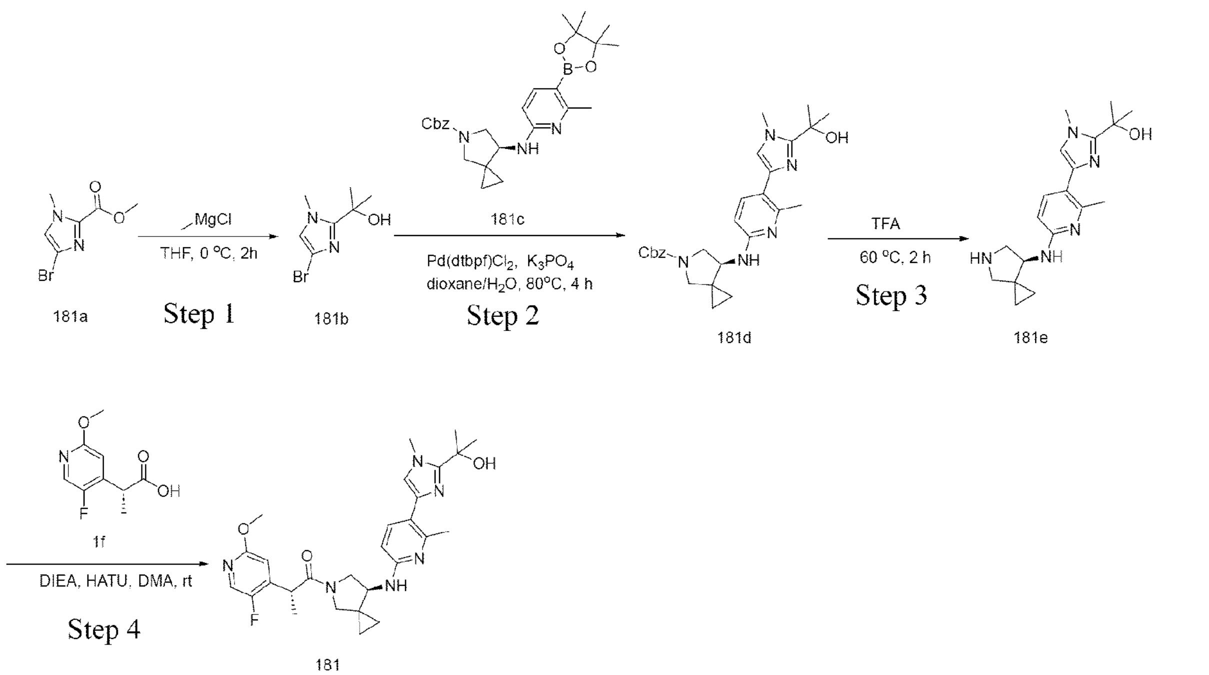


Step 1: 2-(4-bromo-1-methyl-1*H*-imidazol-2-yl)propan-2-ol 181b

To a solution of 181a (1 g) in tetrahydrofuran (15 mL) was added dropwise methylmagnesium chloride (13.5 mL, 1 M) under nitrogen protection at 0°C. After the addition, the mixture was stirred at 0°C for 2 h. Then, the reaction mixture was quenched with a saturated aqueous ammonium chloride solution (20 mL) at 0°C. The aqueous phase was subjected to extraction with ethyl acetate (3 × 30 mL). The organic phases were combined, backwashed with saturated saline (2 × 20 mL), and dried over anhydrous sodium sulfate. After the resulting mixture was filtered, the filtrate was concentrated under reduced pressure, to give the compound 181b (670 mg). The resulting mixture was not further purified and was directly subjected to next step.
LC-MS: (ESI, *m/z*) = 218.90 [M+H]+

Step 2: (S)-7-((5-(2-(2-hydroxypropan-2-yl)-1-methyl-1*H*-imidazol-4-yl)-6-methylpyridine-2-yl)amino)-5-azaspiro[2.4] heptane-5-benzyl carboxylate 181d

To a solution of 181b (189.11 mg) and 181c (200 mg) in 1,4-dioxane (1 mL)/water (0.2 mL) were added 1,1'-bis(di-tert-butylphosphino)ferrocene palladium dichloride (28.13 mg) and potassium phosphate (183.23 mg) at room temperature. After the addition, the reaction mixture was stirred at 80°C for 4 h. Then, the reaction mixture was diluted with 10 mL of water. The resulting mixture was subjected to extraction with ethyl acetate (3 × 30 mL). The organic phases were combined, backwashed with saturated saline (2 × 30 mL), and dried over anhydrous sodium sulfate. The resulting mixture was filtered, and then the filtrate was concentrated under reduced pressure. The resulting residue was purified by silica gel column chromatography with petroleum ether/ethyl acetate (2:3), to give the compound 181d (175 mg).
LC-MS: (ESI, *m/z*) = 476.10 $[M+H]^+$

Step 3: (S)-2-(4-(6-((5-azaspiro[2.4]hept-7-yl)amino)-2-methylpyridine-3-yl)-1-methyl-1H-imidazol-2-yl)propan-2-ol 181e

181d (60 mg) was dissolved in a trifluoroacetic acid (1 mL) solution, and stirred at 60°C under nitrogen protection for 2 h. The resulting residue was directly concentrated under reduced pressure, to give a hydrochloride salt (20 mg) of the compound 181e. The resulting mixture was not further purified and was directly subjected to next step.
LC-MS: (ESI, *m/z*) = 342.00 $[M+H]^+$

Step 4: (R)-2-(5-fluoro-2-methoxypyridine-4-yl)-1-((S)-7-((5-(2-(2-hydroxypropan-2-yl)-1-methyl-1*H*-imidazol-4-yl)-6-methylpyridine-2-yl)amino)-5-azaspiro[2.4]hept-5-yl)propan-1-one 181

To a solution of the hydrochloride salt (20 mg) of 181e and the compound 1f in N,N-dimethylacetamide (0.57 mL) were added dropwise N,N-diisopropylethylamine (37.85 mg) and N,N,N',N'-tetramethyl-O-(7-azabenzotriazol-1-yl)uronium hexafluorophospate (33.41 mg), the mixture was stirred at room temperature for 30 min. The reaction solution was purified by high-performance liquid chromatography (chromatographic column configurations: YMC Triart C18 ExRs 5 μm, 30 × 150 mm; mobile phase A: water (10 mmol/L ammonium bicarbonate), mobile phase B: acetonitrile; flow rate: 60 mL/min; gradient: 28% B to 51% B within 10 min), to give the compound 181 (10 mg).

LC-MS: (ESI, *m/z*) = 522.90 $[M+H]^+$
$^1$H NMR (400 MHz, DMSO-$d_6$) δ 8.12-8.08 (m, 1H), 7.74-7.69 (m, 1H), 7.07 (d, 1H), 6.72-6.68 (m, 1H), 6.51 (d, 0.5H), 6.44-6.33 (m, 1.5H), 5.26 (d, 1H), 4.16-4.03 (m, 2H), 3.85-3.79 (m, 6H), 3.75-3.68 (m, 0.5H), 3.65-3.58 (m, 1.5H), 3.55-3.41 (m, 1.5H), 3.23 (d, 0.5H), 2.41 (d, 3H), 1.53 (s, 6H), 1.35-1.27 (m, 3H), 0.89-0.77 (m, 1H), 0.70-0.41 (m, 3H).

Synthesis of 181c

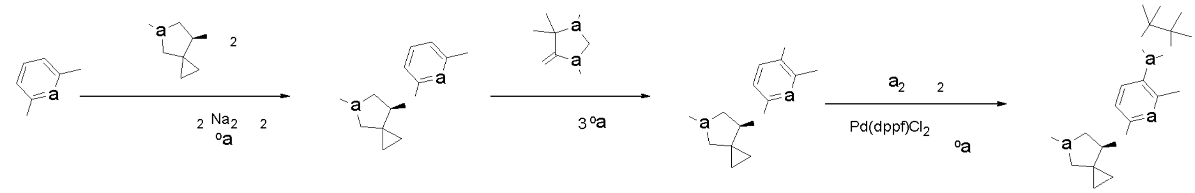


Step 1: (S)-7-((6-methylpyridine-2-yl)amino)-5-azaspiro[2.4]heptane-5-benzyl carboxylate 181g

2-bromo-6-methylpyridine 181f (2.5 g) and (S)-7-amino-5-azaspiro[2.4]heptane-5-benzyl carboxylate (3.6 g) were dissolved in 1,4-dioxane (30 mL), then methanesulfonato(2-dicyclohexylphosphino-2',6'-diisopropoxy-1,1'-biphenyl)(2'-amino-1,1'-biphenyl-2-yl)palladium(II) (1.2 g) and cesium carbonate (9.5 g) were added, heated to 90°C, and stirred under nitrogen atmosphere overnight. The reaction mixture was filtered. The filtrate was diluted with water (50 mL). Then, the mixture was subjected to extraction with ethyl acetate (3 × 100 mL). The organic phases were combined, backwashed with a saturated sodium chloride solution (2 × 100 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-25%), to give the compound 181g (2.5 g).

LC-MS: (ESI, m/z) = 338.05 $[M+H]^+$

Step 2: (S)-7-((5-bromo-6-methylpyridine-2-yl)amino)-5-azaspiro[2.4]heptane-5-benzyl carboxylate 181h

181g (2.5 g) was dissolved in dichloromethane (25 mL), and a mixed solution of 1,3-dibromo-5,5-dimethylimidazolidine-4-one (1.05 g) and dichloromethane (10 mL) was added with an ice bath, and stirred for 1 h. The reaction mixture was quenched with a saturated aqueous sodium bicarbonate solution (50 mL). Then, the mixture was subjected to extraction with dichloromethane (3 × 50 mL). The organic phases were combined, backwashed with a saturated sodium chloride solution (2 × 50 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-16%), to give the compound 181h (2.2 g).
LC-MS: (ESI, m/z) = 416.00 $[M+H]^+$

Step 3: (S)-7-((6-methyl-5-(4,4,5,5-tetramethyl-1,3,2-dioxaboran-2-yl)pyridine-2-yl)amino)-5-azaspiro[2.4]heptane-5-benzyl carboxylate 181c

181h (1.07 g) and bis(pinacolato)diboron (1.31 g) were dissolved in 1,4-dioxane (10 mL), then [1,1'-bis(diphenylphosphino)ferrocene]dichloropalladium(II) dichloromethane complex (210 mg) and potassium acetate (560 mg) were added. The mixture was purged with nitrogen for three times, and heated to 100°C and stirred for 2 h. The reaction mixture was cooled down to room temperature and then diluted with water (30 mL). Then, the mixture was subjected to extraction with ethyl acetate (3 × 50 mL). The organic phases were combined, backwashed with a saturated sodium chloride solution (2 × 20 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-33%), to give the compound 181c (780 mg).
LC-MS: (ESI, m/z) = 464.10 $[M+H]^+$

**Example** 25: (*R*)-1-((*S*)-7-((5-(2-(difluoromethoxy)-1-methyl-1*H*-imidazol-4-yl)-6-methylpyridine-2-yl)amino)-5-azaspiro[2.4]hept-5-yl)-2-(5-fluoro-2-methoxypyridine-4-yl)propan-1-one 185

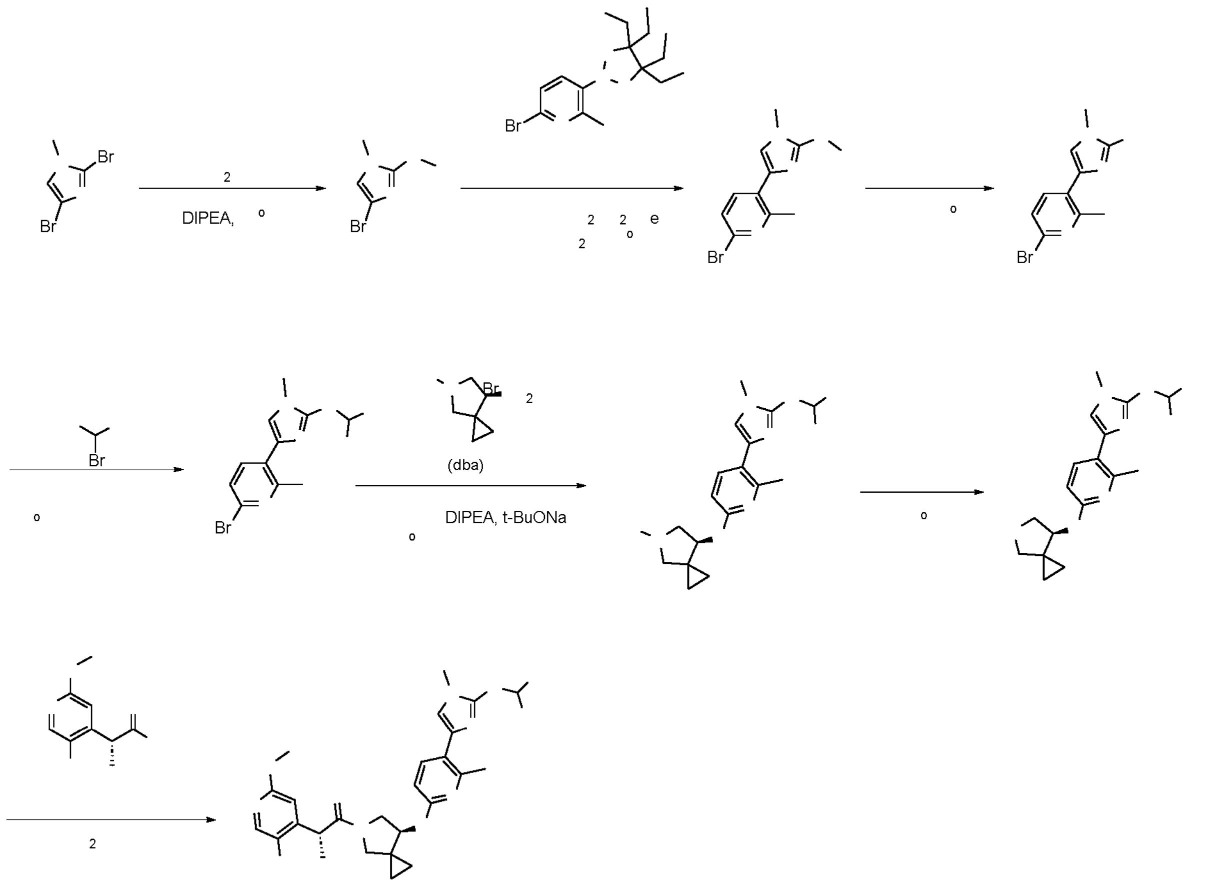


Step 1: 4-bromo-2-methoxy-1-methyl-1*H*-imidazole 185b

A compound 185a (3 g) was dissolved in anhydrous methanol (30 mL), a solution (30 wt%, 45 g) of sodium methoxide in methanol was slowly added dropwise at room temperature, and then heated to 70°C and stirred overnight. Then, the reaction mixture was cooled down to room temperature and diluted with water (100 mL). Then, the mixture was subjected to extraction with ethyl acetate (3 × 100 mL). The organic phases were combined, backwashed with a saturated sodium chloride solution (2 × 100 mL), and dried over anhydrous sodium sulfate. After the resulting mixture was filtered, the filtrate was concentrated under reduced pressure, to give the compound 185b (1.1 g).
LC-MS: (ESI, *m*/*z*) = 191.25 $[M+H]^+$

Step 2: 6-chloro-3-(2-methoxy-1-methyl-1*H*-imidazol-4-yl)-2-methylpyridine 185d

185b (600 mg) and 185c (976 mg) were dissolved in a mixed solution of 1,4-dioxane (6 mL) and water (1.5 mL), then [1,1'-bis(di-tert-butylphosphino)ferrocene]palladium dichloride (206 mg) and potassium phosphate (1.34 g) were added. The mixture was purged with nitrogen for three times, and heated to 80°C and stirred for 3 h. The reaction mixture was cooled down to room temperature and then filtered. The filtrate was diluted with water (30 mL). Then, the mixture was subjected to extraction with ethyl acetate (3 × 50 mL). The organic phases were combined, backwashed with a saturated sodium chloride solution (2 × 30 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-18%), to give the compound 185d (350 mg).
LC-MS: (ESI, *m*/*z*) = 238.00 $[M+H]^+$

Step 3: 4-(6-chloro-2-methylpyridine-3-yl)-1-methyl-1*H*-imidazol-2-ol 185e

185d (350 mg) was dissolved in tetrahydrofuran (4 mL), then a dilute hydrochloric acid solution (1 M, 4 mL) was added, and heated to 60°C and stirred for 1 h. After the reaction mixture was cooled down to room temperature, a saturated sodium bicarbonate solution was added to adjust pH to weak acidity. Then, the mixture was subjected to extraction with ethyl acetate (3 × 30 mL). The organic phases were combined, backwashed with a saturated sodium chloride solution (2 × 30 mL), and dried over anhydrous sodium sulfate. After the resulting mixture was filtered, the filtrate was concentrated

under reduced pressure, to give the compound 185e (300 mg).
LC-MS: (ESI, *m*/*z*) = 224.00 $[M+H]^+$

Step 4: 6-chloro-3-(2-(difluoromethoxy)-1-methyl-1*H*-imidazol-4-yl)-2-methylpyridine 185f

185e (300 mg) was dissolved in 1,4-dioxane (10 mL), a sodium hydroxide solution (20 wt%, 10 mL) was added, heated to 65°C, with monochlorodifluoromethane gas introduced, and stirred at 65°C for 1 h. The reaction mixture was cooled down to room temperature and then diluted with water (30 mL). Then, the mixture was subjected to extraction with ethyl acetate (3 × 50 mL). The organic phases were combined, backwashed with a saturated sodium chloride solution (2 × 30 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-18%), to give the compound 185f (230 mg).
LC-MS: (ESI, *m*/*z*) = 273.95 $[M+H]^{+7}$

Step 5: (S)-7-((5-(2-(difluoromethoxy)-1-methyl-1*H*-imidazol-4-yl)-6-methylpyridine-2-yl)amino)-5-azaspiro[2.4]heptane-5-benzyl carboxylate 185h

185f (200 mg) and 185g (198 mg) were dissolved in toluene (2 mL), then tris(dibenzylideneacetone)dipalladium (67 mg), 2-dicyclohexylphosphino-2',6'-dimethoxy-biphenyl (60 mg), and sodium tert-butoxide (140 mg) were added. The mixture was purged with nitrogen for three times, and heated to 90°C and stirred for 1 h. The reaction mixture was cooled down to room temperature and then filtered. The filtrate was diluted with water (30 mL). Then, the mixture was subjected to extraction with ethyl acetate (3 × 50 mL). The organic phases were combined, backwashed with a saturated sodium chloride solution (2 × 30 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-38%), to give the compound 185h (240 mg).
LC-MS: (ESI, *m*/*z*) = 484.10 $[M+H]^+$

Step 6: (S)-N-(5-(2-(difluoromethoxy)-1-methyl-1*H*-imidazol-4-yl)-6-methylpyridine-2-yl)-5-azaspiro[2.4]hept-7-amine 185i

To a reaction flask was added 185h (240 mg), and trifluoroacetic acid (5 mL) was added at room temperature, and heated to 60°C and stirred for 1 h. The reaction mixture was concentrated under reduced pressure to give a trifluoroacetate salt (180 mg, crude product) of the compound 185i. The crude product was not further purified and was directly subjected to next step.
LC-MS: (ESI, *m*/*z*) = 350.05 $[M+H]^+$

Step 7: (R)-1-((S)-7-((5-(2-(difluoromethoxy)-1-methyl-1*H*-imidazol-4-yl)-6-methylpyridine-2-yl)amino)-5-azaspiro[2.4]hept-5-yl)-2-(5-fluoro-2-methoxypyridine-4-yl)propan-1-one 185

The trifluoroacetate salt (90 mg, crude product) of 185i and the compound 1f (50 mg) were dissolved in N,N-dimethylacetamide (2 mL), and N,N-diisopropylethylamine (322 mg) was added to adjust the mixture to be basic. Then, 1-propylphosphonic anhydride (in 50 wt% ethyl acetate solution, 800 mg) was added, and stirred at room temperature for 1 h. The resulting mixture was filtered. The filter cake was washed with N,N-dimethylacetamide (2 × 1 mL). The crude product was purified by high-performance liquid chromatography (chromatographic column configurations: Kinetex 5 μm EVO C18, 30 × 150 mm; mobile phase A: water (10 mmol/L ammonium bicarbonate), mobile phase B: acetonitrile; flow rate: 60 mL/min; gradient: 39% B to 54% B within 10 min), to give the compound 185 (46.67 mg).

LC-MS: (ESI, *m*/*z*) = 530.90 $[M+H]^+$
$^1$H NMR (400 MHz, DMSO-$d_6$) δ 8.12-8.08 (m, 1H), 7.71-7.31 (m, 2H), 7.01 (d, 1H), 6.73-6.68 (m, 1H), 6.64-6.49 (m, 1H), 6.45-6.33 (m, 1H), 4.16-4.03 (m, 2H), 3.82 (d, 3H), 3.75-3.41 (m, 6.5H), 3.23 (d, 0.5H), 2.40 (d, 3H), 1.36-1.27 (m, 3H), 0.89-0.76 (m, 1H), 0.70-0.42 (m, 3H).

Synthesis of the intermediate 185c

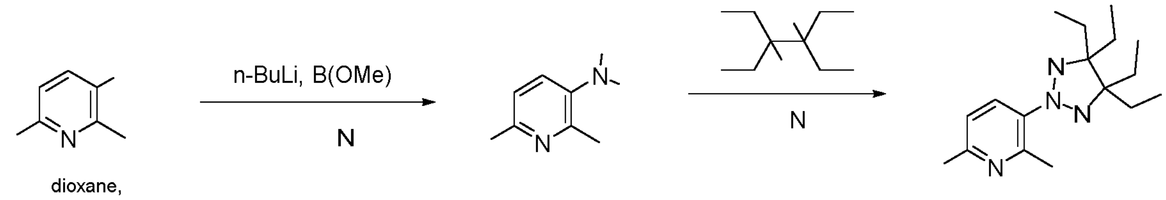


Step 1: (6-chloro-2-methylpyridine-3-yl)boronic acid 185j

To a 2 L three-necked flask were added 083-1a (50 g) and tetrahydrofuran (500 mL) under nitrogen protection, and the mixture was cooled down to -78°C. An n-butyllithium solution (2.5 mol/L, in a tetrahydrofuran/n-hexane solution, 117 mL) was added dropwise at -78°C, and then stirred at -78°C for 40 min. Then, trimethyl borate (50.33 g) was added dropwise at -78°C to react at -78°C for 60 min. Then, a saturated aqueous ammonium chloride solution (400 mL) was added dropwise for quenching. The mixture was subjected to extraction with ethyl acetate (2 × 300 mL). The organic phases were combined. The aqueous phase was then adjusted with hydrochloric acid (2 M) to pH = 6. The aqueous phase was subjected to extraction with methanol/dichloromethane (1/5) (4 × 500 mL). The organic phases were combined, dried over anhydrous sodium sulfate, and concentrated, to give the compound 185j (42 g, crude product). The crude product was not further purified and was directly subjected to next step.
LC-MS: (ES, m/z) = 172.05 $[M+H]^+$

Step 2: 6-chloro-2-methyl-3-(4,4,5,5-tetraethyl-1,3,2-dioxaboran-2-yl)pyridine 185c

185j (17 g) and 3,4-diethylhexane-3,4-diol (15.56 g) were dissolved in a dichloromethane (170 mL) solution, to react at room temperature for 12 h. The reaction mixture was diluted with water (150 mL), and subjected to extraction with dichloromethane (4 × 150 mL). The organic phases were combined, and dried over anhydrous sodium sulfate. The resulting mixture was filtered, and then the filtrate was concentrated under reduced pressure. The resulting residue was purified by silica gel column chromatography with ethyl acetate/petroleum ether (0-15%), to give the compound 185c (20 g).
LC-MS: (ESI, *m/z*) = 310.50 $[M+H]^+$

Synthesis of the intermediate 185g

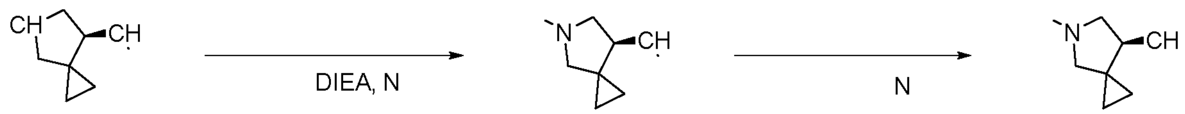


**115c**

Step 1: (*S*)-7-((tert-butoxycarbonyl)amino)-5-azaspiro[2.4]heptane-5-benzyl carboxylate 185l

At 0°C, 185k (30 g) and triethylamine (42.90 g) were added to a 1 L three-necked flask and dissolved in dichloromethane (300 mL), and benzyl chloroformate (36.16 g) was added dropwise. After the addition, the mixture was stirred at room temperature for 2 h. The reaction mixture was quenched with ice water (100 mL) at room temperature. The reaction mixture was subjected to extraction with ethyl acetate (3 × 300 mL). The organic phases were combined, backwashed with saturated saline (1 × 100 mL), and dried over anhydrous sodium sulfate. The resulting mixture was filtered, and then the filtrate was concentrated under reduced pressure. The resulting residue was purified by silica gel column chromatography with petroleum ether/ethyl acetate (3:2), to give the compound 185l (46.0 g).
LC-MS: (ES, m/z) = 347.10 $[M+H]^+$

Step 2: (S)-7-amino-5-azaspiro[2.4]heptane-5-benzyl carboxylate 185g

At 0°C, to a 1 L three-necked flask was added 185l (46 g) in 1,4-dioxane (100 mL), and a solution of hydrogen chloride in 1,4-dioxane (4 M, 150 mL) was added dropwise. After the addition, the mixture was stirred at room temperature

for 1 h. The reaction mixture was quenched with a saturated aqueous sodium bicarbonate solution at room temperature. The reaction mixture was subjected to extraction with ethyl acetate (3 × 500 mL). The organic phases were combined, backwashed with saturated saline (1 × 300 mL), and dried over anhydrous sodium sulfate. After the resulting mixture was filtered, the filtrate was concentrated under reduced pressure, to give the compound 185g (28.0 g).
LC-MS: (ES, m/z) = 247.05 $[M+H]^+$

**Example 26:** (R)-1-((S)-7-((5-(7,7-difluoro-6,7-dihydro-5H-pyrrolo[1,2-a]imidazol-2-yl)-6-methylpyridine-2-yl)amino)-5-azaspiro[2.4]hept-5-yl)-2-(5-fluoro-2-methoxypyridine-4-yl)propan-1-one 189

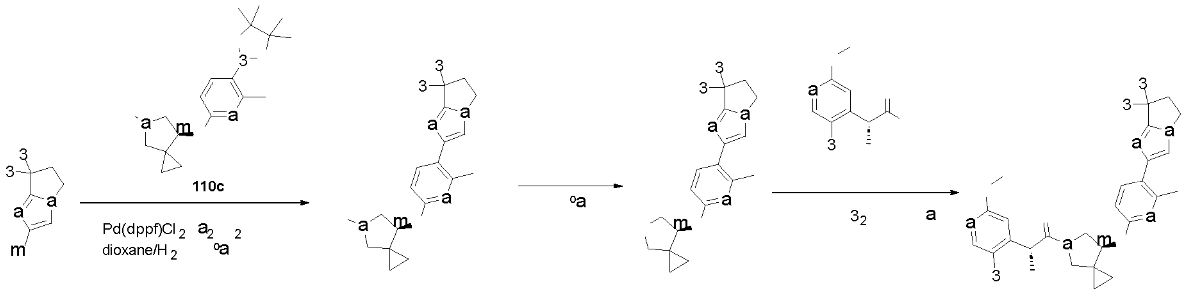


110c 110c 110c

Step 1: (S)-7-((5-(7,7-difluoro-6,7-dihydro-5H-pyrrolo[1,2-a]imidazol-2-yl)-6-methylpyridine-2-yl)amino)-5-azaspiro[2.4] heptane-5-benzyl carboxylate 189b

189a (300 mg, prepared with reference to the patent "WO2023280254A1") and 181c (626 mg) were dissolved in a mixed solution of 1,4-dioxane (4 mL) and water (1 mL), then [1,1'-bis(di-tert-butylphosphino)ferrocene]palladium dichloride (91 mg) and potassium phosphate (572 mg) were added. The mixture was purged with nitrogen for three times, and heated to 80°C and stirred for 1 h. The reaction mixture was cooled down to room temperature and then diluted with water (30 mL). Then, the mixture was subjected to extraction with ethyl acetate (3 × 50 mL). The organic phases were combined, backwashed with a saturated sodium chloride solution (2 × 30 mL), and dried over anhydrous sodium sulfate. The resulting mixture was filtered, and then the filtrate was concentrated under reduced pressure. The resulting residue was purified by silica gel column chromatography with the eluent of ethyl acetate/petroleum ether (0-100%), to give the compound 189b (400 mg).
LC-MS: (ESI, *m/z*) = 480.10 $[M+H]^+$

Step 2: (S)-N-(5-(7,7-difluoro-6,7-dihydro-5H-pyrrolo[1,2-a]imidazol-2-yl)-6-methylpyridine-2-yl)-5-azaspiro[2.4]hept-7-amine 189c

To a reaction flask was added 189b (200 mg), and trifluoroacetic acid (5 mL) was added at room temperature, and heated to 60°C and stirred for 1 h. The reaction mixture was concentrated under reduced pressure to give the compound 189c (160 mg, crude product). The crude product was not further purified and was directly subjected to next step.
LC-MS: (ESI, *m/z*) = 346.00 $[M+H]^+$

Step 3: (R)-1-((S)-7-((5-(7,7-difluoro-6,7-dihydro-5H-pyrrolo[1,2-a]imidazol-2-yl)-6-methylpyridine-2-yl)amino)-5-azaspiro[2.4]hept-5-yl)-2-(5-fluoro-2-methoxypyridine-4-yl)propan-1-one 189

189c (80 mg, crude product) and the compound 1f (42 mg) were dissolved in N,N-dimethylacetamide (2 mL), and N,N-diisopropylethylamine (271 mg) was added to adjust the mixture to be basic. Then, 1-propylphosphonic anhydride (in 50 wt% ethyl acetate solution, 334 mg) was added, and stirred at room temperature for 1 h. The resulting mixture was filtered. The filter cake was washed with N,N-dimethylacetamide (2 × 1 mL). The crude product was purified by high-performance liquid chromatography (chromatographic column configurations: XBridge BEH Shield RP18 5 μm, 30 × 150 mm; mobile phase A: water (10 mmol/L ammonium bicarbonate), mobile phase B: acetonitrile; flow rate: 60 mL/min; gradient: 32% B to 50% B within 8 min), to give the compound 189 (45.3 mg).

LC-MS: (ESI, *m/z*) = 526.90 $[M+H]^+$
$^1H$ NMR (400 MHz, DMSO-$d_6$) δ 8.13-8.08 (m, 1H), 7.70-7.64 (m, 1H), 7.43 (d, 1H), 6.74-6.58 (m, 2H), 6.46-6.35 (m,

1H), 4.29-4.22 (m, 2H), 4.17-4.04 (m, 2H), 3.82 (d, 3H), 3.75-3.42 (m, 3.5H), 3.24 (d, 0.5H), 3.20-3.07 (m, 2H), 2.43 (d, 3H), 1.36-1.27 (m, 3H), 0.89-0.77 (m, 1H), 0.71-0.42 (m, 3H).

The compounds shown in Table 1 were prepared using methods similar to those in Example 11, Example 14, Example 16, Examples 24-26, or the like. The structural characterization data of these compounds are listed together in Table 1.

Table 1

| Compound No. | Structure | Name | $[M+H]^+$ | $^1$H-NMR data |
|---|---|---|---|---|
| 001-1 | 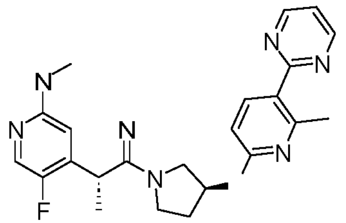 | (*R*)-2-(5-fluoro-2-methoxypyridine-4-yl)-1-((*S*)-3-((6-methyl-5-(pyrimidine-2-yl)pyridine-2-yl)amino)pyrrolidine-1-yl)propan-1-one | 436.90 | $^1$H NMR (400 MHz, DMSO-$d_6$) δ 8.84-8.80 (d, 2H), 8.14-8.09 (m, 1H), 8.05-8.01 (m, 1H), 7.33-7.28 (m, 1H), 7.17-7.00 (m, 1H), 6.74-6.66 (m, 1H), 6.47-6.39 (m, 1H), 4.41-4.32 (m, 1H), 4.19-4.04 (m, 1H), 3.82 (d, 3H), 3.77-3.38 (m, 3H), 3.27-3.19 (m, 1H), 2.63 (s, 1.5H), 2.59 (s, 1.5H), 2.29-1.81 (m, 2H), 1.38-1.28 (m, 3H) |
| 051-2 | 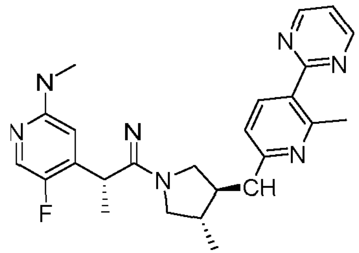 | (*R*)-2-(5-fluoro-2-methoxypyridine-4-yl)-1-((3*R*,4*S*)-3-methyl-4-((6-methyl-5-(pyrimidine-2-yl)pyridine-2-yl)amino)pyrrolidine-1-yl)propan-1-one | 450.95 | $^1$H NMR (400 MHz, DMSO-$d_6$) δ 8.84-8.81 (m, 2H), 8.14-8.10 (m, 1H), 8.05-8.00 (m, 1H), 7.33-7.28 (m, 1H), 7.16-6.99 (m, 1H), 6.73-6.67 (m, 1H), 6.48-6.42 (m, 1H), 4.17-3.78 (m, 6H), 3.75-3.53 (m, 1H), 3.37-3.22 (m, 1H), 3.04-2.94 (m, 1H), 2.63 (s, 1.3H), 2.55 (s, 1.7H), 2.27-2.14 (m, 1H), 1.37-1.28 (m, 3H), 1.08-1.00 (m, 3H) |
| 145 | 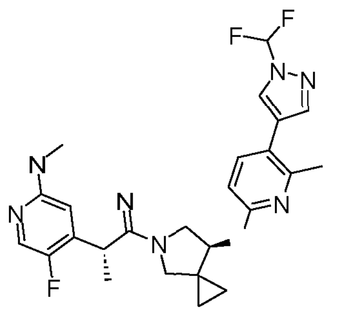 | (*R*)-1-((*S*)-7-((5-(1-(difluoromethyl)-1*H*-pyrazol-4-yl)-6-methylpyridine-2-yl)amino)-5-azaspiro[2.4]hept-5-yl)-2-(5-fluoro-2-methoxypyridine-4-yl)propan-1-one | 501.20 | $^1$H NMR (400 MHz, DMSO-$d_6$) δ 8.30 (d, 1H), 8.12-8.09 (m, 1H), 7.96-7.65 (m, 2H), 7.45-7.38 (m, 1H), 6.75-6.62 (m, 2H), 6.47-6.36 (m, 1H), 4.17-4.04 (m, 2H), 3.82 (d, 3H), 3.75-3.43 (m, 3.5H), 3.24 (d, 0.5H), 2.35 (d, 3H), 1.35-1.28 (m, 3H), 0.88-0.76 (m, 1H), 0.71-0.44 (m, 3H). |

(continued)

| Compound No. | Structure | Name | [M+H]+ | 1H-NMR data |
|---|---|---|---|---|
| 146 | 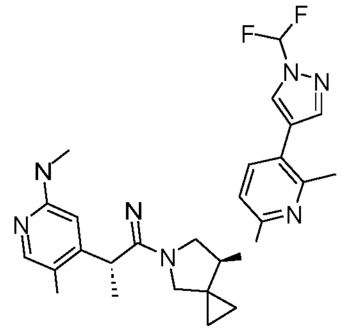 | (*R*)-2-(5-chloro-2-methoxypyridine-4-yl)-1-((*S*)-7-((5-(1-(difluoromethyl)-1*H*-pyrazol-4-yl)-6-methylpyridine-2-yl)amino)-5-azaspiro[2.4]hept-5-yl)propan-1-one | 517.20 | 1H NMR (400 MHz, DMSO-$d_6$) δ 8.30 (d, 1H), 8.23 (s, 0.5H), 8.19 (s, 0.5H), 7.97-7.65 (m, 2H), 7.44-7.38 (m, 1H), 6.75-6.62 (m, 2H), 6.47-6.36 (m, 1H), 4.14-4.00 (m, 2H), 3.84 (d, 3H), 3.71-3.47 (m, 3.5H), 3.25 (d, 0.5H), 2.35 (d, 3H), 1.35-1.25 (m, 3H), 0.87-0.76 (m, 1H), 0.72-0.45 (m, 3H). |
| 147 | 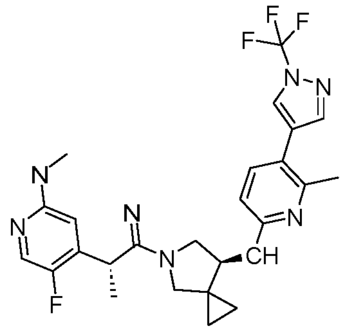 | (*R*)-2-(5-fluoro-2-methoxypyridine-4-yl)-1-((*S*)-7-((6-methyl-5-(1-(trifluoromethyl)-1*H*-pyrazol-4-yl)pyridine-2-yl)amino)-5-azaspiro[2.4]hept-5-yl)propan-1-one | 518.95 | 1H NMR (400 MHz, DMSO-$d_6$) δ 8.53 (d, 1H), 8.14-8.09 (m, 2H), 7.46-7.40 (m, 1H), 6.81-6.67 (m, 2H), 6.47-6.37 (m, 1H), 4.17-4.04 (m, 2H), 3.82 (d, 3H), 3.75-3.60 (m, 2H), 3.55-3.43 (m, 1.5H), 3.24 (d, 0.5H), 2.36 (d, 3H), 1.36-1.28 (m, 3H), 0.86-0.76 (m, 1H), 0.71-0.44 (m, 3H). |
| 148 | 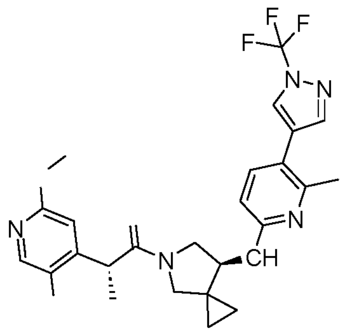 | (*R*)-2-(5-chloro-2-methoxypyridine-4-yl)-1-((*S*)-7-((6-methyl-5-(1-(trifluoromethyl)-1*H*-pyrazol-4-yl)pyridine-2-yl)amino)-5-azaspiro[2.4]hept-5-yl)propan-1-one | 534.90 | 1H NMR (400 MHz, DMSO-$d_6$) δ 8.53 (d, 1H), 8.23 (s, 0.5H), 8.19 (s, 0.5H), 8.12 (d, 1H), 7.46-7.40 (m, 1H), 6.81-6.68 (m, 2H), 6.48-6.37 (m, 1H), 4.14-4.00 (m, 2H), 3.84 (d, 3H), 3.70-3.47 (m, 3.5H), 3.25 (d, 0.5H), 2.36 (d, 3H), 1.35-1.26 (m, 3H), 0.86-0.76 (m, 1H), 0.72-0.46 (m, 3H). |

(continued)

| Compound No. | Structure | Name | $[M+H]^+$ | $^1$H-NMR data |
|---|---|---|---|---|
| 155 | 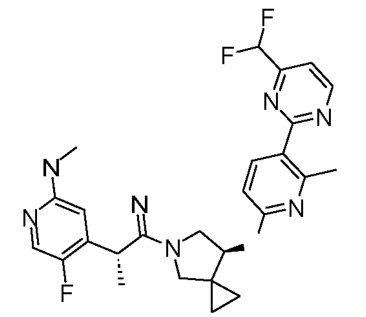 | (*R*)-1-((*S*)-7-((5-(4-(difluoromethyl)pyrimidine -2-yl)-6-methylpyridine-2-yl)amino)-5-azaspiro[2.4]hept-5-yl)-2-(5-fluoro-2-methoxypyridine-4-yl)propan-1-one | 512.90 | $^1$H NMR (400 MHz, DMSO-$d_6$) δ 9.06-9.02 (m, 1H), 8.14-8.04 (m, 2H), 7.57-7.53 (m, 1H), 7.25-6.84 (m, 2H), 6.73-6.69 (m, 1H), 6.55-6.45 (m, 1H), 4.24-4.05 (m, 2H), 3.82 (d, 3H), 3.78-3.60 (m, 2H), 3.56-3.46 (m, 1.5H), 3.23 (d, 0.5H), 2.63 (d, 3H), 1.36-1.28 (m, 3H), 0.88-0.79 (m, 1H), 0.74-0.47 (m, 3H). |
| 165 | 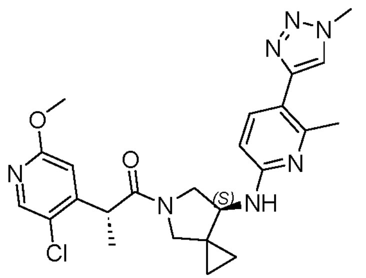 | (*R*)-2-(5-chloro-2-methoxypyridine-4-yl)-1-((*S*)-7-((6-methyl-5-(1-methyl-1*H*-1,2,3-triazol-4-yl)pyridine-2-yl)amino)-5-azaspiro[2.4]hept-5-yl)propan-1-one | 481.85 | $^1$H NMR (400 MHz, DMSO-$d_6$) δ 8.23 (s, 0.5H), 8.19 (s, 0.5H), 8.15 (d, 1H), 7.70-7.65 (m, 1H), 6.80-6.67 (m, 2H), 6.50-6.39 (m, 1H), 4.16-4.00 (m, 5H), 3.84 (d, 3H), 3.72-3.47 (m, 3.5H), 3.25 (d, 0.5H), 2.40 (d, 3H), 1.35-1.26 (m, 3H), 0.88-0.77 (m, 1H), 0.73-0.45 (m, 3H). |
| 166 | 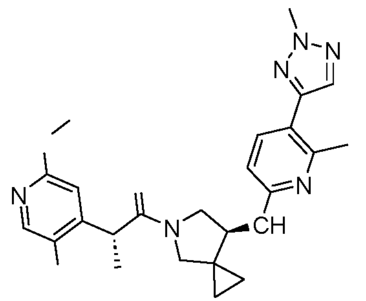 | (*R*)-2-(5-chloro-2-methoxypyridine-4-yl)-1-((*S*)-7-((6-methyl-5-(2-methyl-2*H*-1,2,3-triazol-4-yl)pyridine-2-yl)amino)-5-azaspiro[2.4]hept-5-yl)propan-1-one | 481.90 | $^1$H NMR (400 MHz, DMSO-$d_6$) δ 8.23 (s, 0.5H), 8.19 (s, 0.5H), 7.87 (d, 1H), 7.62-7.55 (m, 1H), 6.87-6.74 (m, 1H), 6.72 (d, 1H), 6.50-6.38 (m, 1H), 4.19-4.02 (m, 5H), 3.84 (d, 3H), 3.74-3.46 (m, 3.5H), 3.25 (d, 0.5H), 2.43 (d, 3H), 1.37-1.25 (m, 3H), 0.90-0.77 (m, 1H), 0.73-0.45 (m, 3H). |

(continued)

| Compound No. | Structure | Name | $[M+H]^+$ | $^1$H-NMR data |
|---|---|---|---|---|
| 167 | 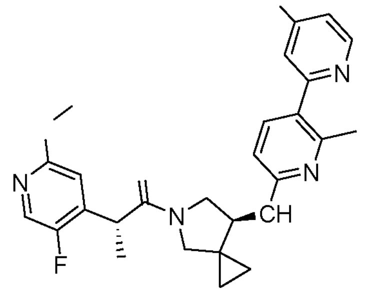 | 6'-(((S)-5-((R)-2-(5-fluoro-2-methoxypyridine-4-yl) propionyl)-5-azaspiro[2.4]hept-7-yl)amino)-2'-methyl-[2,3'-bipyridyl]-4-carbonitrile | 487.10 | $^1$H NMR (400 MHz, DMSO-$d_6$) δ 8.85-8.82 (m, 1H), 8.13-8.09 (m, 1H), 7.98-7.95 (m, 1H), 7.73-7.68 (m, 1H), 7.60-7.54 (m, 1H), 7.04-6.89 (m, 1H), 6.74-6.68 (m, 1H), 6.53-6.42 (m, 1H), 4.22-4.04 (m, 2H), 3.82 (d, 3H), 3.77-3.59 (m, 2H), 3.57-3.45 (m, 1.5H), 3.25 (d, 0.5H), 2.40 (d, 3H), 1.37-1.28 (m, 3H), 0.90-0.78 (m, 1H), 0.74-0.45 (m, 3H). |
| 168 | 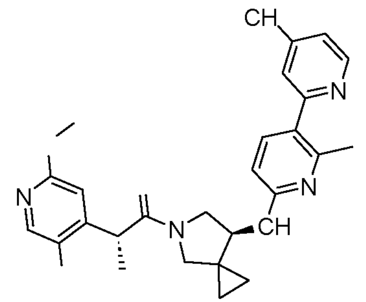 | 6'-(((S)-5-((R)-2-(5-chloro-2-methoxypyridine-4-yl) propionyl)-5-azaspiro[2.4]hept-7-yl)amino)-2'-methyl-[2,3'-bipyridyl]-4-carbonitrile | 503.05 | $^1$H NMR (400 MHz, DMSO-$d_6$) δ 8.84 (d, 1H), 8.24 (s, 0.5H), 8.20 (s, 0.5H), 7.99-7.94 (m, 1H), 7.73-7.68 (m, 1H), 7.61-7.53 (m, 1H), 7.04-6.89 (m, 1H), 6.73 (d, 1H), 6.53-6.42 (m, 1H), 4.19-4.03 (m, 2H), 3.84 (d, 3H), 3.74-3.47 (m, 3.5H), 3.26 (d, 0.5H), 2.40 (d, 3H), 1.38-1.24 (m, 3H), 0.89-0.78 (m, 1H), 0.75-0.47 (m, 3H). |
| 169 | 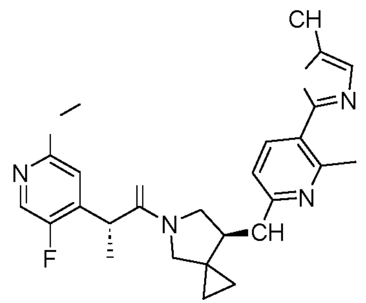 | 2-(6-(((S)-5-((R)-2-(5-fluoro-2-methoxypyridine-4-yl) propionyl)-5-azaspiro[2.4]heptan-7-yl)amino)-2-methylpyridine-3-yl)oxazole-5-carbonitrile | 477.10 | $^1$H NMR (400 MHz, DMSO-$d_6$) δ 8.33 (d, 1H), 8.13-8.08 (m, 1H), 7.98-7.90 (m, 1H), 7.63-7.47 (m, 1H), 6.74-6.67 (m, 1H), 6.58-6.46 (m, 1H), 4.25-4.04 (m, 2H), 3.82 (d, 3H), 3.78-3.45 (m, 3.5H), 3.22 (d, 0.5H), 2.65 (d, 3H), 1.38-1.25 (m, 3H), 0.87-0.75 (m, 1H), 0.74-0.48 (m, 3H). |

(continued)

| Compound No. | Structure | Name | [M+H]+ | 1H-NMR data |
|---|---|---|---|---|
| 170 | 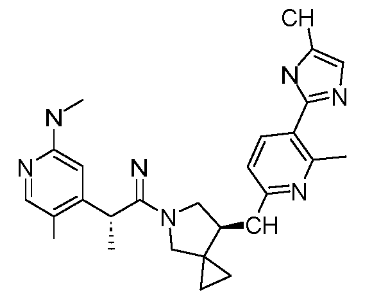 | 2-(6-(((S)-5-((R)-2-(5-chloro-2-methoxypyridine-4-yl)propionyl)-5-azaspiro[2.4]heptan-7-yl)amino)-2-methylpyridine-3-yl)oxazole-5-carbonitrile | 493.05 | $^1$H NMR (400 MHz, DMSO-$d_6$) δ 8.33 (d, 1H), 8.23 (s, 0.5H), 8.20 (s, 0.5H), 7.98-7.90 (m, 1H), 7.63-7.48 (m, 1H), 6.72 (d, 1H), 6.58-6.47 (m, 1H), 4.21-4.03 (m, 2H), 3.84 (d, 3H), 3.74-3.45 (m, 3.5H), 3.23 (d, 0.5H), 2.65 (d, 3H), 1.37-1.24 (m, 3H), 0.88-0.76 (m, 1H), 0.76-0.49 (3H). |
| 182 | 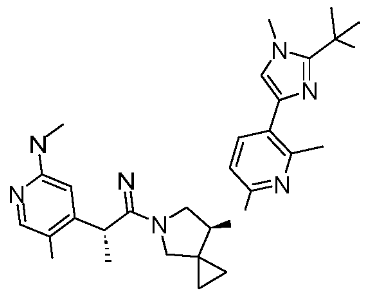 | (R)-2-(5-chloro-2-methoxypyridine-4-yl)-1-((S)-7-((5-(2-(2-hydroxypropan-2-yl)-1-methyl-1*H*-imidazol-4-yl)-6-methylpyridine-2-yl)amino)-5-azaspiro[2.4]hept-5-yl)propan-1-one | 538.90 | $^1$H NMR (400 MHz, DMSO-$d_6$) δ 8.23 (s, 0.5H), 8.19 (s, 0.5H), 7.75-7.68 (m, 1H), 7.10-7.04 (m, 1H), 6.72 (d, 1H), 6.55-6.49 (m, 0.5H), 6.47-6.33 (m, 1.5H), 5.27 (s, 1H), 4.13-3.98 (m, 2H), 3.87-3.78 (m, 6H), 3.71-3.46 (m, 3.5H), 3.24 (d, 0.5H), 2.40 (d, 3H), 1.53 (s, 6H), 1.35-1.23 (m, 3H), 0.87-0.77 (m, 1H), 0.71-0.43 (m, 3H). |
| 184 | 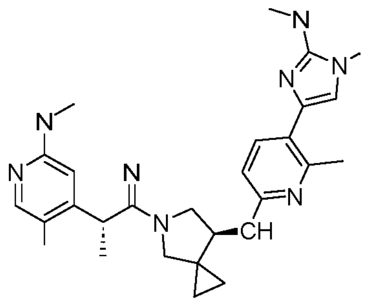 | (R)-2-(5-chloro-2-methoxypyridine-4-yl)-1-((S)-7-((5-(2-methoxy-1-methyl-1*H*-imidazol-4-yl)-6-methylpyridine-2-yl)amino)-5-azaspiro[2.4]hept-5-yl)propan-1-one | 510.90 | $^1$H NMR (400 MHz, DMSO-$d_6$) δ 8.23 (s, 0.5H), 8.19 (s, 0.5H), 7.74-7.68 (m, 1H), 6.79 (d, 1H), 6.72 (d, 1H), 6.55-6.33 (m, 2H), 4.13-4.00 (m, 2H), 3.95 (s, 3H), 3.84 (d, 3H), 3.71-3.46 (m, 3.5H), 3.39-3.36 (m, 3H), 3.24 (d, 0.5H), 2.39 (d, 3H), 1.35-1.25 (m, 3H), 0.88-0.76 (m, 1H), 0.71-0.43 (m, 3H) |

(continued)

| Compound No. | Structure | Name | $[M+H]^+$ | $^1$H-NMR data |
|---|---|---|---|---|
| 186 | 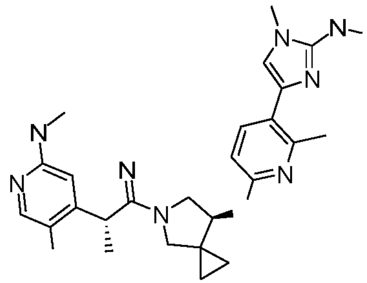 | (R)-2-(5-chloro-2-methoxypyridine-4-yl)-1-((S)-7-((5-(2-(difluoromethoxy)-1-methyl-1*H*-imidazol-4-yl)-6-methylpyridine-2-yl)amino)-5-azaspiro[2.4]hept-5-yl)propan-1-one | 546.90 | $^1$H NMR (400 MHz, DMSO-$d_6$) δ 8.23 (s, 0.5H), 8.19 (s, 0.5H), 7.70-7.31 (m, 2H), 7.01 (d, 1H), 6.72 (d, 1H), 6.63-6.49 (m, 1H), 6.45-6.34 (m, 1H), 4.14-4.00 (m, 2H), 3.84 (d, 3H), 3.72-3.45 (m, 6.5H), 3.24 (d, 0.5H), 2.39 (d, 3H), 1.35-1.25 (m, 3H), 0.90-0.75 (m, 1H), 0.71-0.43 (m, 3H). |
| 187 | 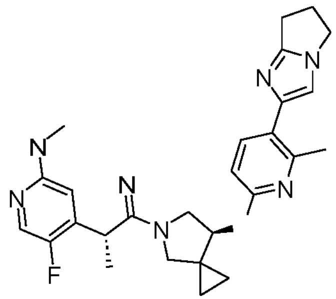 | (R)-1-((S)-7-((5-(6,7-dihydro-5H-pyrrolo[1,2-a]imidazol-2-yl)-6-methylpyridine-2-yl)amino)-5-azaspiro[2.4]hept-5-yl)-2-(S-fluoro-2-methoxypyridine-4-yl)propan-1-one | 491.15 | $^1$H NMR (400 MHz, Chloroform-d) δ 7.98-7.89 (m, 2H), 6.93-6.89 (m, 1H), 6.79-6.75 (m, 1H), 6.31-6.24 (m, 1H), 4.70 (brs, 1H), 4.07-3.99 (m, 3H), 3.99-3.91 (m, 0.5H), 3.90-3.86 (m, 3H), 3.86-3.53 (m, 3.5H), 3.39-3.32 (m, 1H), 2.96-2.88 (m, 2H), 2.68-2.58 (m, 2H), 2.56-2.50 (m, 3H), 1.47-1.37 (m, 3H), 0.98-0.53 (m, 4 H). |
| 188 | 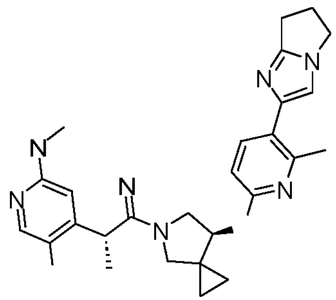 | (R)-2-(5-chloro-2-methoxypyridine-4-yl)-1-((S)-7-((5-(6,7-dihydro-5H-pyrrolo[1,2-a]imidazol-2-yl)-6-methylpyridine-2-yl)amino)-5-azaspiro[2.4]hept-5-yl)propan-1-one | 506.85 | $^1$H NMR (400 MHz, Chloroform-d) δ 8.13-8.10 (m, 1H), 6.93-6.89 (m, 1H), 6.81-6.79 (m, 1H), 7.93 (d, 1H), 6.27 (d, 1H), 4.67 (brs, 1H), 4.12-3.99 (m, 3H), 3.97-3.70 (m, 6H), 3.58-3.47 (m, 1H), 3.39-3.30 (m, 1H), 2.96-2.88 (m, 2H), 2.68-2.58 (m, 2H), 2.56-2.50 (m, 3H), 1.44-1.34 (m, 3H), 0.98-0.50 (m, 4H). |

(continued)

| Compound No. | Structure | Name | $[M+H]^+$ | $^1H$-NMR data |
|---|---|---|---|---|
| 190 | 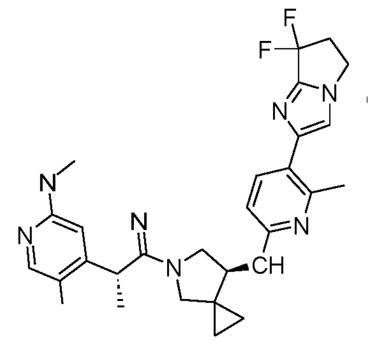 | (R)-2-(5-chloro-2-methoxypyridine-4-yl)-1-((S)-7-((5-(7,7-difluoro-6,7-dihydro-5H-pyrrolo[1,2-a]imidazol-2-yl)-6-methylpyridine-2-yl)amino)-5-azaspiro[2.4]hept-5-yl)propan-1-one | 543.05 | $^1H$ NMR (400 MHz, DMSO-$d_6$) δ 8.23 (s, 0.5H), 8.20 (s, 0.5H), 7.70-7.64 (m, 1H), 7.43 (d, 1H), 6.74-6.59 (m, 2H), 6.47-6.36 (m, 1H), 4.29-4.21 (m, 2H), 4.15-4.00 (m, 2H), 3.84 (d, 3H), 3.71-3.47 (m, 3.5H), 3.25 (d, 0.5H), 3.20-3.07 (m, 2H), 2.43 (d, 3H), 1.35-1.26 (m, 3H), 0.89-0.77 (m, 1H), 0.73-0.44 (m, 3H). |
| 195 | 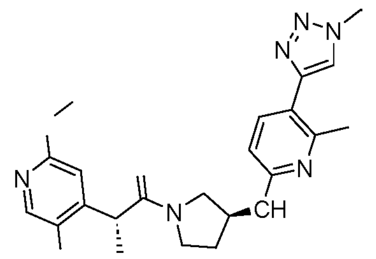 | (R)-2-(5-chloro-2-methoxypyridine-4-yl)-1-((S)-3-((6-methyl-5-(1-methyl-1*H*-1,2,3-triazol-4-yl)pyridine-2-yl)amino)pyrrolidine-1-yl)propan-1-one | 455.85 | $^1H$ NMR (400 MHz, DMSO-$d_6$) δ 8.23 (d, 1H), 8.17 (d, 1H), 7.72-7.68 (m, 1H), 6.92-6.74 (m, 1H), 6.72 (d, 1H), 6.43-6.36 (m, 1H), 4.36-4.23 (m, 1H), 4.19-4.08 (m, 1H), 4.07 (d, 3H), 3.84 (d, 3H), 3.72-3.48 (m, 2H), 3.45-3.34 (m, 1.5H), 3.28-3.21 (m, 0.5H), 2.41 (d, 3H), 2.28-1.79 (m, 2H), 1.35-1.26 (m, 3H). |
| 196 | 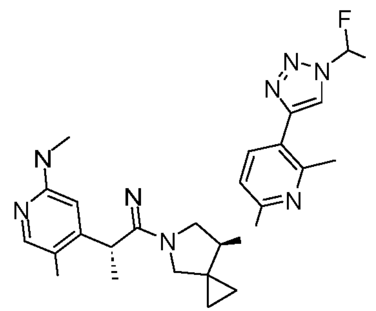 | (*R*)-2-(5-chloro-2-methoxypyridine-4-yl)-1-((*S*)-7-((S-(1-(difluoromethyl)-1*H*-1,2,3-triazol-4-yl)-6-methylpyridine-2-yl)amino)-5-azaspiro[2.4]hept-5-yl)propan-1-one | 517.85 | $^1H$ NMR (400 MHz, DMSO-$d_6$) δ 8.80 (d, 1H), 8.43-8.11 (m, 2H), 7.75-7.68 (m, 1H), 6.98-6.84 (m, 1H), 6.72 (d, 1H), 6.53-6.42 (m, 1H), 4.17-4.01 (m, 2H), 3.84 (d, 3H), 3.73-3.46 (m, 3.5H), 3.25 (d, 0.5H), 2.43 (d, 3H), 1.36-1.25 (m, 3H), 0.88-0.78 (m, 1H), 0.74-0.46 (m, 3H). |

(continued)

| Compound No. | Structure | Name | $[M+H]^+$ | $^1$H-NMR data |
|---|---|---|---|---|
| 197 | 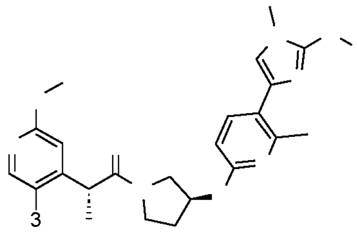 | (*R*)-1-((*S*)-3-((5-(2-(difluoromethoxy)-1-methyl-1*H*-imidazol-4-yl)-6-methylpyridine-2-yl)amino)pyrrolidine-1-yl)-2-(5-fluoro-2-methoxypyridine-4-yl)propan-1-one | 504.90 | $^1$H NMR (400 MHz, DMSO-$d_6$) δ 8.13-8.09 (m, 1H), 7.71-7.33 (m, 2H), 7.03 (d, 1H), 6.75-6.59 (m, 2H), 6.38-6.31 (m, 1H), 4.33-4.23 (m, 1H), 4.19-4.02 (m, 1H), 3.82 (d, 3H), 3.73-3.66 (m, 1H), 3.63-3.51 (m, 1H), 3.50 (d, 3H), 3.43-3.35 (m, 1.5H), 3.22-3.16 (m, 0.5H), 2.43 (s, 1.5H), 2.38 (s, 1.5H), 2.25-1.77 (m, 2H), 1.36-1.28 (m, 3H). |
| 198 | 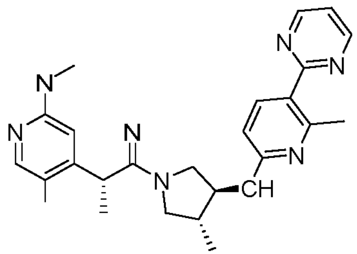 | (*R*)-2-(5-chloro-2-methoxypyridine-4-yl)-1-((3*R*,4*S*)-3-methyl-4-((6-methyl-5-(pyrimidine-2-yl)pyridine-2-yl)amino)pyrrolidine-1-yl)propan-1-one | 467.00 | $^1$H NMR (400 MHz, DMSO-$d_6$) δ 8.84-8.81 (m, 2H), 8.23 (d, 1H), 8.05-8.00 (m, 1H), 7.32-7.28 (m, 1H), 7.16-6.98 (m, 1H), 6.73 (d, 1H), 6.48-6.41 (m, 1H), 4.17-3.81 (m, 6H), 3.74-3.52 (m, 1H), 3.29-3.22 (m, 1H), 3.05-2.95 (m, 1H), 2.63 (s, 1.3H), 2.55 (s, 1.7H), 2.26-2.14 (m, 1H), 1.35-1.27 (m, 3H), 1.08-1.00 (m, 3H). |
| 199 | 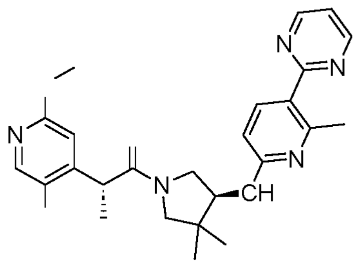 | (*R*)-2-(5-chloro-2-methoxypyridine-4-yl)-1-((*S*)-3,3-dimethyl-4-((6-methyl-5-(pyrimidine-2-yl)pyridine-2-yl)amino)pyrrolidine-1-yl)propan-1-one | 480.95 | $^1$H NMR (400 MHz, DMSO-$d_6$) δ 8.84-8.80 (m, 2H), 8.23 (s, 0.4H), 8.17 (s, 0.6H), 8.05-7.98 (m, 1H), 7.32-7.28 (m, 1H), 6.92-6.77 (m, 1H), 6.75 (s, 0.4H), 6.69 (s, 0.6H), 6.56-6.46 (m, 1H), 4.59-4.31 (m, 1H), 4.16-3.73 (m, 5H), 3.56-3.43 (m, 1H), 3.24-3.02 (m, 2H), 2.63-2.60 (m, 3H), 1.37-1.29 (m, 3H), 1.08-0.85 (m, 6H). |
| 200 | 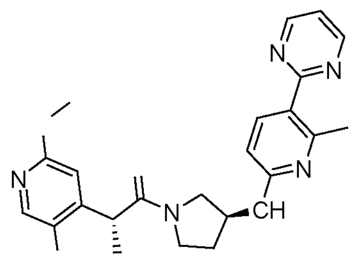 | (*R*)-2-(5-bromo-2-methoxypyridine-4-yl)-1-((*S*)-3-((6-methyl-5-(pyrimidine-2-yl)pyridine-2-yl)amino)pyrrolidine-1-yl)propan-1-one | 496.90 | $^1$H NMR (400 MHz, DMSO-$d_6$) δ 8.84-8.80 (m, 2H), 8.33 (d, 1H), 8.05-8.01 (m, 1H), 7.32-7.28 (m, 1H), 7.19-6.99 (m, 1H), 6.71 (d, 1H), 6.46-6.38 (m, 1H), 4.40-4.28 (m, 1H), 4.13-3.99 (m, 1H), 3.84 (d, 3H), 3.74-3.49 (m, 2H), 3.46-3.35 (m, 1H), 3.30-3.24 (m, 1H), 2.63 (s, 1.3H), 2.59 (s, 1.7H), 2.30-1.82 (m, 2H), 1.34-1.25 (m, 3H). |

(continued)

| Compound No. | Structure | Name | $[M+H]^+$ | $^1$H-NMR data |
|---|---|---|---|---|
| 201 | 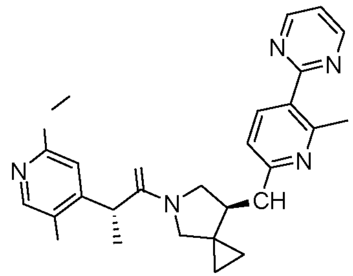 | (*R*)-2-(5-bromo-2-methoxypyridine-4-yl)-1-((*S*)-7-((6-methyl-5-(pyrimidine-2-yl)pyridine-2-yl)amino)-5-azaspiro[2.4]hept-5-yl)propan-1-one | 522.95 | $^1$H NMR (400 MHz, DMSO-$d_6$) δ 8.84-8.80 (m, 2H), 8.34 (s, 0.5H), 8.29 (s, 0.5H), 8.03-7.97 (m, 1H), 7.32-7.27 (m, 1H), 7.11-6.97 (m, 1H), 6.72 (d, 1H), 6.52-6.42 (m, 1H), 4.19-3.98 (m, 2H), 3.84 (d, 3H), 3.72-3.46 (m, 3.5H), 3.25 (d, 0.5H), 2.60 (d, 3H), 1.35-1.25 (m, 3H), 0.89-0.79 (m, 1H), 0.76-0.48 (m, 3H). |

**Test Example 1:** Assay of MC4R antagonist activity in cells in vitro

a. Experimental consumables:

| Reagents and consumables | Supplier | Product No. |
|---|---|---|
| Fetal bovine serum, 500 mL | AusGenex | FBS500-S |
| DMEM (500 mL) | Gibco | 10566-016 |
| Penicillin-Streptomycin, liquid, 100 mL (100X) | Gibco | 15140122 |
| Bovine serum albumin 7.5%, 50 mL | Perkin Elmer | CR84-100 |
| cAMP assay kit | Perkin Elmer | TRF0263 |
| IBMX, phosphodiesterase inhibitor | Sigma | 15879 |
| HEPES buffer, 1 M, 100 mL | Gibco | 15630080 |
| 384-well cell culture plate, white opaque, sterile | Perkin Elmer | 6007680 |
| 96-well V-bottom, natural RNA/DNAase-free plate | ThermoFisher | 249944 |
| **Instrument** | **Supplier** | **Product No. and Model No.** |
| EnVision | Perkin Elmer | 2105 |

b. Experimental steps

✓ Cell line: flipin-293-MC4
✓ Cell culture medium: DMEM, 10% fetal bovine serum, 1 × PS, 200 μg/mL hygromycin
✓ Experimental buffer: HBSS, 20 mM HEPES, 0.1% BSA, 500 μM IBMX
✓ Positive compound: ML00253764

c. Antagonist detection

1. Digested cells were suspended in the experimental buffer, and then inoculated into the 384-well cell culture plate.
2. The to-be-detected compound was added to the cell plate, and incubated at 37°C for 10 min.
3. Melanotan I was added to the cell plate, and incubated at 37°C for 30 min.
4. Two detection reagents EU cAMP tracer and ulight anti camp were thawed and diluted with a lysis solution in the kit.
5. The diluted detection reagents were added to the cell plate and incubated at room temperature for 1 h.
6. Reading was carried out by Envision. (Excitation light: 340 nm, emission light: 615 nm and 665 nm)

d. Data analysis

1. %Inhibition calculation:

$$\%\text{Inhibition} = (\text{Signalcmpd} - \text{SignalAve_VC})/(\text{SignalAve_PC} - \text{SignalAve_VC}) \times 100.$$

Signalcmpd: compound signal value;
SignalAve_VC: negative control signal value;
SignalAve_PC: positive control signal value;
%inhibition: inhibition percentage.

2. Calculation of $IC_{50}$ of the compound using the nonlinear fitting equation in GraphPad:

$$Y = \text{Bottom} + (\text{Top} - \text{Bottom})/(1 + 10\^{}((\text{LogIC}_{50} - X) \times \text{HillSlope}))$$

X: logarithmic value of the concentration of the compound; Y: inhibition percentage

The activities of the tested compounds antagonizing MC4R are shown in Table 2.

Table 2

| Compound No. | MC4R antagonist activity in cells $IC_{50}$ (nM) |
|---|---|
| 001 | 16.36 |
| 001-1 | 3.95 |
| 051-1 | 2.96 |
| 051-2 | 10.6 |
| 056-1 | 4.23 |
| 083-1 | 1.82 |
| 110 | 26.20 |
| 111 | 3.40 |
| 112 | 2.30 |
| 113 | 0.90 |
| 114 | 2.45 |
| 115 | 2.78 |
| 116 | 2.15 |
| 117 | 1.57 |
| 118 | 3.75 |
| 119 | 1.71 |
| 132 | 1.06 |
| 136 | 1.28 |
| 141 | 2.88 |
| 142 | 3.29 |
| 143 | 5.73 |
| 144 | 1.83 |
| 149 | 1.74 |
| 150 | 1.84 |
| 155 | 3.04 |
| 156 | 1.85 |
| 165 | 2.86 |
| 166 | 4.03 |
| 168 | 3.68 |
| 181 | 1.59 |
| 182 | 0.86 |
| 184 | 1.49 |
| 185 | 0.54 |
| 186 | 0.55 |
| 187 | 3.42 |
| 188 | 2.11 |
| 189 | 2.25 |
| 190 | 1.10 |
| 195 | 0.99 |

(continued)

| Compound No. | MC4R antagonist activity in cells $IC_{50}$ (nM) |
|---|---|
| 196 | 4.88 |
| 198 | 4.9 |
| 199 | 366 |
| 200 | 0.98 |
| 201 | 1.41 |

**Test Example 2:** Pharmacokinetic study on the tested compound in SD male rats

2.1 Experimental method

On the day of administration, the tested compound was prepared with 5% DMSO + 5% Solutol + 90% Saline as a solvent into a solution for administration for use.

The solution for administration was administered to an intravenous injection IV group at a dose of 1 mg/kg in a concentration of 0.2 mg/mL. The solution for administration was administered to an oral gavage PO group at a dose of 2 mg/kg in a concentration of 0.2 mg/mL. The animals in the PO group were required to fast overnight in advance, drink water freely, and be fed 4 hours after administration. The IV group was not required to fast.

The animals were weighed before administration. On the day of administration, the solution was IV/PO-administered once in an administration amount calculated based on the weight. In the IV group, blood was collected from veins at 0.083 h, 0.25 h, 0.5 h, 1.0 h, 2.0 h, 4.0 h, 8.0 h, and 24 h after administration. In the PO group, blood was collected from veins at 0.25 h, 0.5 h, 1.0 h, 2.0 h, 4.0 h, 6.0 h, 8.0 h, and 24 h after administration. The amount of blood collected was about 0.20 mL. K2-EDTA was used in blood collection tubes as an anticoagulant. The collected blood sample was centrifuged (centrifugation conditions: 6800 g, 6 min, 2°C to 8°C) within 1 h to obtain plasma. The to-be-tested sample was stored in a refrigerator at -80°C before analysis.

The concentration of the tested compound in the plasma of the SD male rats was detected by the validated LC-MS/MS method.

2.2 Experimental results

The pharmacokinetic study on the compound in the present invention in the SD male rats was determined through the foregoing experiment. The results are shown in Table 3.

Table 3 Experimental results of the pharmacokinetic study on the tested compound in the SD male rats (IV 1 mpk/PO 2 mpk)

| Compound | $C_{max}$ (ng/mL) | AUC (h×ng/mL) | $T_{1/2}$ (h) | F | Cl (mL/min/kg) |
|---|---|---|---|---|---|
| 001-1 | 338.5 | 441.9 | 0.71 | 86.8% | 65.3 |
| 051-1 | 329 | 317 | 0.25 | 52.7% | 55.2 |
| 056-1 | 380.1 | 566.4 | 0.89 | 61.5% | 35.8 |
| 083-1 | 155.9 | 239.9 | 0.65 | 31.3% | 43.7 |
| 112 | 157 | 179 | 0.25 | 33.8% | 62.5 |
| 114 | 197.2 | 449.9 | 0.98 | 54.0% | 39.9 |
| 165 | 577 | 858 | 0.65 | 56.0% | 22.3 |
| 197 | 35 | 43 | 1.2 | 11.1% | 86.9 |
| 185 | 129 | 170 | 0.68 | 22.9% | 45.4 |

2.3 Experimental conclusion

The compound 056-1 has a higher AUC than the compounds 001-1 and 051-1, and a lower clearance rate than the compounds 001-1 and 051-1 in the SD male rats. The compound 114 has a significantly higher AUC and significantly

higher bioavailability than the compounds 083-1 and 112, and a lower clearance rate than the compounds 083-1 and 112 in the SD male rats. The compound 185 has a significantly higherAUC and significantly higher bioavailability than the compound 197, and a lower clearance rate than the compound 197 in the SD male rats. In conclusion, it is found from the study on structure-activity relationships that in the structures of the MC4R antagonist compounds disclosed in the present invention, the specific structural modification by introducing a spiro three-membered ring on a tetrahydropyrrole ring can significantly improve in vivo pharmacokinetic properties of such type of compounds, improving the druggability of the compounds.

**Test Example 3:** Experiment on inhibition of P450 enzyme by the tested compound

3.1 Experimental purpose

The inhibitory effect of the tested compound on 3A4-M (with midazolam as a substrate) in human liver microsomes is evaluated in vitro to assess the safety of the compound.

3.2 Experimental method

In this study, the inhibition of CYP 3A4 enzyme in human liver microsomes by the tested compound was investigated using the human liver microsomal system and the cytochrome P450 enzyme probe substrate recommended by the FDA Guidance for In Vitro Drug Interaction Studies. The human liver microsomes were incubated separately with the tested compound of different concentrations (0 μM to 30.0 μM) and corresponding probe substrates. Then, the changes of activity of the CYP enzyme were determined, and the $IC_{50}$ value was calculated, to evaluate the inhibitory potential of the tested compound on the CYP 3A4 enzyme.

3.2.1 Test concentrations

Seven test concentrations were given, respectively 30.00 μM, 10.00 μM, 3.33 μM, 1.11 μM, 0.370 μM, 0.123 μM, and 0.0412 μM.

3.2.2 Test group and control group

Test group (TG): The human liver microsomes were incubated separately with the tested compound of different concentrations, NADPH (in a final concentration of 2.00 mM), and probe substrates (Table 4) of different enzymes in subfamilies for 5 min.

Positive control group (PC): The human liver microsomes were incubated separately with an inhibitor (Table 4), NADPH (in a final concentration of 2.00 mM), and a probe substrate (Table 4) of 3A4M for 5 min.

Table 4 Probe substrate of each enzyme in the subfamily, inhibitor, and incubation system

| CYP450 enzyme | Concentration of liver microsomes (final concentration, mg/mL) | Probe substrate (final concentration, μM) | Inhibitor (final concentration, μM) | Incubation time (min) |
|---|---|---|---|---|
| CYP3A4 | 0.100 | Midazolam (5.00) | Ketoconazole (2.50) | 5 |

At the end of the incubation, the reaction was terminated with a stop solution.

3.2.3 Preparation of experimental solutions

3.2.3.1 Stop solution containing internal standard

To 4 L of acetonitrile/methanol (in a volume ratio of 1:1) were added 18.26 μL of 4.379 mg/mL tolbutamide stock solution and 13.02 μL of 3.071 mg/mL verapamil stock solution, to give the stop solution containing 20.0 ng/mL tolbutamide and 10.0 ng/mL verapamil.

3.2.3.2 Preparation of human liver microsomes (HLM) working solution

A 20.0 mg/mL HLM stock solution was diluted with a phosphate buffer to give a 0.2 mg/mL HLM working solution.

3.2.3.3 Preparation of NADPH working solution

An appropriate amount of NADPH was weighed and prepared with a phosphate buffer into an 8.00 mM NADPH working solution, and pre-warmed at 37°C.

3.2.3.4 Preparation of test sample stock solution

An appropriate amount of test sample was weighed and dissolved in DMSO to give a 30 mM stock solution.

3.2.3.5 Preparation of substrate working solution

An 80 mM testosterone stock solution was diluted with a phosphate buffer to give a 320 μM working solution. A 5 mM midazolam stock solution was diluted with a phosphate buffer to give a 20 μM working solution.

3.2.4 Experimental process

3.2.4.1 Pre-warming a phosphate buffer (pH 7.40±0.05)

3.2.4.2 Preparation of a test sample in serial concentrations or a positive control intermediate solution

8 μL of 30 mM test sample was transferred to 12 μL of DMSO to give a 12 mM test sample intermediate solution, and then diluted with DMSO to give test sample working solutions in different concentrations (12 mM, 4 mM, 1.33 mM, 0.433 mM, 0.147 mM, 0.049 mM, and 0.0164 mM).

To 12 μL of DMSO was added 8 μL of 2.5 mM CYP3A4 inhibitor ketoconazole stock solution to give a 1 mM positive control inhibitor working solution.

3.2.4.3 Test group (TG)

199 μL of 0.2 mg/mL HLM and 1 μL of test sample working solution (12 mM, 4 mM, 1.33 mM, 0.433 mM, 0.147 mM, 0.049 mM, and 0.0164 mM) were mixed uniformly. 30 μL of this liver microsome solution was added to a 96-well plate, and then 10 μL of substrate working solution (320 μM testosterone or 20 μM midazolam) was added. This preparation process was carried out in duplicate. After pre-incubation at 37°C for 5 min, 15 μL of 8 mM NADPH solution pre-warmed at 37°C for 5-10 min was added to initiate the reaction. After incubation in the 96-well plate at 37°C for 5 min, 180 μL of acetonitrile/methanol (ACN:MeOH = 1:1) solution containing the internal standard (IS) was added to terminate the reaction. The 96-well plate was shaken at 600 rpm for 5-10 min, and then centrifuged at 6000 rpm for 15 min. 80 μL of supernatant was taken and added to 120 μL of ultrapure water, shaken uniformly, and then detected by LC-MS/MS.

3.2.4.4 Positive control group (PC)

199 μL of 0.2 mg/mL HLM and 1 μL of 1 mM positive control inhibitor (ketoconazole) working solution were mixed uniformly. 30 μL of this liver microsome solution was added to a 96-well plate, and then 10 μL of substrate working solution (20 μM midazolam) was added. This preparation process was carried out in duplicate. After pre-incubation at 37°C for 5 min, 15 μL of 8 mM NADPH solution pre-warmed at 37°C for 5-10 min was added to initiate the reaction. After incubation in the 96-well plate at 37°C for 5 min, 180 μL of ACN:MeOH (1:1) solution containing IS was added to terminate the reaction. The 96-well plate was shaken at 600 rpm for 5-10 min, and then centrifuged at 6000 rpm for 15 min. 80 μL of supernatant was taken and added to 120 μL of ultrapure water, shaken uniformly, and then detected by LC-MS/MS.

3.2.5 Data analysis

3.2.5.1 The relative activity of CYP450 enzyme in the subfamily in different concentrations was calculated by dividing a peak area ratio (peak area of the analyte/peak area of the internal standard) of a metabolite obtained in the presence of the test sample or positive control solvent by a peak area ratio of a metabolite obtained in the absence of the test sample and positive control solvent, according to the following formulas:

Relative activity (% of NC) = peak area ratio of the metabolite in the test group or the positive control group/peak area ratio of the metabolite in the negative control group × 100

Enzyme activity inhibition % in the PC group = 100% - relative enzyme activity % in the test concentration

3.2.5.2 Half-maximal inhibitory concentration ($IC_{50}$) obtained through fitting by the Prism software according to the following model formula:

$$Y = Bottom + (Top - Bottom)/(1 + 10^{\wedge}((LogIC_{50} - X) \times HillSlope))$$

X is the concentration of the tested compound converted to a logarithmic value (Log μM), Y is the relative activity (% of NC), Bottom and Top represent the percentages of enzyme activity respectively at the bottom and top plateaus of the curve, and HillSlope represents the slope. To optimize a fitting effect, fitting with another (nonlinear) dose-response model is not considered as a deviation from the solution.

When the relative activity (% of NC) in the PC group is ≤ 50.0%, the known inhibitor exhibits the inhibitory effect, indicating that the test system is qualified.

3.3 Experimental results

The experimental results of the experiment on inhibition of P450 enzyme by the tested compound are shown in Table 5.

Table 5

| Compound | CYP 3A4M $IC_{50}$ (μM) |
|---|---|
| 083-1 | 5.8 |
| 114 | 29.5 |
| 111 | 2.94 |
| 116 | > 30 |
| 200 | 3.75 |
| 201 | > 30 |

3.4 Experimental conclusion

The inhibition of CYP 3A4M by the compound 083-1 is much stronger than the inhibition of CYP 3A4M by the compound 114. Similarly, the inhibition of CYP 3A4M by the compound 111 is much stronger than the inhibition of CYP 3A4M by the compound 116, and the inhibition of CYP 3A4M by the compound 200 is much stronger than the inhibition of CYP 3A4M by the compound 201.

It can be learned that in the structures of the MC4R antagonist molecules of this structural type, the specific structure obtained by introducing a spiro three-membered ring on a tetrahydropyrrole ring can significantly reduce the inhibition of CYP 3A4M by the compounds, and reduce the risk caused by drug interactions, improving the druggability of the compounds.

The foregoing exemplarily describes the embodiments of the technical solutions of the present invention. It should be understood that the protection scope of the present invention is not limited to the foregoing embodiments. Any modification, equivalent replacement, or improvement made by a person skilled in the art within the spirit and principle of the present invention shall fall within the protection scope of the claims of this application.

## Claims

1. A compound represented by formula I, and a racemate, a stereoisomer, a tautomer, an isotopic label, a nitrogen oxide, a solvate, a polymorph, a metabolite, an ester, a prodrug, or a pharmaceutically acceptable salt thereof:

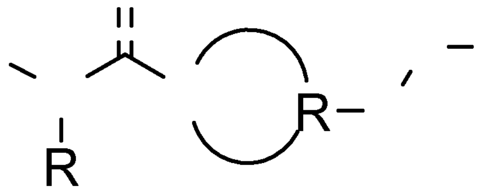

**formula I**

wherein X is selected from NR', O, or S; R' is selected from H, $C_{1-12}$ alkyl, or $C_{3-12}$ cycloalkyl;
Y is selected from CH or N;
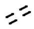 is present or absent;
R is selected from $C_{1-12}$ alkyl, deuterated $C_{1-12}$ alkyl, or halogenated $C_{1-12}$ alkyl;
A is selected from the following groups unsubstituted or optionally substituted with one, two, or more $R_a$: $C_{6-14}$ aryl, 5- to 14-membered heteroaryl, 3- to 14-membered heterocyclyl, and $C_{3-12}$ cycloalkyl; each $R_a$ is the same or different, and is independently selected from H, deuterium, halogen, CN, or the following groups unsubstituted or optionally substituted with one, two, or more $R_{a1}$: $C_{1-12}$ alkyl and $C_{1-12}$ alkoxy; each $R_{a1}$ is the same or different, and is independently selected from deuterium, halogen, CN, $C_{1-12}$ alkyl, or $C_{1-12}$ alkoxy;
E is selected from 3- to 14-membered N-containing heterocyclyl unsubstituted or optionally substituted with one, two, or more $R_e$; each $R_e$ is the same or different, and is independently selected from H, deuterium, halogen, CN, OH, oxo (=O), $C_{1-12}$ alkyl, or $C_{1-12}$ alkoxy; or two $R_e$ with an atom linked thereto form $C_{3-12}$ cycloalkyl or 3- to 14-membered heterocyclyl; N in E is linked to

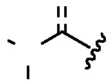,

and C in E is linked to X;
G is selected from the following groups unsubstituted or optionally substituted with one, two, or more $R_g$: $C_{6-14}$ aryl and 5- to 14-membered heteroaryl; each $R_g$ is the same or different, and is independently selected from H, deuterium, halogen, CN, or the following groups unsubstituted or optionally substituted with one, two, or more $R_{g1}$: amino, $C_{1-12}$ alkyl, $C_{1-12}$ alkoxy, $C_{3-12}$ cycloalkyl, $C_{6-14}$ aryl, and 5- to 14-membered heteroaryl; each $R_{g1}$ is the same or different, and is independently selected from deuterium, halogen, CN, $C_{1-12}$ alkyl, or $C_{1-12}$ alkoxy; and
M is absent or is selected from the following groups unsubstituted or optionally substituted with one, two, or more $R_m$: $C_{6-14}$ aryl, 5- to 14-membered heteroaryl, 3- to 14-membered heterocyclyl, and $C_{3-12}$ cycloalkyl; each $R_m$ is the same or different, and is independently selected from H, deuterium, halogen, CN, or the following groups unsubstituted or optionally substituted with one, two, or more $R_{m1}$: $C_{1-12}$ alkyl, $C_{1-12}$ alkoxy, and $C_{3-12}$ cycloalkyl; each $R_{m1}$ is the same or different, and is independently selected from deuterium, halogen, CN, OH, $C_{1-12}$ alkyl, or $C_{1-12}$ alkoxy.

**2.** The compound according to claim 1, wherein X is selected from NR', O, or S; R' is selected from H, $C_{1-6}$ alkyl, or $C_{3-8}$ cycloalkyl, such as methyl, ethyl, or cyclopropyl;

preferably, R is selected from $C_{1-6}$ alkyl, deuterated $C_{1-6}$ alkyl, or halogenated $C_{1-6}$ alkyl, such as methyl;
preferably, A is selected from the following groups unsubstituted or optionally substituted with one, two, or more $R_a$: $C_{6-10}$ aryl, 5- to 10-membered heteroaryl, 3- to 10-membered heterocyclyl, and $C_{3-8}$ cycloalkyl; each $R_a$ is the same or different, and is independently selected from H, deuterium, halogen, CN, or the following groups unsubstituted or optionally substituted with one, two, or more $R_{a1}$: $C_{1-6}$ alkyl and $C_{1-6}$ alkoxy; each $R_{a1}$ is the same or different, and is independently selected from deuterium, halogen, CN, $C_{1-6}$ alkyl, or $C_{1-6}$ alkoxy;
preferably, A is selected from the following groups unsubstituted or optionally substituted with one, two, or more $R_a$:

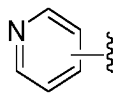, 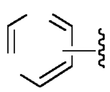, 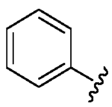, 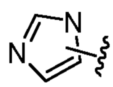, 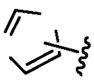, 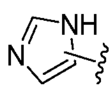,

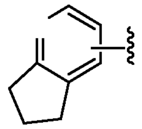, 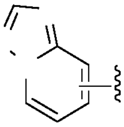, 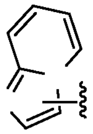, 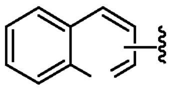, 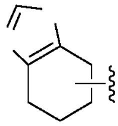, 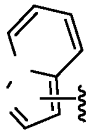, 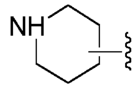,

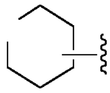, 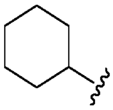, 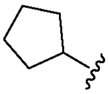, 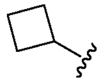, and 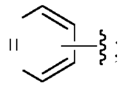;

each $R_a$ is the same or different, and is independently selected from H, F, Cl, Br, I, CN, $C_{1-6}$ alkyl, $C_{1-6}$ alkoxy, halogenated $C_{1-6}$ alkyl, or halogenated $C_{1-6}$ alkoxy;
preferably, each $R_a$ is the same or different, and is independently selected from H, F, Cl, Br, CN, methyl, methoxy, trifluoromethyl, or difluoromethoxy; and
preferably, A is selected from

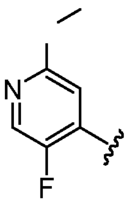, 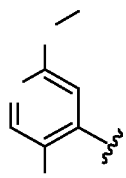, 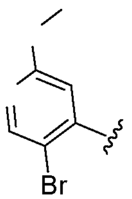, 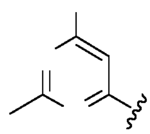,

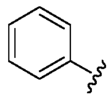, 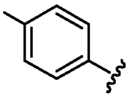, 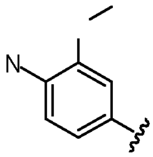, 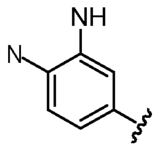, 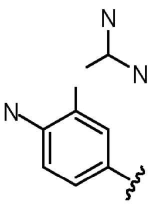, 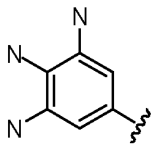,

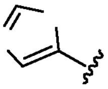, 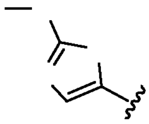, 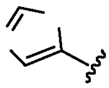, 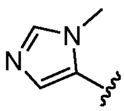, 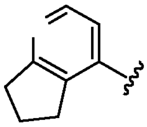, 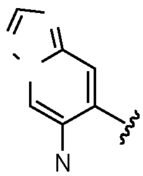, 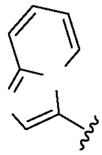,

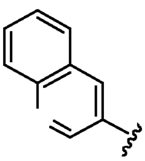, 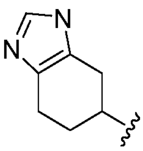, 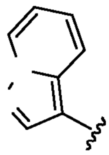, 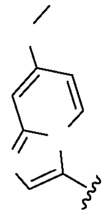, 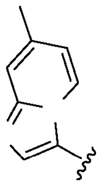, 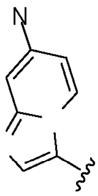, 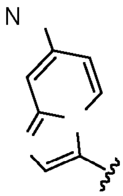,

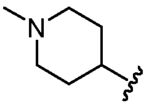, 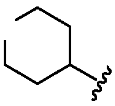, 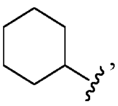, 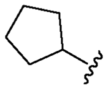, , , , or .

**3.** The compound according to claim 1 or 2, wherein E is selected from 3- to 8-membered N-containing heterocyclyl unsubstituted or optionally substituted with one, two, or more $R_e$; each $R_e$ is the same or different, and is independently selected from H, deuterium, halogen, CN, OH, oxo (=O), $C_{1-6}$ alkyl, or $C_{1-6}$ alkoxy; or two $R_e$ with an atom linked thereto form $C_{3-8}$ cycloalkyl; N in E is linked to

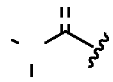,

and C in E is linked to X;

preferably, E is selected from the following groups unsubstituted or optionally substituted with one, two, or more $R_e$:

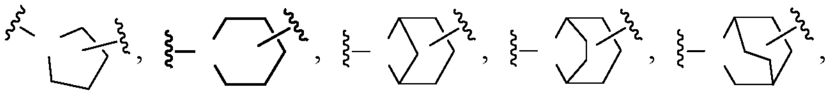

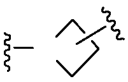, 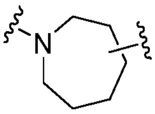, and 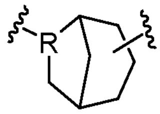;

each $R_e$ is the same or different, and is independently selected from H, F, Cl, Br, I, CN, OH, oxo (=O), or $C_{1-6}$ alkyl; or two $R_e$ with an atom linked thereto form $C_{3-8}$ cycloalkyl;
preferably, each $R_e$ is the same or different, and is independently selected from H, F, CN, OH, oxo (=O), or methyl; or two $R_e$ with an atom linked thereto form cyclopropyl; and
preferably, E is selected from

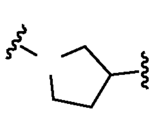, 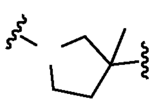, 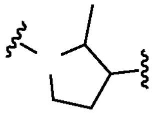, 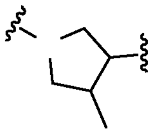,

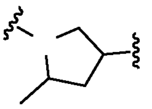, 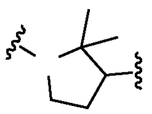, 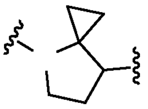, 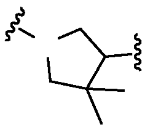, 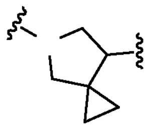, 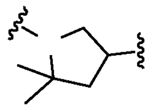,

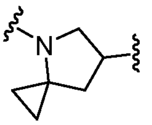, 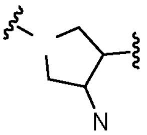, 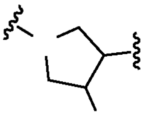, 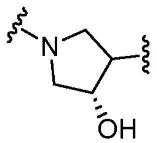, 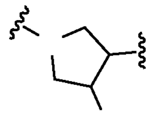, 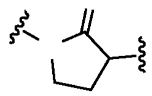,

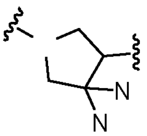, 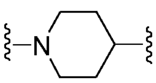, 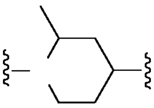, 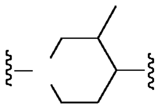, 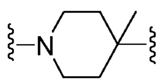, 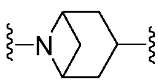,

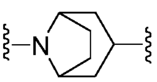, 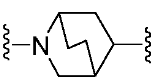, 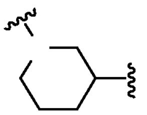, 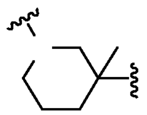, 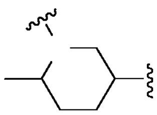, 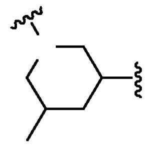,

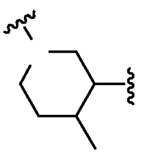, 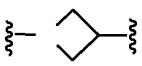, 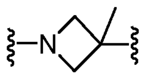, 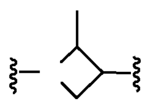, 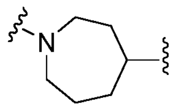, 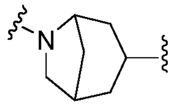, or

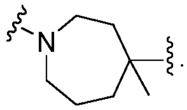

4. The compound according to any one of claims 1 to 3, wherein G is selected from the following groups unsubstituted or optionally substituted with one, two, or more $R_g$: $C_{6-10}$ aryl and 5- to 10-membered heteroaryl; each $R_g$ is the same or different, and is independently selected from H, deuterium, halogen, CN, or the following groups unsubstituted or optionally substituted with one, two, or more $R_{g1}$: amino, $C_{1-6}$ alkyl, $C_{1-6}$ alkoxy, $C_{3-8}$ cycloalkyl, $C_{6-10}$ aryl, and 5- to 10-membered heteroaryl; each $R_{g1}$ is the same or different, and is independently selected from deuterium, $C_{1-6}$ alkyl, or $C_{1-6}$ alkoxy;

preferably, G is selected from the following groups unsubstituted or optionally substituted with one, two, or more $R_g$:

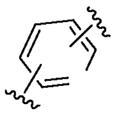, 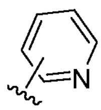, 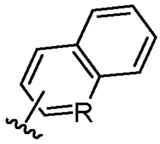, 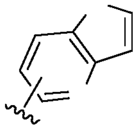, 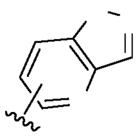,

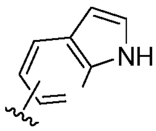, 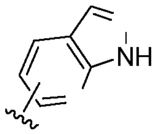, 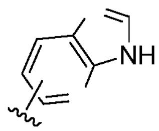, 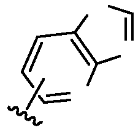, 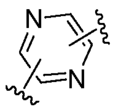, 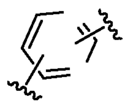, 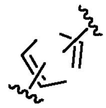,

and 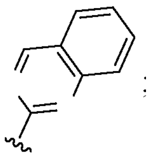;

each $R_g$ is the same or different, and is independently selected from H, halogen, CN, $C_{1-6}$ alkyl, $C_{1-6}$ alkoxy, $C_{3-8}$ cycloalkyl, amino, $C_{1-6}$ alkylamino, ($C_{1-6}$ alkyl)$_2$amino, $C_{6-10}$ aryl, or $C_{1-6}$ alkyl 5- to 8-membered heteroaryl; preferably, each $R_g$ is the same or different, and is independently selected from H, F, methyl, ethyl, cyclopropyl, cyclobutyl, methoxy, methylamino ($CH_3NH$-), dimethylamino ($(CH_3)_2N$-), phenyl, or

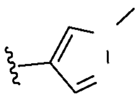;

and

preferably, G is selected from

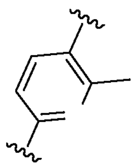, 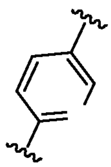, 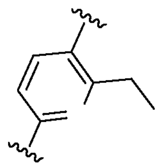, 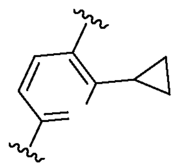,

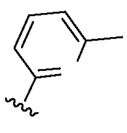, 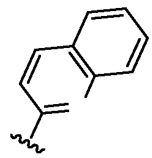, 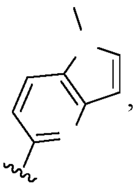, 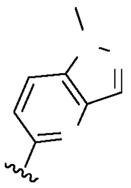, 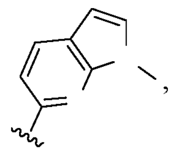, 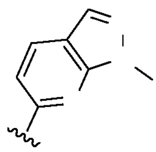, 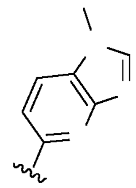,

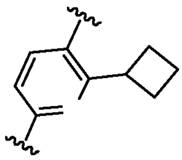, 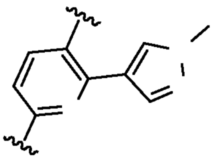, 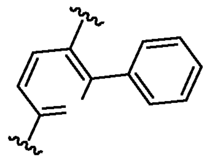, 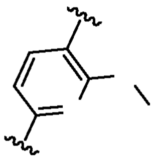, 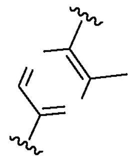, 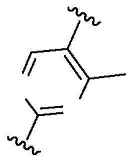,

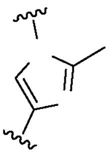, 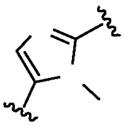, 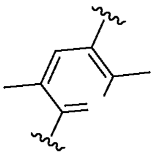, 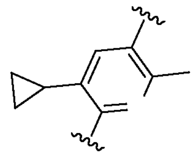, 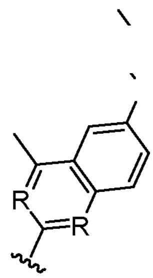, 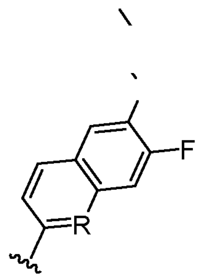,

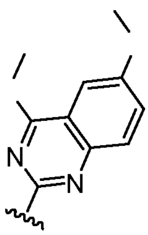, 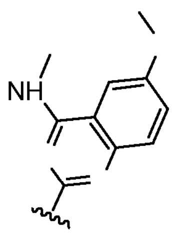, 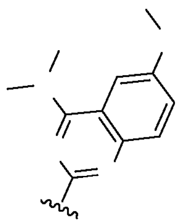, 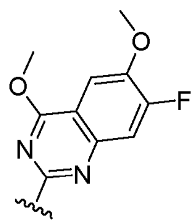, 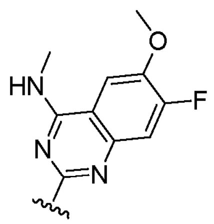,

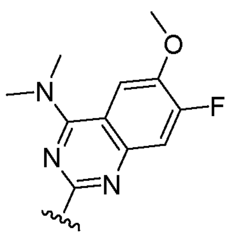, or 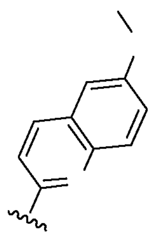.

**5.** The compound according to any one of claims 1 to 4, wherein M is absent or is selected from the following groups unsubstituted or optionally substituted with one, two, or more $R_m$: $C_{6-10}$ aryl, 5- to 10-membered heteroaryl, and 3- to 8-membered heterocyclyl; each $R_m$ is the same or different, and is independently selected from H, deuterium, halogen, CN, or the following groups unsubstituted or optionally substituted with one, two, or more $R_{m1}$: $C_{1-6}$ alkyl, $C_{1-6}$ alkoxy, and $C_{3-6}$ cycloalkyl; each $R_{m1}$ is the same or different, and is independently selected from deuterium, halogen, CN, OH, $C_{1-6}$ alkyl, or $C_{1-6}$ alkoxy;

preferably, M is absent or is selected from the following groups unsubstituted or optionally substituted with one, two, or more $R_m$: pyridinyl, pyrimidinyl, phenyl, oxazolyl, pyrazolyl, triazolyl, 1,2,3-triazolyl, tetrazolyl, thiazolyl, tetrahydropyrrolyl, piperidinyl, morpholinyl, piperazinyl, naphthyl, quinolyl, azepanyl, imidazolyl,

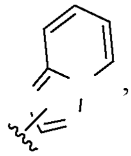, 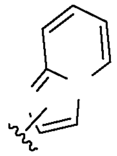, 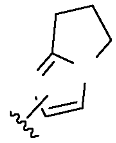,

and

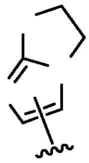 ;

each $R_m$ is the same or different, and is independently selected from H, deuterium, F, Cl, Br, I, CN, $C_{1-6}$ alkyl, $C_{1-6}$ alkoxy, $C_{3-6}$ cycloalkyl, halogenated $C_{1-6}$ alkyl, halogenated $C_{1-6}$ alkoxy, or hydroxyl-$C_{1-6}$ alkyl;
preferably, each $R_m$ is the same or different, and is independently selected from H, F, CN, methyl, methoxy, difluoromethyl, difluoromethoxy, trifluoromethyl, cyclopropyl, or

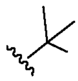 ;

preferably, each $R_m$ is the same or different, and is independently selected from H, F, CN, methyl, methoxy, difluoromethyl, difluoromethoxy, trifluoromethyl, or cyclopropyl;
preferably, $R_m$ is selected from CN;
preferably, M is absent or is selected from

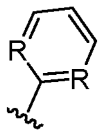 , 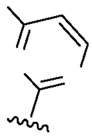 , 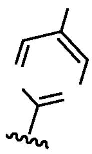 , 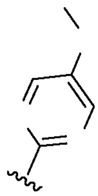 ,

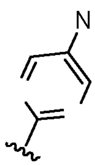 , 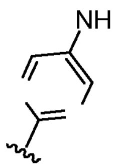 , 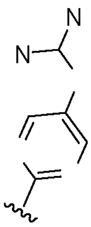 , 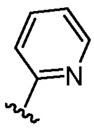 , 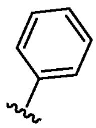 , 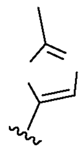 , 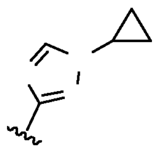 , 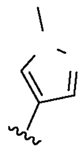 , 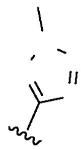 ,

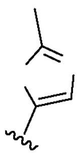 , 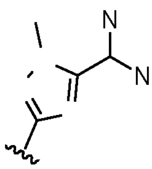 , 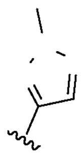 , 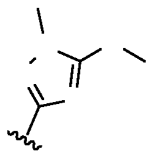 , 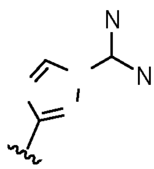 , 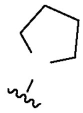 , 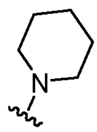 , 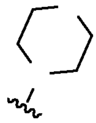 ,

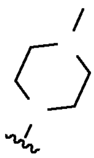 , 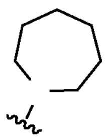 , 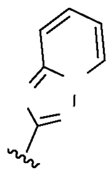 , 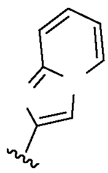 , 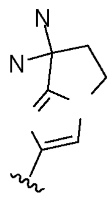 , 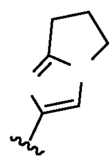 , 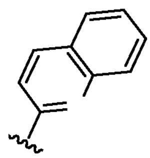 , 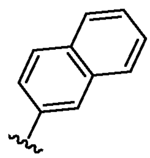 ,

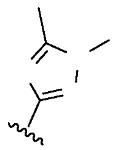, 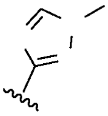, 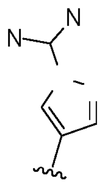, 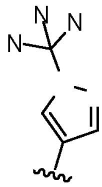, 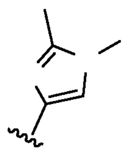, 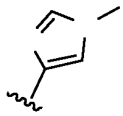, 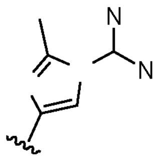, 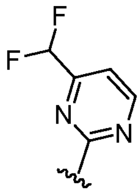,

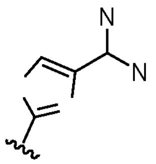, 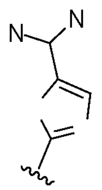, 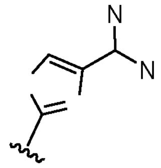, 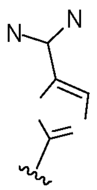, 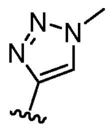, 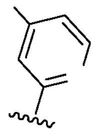, 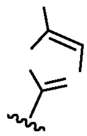, 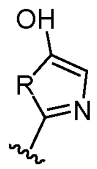,

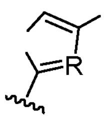, 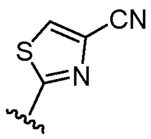, 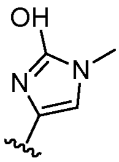, 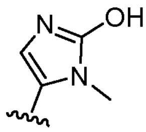, 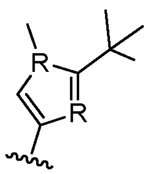, 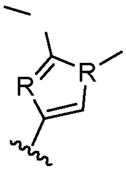,

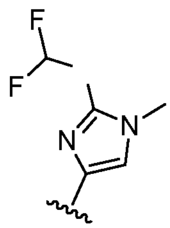, 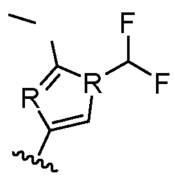, 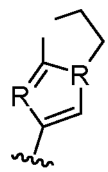, or 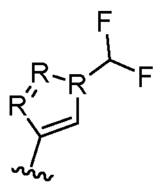;

preferably, M is selected from 5- and 6-membered heteroaryl substituted with one, two, or more $R_m$, wherein at least one of $R_m$ is CN, and the other of $R_m$ is present or absent, when the other of $R_m$ is present, the other of $R_m$ is the same or different, and is independently selected from halogen, CN, $C_{1-6}$ alkyl, $C_{1-6}$ alkoxy, $C_{3-6}$ cycloalkyl, halogenated $C_{1-6}$ alkyl, or halogenated $C_{1-6}$ alkoxy; and

preferably, M is selected from 5- and 6-membered heteroaryl substituted with CN.

**6.** The compound according to any one of claims 1 to 5, wherein the compound represented by formula I is selected from the following structures:

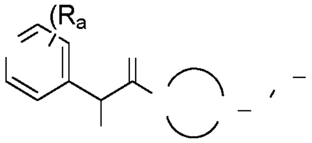

formula I-1

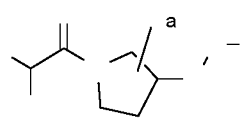

formula I-2

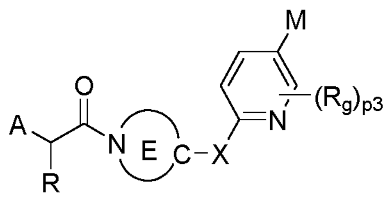

formula I-3

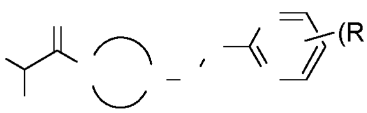

formula I-4

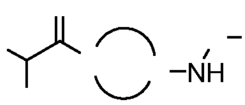

formula I-5

wherein X, R, A, E, G, M, $R_a$, $R_e$, $R_g$, and $R_m$ are as defined according to any one of claims 1 to 5; and p1, p2, p3, and p4 are each independently selected from 0, 1, 2, 3, 4, or 5;

preferably, the compound represented by formula I has a structure represented by the following formula II:

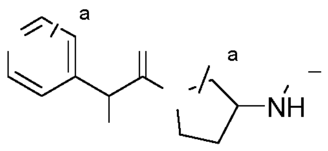

formula II

wherein R, M, G, $R_a$, and $R_e$ are as defined according to any one of claims 1 to 5; and p1 and p2 are each selected from 0, 1, 2, 3, 4, or 5;
preferably, the compound represented by formula I has a structure represented by the following formula II-1, II-2, or II-3:

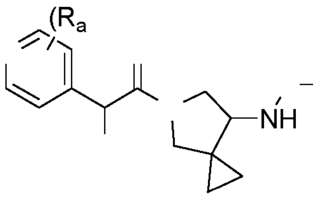

formula II-1

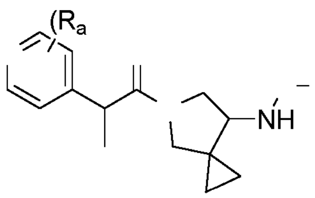

formula II-2

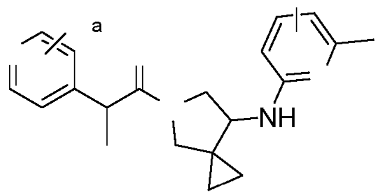

formula II-3

wherein R, M, G, and $R_a$ are as defined according to any one of claims 1 to 5; and p1 is selected from 0, 1, 2, 3, 4, or 5;
preferably, the compound represented by formula I has a structure represented by the following formula III:

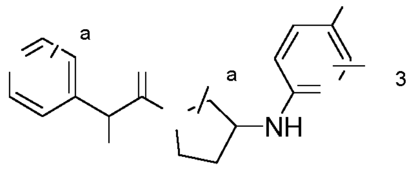

formula III

wherein M, R, $R_a$, $R_e$, and $R_g$ are as defined according to any one of claims 1 to 5; and p1, p2, and p3 are each selected from 0, 1, 2, 3, 4, or 5;
preferably, the compound represented by formula I is selected from the following structures:

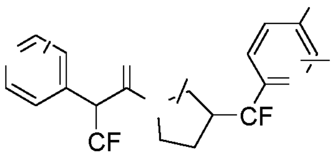

formula III-1

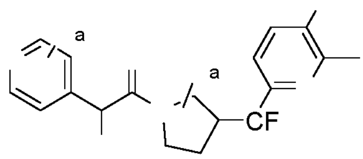

formula III-2

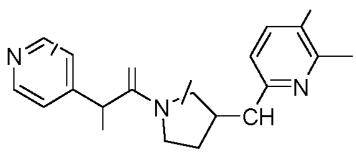

formula III-3

wherein M, R, $R_a$, $R_e$, and $R_g$ are as defined according to any one of claims 1 to 5; and p1, p2, and p3 are each selected from 0, 1, 2, 3, 4, or 5;
preferably, the compound represented by formula I has a structure represented by the following formula IV:

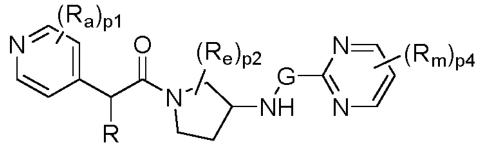

formula IV

wherein R, G, $R_a$, $R_e$, and $R_m$ are as defined according to any one of claims 1 to 5; and p1, p2, and p4 are each

selected from 0, 1, 2, 3, 4, or 5;
preferably, the compound represented by formula I has a structure represented by the following formula V:

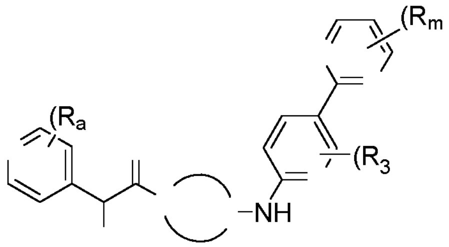

formula V

wherein R, E, $R_a$, $R_g$, and $R_m$ are as defined according to any one of claims 1 to 5; and p1, p3, and p4 are each selected from 0, 1, 2, 3, 4, or 5;
preferably, the compound represented by formula I has a structure represented by the following formula VI:

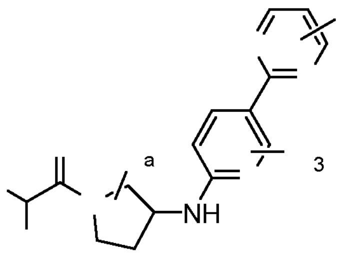

formula VI

wherein A, R, $R_e$, $R_g$, and $R_m$ are as defined according to any one of claims 1 to 5; and p2, p3, and p4 are each selected from 0, 1, 2, 3, 4, or 5;
preferably, the compound represented by formula I has a structure represented by the following formula VII:

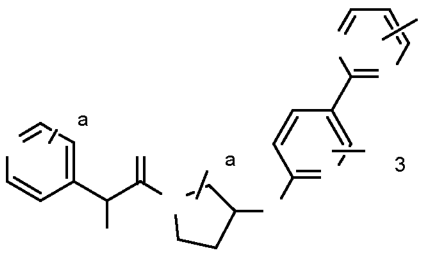

formula VII

wherein R, $R_a$, $R_e$, $R_g$, and $R_m$ are as defined according to any one of claims 1 to 5; and p1, p2, p3, and p4 are each selected from 0, 1, 2, 3, 4, or 5;
preferably, the compound represented by formula I has a structure represented by the following formula VIII or VIII-A:

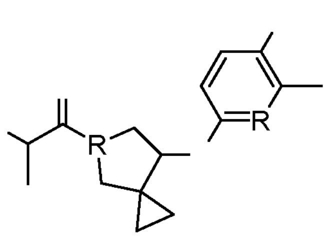

formula VIII

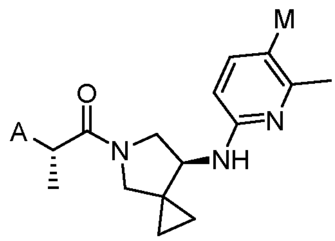

formula VIII-A

wherein A and M are as defined according to any one of claims 1 to 5;
preferably, the compound represented by formula I has a structure represented by the following formula VIII-1 or VIII-2:

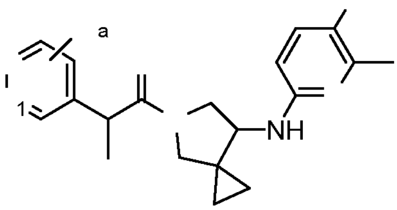

formula VIII-1

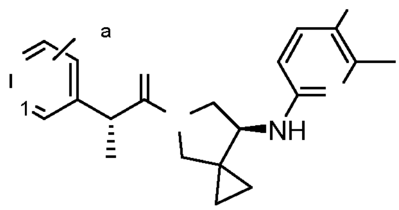

formula VIII-2

wherein $R_a$ and M are as defined according to any one of claims 1 to 5; p1 is selected from 0, 1, 2, 3, 4, or 5; and $Y_1$ is selected from CH or N; and
preferably, the compound represented by formula I has a structure represented by the following formula IX or IX-1:

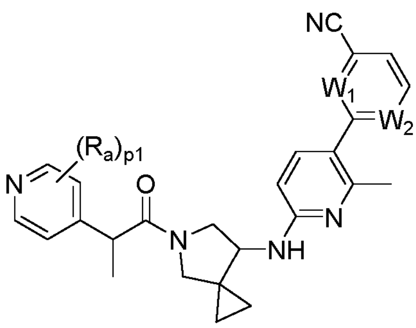

formula IX

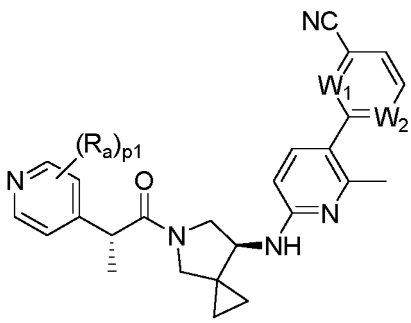

formula IX-1

wherein $R_a$ is as defined according to any one of claims 1 to 5; p1 is selected from 0, 1, 2, 3, 4, or 5; and $W_1$ and $W_2$ are the same or different, and are each independently selected from CH or N.

**7.** The compound according to any one of claims 1 to 6, wherein the compound represented by formula I is selected from the following structures:

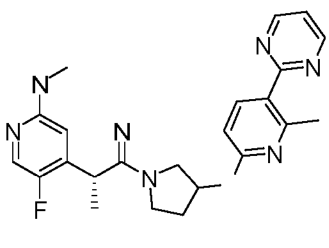

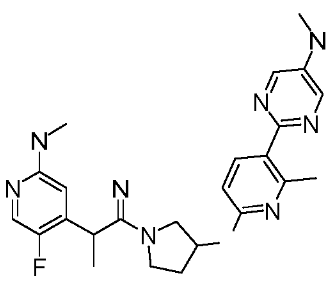

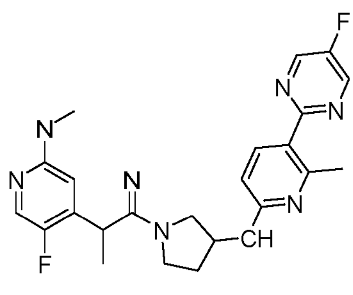

CH

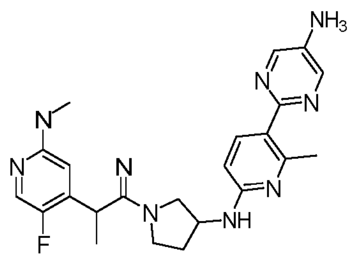
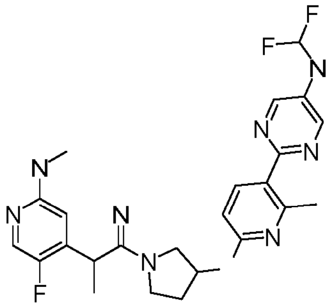
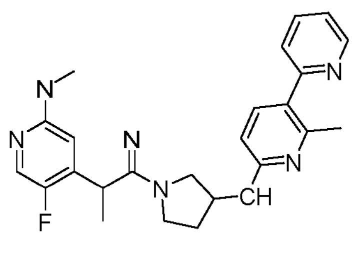

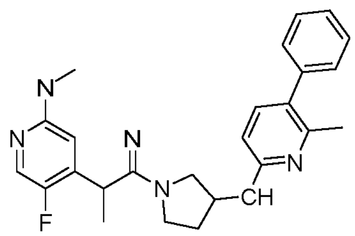
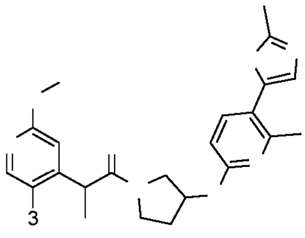

(dba)

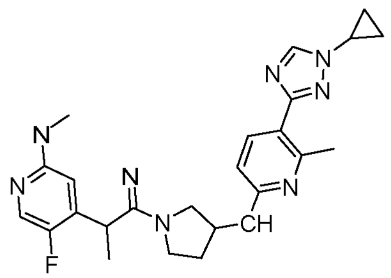

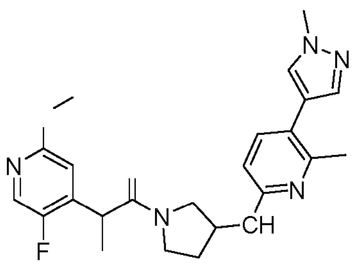
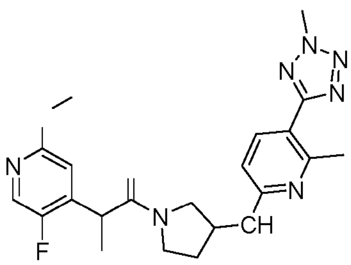
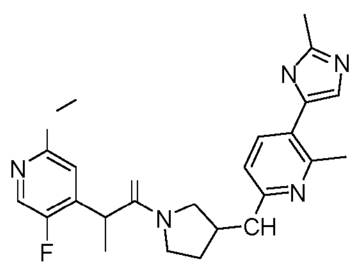

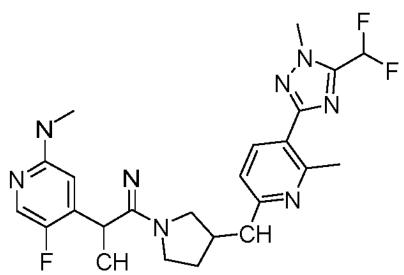
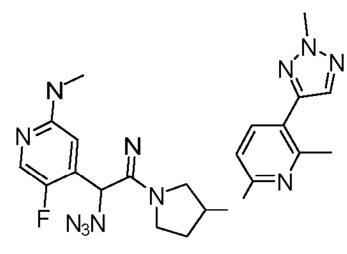
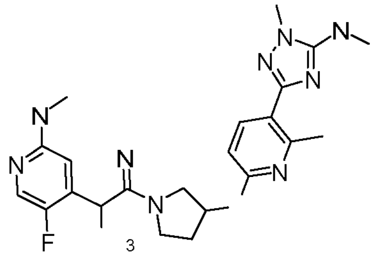

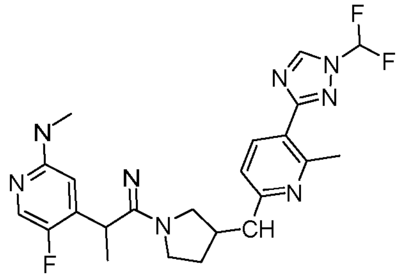
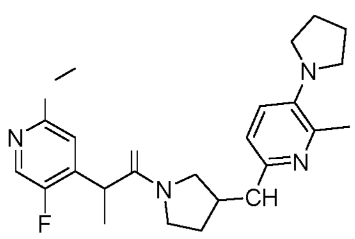
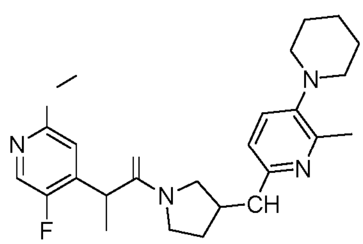

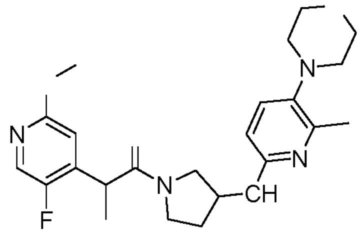
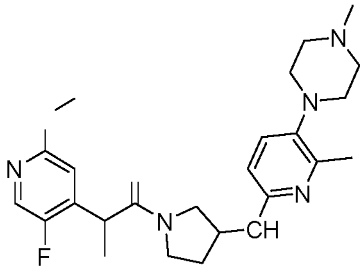
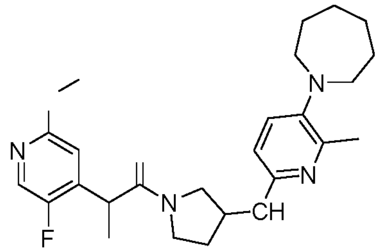

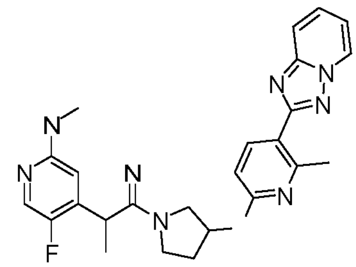
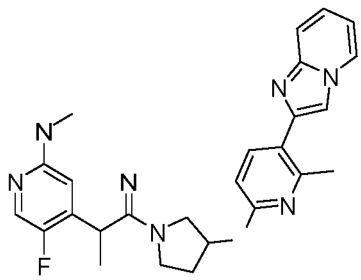
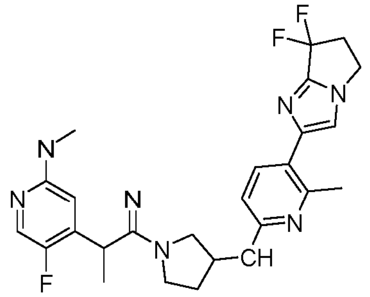

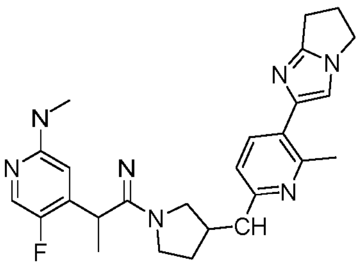
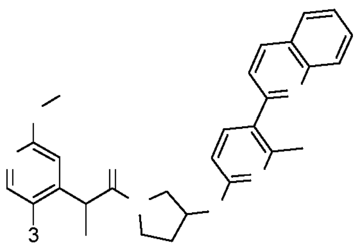
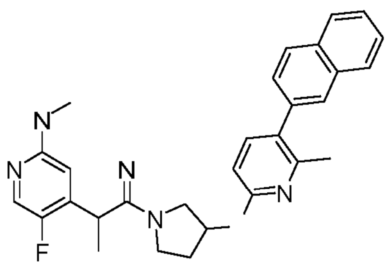

(dba)

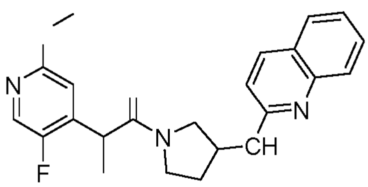
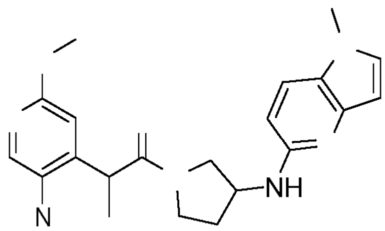
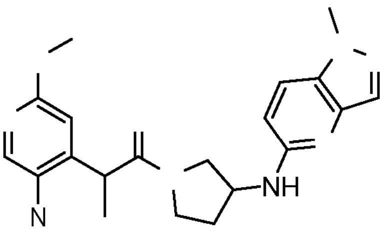

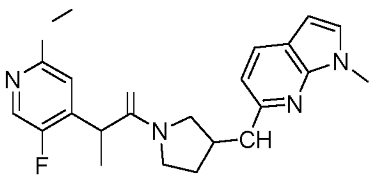
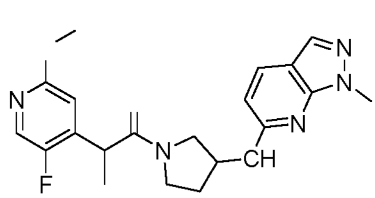
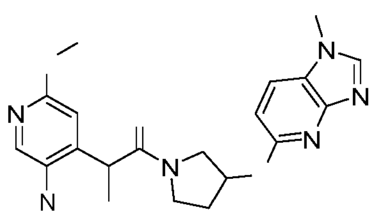

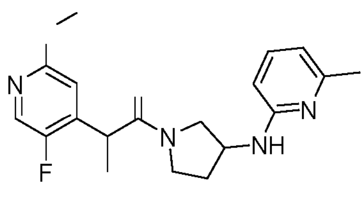

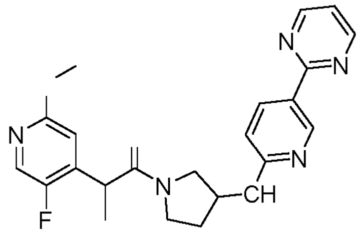

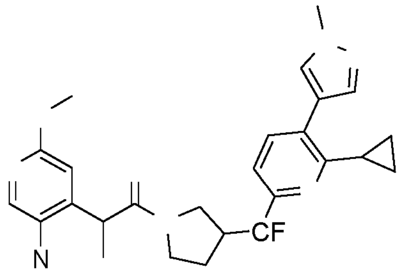

024

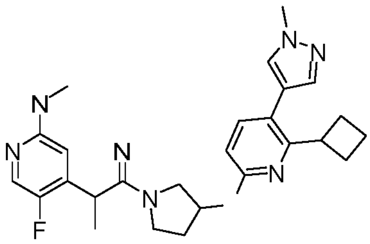

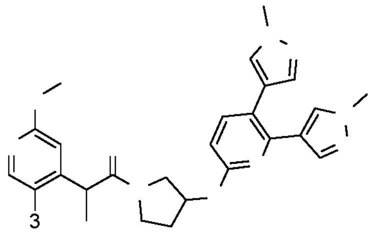

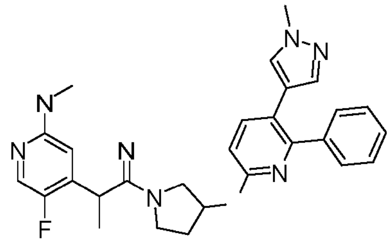

(dba)

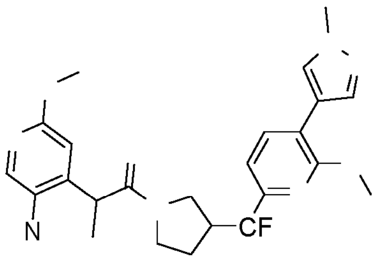

024

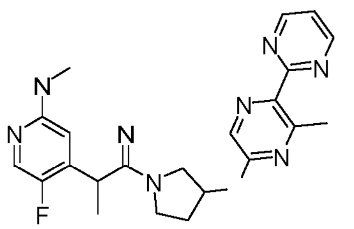

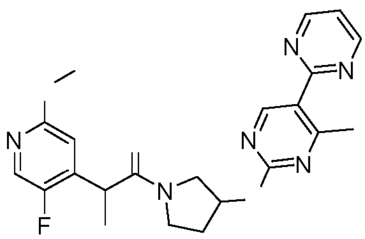

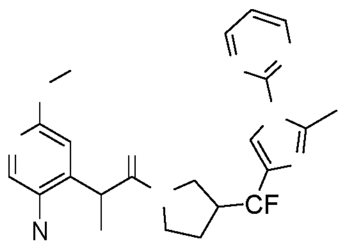

024

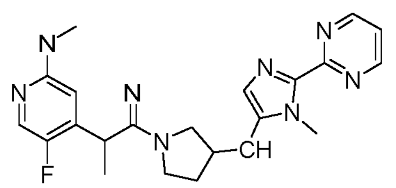

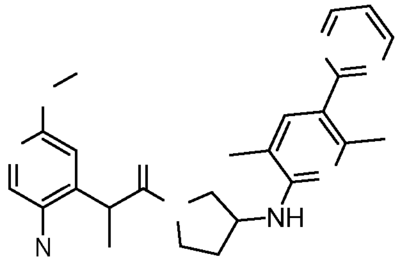

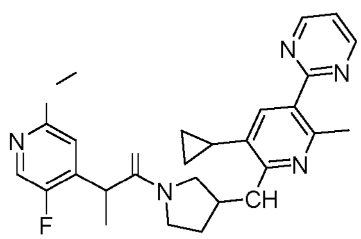

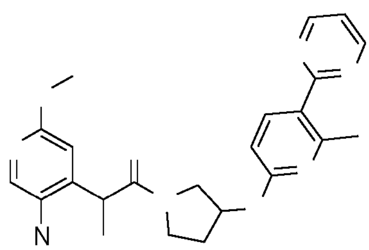

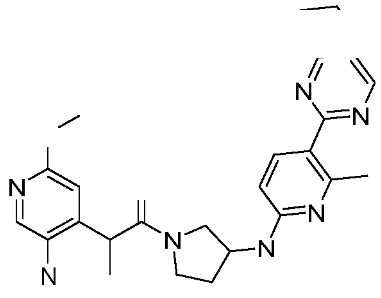

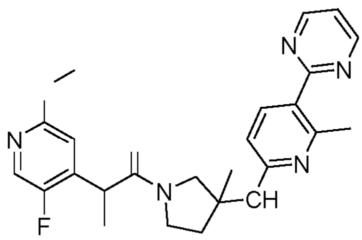

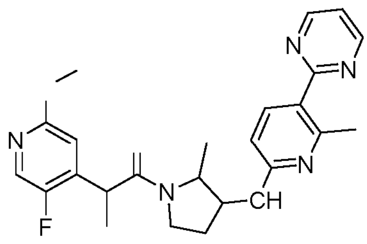

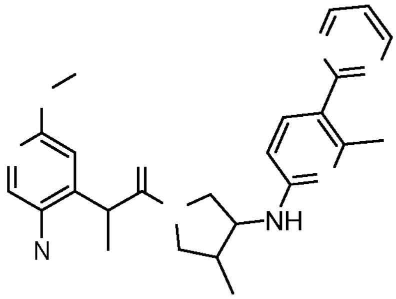

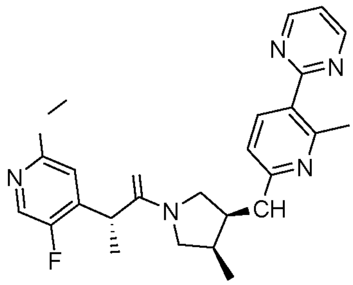

111-X

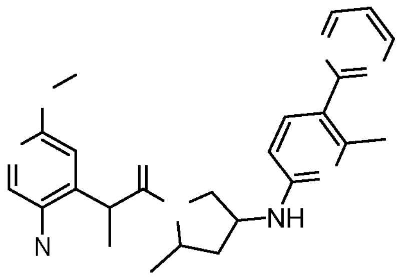

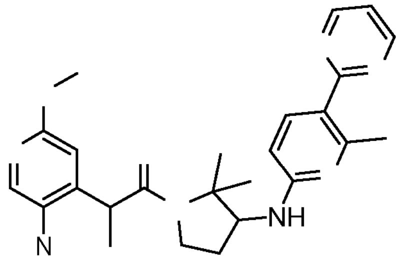

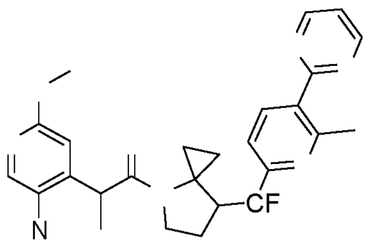

024

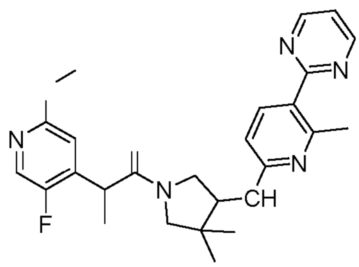

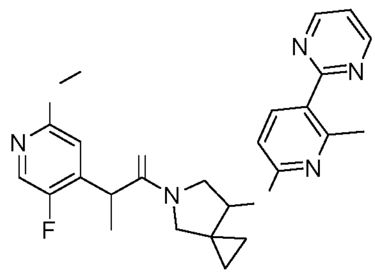

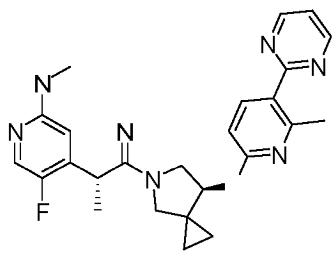

114-a

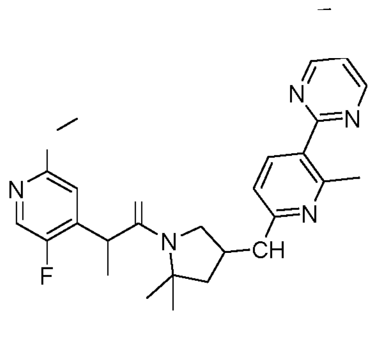

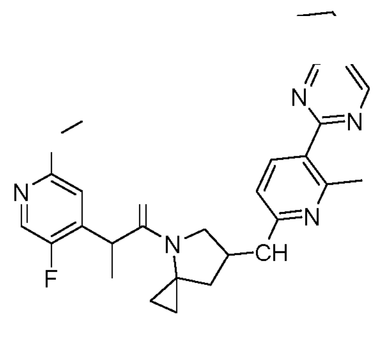

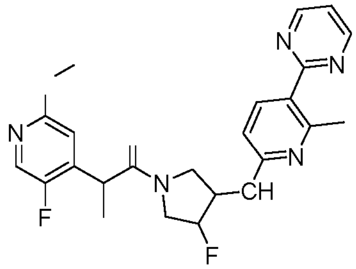

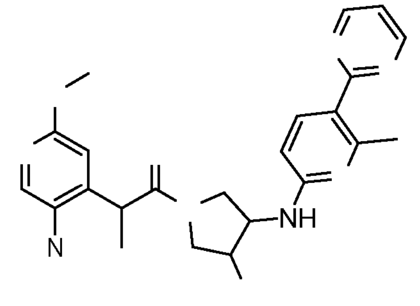

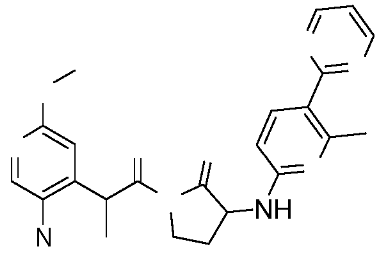

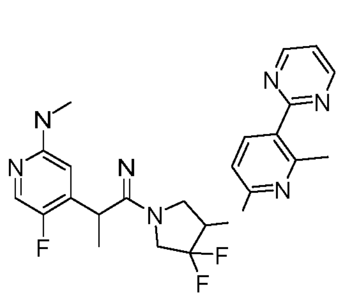

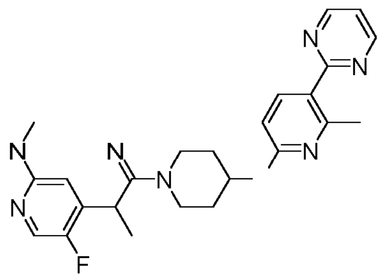

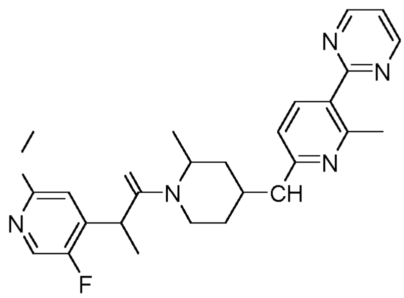

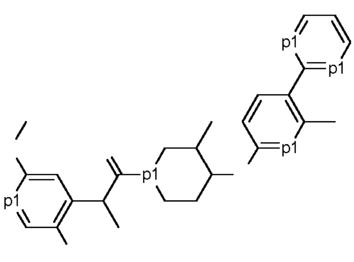

(dba)

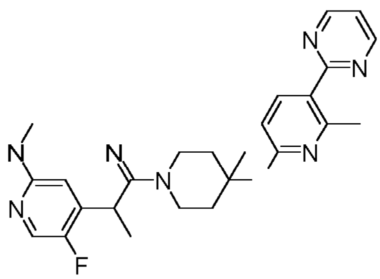

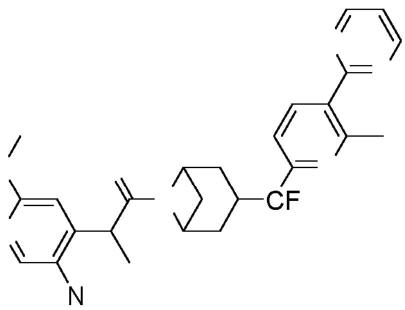

024

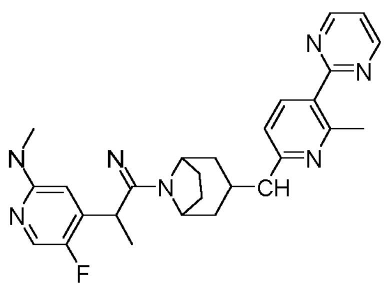

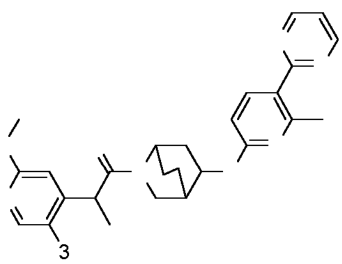

(dba)

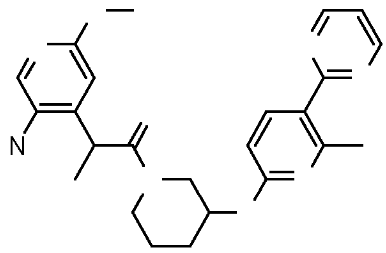

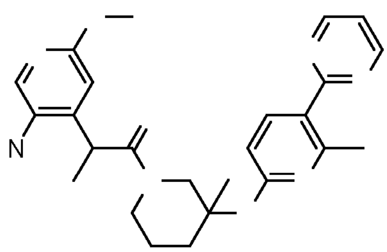

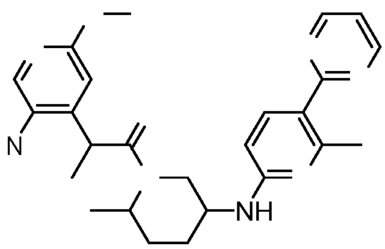

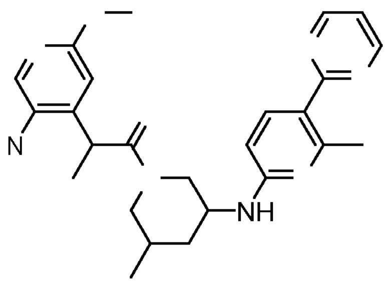

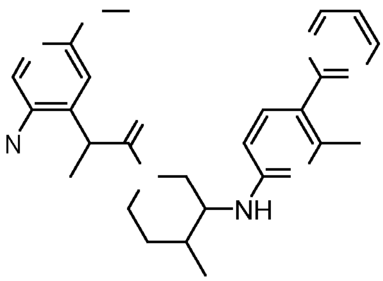

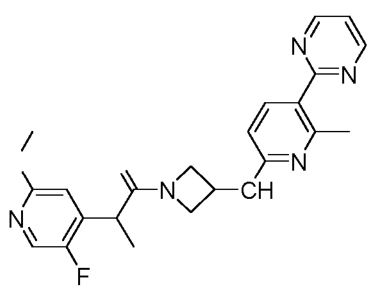

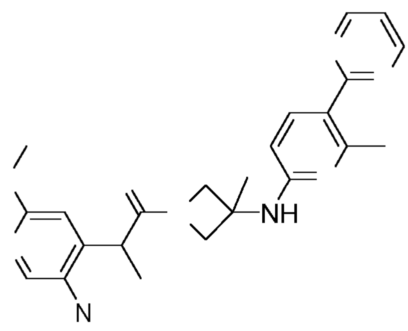

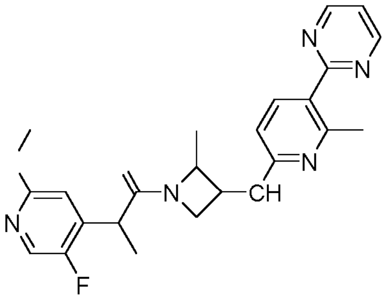

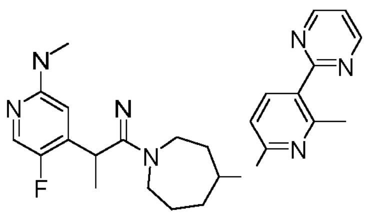

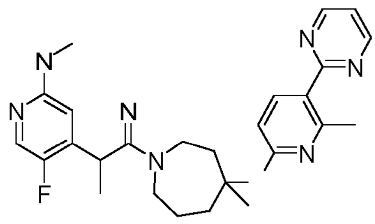

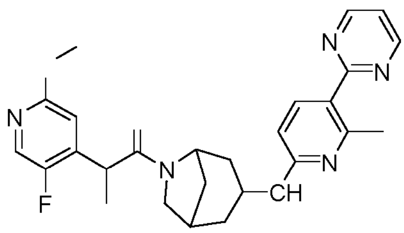

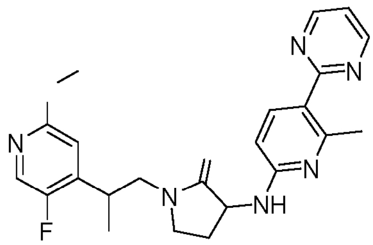

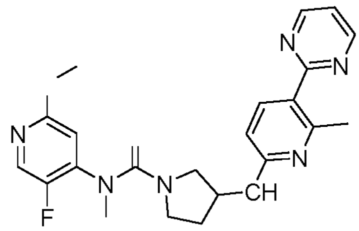

NH

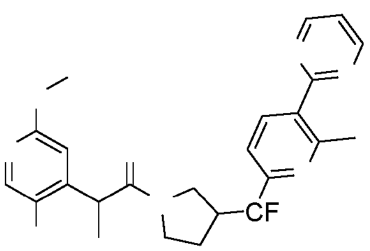

024

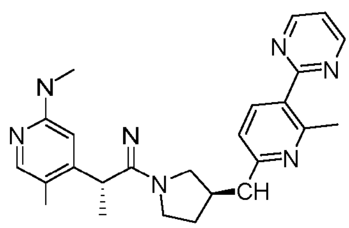

HATU,

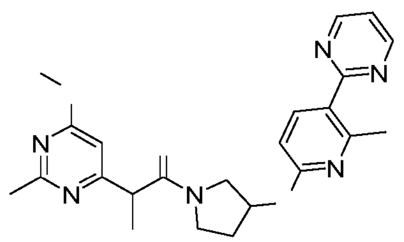

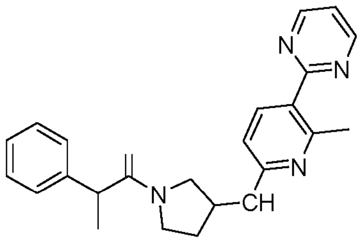

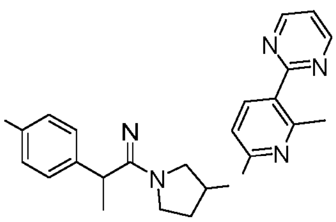

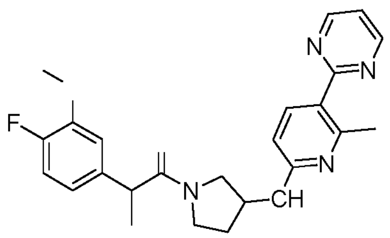

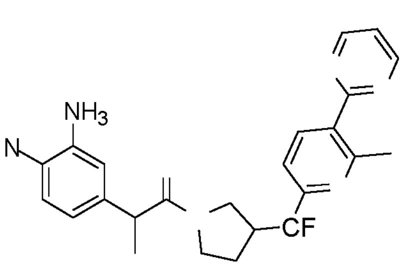

024

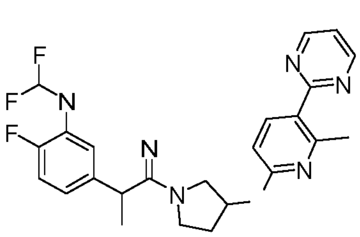

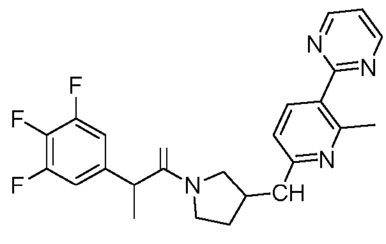

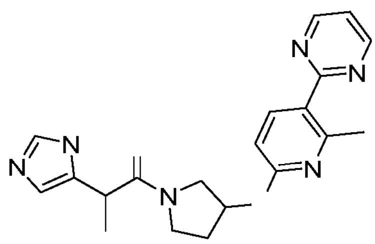

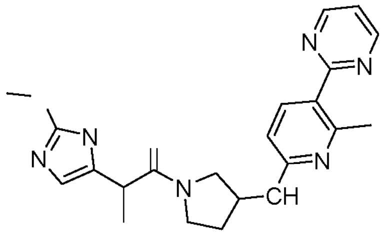

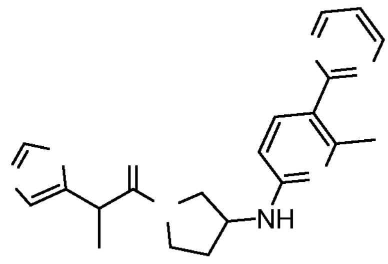

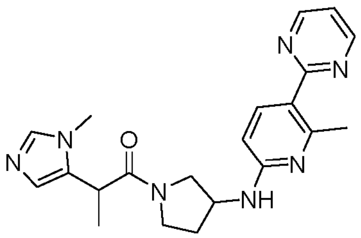

094

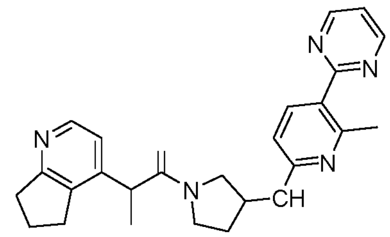

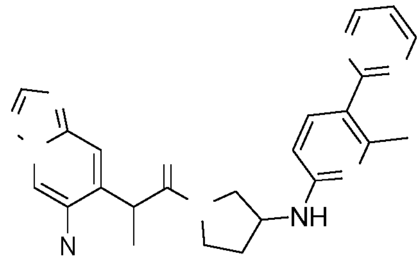

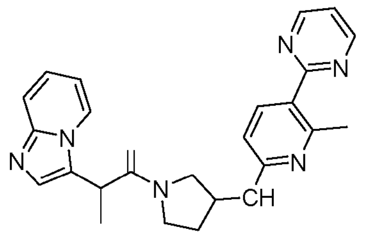

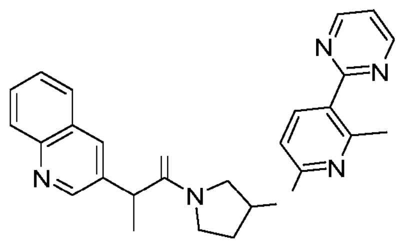

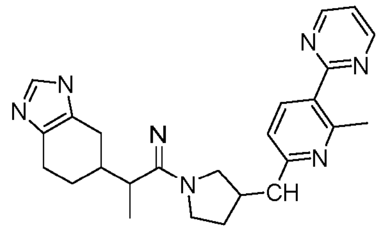

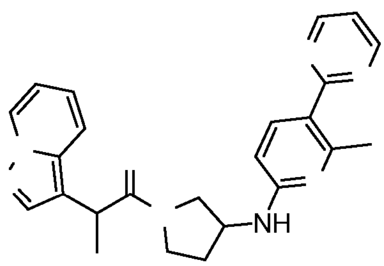

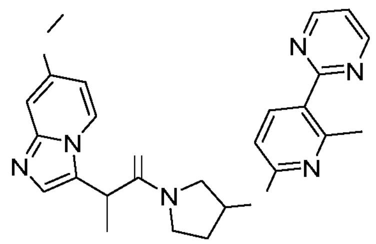

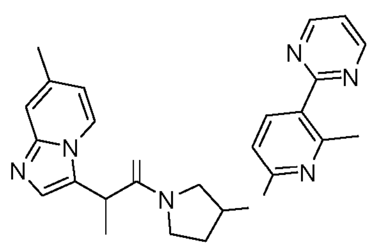

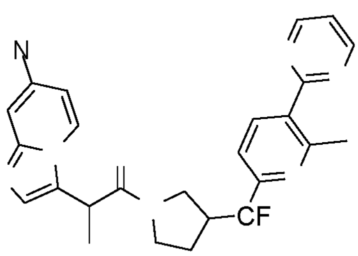

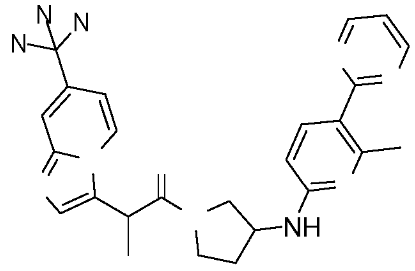

024

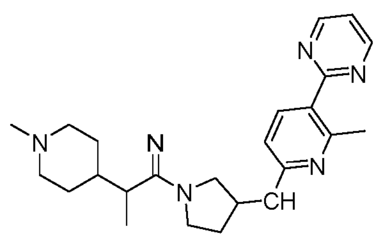

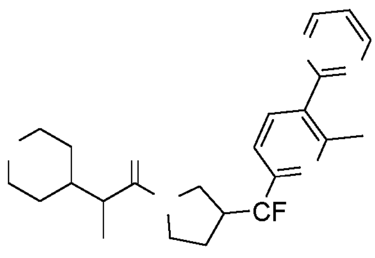
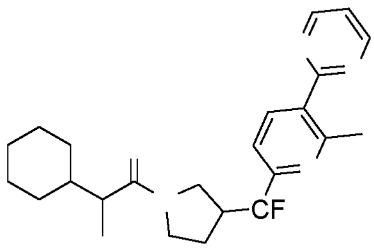
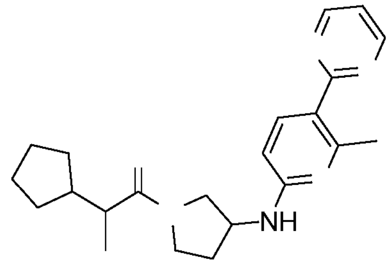

024

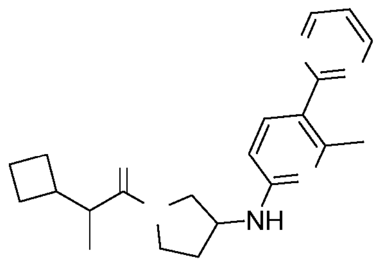

024

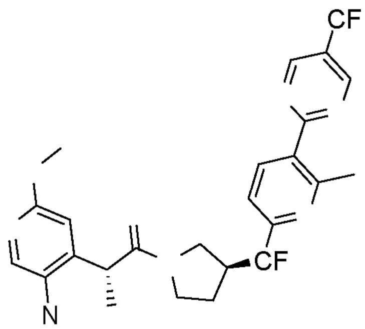
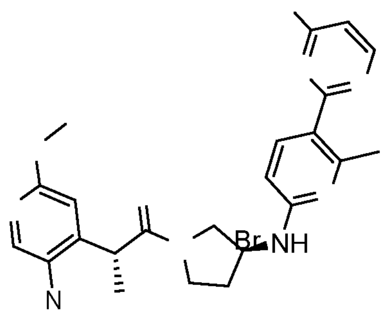

CF

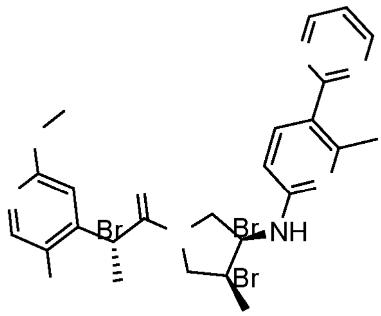
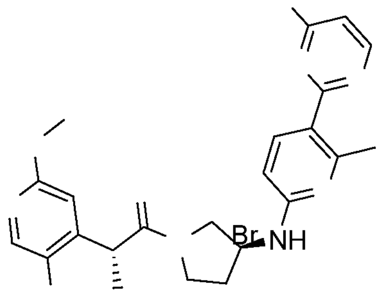
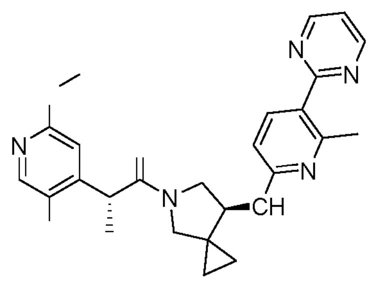

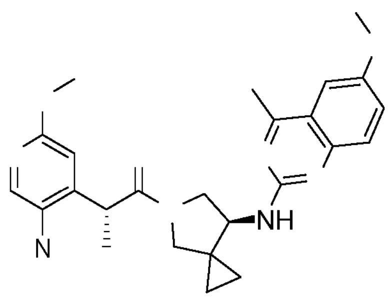
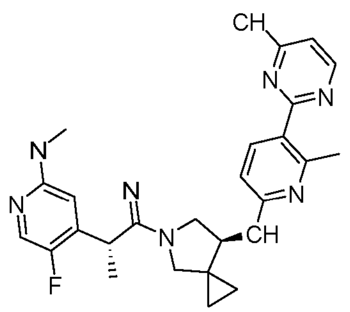
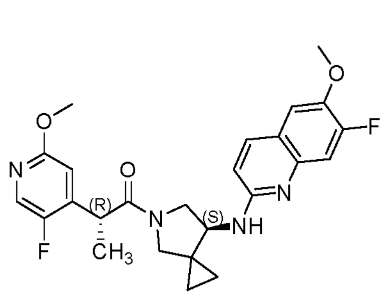

117

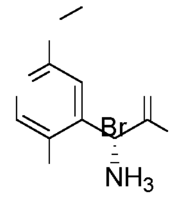
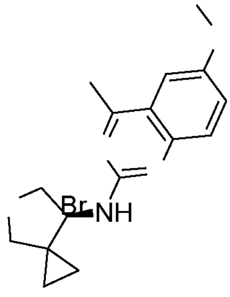
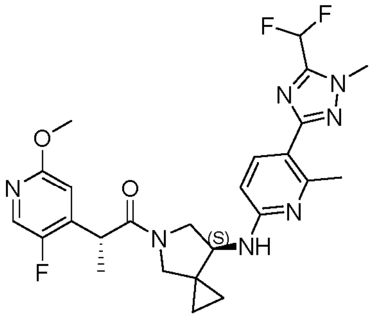
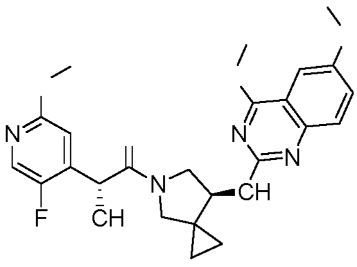

119

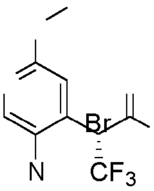
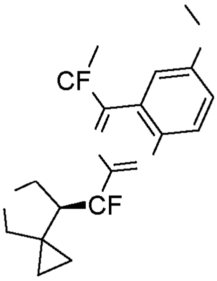
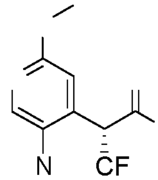
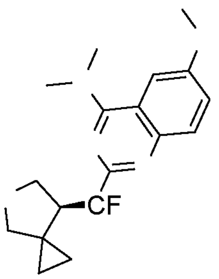
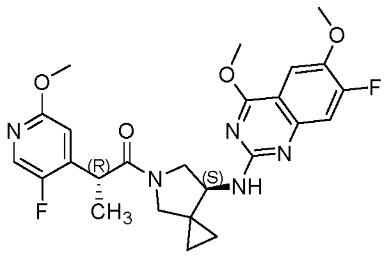

123

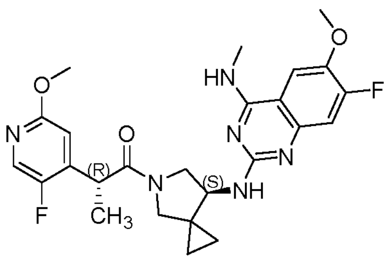

124

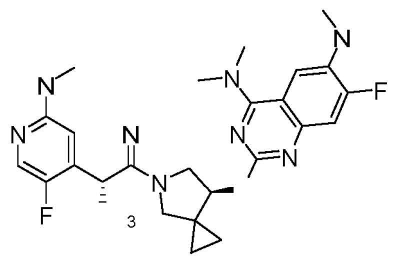
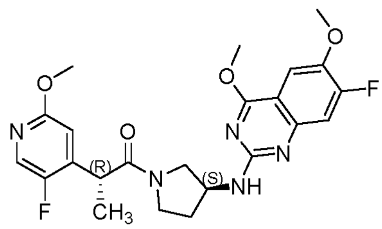

126

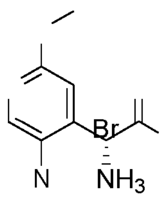
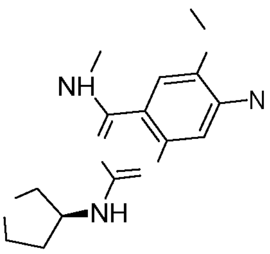

024

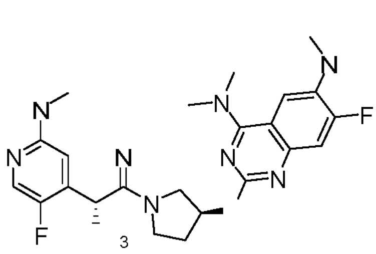
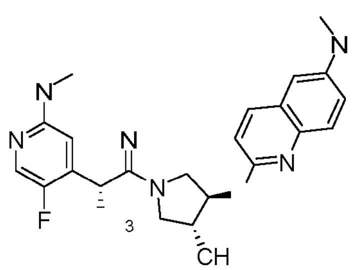

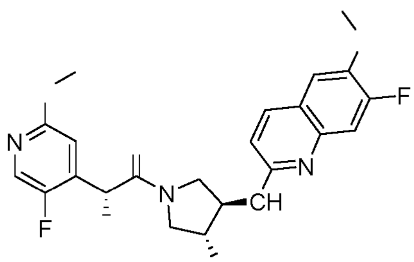
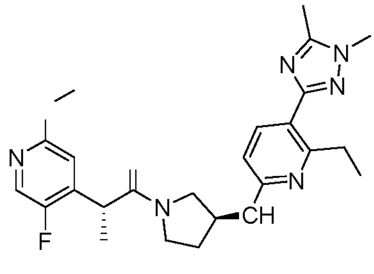
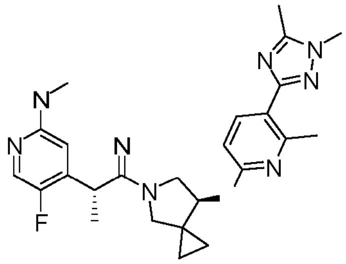

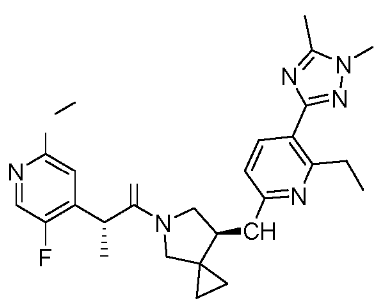

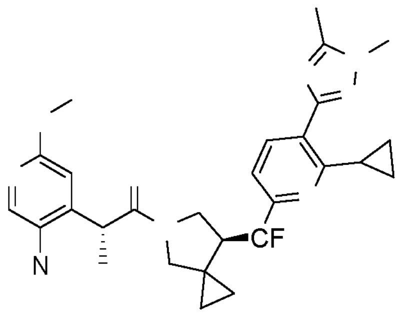

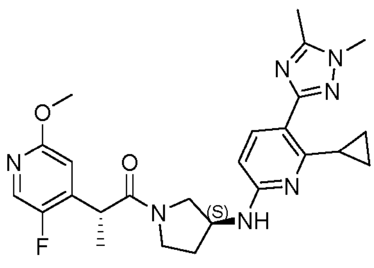

135

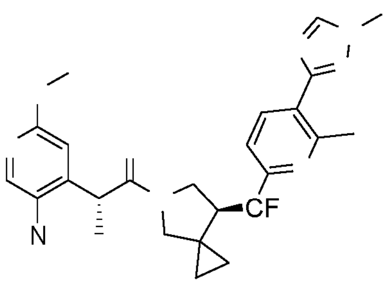

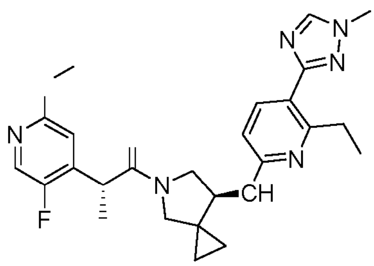

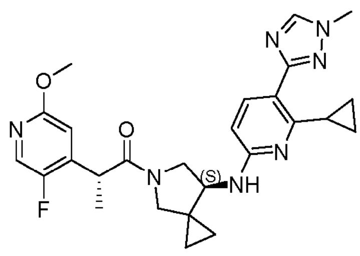

138

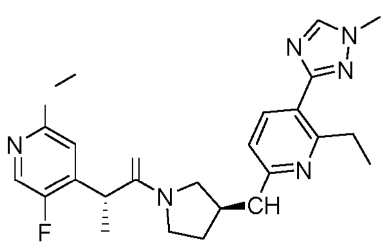

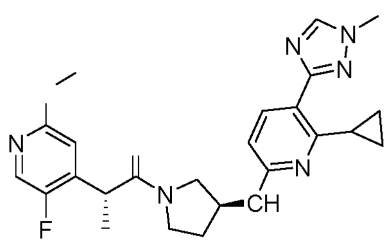

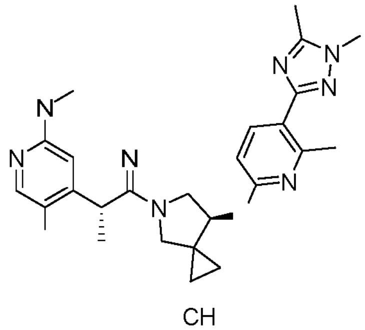

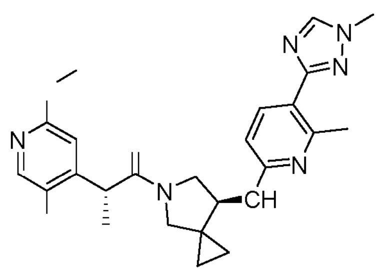

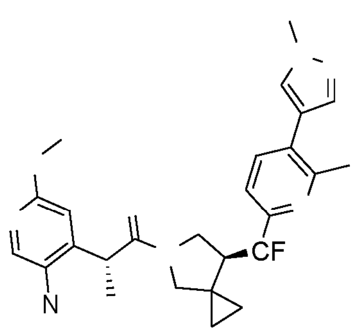

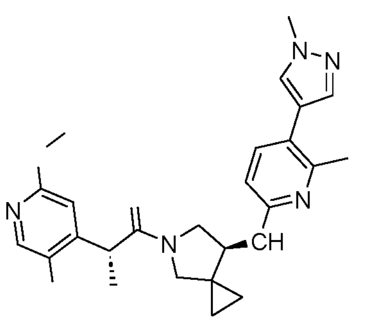

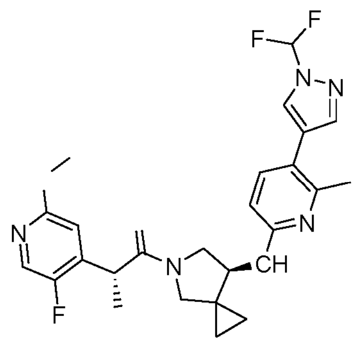

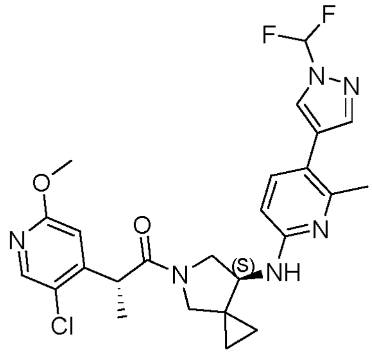

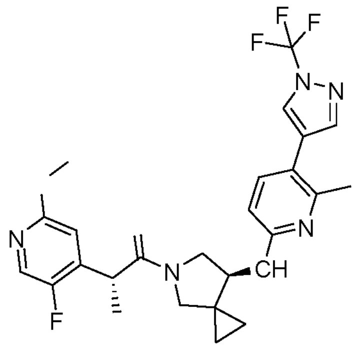

146

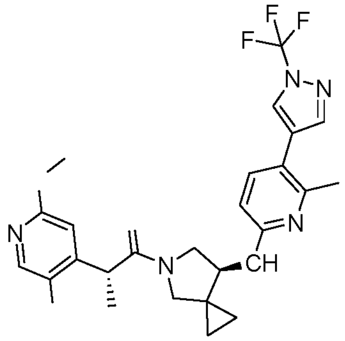

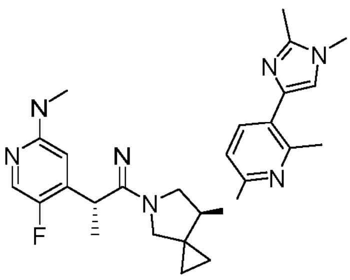

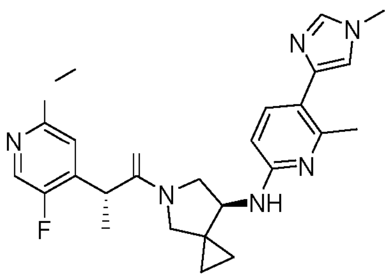

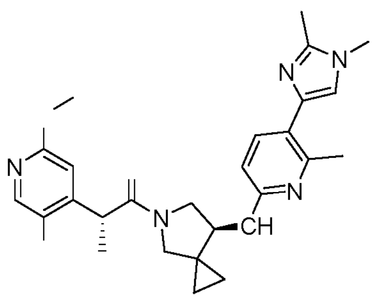

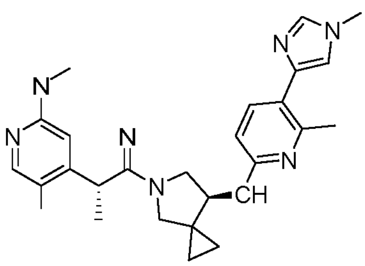

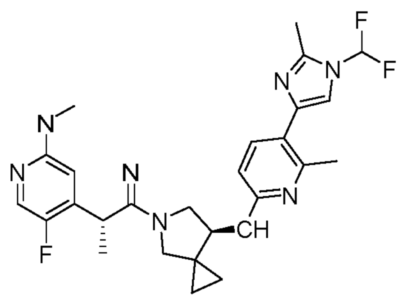

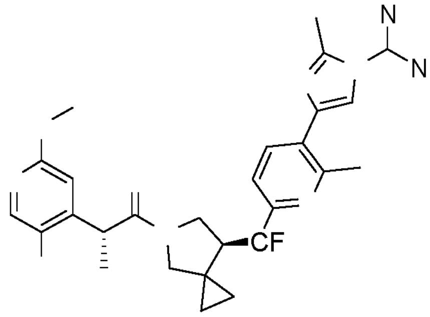

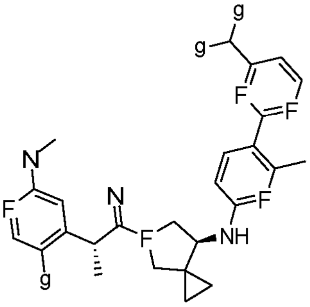

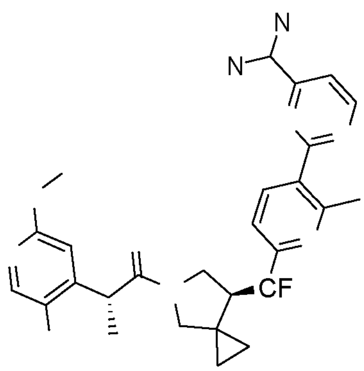

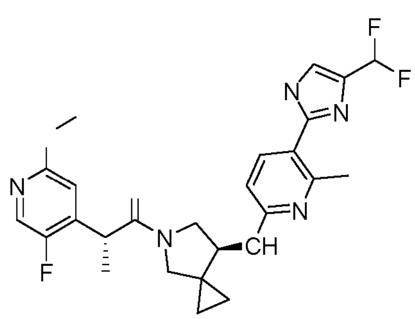

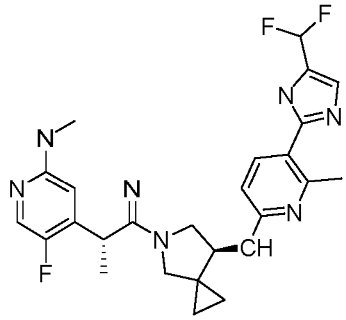

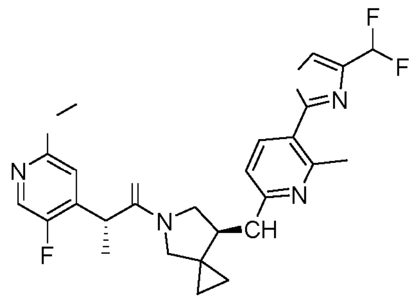

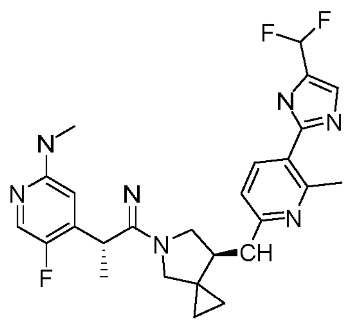

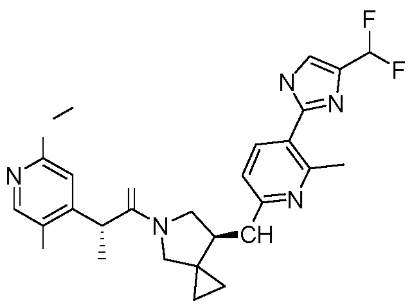

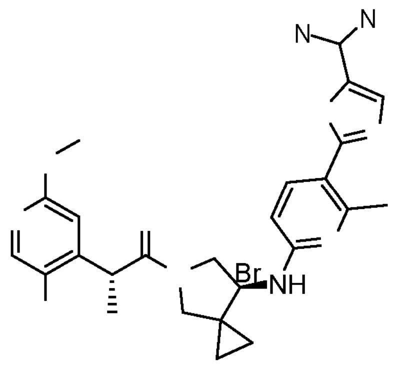

024

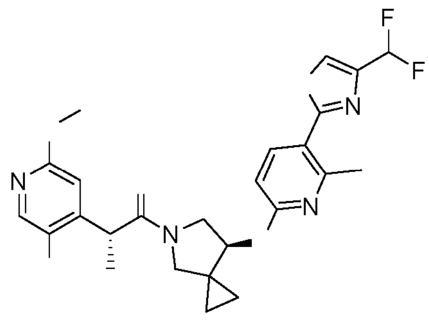

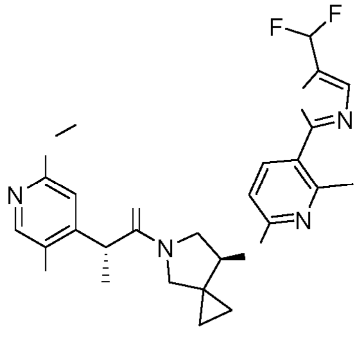

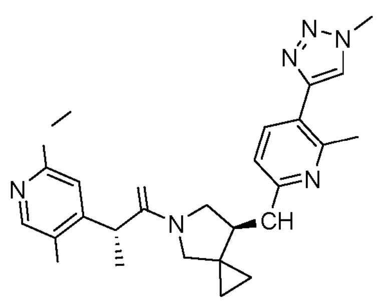

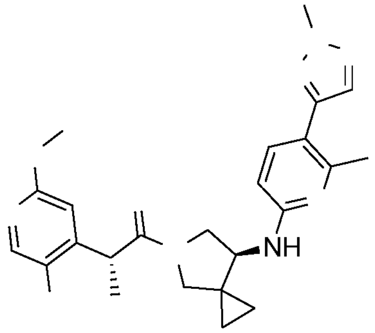

024

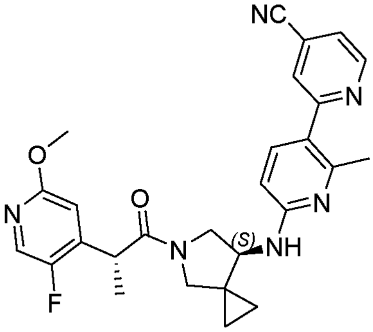

167

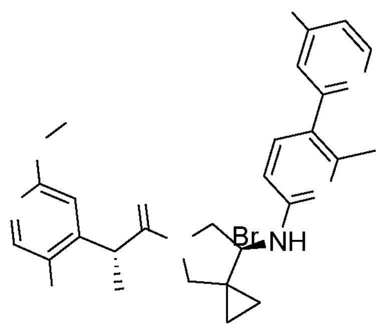

024

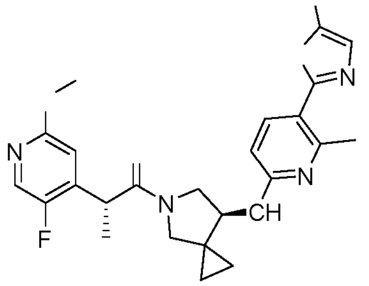

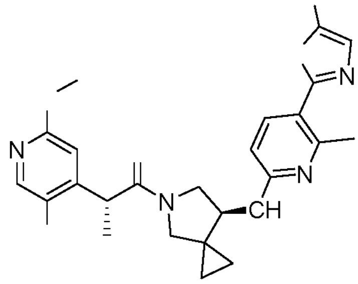

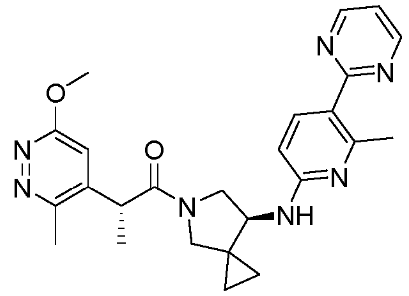

171

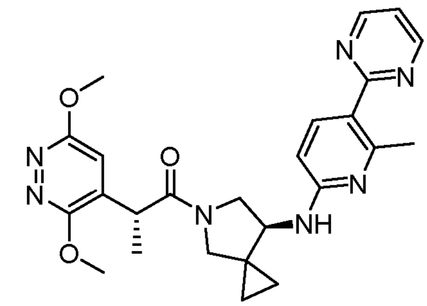

172

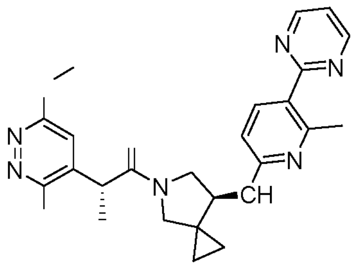

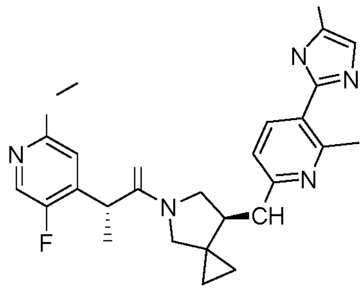

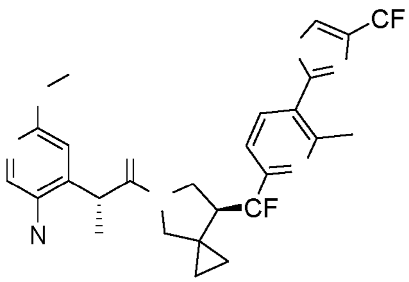

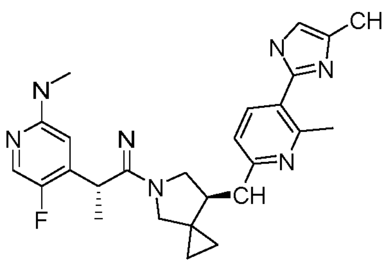

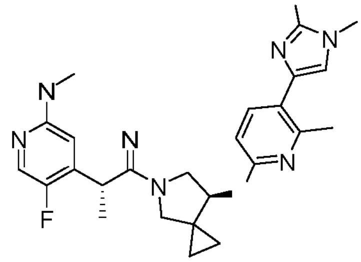

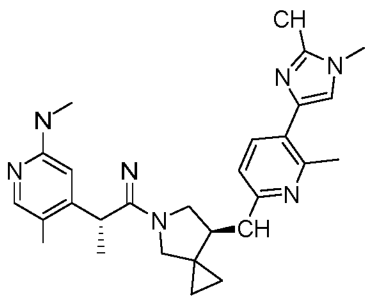

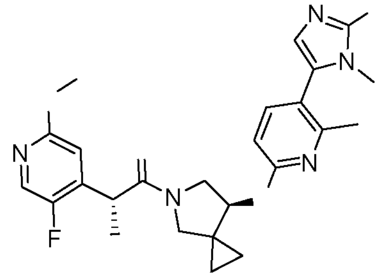

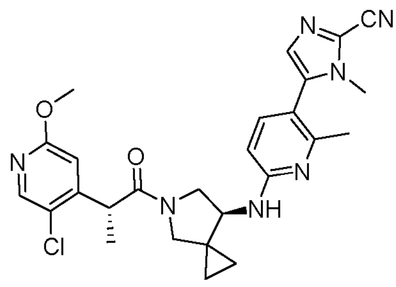

180

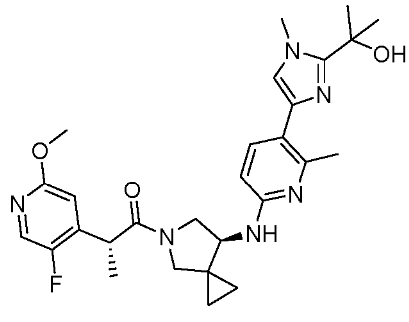

181

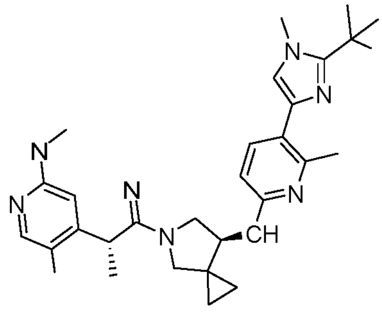

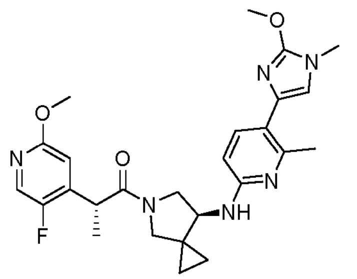

183

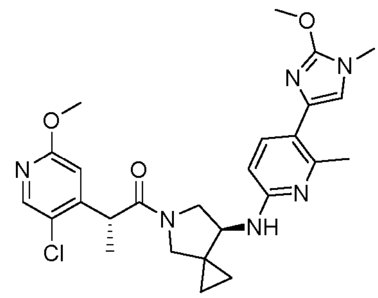

184

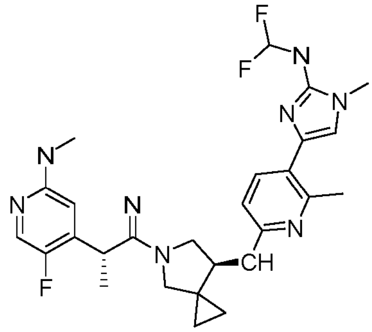

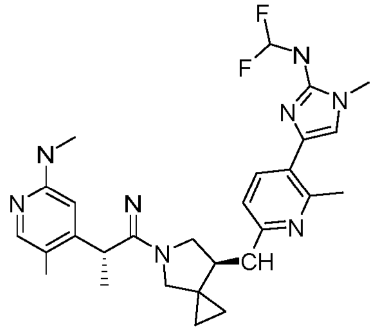

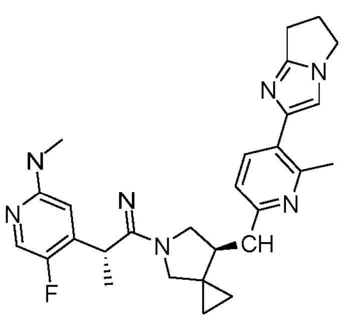

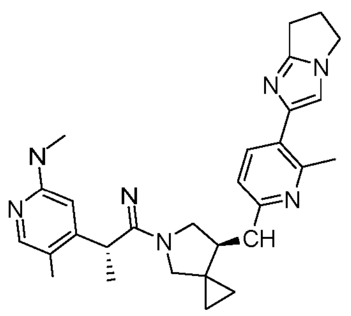

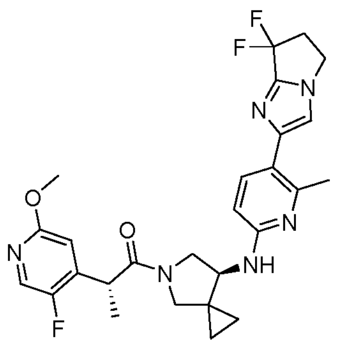

189

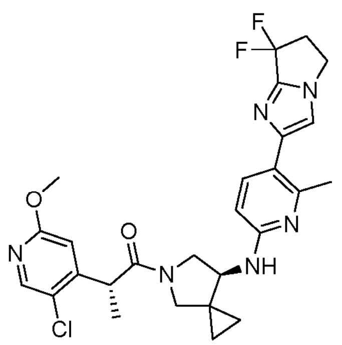

190

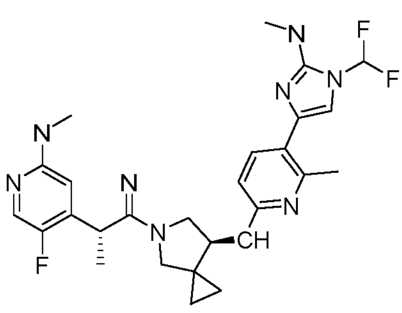

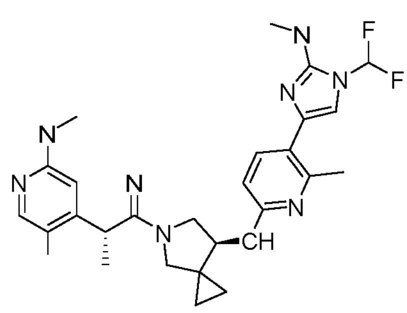

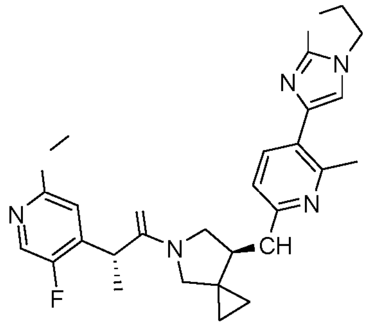

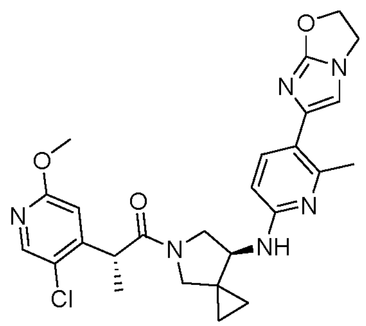

194

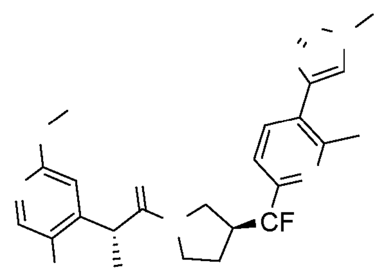

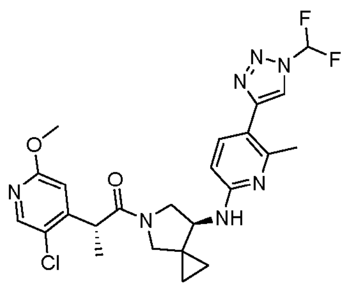

196

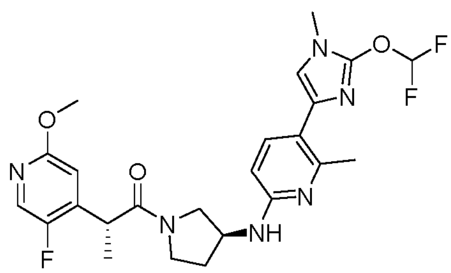

197

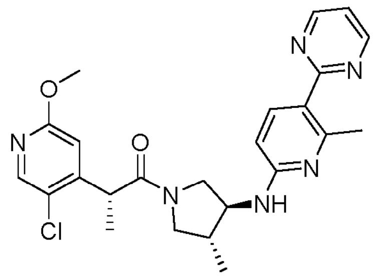

198

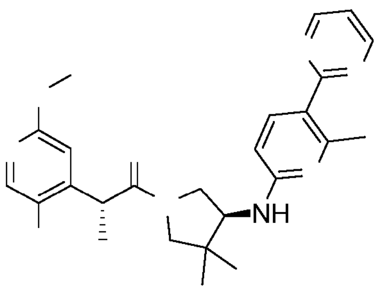

024

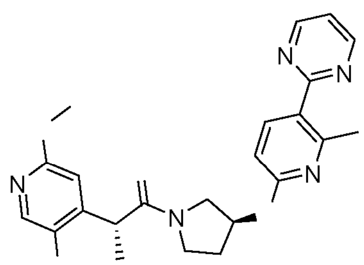

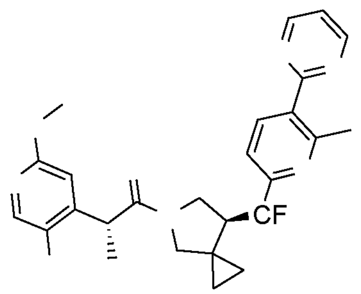

;

and/or the compound represented by formula I is selected from the following structures:

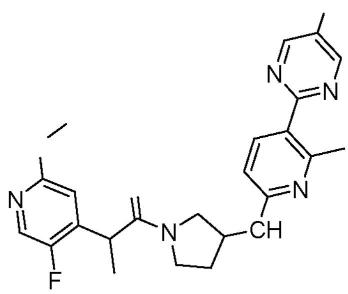

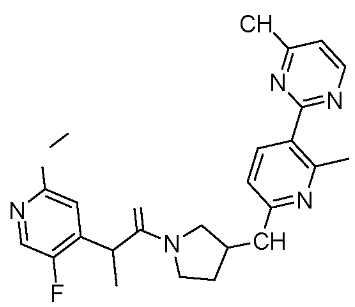

111-X

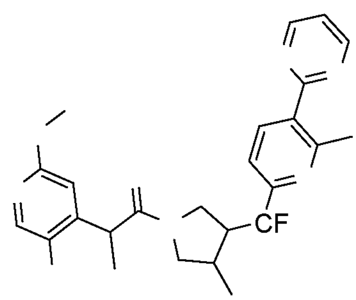

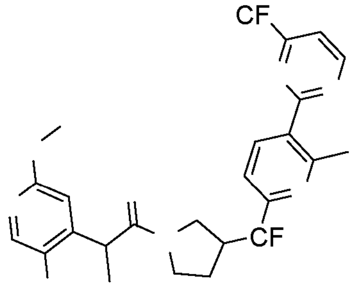

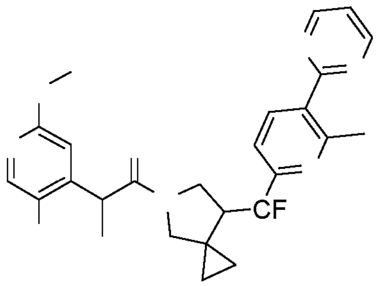

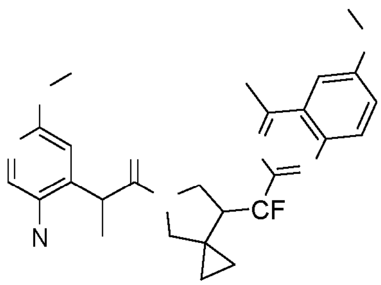

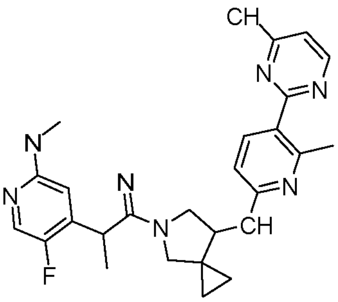

111-X

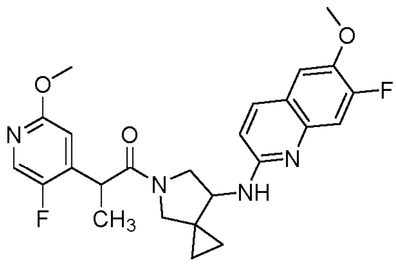

117-X

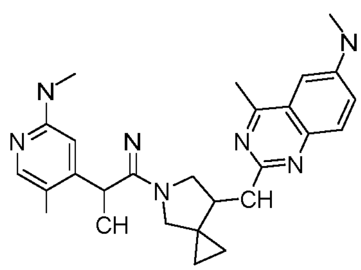

111-X

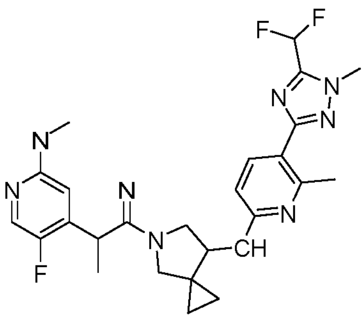

111-X

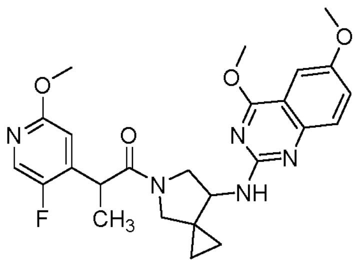

120-X

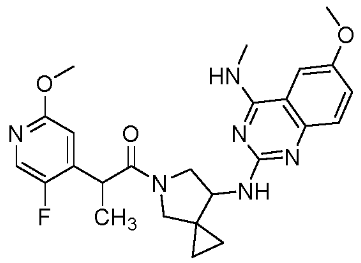

121-X

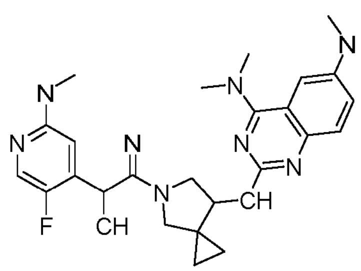

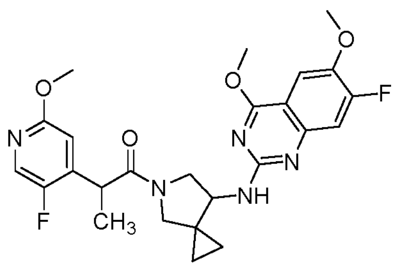

123-X

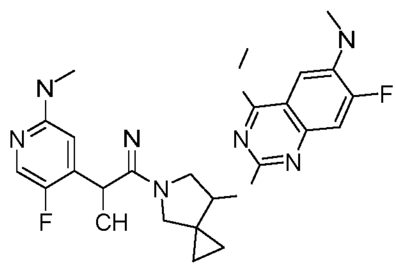

111-X

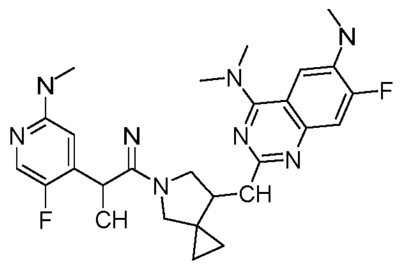

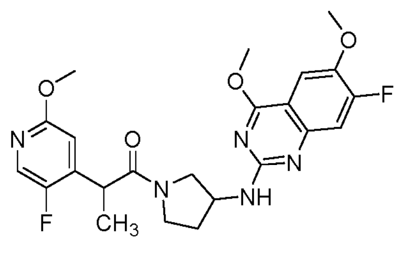

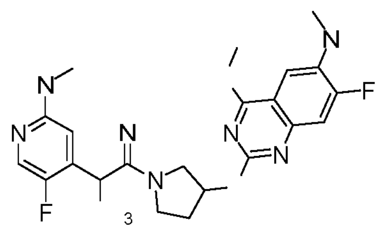

126-X

111-X

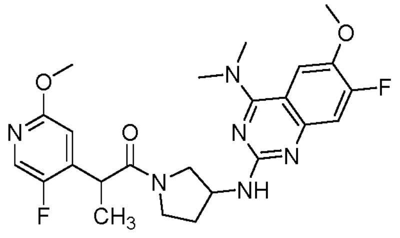

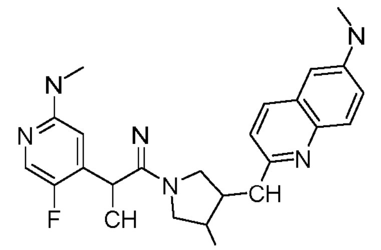

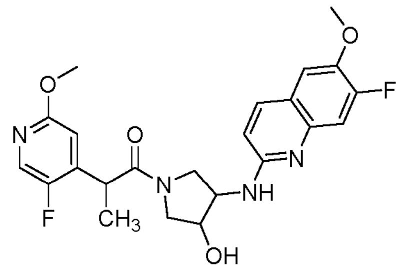

128-X

130-X

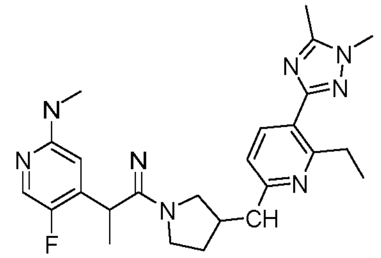

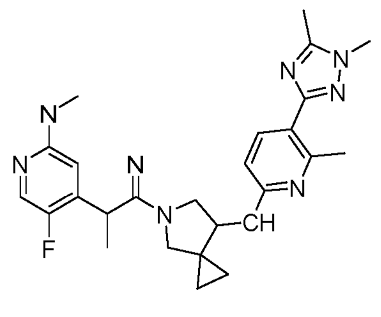

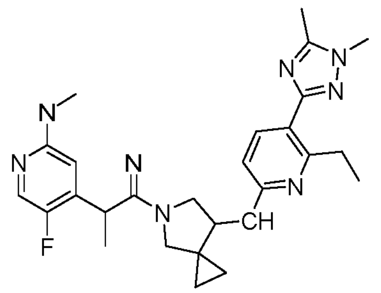

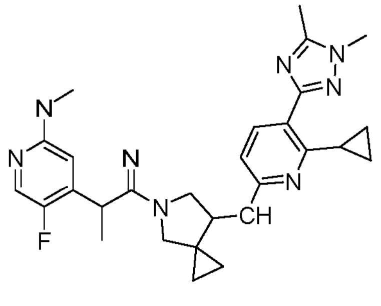

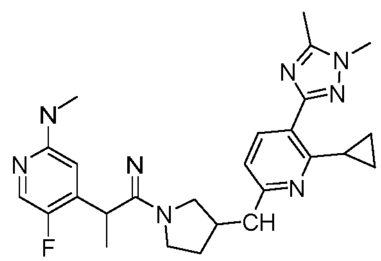

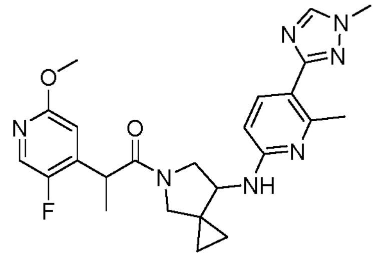

136-X

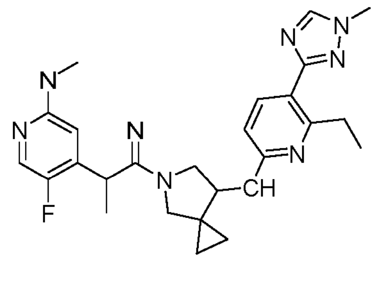

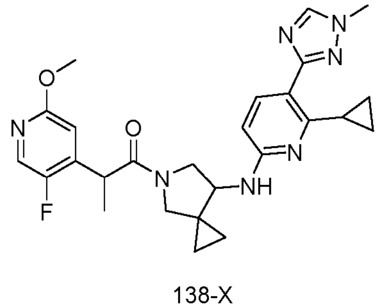

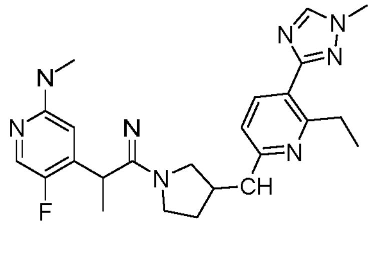

138-X

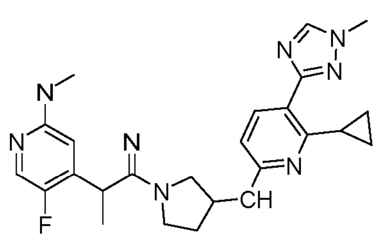
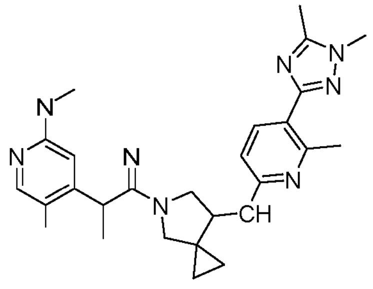
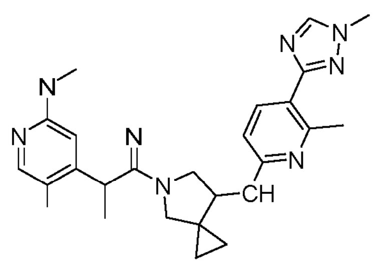

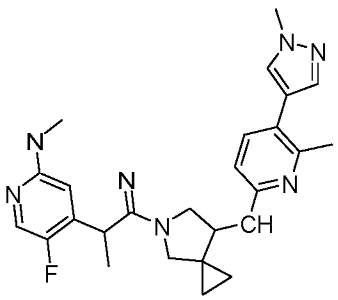
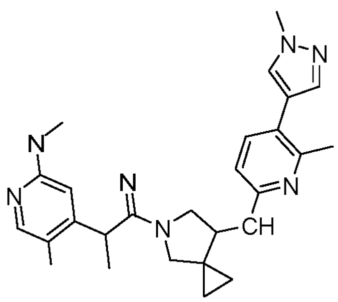
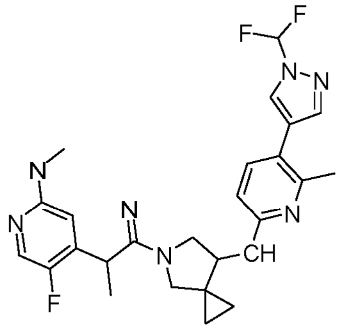

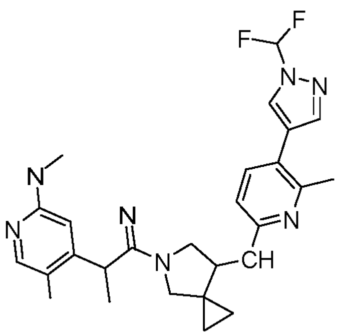
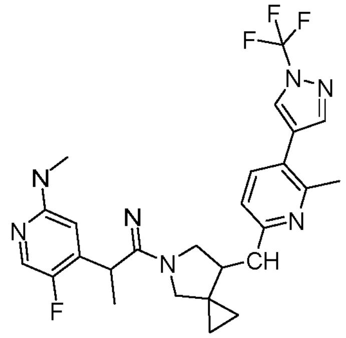
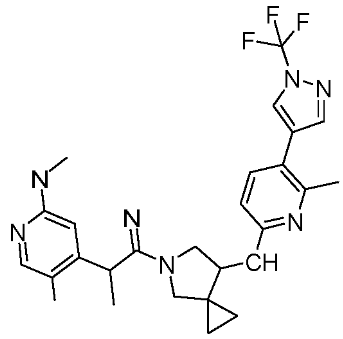

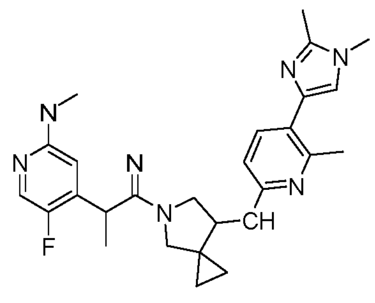
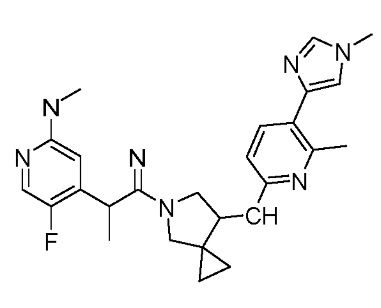
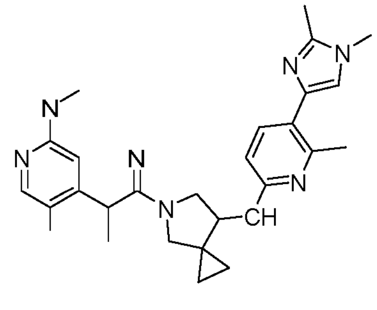

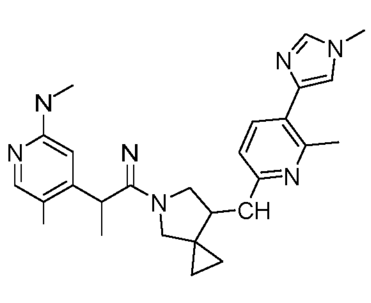
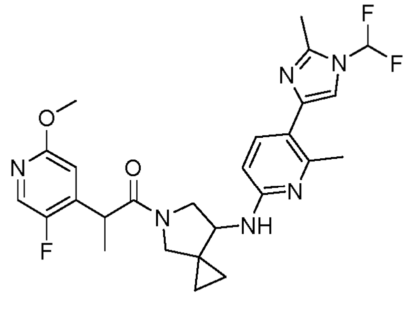

153-X

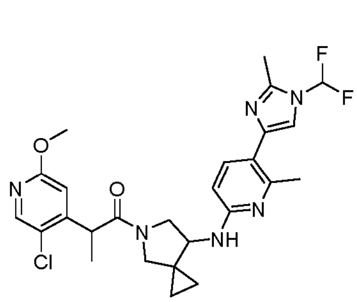

154-X

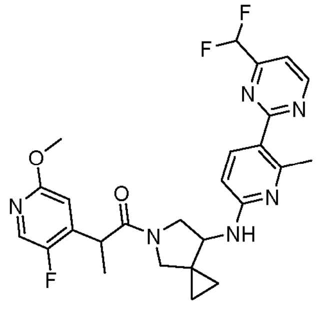

155-X

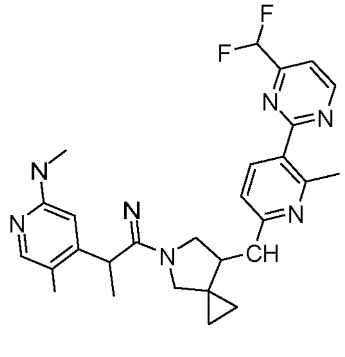

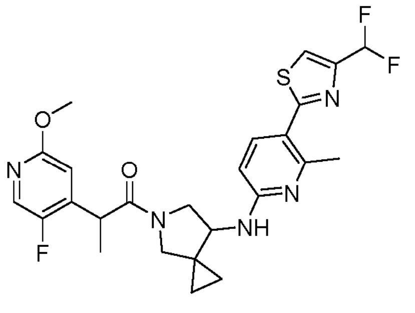

157-X

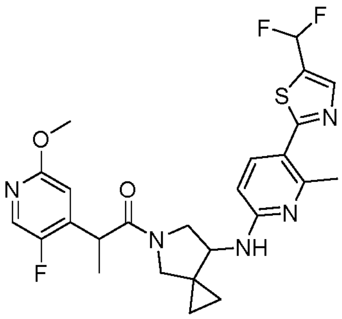

158-X

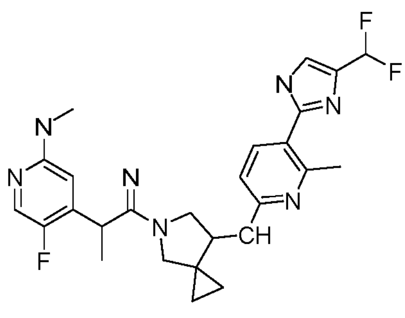

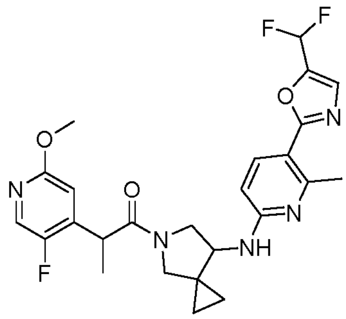

160-X

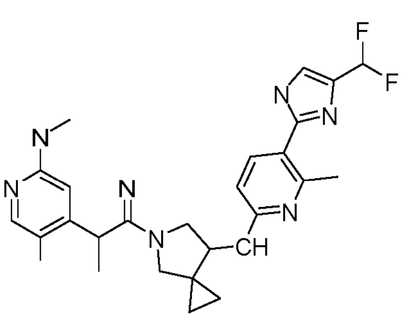

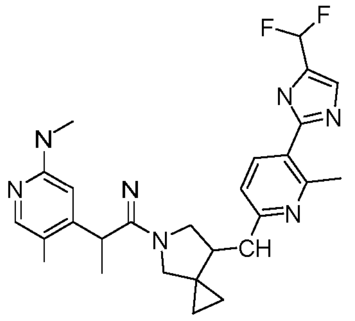

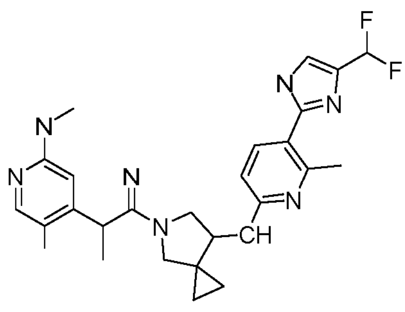

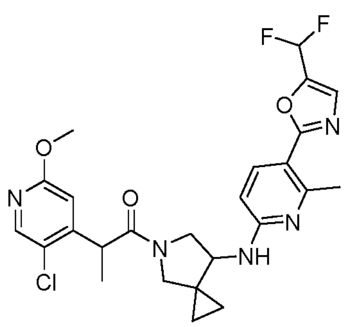

164-X

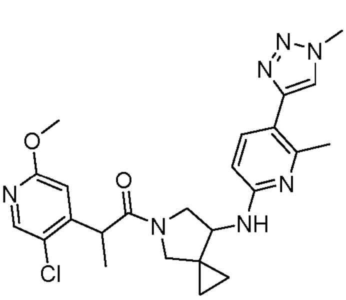

165-X

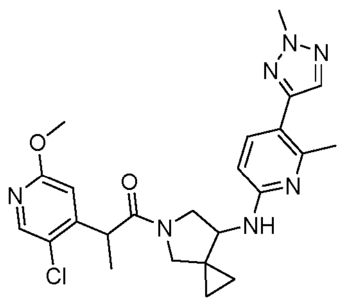

166-X

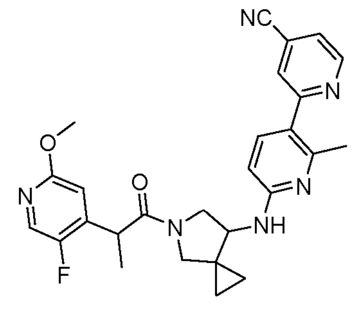

167-X

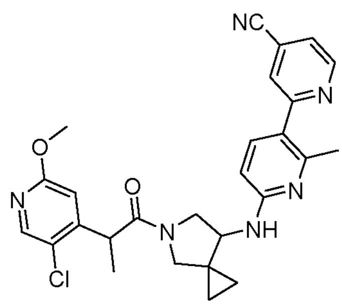

168-X

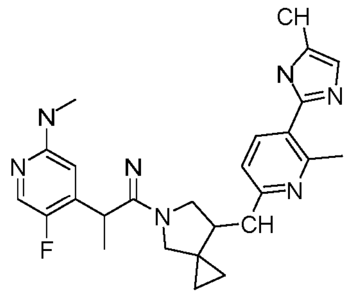

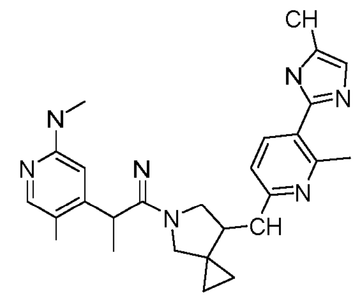

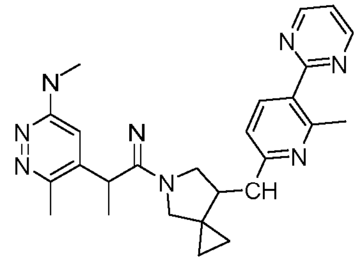

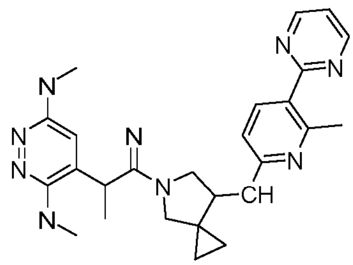

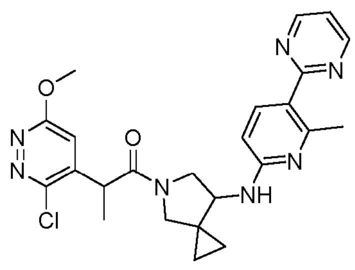

173-X

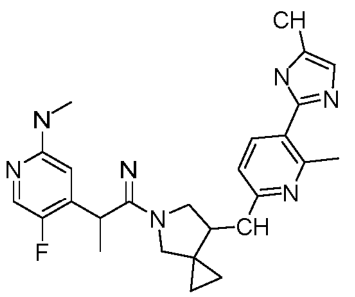

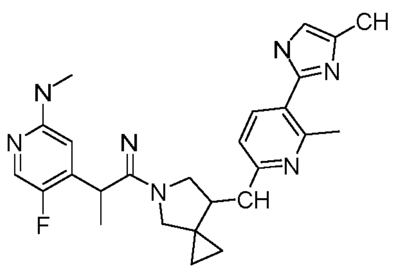

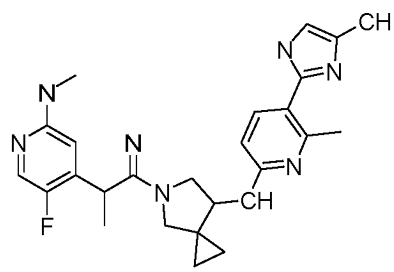

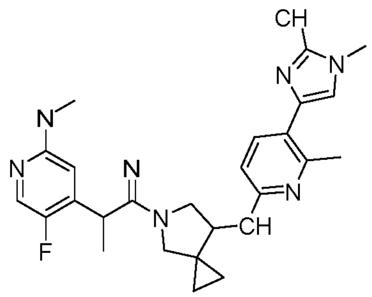

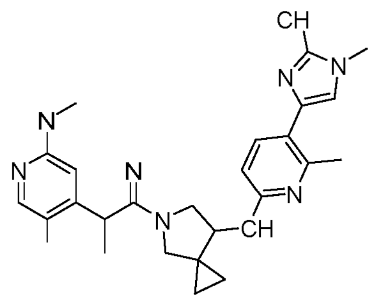

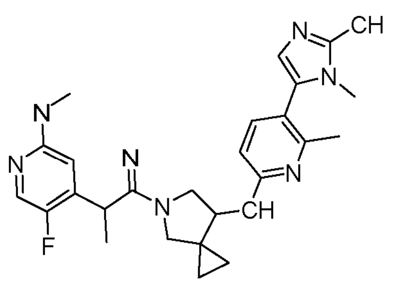

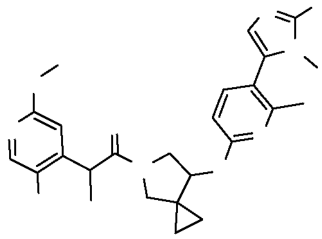

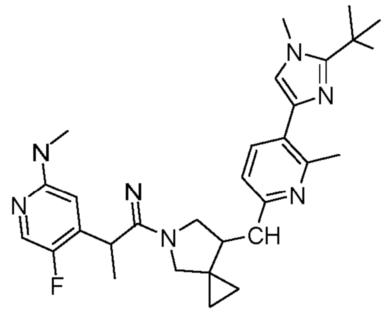

180-X

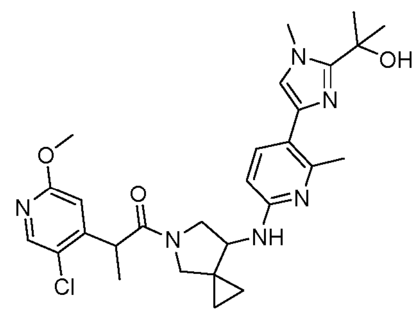

182-X

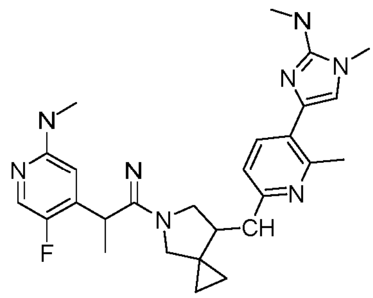

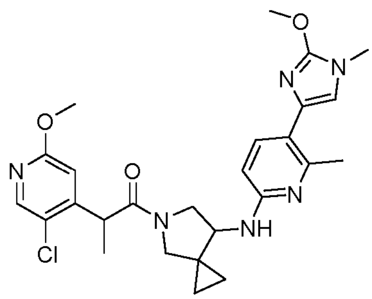

184-X

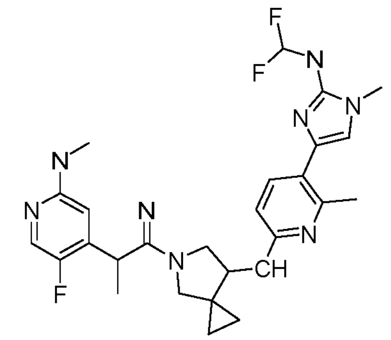

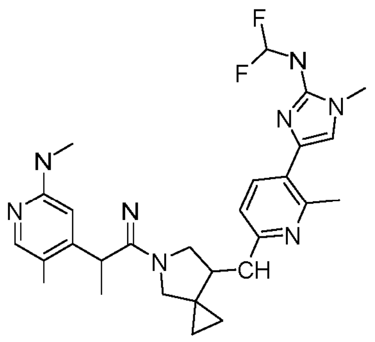

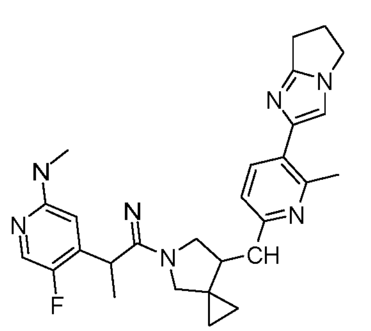

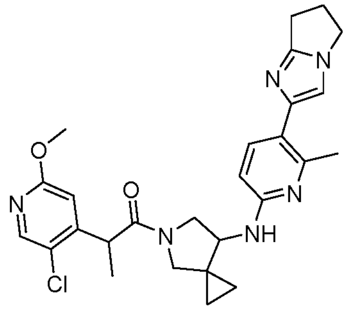

188-X

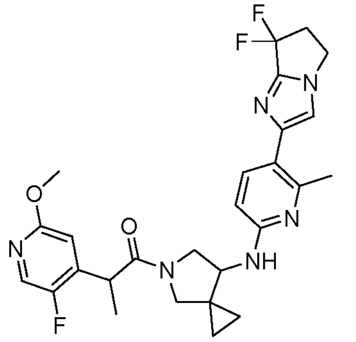

189-X

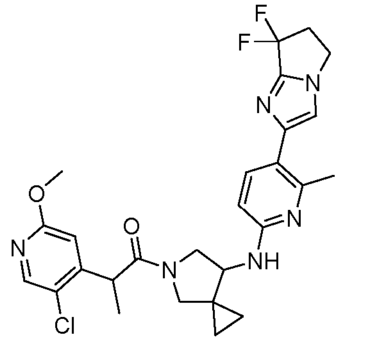

190-X

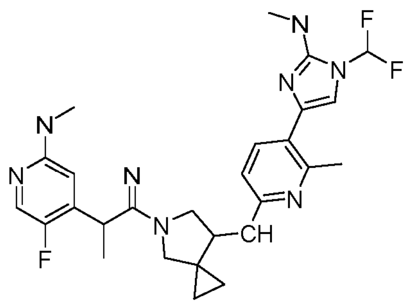

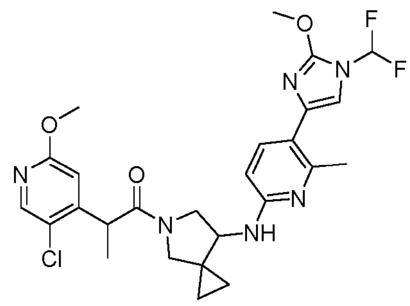

192-X

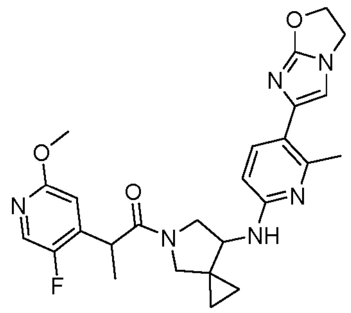

193-X

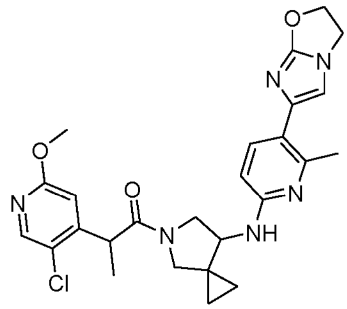

194-X

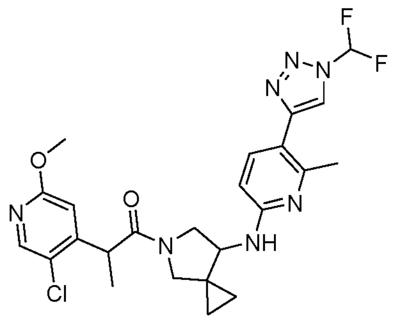

196-X

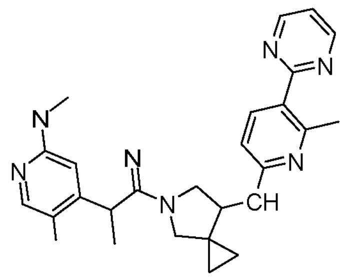

**8.** A method for preparing the compound according to any one of claims 1 to 7, comprising the following step:

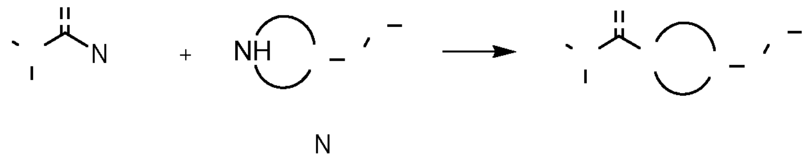

wherein X, Y, R, A, E, G, and M are as defined according to any one of claims 1 to 7; and L is selected from a leaving group, such as OH, OTf, F, Cl, Br, I, tributyltin ($SnBu_3$), 4,4,5,5-tetramethyl-1,3,2-dioxaborolan-2-yl, or methylsulfonyl;

preferably, the preparation method comprises the following step:

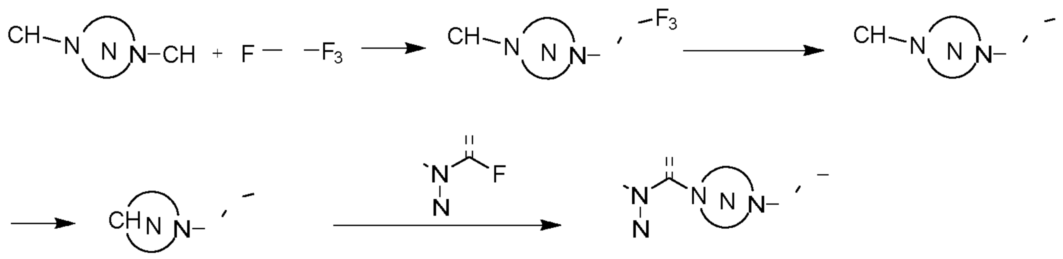

wherein X, Y, R, A, E, G, and M are as defined according to any one of claims 1 to 7; L, $L_1$, $L_2$, and $L_3$ are each selected from a leaving group, such as OH, OTf, F, Cl, Br, I, tributyltin ($SnBu_3$), 4,4,5,5-tetramethyl-1,3,2-dioxaborolan-2-yl, or methylsulfonyl; and PG is selected from an amino-protecting group, such as benzyloxycarbonyl (Cbz), tert-butoxycarbonyl (Boc), fluorenylmethoxycarbonyl (Fmoc), benzyl (Bn), or p-methoxybenzyl (PMB);

preferably, the preparation method comprises the following step:

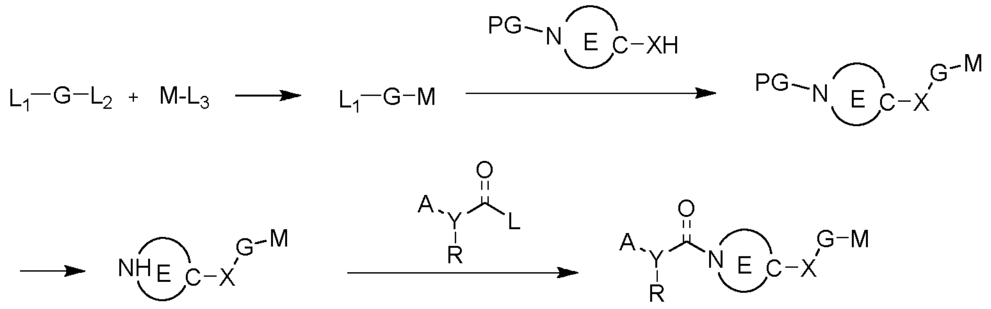

wherein X, Y, R, A, E, G, and M are as defined according to any one of claims 1 to 7; L, $L_1$, $L_2$, and $L_3$ are each selected from a leaving group, such as OH, OTf, F, Cl, Br, I, tributyltin ($SnBu_3$), 4,4,5,5-tetramethyl-1,3,2-dioxaborolan-2-yl, or methylsulfonyl; and PG is selected from an amino-protecting group, such as benzyloxycarbonyl (Cbz), tert-butoxycarbonyl (Boc), fluorenylmethoxycarbonyl (Fmoc), benzyl (Bn), or p-methoxybenzyl (PMB);

preferably, the preparation method comprises the following step:

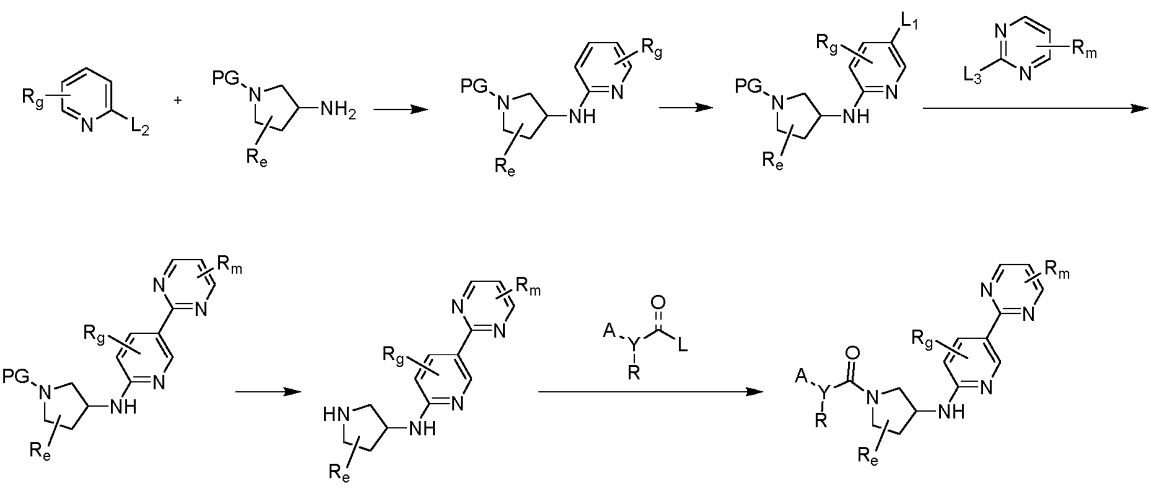

wherein Y, R, A, $R_e$, $R_g$, $R_m$, PG, L, $L_1$, $L_2$, and $L_3$ are as defined according to any one of claims 1 to 8.

**9.** A pharmaceutical composition, comprising the compound according to any one of claims 1 to 7, and a racemate, a stereoisomer, a tautomer, an isotopic label, a nitrogen oxide, a solvate, a polymorph, a metabolite, an ester, a prodrug, or a pharmaceutically acceptable salt thereof.

**10.** Use of the compound according to any one of claims 1 to 7 and a racemate, a stereoisomer, a tautomer, an isotopic label, a nitrogen oxide, a solvate, a polymorph, a metabolite, an ester, a prodrug, or a pharmaceutically acceptable salt thereof, or the pharmaceutical composition according to claim 9 for the manufacture of a medicament, wherein,

preferably, the medicament is for diagnosis, prevention, and/or treatment of an MC4R-mediated disease or condition;

preferably, the medicament is an MC4R antagonist; and

preferably, the disease or condition is cachexia (cancer-associated cachexia, acquired immunodeficiency syndrome (AIDS)-associated cachexia, congestive heart failure (CHF)-associated cachexia, chronic kidney disease (CKD)-associated cachexia, and cachexia associated with the treatment of other chronic diseases); anorexia or anorexia nervosa (anorexia of aging, and anorexia associated with chemotherapy and/or radiotherapy); nausea and vomiting; weight loss (involuntary weight loss); failure to thrive; sarcopenia; muscular dystrophy; muscle weakness; fragility; osteoporosis; bone disorders (bone loss); pain (neuropathic pain); anxiety (post-traumatic stress disorder or PTSD); depression; hypertension; malnutrition in obesity (for example, sarcopenia caused by chronic obesity); sexual dysfunction; and inflammatory disorders (inflammatory disorders associated with anorexia, cachexia, sarcopenia, or muscular dystrophy).

## INTERNATIONAL SEARCH REPORT

International application No.
**PCT/CN2023/132220**

### A. CLASSIFICATION OF SUBJECT MATTER

C07D401/14(2006.01)i；C07D413/14(2006.01)i；C07D471/04(2006.01)i；C07D498/04(2006.01)i；A61P1/08(2006.01)i；A61K31/4439(2006.01)i；A61P3/04(2006.01)i；A61K31/444(2006.01)i；A61K31/437(2006.01)i；A61P29/00(2006.01)i

According to International Patent Classification (IPC) or to both national classification and IPC

### B. FIELDS SEARCHED

Minimum documentation searched (classification system followed by classification symbols)

IPC:C07D,A61P,A61K

Documentation searched other than minimum documentation to the extent that such documents are included in the fields searched

Electronic data base consulted during the international search (name of data base and, where practicable, search terms used)

VCN, CNTXT, ENTXTC, CAPLUS(STN), REGISTRY(STN), MARPAT(STN): 长春金赛, 黑皮质素, 黑皮质素4受体, 金磊, 陆标, 杨方龙, 赵盛, 王思勤, 拮抗, 肥胖, MC4R, Melanocortin, Melanocortin 4 Receptor, Antagonism, Obesity, 结构式检索, structural formula search

### C. DOCUMENTS CONSIDERED TO BE RELEVANT

| Category* | Citation of document, with indication, where appropriate, of the relevant passages | Relevant to claim No. |
|---|---|---|
| PX | GARNSEY, Michelle R. et al. "Discovery of the Potent and Selective MC4R Antagonist PF-07258669 for the Potential Treatment of Appetite Loss" *Journal of Medicinal Chemistry,* Vol. vol. 66, 19 February 2023 (2023-02-19), 3195-3211<br>abstract, Table 1, and Schemes 3 and 4 | 6-10 |
| PX | WO 2023105387 A1 (PFIZER) 15 June 2023 (2023-06-15)<br>description, page 2, lines 24-29, and page 4, line 23 to page 6, line 3, and embodiment 6 | 6-10 |
| X | STN. "CAS registration number"<br>*Registry,* 29 July 2022 (2022-07-29),<br>2799063-02-6 | 6 |
| X | US 2009291940 A1 (TAISHO PHARMA CO., LTD. et al.) 26 November 2009 (2009-11-26)<br>description, paragraphs 0012-0040, tables 2-5, and preparation embodiments 1-4 | 6-10 |
| A | WO 2010081666 A1 (SANTHERA PHARMACEUTICALS AG et al.) 22 July 2010 (2010-07-22)<br>entire description | 6-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 January 2024** | **07 February 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/132220**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-5, 6 (in part), 7 (in part), 8 (in part), 9 (in part) and 10 (in part)**
because they relate to parts of the international application that do not comply with the prescribed requirements to such an extent that no meaningful international search can be carried out, specifically:

General formula I involved in claim 1 comprises a plurality of substitution variables, the situation of layer-by-layer substitution appears in each substitution variable, and thus it is difficult for the examiner to carry out an exhaustive search of the prior art with regard to the current scope of the claim. Meanwhile, the layer-by-layer substitution of a substituent in formula I causes said claim to cover a relatively large scope of protection, but the description of the present application only verifies the pharmacological activity of specific compounds with limited number of substitution situations. According to the content disclosed in the present application, a person skilled in the art would not have been able to determine that claim 1, as a whole technical solution, can solve the technical problem of the present application. Therefore, claim 1 is not supported by the description. Similarly, claims 2-5, 6 (in part), 7 (in part), 8 (in part), 9 (in part) and 10 (in part) also have the same defect. The present search report is provided in the following range: general formulae II, II-1, II-2, II-3, III, III-1, III-2, III-3, IV, VII, IX and IX-1 set forth in claim 6, wherein the definition of a substituent is the same as that of claim 1; and specific compounds of claim 7 that fall within the scope of claim 6 and the technical solutions of claims 8-10 when referring to claims 6-7.

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/132220**

| Patent document cited in search report | | | Publication date (day/month/year) | Patent family member(s) | | | Publication date (day/month/year) |
|---|---|---|---|---|---|---|---|
| WO | 2023105387 | A1 | 15 June 2023 | None | | | |
| US | 2009291940 | A1 | 26 November 2009 | WO | 2007114323 | A1 | 11 October 2007 |
| | | | | EP | 2003131 | A1 | 17 December 2008 |
| | | | | EP | 2003131 | A4 | 16 December 2009 |
| | | | | JPWO | 2007114323 | A1 | 20 August 2009 |
| | | | | US | 8044068 | B2 | 25 October 2011 |
| WO | 2010081666 | A1 | 22 July 2010 | EP | 2210885 | A1 | 28 July 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202211461838 A
- WO 202310223815 A
- WO 202310388373X A
- WO 202310578810 A
- WO 202310895827 A
- WO 202311158862 A
- WO 2021250541 A1
- WO 2012083165 A1
- WO 2023280254 A1